# EUROPEAN PATENT APPLICATION

(11) **EP 1 712 641 A1**
(43) Date of publication of application: **18.10.2006**
(21) Application number: 05090108.1
(22) Date of filing: 14.04.2005
(51) Int. Cl.: C12Q 1/68

(54) **Use of novel HNF4a target genes and their gene products**

(71) Applicant: Fraunhofer-Gesellschaft zur Förderung der angewandten Forschung e.V., 80686 München (DE)
(72) Inventor: Borlak, Jürgen, Prof. Dr., 31275 Lehrte/OT Immensen (DE); Niehof, Monika, Dr., 30161 Hannover (DE)
(74) Representative: Baumbach, Friedrich

(57) **Abstract**

Dysfunction of HNF4α may lead to disease and an identification of genes targeted by this factor provides insights into mechanisms of disease. In accordance with the invention thirteen new HNF4α target genes were found (C20orf13, KIAA0774, EPS15R, PLCB1, UGTREL1, RSK4, PAK5, FMR2, NEB, NFYC, KCNQ4, PRPF3, TRPC1). These genes were identified by means of molecular biological and molecular genetic methods. The genes code for various biological functions (metabolism, regulation of cell cycle and signal transduction, differentiation, ion channels, mRNA processing, see table A) and are thus important for the therapy of metabolic disorders, diabetic diseases and tumor growth.

The HNF4α target genes RSK4 and PAK5 are already reported. These genes encode kinases which are selective regulated in animal models of diabetes. The kinases RSK4 and PAK5 are of fundamental importance for the therapy of diabetic nephropathy and neuroropathy (Niehof, M. and Borlak, J. 2005, RSK4 and PAK5 are novel candidate genes in diabetic rat kidney and brain, *Mol. Pharmacol.* 67 604-611; European Patent application 04016948.4).

In the present invention, additionally eleven new HNF4α target genes are described.

The gene TPRC1 encodes a non-selective cation channel, which is activated via a receptor or after emptying of intracellular stores. The TPRC1-channel controls the influx of calcium into the cell. TPRC1 proteins are expressed in most cells and are the most important channels for the uptake of calcium into the cell. TPRC1 is for example expressed in the pancreas and the kidney. In the pancreas TPRC1 can support the calciumhomeostasis. A defective regulation of TPRC1 can effect the secretion of insulin.

It was shown that HNF4α and TPRC1 are regulated in animal models of diabetes. Thus, TPRC1 is a candidate gene for the treatment of diabetic nephropathy.

KCNQ4 encodes a voltage-dependent potassium channel. This channel is expressed in sensory haircells of the cochlea. The dysfuction of this channel can cause loss of hearing. Impairments in hearing are one of the symptoms caused by diabetes.

The HNF4α transcription factor targets the genes EPS15R and PLCB1, which are involved in the regulation of signal cascades. Via these genes HNF4α controls the cell cycle. It was found that the EPS15R protein binds to the EGF-receptor. This knowledge can be used to develop strategies to treat tumors.

The discovery of the above described target genes of HNF4α and their function comprises an enormous potential for the treatment of metabolic diseases including diabetes and diabetic caused diseases and tumor growth. By cooperation with pharmaceutic companies this knowledge can be excellently used to develop new straightforward strategies and outstanding agents for the simple treatment of metabolic disorders. Such agents can be, for example, drugs which affect the genes which are dysregulated due to the disease.

## Description

The invention relates to the use of novel target genes of the transcription factor HNF4α and their gene products or their functions to screen for and to identify drugs directed against metabolic diseases, including type 1 and/or type 2 diabetes mellitus, and/or diabetes-caused diseases, including diabetic nephropathy, hearing dysfunction, diabetic neuropathy, cardiovascular diseases or diseases of the retina, and/or tumor growth. Furthermore the invention concerns methods to use thereof and substances which regulate said gene products.

### Background of the invention

Certain metabolic diseases, like for example diabetes type II, are widespread diseases. Prognoses predict an amount of 366 million of diabetic patients worldwide (4.4 % of the population) in 2030. This is twice as much patients as in 2000. Additionally to a defective regulation of the glucose metabolism diabetic patients suffer from diabetic nephropathy, diabetic neuropathy, diseases of the retina, cardiovascular diseases and an impairment of hearing. Worldwide great efforts are made to understand the molecular basis of diabetes. There is evidence that a non-functional HNF4α gene plays a critical role for the development of diabetes. The zinc-finger transcription factor HNF4α is of pivotal importance for liver development and hepatocellular differentiation and plays an essential role in a regulatory circuity to control a wide range of metabolic processes.

HNF4α is a zinc-finger transcription factor and a member of the hepatic transcription factor network. It is a key player in liver biology and drives hepatocyte differentiation (7;28;44;54). Specifically, HNF4α contacts regulatory elements of several genes of various metabolic pathways including carbohydrate, lipid, steroid, xenobiotic and amino acid metabolism, respectively (63;66). This factor also participates in the glucose-dependent insulin secretory pathways (63;66). The role of HNF4α in glucose metabolism is particularly obvious through its functional link to diabetes. Indeed, one form of a monogenetic disorder termed maturity onset diabetes of the young (MODY) was mapped to mutations within the HNF4α gene (MODY-1). There is conclusive evidence for a unique and pivotal role of HNF4α in pancreatic β-cell function (63;66) and HNF4α dysfunction is strongly associated with multifactorial Type 2 diabetes (45;65). Further, metabolic disposalof endogenous compounds including cholesterol and/or fatty acids relies on the proper function of cytochrome P450 (CYP) monooxygenases and the expression of a broad range of CYP-isozymes is regulated by HNF4α as well (33). Notably, treatment of rat hepatocyte cultures with Aroclor 1254, i.e. a complex mixture of polychlorinated biphenyls, resulted in the simultaneous induction of HNF4α and of several CYP isoforms and this points to a coordinate response in the regulation of HNF4α and of genes targeted by this factor (4). Besides its pivotal functions in liver metabolism, HNF4α also targets genes in other tissues and organs, such as kidney, intestine and colon (66). In general, HNF4α is a dominant regulator for an epithelial phenotype, triggers de novo formation of functional tight junctions and contributes to epithelial cell polarity (8). Because of its role in the differentiation of epithelium, it is probable that HNF4α plays an additional role in the control of cell proliferation. Indeed, Chiba et al (9) reported overexpression of HNF4α to inhibit cell growth in F9 cells presumably due to exaggregated expression of cyclin-dependent kinase inhibitor p21^{CIP1/WAF1}. As of today a total of 76 genes targeted by HNF4α have been studied in some detail. Results from these investigations suggest 95 bona fide recognition sites for HNF4α. Different experimental strategies were, however, employed to identify novel HNF4α gene targets and this included an in-silico approach (17) or transfection of HNF4α into a human hepatoma cell line (49) or in a rat insulinoma cell line (70). Most recently, the ChIP-chip assay enabled a comprehensive search for novel HNF4α candidate genes (53) and this approach yielded an unexpected high number of putative candidate genes, i.e. 1575. Though, there is concern about proper validation of results obtained by the ChIP-chip assay, it unprecedently demonstrates the tremendous versatility of HNF4α in contacting promoters of many different genes. The invention is related on the successful search and surprising identification of novel HNF4α gene targets by the use of the chromatin immunoprecipitation (ChIP) assay and cloning of targeted DNA.

The aim of the present invention is therefore to make available the fast and efficient screening and identifying of drugs directed against metabolic diseases, including type 1 and/or type 2 diabetes mellitus, and/or late stage complications of diabetes-caused diseases, including diabetic nephropathy, hearing dysfunction, diabetic neuropathy, cardiovascular diseases or diseases of the retina, and/or tumor growth as well as beneficial methods to use thereof and substances for treating said diseases. To this end, the implementation of the actions as described in the claims provides appropriate means to fulfill these demands in a satisfying manner.

Dysfunction of HNF4α may lead to disease and an identification of genes targeted by this factor provides insights into mechanisms of disease. In accordance with the invention thirteen new HNF4α target genes were found (C20orf13, KIAA0774, EPS15R, PLCB1, UGTREL1, RSK4, PAK5, FMR2, NEB, NFYC, KCNQ4, PRPF3, TRPC1). These genes were identified by means of molecular biological and molecular genetic methods. The genes code for various biological functions (metabolism, regulation of cell cycle and signal transduction, differentiation, ion channels, mRNA processing, see table A) and are thus important for the therapy of metabolic disorders, diabetic diseases and tumor growth.
The HNF4α target genes RSK4 and PAK5 are already reported. These genes encode kinases which are selective regulated in animal models of diabetes. The kinases RSK4 and PAK5 are of fundamental importance for the therapy of diabetic nephropathy and neuroropathy (Niehof, M. and Borlak, J. 2005, RSK4 and PAK5 are novel candidate genes in diabetic rat kidney and brain. Mol. Pharmacol. 67 604-611.; European Patent application 04016948.4).
In the present invention, additionally eleven new HNF4α target genes are described. The gene TPRC1 encodes a non-selective cation channel, which is activated via a receptor or after emptying of intracellular stores. The TPRC1-channel controls the influx of calcium into the cell. TPRC1 proteins are expressed in most cells and are the most important channels for the uptake of calcium into the cell. TPRC1 is for example expressed in the pancreas and the kidney. In the pancreas TPRC1 can support the calciumhomeostasis. A defective regulation of TPRC1 can effect the secretion of insulin.
It was shown that HNF4α and TPRC1 are regulated in animal models of diabetes. Thus, TPRC1 is a candidate gene for the treatment of diabetic nephropathy.
KCNQ4 encodes a voltage-dependent potassium channel. This channel is expressed in sensory haircells of the cochlea. The dysfuction of this channel can cause loss of hearing. Impairments in hearing are one of the symptoms caused by diabetes.
The HNF4α transcription factor targets the genes EPS15R and PLCB1, which are involved in the regulation of signal cascades. Via these genes HNF4α controls the cell cycle. It was found that the EPS15R protein binds to the EGF-receptor. This knowledge can be used to develop strategies to treat tumors.

### Summary of the invention

The invention is realized according to the claims.
With the help of molecular biological and molecular genetic methods new HNF4α target genes (C20orf13, KIAA0774, EPS15R, PLCB1, UGTREL1, RSK4, PAK5, FMR2, NEB, NFYC, KCNQ4, PRPF3, TRPC1) were identified. These 13 genes encode for gene products, herewith identified as being important for cell cycle regulation, signal transduction, metabolism, nutrient transport, ion channels, development and mRNA splicing. An overview of these genes is given in Table A, providing also the links to their database entries and sequences which herewith are referred to this invention.

**Table A. Data base entries and biological relevance of the new HNF4α target genes and their gene products.**

| gene name | Swiss-Prot/mRNA NCBI | molecular function | biological process |
|---|---|---|---|
| C20orf13 | 09H6P5/NM_017714 | asparaginase activity (prediction) | metabolism / glycoprotein catabolism (prediction) |
| KIAA0774 | 094872/XM_166270 | hypothetical protein | Unknown |
| EPS15R (EPS15L1) | Q9UBC2/NM_021235 | receptor activity | Endocytosis signal transduction |
| PLCB1 | Q9NQ66/NM_015192 | phospholipase activity | cell communication / signal transduction regulation of cell cycle |
| UGTREL1 (SLC35B1) | P78383/NM_005827 | UDP-galactose transporter activity | Transport |
| RSK4 (RPS6KA6) | Q9UK32/NM_0144496 | protein kinase activity | cell communication / signal transduction |
| PAK5 (PAK7) | Q9P286/NM_045653 | protein kinase activity | cell communication / signal transduction |
| FMR2 | P51816/NM_002025 | transcription regulator activity (prediction) | Development |
| NEB | P20929/NM_004543 | structural constituent of muscle | Development |
| NFYC | Q13952/NM_014223 | transcription regulator activity | transcription redox response |
| KCNQ4 | P56696/NM_004700 | ion channel activity | ion transport / ion channel |
| PRPF3 (HPRP3, PRP3) | 043395/NM_004698 | mRNA splicing factor activity | nuclear mRNA splicing |
| TRPC1 | P48995/NM_003304 | ion channel activity | ion transport / ion channel |

The identification of these genes and their fundamental importance for a therapy of metabolic diseases, including type 1 and/or type 2 diabetes mellitus, and/or diabetes-caused diseases, including diabetic nephropathy, hearing dysfunction, diabetic neuropathy, cardiovascular diseases or diseases of the retina, and/or tumor growth is described. Agents can now be identified for the control of genes,where regulation is for example disturbed by these diseases. Furthermore, it enables the screening and identification of drugs controlling mutants or variations of these genes or partial sequences playing a role in the pathologic outcome of metabolic dysfunctions.

Therefore new therapeutic concepts are possible for the treatment of metabolic diseases, including type 1 and/or type 2 diabetes mellitus, and/or diabetes-caused diseases, including diabetic nephropathy, hearing dysfunction, diabetic neuropathy, cardiovascular diseases or diseases of the retina, and/or tumor growth.

The term gene in this invention comprises single or double stranded DNA, possibly combined with regulatory elements for their transcription, including sequences as being reffered by the data base codes (s. Table A), but also the use of accordant single stranded (sense or anti-sense) DNA is possible. The term mutant in this context is related to nucleic acid sequences including at least one genetic mutation, for example sequence or chromosomal mutations, compared to the native sequence. The term variation is directed towards all possible parts and/or their combinations of the referred sequences, as well as variations leading to processing or splicing variants or to combinations of the referred mRNA or to respective mutants, as well as sequences including naturally or unnaturally labelled nucleotides, for example methylation patterns. The term part thereof concerns shorter sequences related from the referred sequences, for example coding for a protein domain, but also can include for example parts of introns and/or exons or their combinations, as well as primers for the use in a polymerase chain reaction, and also the use of sequences interacting with mRNA or siRNA is practical, but also interaction with non natural molecules, for example PNA sequences, is appropriate. All of the mentioned elements in the invention or their combinations can also be used appropriately in a non natural environment, for example as being part of a plasmid / transcription vector and/or being expressed in cell culture and/or in an in vitro translation system. Furthermore, said sequences can also be used in combinatorial screening systems, for example in yeast-two-hybrid, phage display, enzyme-function-reassembly, random mutagenesis or different systems, as well as being used by array techniques, for example in micro arrays or spot syntheses systems.

An overview of the function of the new HNF4α regulated gene products is given in Table A. Therein, furthermore, an outline of the biological processes these proteins are involved in, and data base entries is supplied and referred herewith in this invention.
The present invention is now adding the knowledge that these gene products are involved in the complex regulation of certain metabolic processes, which, if dysregulated, can cause metabolic diseases, particularly type 1 and/or type 2 diabetes mellitus, and/or diabetes-caused diseases, including diabetic nephropathy, hearing dysfunction, diabetic neuropathy, cardiovascular diseases or diseases of the retina, and/or tumor growth. Hence, these gene products or, for example their mutants, are now usable as targets for the identification of pharmaceutical leads concerning said diseases. Also, their variations, for example the gene products being phosphorylated or glycosylated, or parts thereof is approriate to derive means, and in particular molecules, for example peptides or peptides including non natural building blocks or D-amino acids and/or peptidomimetics having the desired properties.
Therefore, the inventive use of said gene products enables an easy and effective screening and identification of agents for the treatment of metabolic diseases.
Furthermore, as their dysregulation can lead to metabolic diseases, the invention is also related to mutants, variations and parts of these gene products.

In general, the inventive use of the identified HNF4α target genes and/or their gene products as well as their related/derived molecules furthermore allows for the first time easy and straightforward strategies for identifying means for ameliorating metabolic disorders / dysfunctions, particularly said diseases, if combinations of several genes or gene products or their derived molecules / sequences are used.
The term gene products comprises all possible translational products being coded by the above specified nucleotide sequences their mutants. Moreover, it relates to all possible unfolded and folded proteins, protein complexes or protein derived structures, comprising glycoproteins or lipoproteins, including amino acid sequences which are coded by said nucleotide sequences. The term variations concerns all possible posttranslational modifications, for example phosphorylations or the formation of disulfide bridges. The term mutant in this context relates to all sequences differing from the native sequence by at least one amino acid residue, or any other modifications leading to a misfolded or dysfunctional structure. The terms parts thereof also includes fragments or enzymatic cleavage products.
In other aspects of the invention, the goal oriented use of genes and/or their gene products being coded by the genes TRPC1, KCNQ4, EPS15R, PLCB1, C20orf13 or UGTREL, and/or mutants and/or variations and/or parts thereof for the search and identification of drugs being directed against the specified diseases is described.

In one aspect of the invention one gene and/or gene products being coded by the gene and/or its mutations and/or variations and or parts thereof are selected and used for the screening and identifying of drugs concerning a certain application area, according to the claims 3-8. This aspect has the advantageous characteristic that it enables a simple straightforward screening and identification procedure.

In another aspect of the invention one gene and/or gene products being coded by the gene and/or its mutations and/or variations and or parts thereof are selected and used for the screening and identifying of drugs by termining the function of said gene and/or gene products in certain fields, according to the claims 9-20. This aspect has the preferable characteristic that it eases the identification procedure by reducing the parameters for the screening procedure.
The functions said genes or their gene products are involved in and which are appropriate for the screening and identification of said drugs are summarized as follows:

### EPS15R function

- in binding to EGF-receptor
- in endocytosis and proteosomal degradation of EGF-receptor
- in inhibition of EGF-receptor
- in EGF-receptor mediated signal transduction
- in regulation of tumor growth

### PLCB1 function

- in activation through G-proteins
- in stimulation of PKC
- in nuclear activation through ERK1 and ERK2
- in regulation of differentiation
- in regulation of proliferation
- in regulation of cell cycle
- in regulation of tumor growth

### C20orf13 function

- in taspase function
- in catalysing glycoprotein metabolism
- in decrease of deamidation of asparagine
- in regulation of tumor growth
- in cellular differentiation
- in regulation of HOX-genes
- in organogenesis / development

### UGTREL1 function

- in glycoconjugate synthesis
- in effects on cell adhesion and tumor growth

### RSK4 function

- in mediating MAP/ERK signal transduction
- in regulation of gene expression by phosphorylation of transcription factors (namely CREB, CBP/p300, ERα, IKBα/NFkB, c-Fos)
- in regulation of cell cycle
- in regulation of cell proliferation
- in regulation of cell differentiation
- in the treatment of nephro- and neuropathies

### PAK5 function

- in mediating effects of rho-proteins (such as cdc42 or p21-rac1)
- in regulation of MAPK signaling pathways
- in regulation of cytoskeletal dynamics
- in regulation of cell cycle
- in regulation of cell proliferation
- in regulation of cell differentiation
- in the treatment of nephro- and neuropathies

### FMR2 function

- in transcriptional activation of genes
- in regulation of cell differentiation of for instance neuronal cells

### NEB function

- in maintaining structural integrity of cardiac and skeletal muscle

### NFYC function

- in transcriptional activation of genes
- in regulation through cellular redox potential
- in regulation based on redox response

### KCNQ4 function

- in hearing impairment and hearing lost including late stage complications of metabolic diseases
- in generating potassium currents and membrane potentials in brain to transduce signals
- in generating faultless potassium currents and membrane potentials in other organs, e.g. heart to transduce signals

### PRPF3 function

- in participating in pre-mRNA splicing as component of the spliceosome
- as part of faultless gene expression
- in treatment of disorders linked to RNA-spliceosome

### TRPC1 function

- as part of heterodimers with other TRP proteins
- in primary mode of Ca²⁺ entry after receptor activation or after store-dependent activation
- in calcium homeostasis including kidney and pancreas,
- as non-selective cation channels in beta-cells
- in insulin secretion by regulating pancreatic beta-cell plasma membrane potential in a K_{ATP} channel independent manner
- in glucose-signalling
- in the treatment of metabolic disorders including diabetes and nephropathy

In yet another aspect of the invention drugs regulating the expression of one or more genes and/or the function of one or more gene products or molecules derived thereof are used for the treatment or for the production of means for the treatment of metabolic diseases, according to the claims 21-23. This aspect has the favourable aspect that it allows a fast and easy way to realise the drugs identified towards a product that can be sold.

A further aspect of the invention concerns the use of said genes and/or related molecules and/or gene products or related molecules for preparing a medication for the treatment of metabolic diseases, according to claim 24. This aspect has the preferable characteristic that it allows an easy application of said identified drugs, for example in a beneficial dose.

Yet a further aspect of the invention relates to a method for the identification of compounds directed against metabolic diseases wherein genes and/or their related molecules and/or their gene products and/or derived structures are used as targets and changes in the expression and/or function are determined, according to claim 25. This aspect has the favourable characteristic that it enables a fast and efficient identification procedure and further reduces the screening expense.

Another aspect of the invention is related to methods for the treatment of metabolic diseases, according to the claims 26 and 27. This aspect has the advantageous aspect that it allows the treatment of said diseases by the use of compounds directly directed against the chosen targets, for example antibodies or derived structures.

Yet another aspect of the invention concerns an method for the treatment of metabolic diseases by the use of agonistic compounds according to the claims 28-32. This aspect has the preferable characteristic that it allows the simple treatment of said diseases, for example by the use of shortened sequences or derived molecules, which can compensate or enhance a natural function.

A further aspect of the invention relates to a method for the treatment of metabolic diseases by the use of blocking / antagonistic substances according to the claims 33-36. This aspect of the invention has the favourable characteristic that an upregulated dysfunction can be easily reduced.

Yet another aspect of the invention features a method for the treatment of metabolic diseases by reducing the overexpression of a normal gene, according to the claims 35 and 36. This aspect has the advantageous characteristic that the natural function of a dysregulated gene can be easily downregulated or restored.

Another aspect of the invention provides substances regulating said genes or gene products, according to the claims 37 and 38. This aspect has the preferable characteristic that it enables the easy storage, preparation or use of compounds being able to ameliorate metabolic diseases

Other features and advantages will be apparent from the following detailed description.

### Detailed disclosure of the invention

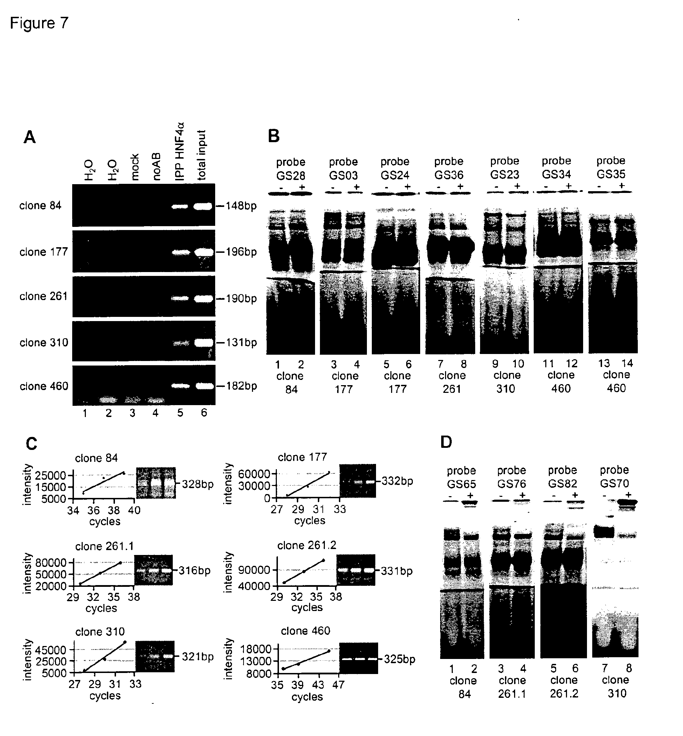

Several independent investigations provide evidence for Caco-2 cells to be valuable for functional studies on HNF4α (27;29). The invention relates on 13 novel genes targeted by HNF4α and was requested in three independent ChIP experiments by repetitive identification of novel gene targets. Additionally, and whenever possible, *in vitro* binding of HNF4α to recognition sites of candidate genes was confirmed by EMSA and mRNA expression of gene targets was verified by quantitative RT-PCR.
Notably, the cDNA of C20orf13 (12) was sequenced from colon mucosa and HepG2 cells and codes for a protein with predicted asparaginase activity, which might participate in glycoprotein metabolism. Likewise, UGTREL1 (31) codes for an isoform of the UDP-galactose transporters and carries nucleotide sugars into the Golgi apparatus to enable glycoconjugate synthesis. Thus, identification of C20orf13 and UGTREL1 provide further evidence for HNF4α to function as a master regulator in metabolism (63;66). A further gene targeted by HNF4α is an isoform of phospholipase C (PLCB1). This protein is involved in cell communication and signal transduction and belongs to one of several phospholipase C-beta isoforms (5). Specifically, PLCBs catalyze the hydrolysis of phosphatidylinositol-bisphosphate (PIP₂) to generate the second messengers diacylglycerol (DAG) and inositol-triphosphate (IP₃) with subsequent Ca²⁺ mobilization and proteinkinase C (PKC) activation. It therefore facilitates cell proliferation and differentiation (5). Within the plasma membrane PLCB1 is activated by members of the alpha-q family of G-proteins (5). However, phospoinositide-metabolism also occurs in the nucleus and nuclear PLCB1 is a physiological target of ERK1 and ERK2 (18). PLCB1 is basically expressed in all human tissues tested so far and nuclear PLCB1-signaling based on increased expression and activity was recently linked to myogenic differentiation (18). It is of considerable importance that the gene coding for an EPS15R (38), epidermal growth factor receptor substrate, was targeted by HNF4α. This protein plays a pivotal role in receptor down-regulation and in clathrin-mediated endocytosis as well as degradation of activated EGF-receptors (18). Thus, HNF4α targets partners of cell cycle regulation presumably with to aim cellular differentiation, rather than cell proliferation. HNF4α also targets KIAA0774. The cDNA of this gene (48) was sequenced from brain and encodes for a hypothetical protein with as yet uncertain function, when assessed on the basis of homology. Furthermore, two kinases were identified, i.e. RSK4 and PAK5, which regulate, in part, cell cycle and signal transduction (51). Because of their importance in diabetic neuro- and nephropathy these kinases were studied in detail in an streptozotocin disease model and reported comprehensively elsewhere (51). Taken collectively, this reports evidence for HNF4α to target genes with novel functions, which are beyond its master functions in metabolism and nutrient transport.
As discussed above, *in vitro* binding of HNF4α to candidate genes (Table 6) was confirmed by EMSA, but for some targets (FMR2, NEB, NFYC, KCNQ4, PRPF3, TRPC1, see Table 7) *in vitro* binding to the ChIP confirmed fragments could not be demonstrated. Nonetheless, these genes are faithful HNF4α targets, because they have been verified in separate immunoprecipitation experiments (Fig. 7A). Indeed, during initiation of transcription, a specific transcription factor interacts with several coactivators and basal factors. Binding of different transcription factors as part of a multiprotein complex leads to combinatorial control of gene expression. As formaldehyde crosslinks lead to both, protein-DNA and protein-protein fixation, ChIP-assays allow for the study of a three-dimensional, higher order structure. Thus, immunoprecipitated HNF4α might not necessarily contact DNA directly, but a cooperating partner, which was in contact through multimeric protein interactions with chromatin. Indeed, Hatzis and Talianidis (27) used the ChIP-assay to investigate the order of recruitment of transcription factors to HNF4α enhancer and promoter and found the promoter to contact long distance (6 kb) immunoprecipitated enhancer associated transcription factors. Thus, the CHIP assay allowed for protein-protein interactions of factors contacting enhancer and promoter regulatory regions. Consequently, the genes listed in Table 7 were fished by crosslinked protein-protein complexes.
Indeed, Fragile X mental retardation protein 2 (FMR2) is shown to be targeted by HNF4α and this protein is a member of a new family of putative transcription activators. Defects in FMR2 are the molecular cause of FRAXE mental retardation (25) and expression of lacZ-constructs in knock-out animals provide evidence for FMR2 to be expressed in several tissues with as yet unknown functions (25). Further, Nebulin (14) is also regulated by HNF4α and codes for a giant muscle protein with functions in actin cytoskeleton. Its expression in tissues other than muscle is, however, unknown. Additionally, HNF4α targets PRPF3, which codes for a component of the spliceosome and participates in pre-mRNA splicing (6). It is highly expressed in liver, kidney, blood and retina and mutations in PRPF3 are implicated in autosomal retinitis pigmentosa (6). TRPC1, transient receptor potential cation channel, subfamily C, member 1, codes for a nonselective cation channel, is widely expressed and allows plasma membrane calcium influx to occur in response to intracellular depletion or to activation by G(q)-coupled receptors (10). Specifically, nonselective cation channels play a role in insulin secretion and regulate pancreatic beta-cell plasma membrane potential, Ca(2+) homeostasis and thus glucose signaling (58). Qian et al (58) proposed TRPCs as good candidates for, as yet, not well-characterized nonselective cation channels in beta-cells. HNF4α regulates several genes involved in glucose metabolism (63) and participates in the glucose-dependent insulin secretory pathways (63;66). HNF4α dysfunction does, however, lead to multifactorial Type 2 diabetes (45;65) and TRPC1 might be an important disease associated HNF4α candidate gene target during diabetes. Furthermore, it was verified by immunoprecipitation of a region located around 7.5 kb upstream of potassium voltage-gated channel, KQT-like subfamily, member 4 (KCNQ4) and around 4.7 kb downstream of nuclear factor Y (NFYC). KCNQ4 belongs to the potassium channel family and regulates the excitability of sensory cells of the cochlea. Defects in KCNQ4 are a cause of nonsyndromic sensorineural deafness type 2, an autosomal dominant form of progressive hearing loss (73). It is expressed in the outer sensory hair cells of the cochlea and also slightly in heart, brain and skeletal muscle with as yet unknown functions. Hearing impairment has been reported to be one of the late complications of diabetes. It appears to be a multifunctional process with cochlear and nerve involvement (15). Therefore, KCNQ4 might represent a further novel HNF4α gene target deregulated during diabetes, but requires further studies. NFYC is one subunit of the highly conserved transcription factor NFY, which binds as trimeric complex with high specificity to CCAAT boxes in the promoter regions of a variety of genes (61). During hypoxia HNF4α physically interacts with HIF-1α, which results in increased erythropoietin gene expression (72), and furthermore, HNF4α regulates redox-mediated iNOS (inducible NO synthase) expression (26). Interestingly, transcriptional activity of NFY is regulated by the cellular redox potential (47) and this might implicate an additional link of HNF4α to redox response. The findings given in Table 7 are likely to be the result of immunoprecipitated protein-protein crosslinks (see above). In addition, it was tested only whether predicted recognition sites of HNF4α in the promoter of these genes can be confirmed, but did not investigate their in vivo binding. Indeed, in vitro binding to putative promoter sites of FMR2, KCNQ4, NFYC, and PRPF3 could be demonstrated. The results therefore provide strong evidence for these genes to be regulated by HNF4α.

HNF4α contacts DNA exclusively as a homodimer and physically interacts with members of the basal transcription machinery, cofactors and several transcription factors (63;66). Protein-protein interaction of HNF4α homodimers with other transcription factors is well documented for HNF1α (41) and COUP-TF (40). Because of these interactions it was analyzed *in vitro* binding of COUP-TF and HNF1α to the newly identified HNF4α binding sites. Besides HNF4α, COUP-TF has the potential to contact HNF4α recognition sites and may compete with HNF4α for the same sites (63;66). Neither COUP-TF nor HNF1α was bound to the novel HNF4α target sites as determined by EMSA and super shift assays. In addition, it was used the Transfac matrixes for COUP-TF and HNF1α with a cut-off matrix similarity of 0.78 to minimize false positive matches (min FP). Applying these matrixes was not suggestive for COUP-TF or HNF1α binding to immunoprecipitated fragments. The results therefore provide strong evidence for specificity of HNF4α binding.
Indeed, HNF4α acts in concert with other, mainly liver specific transcription factors (63) and the described analysis for binding sites of other transcription factors in proximal promoters with Transfac matrixes setting of minFP cut off resulted in promising, but experimentally unproven binding sites. Based on this computation, binding sites for HNF1, HNF3, C/EBPα, C/EBPβ and GATA-4 surrounding the HNF4α sites in proximal promoters (-1 to -3500bp) of the newly identified gene targets are predicted.

In fact, ChIP cloning in combination with carefully performed target confirmation is a time consuming process, but allows for thorough validation of novel gene targets. However, there are methodological considerations that need to be addressed. Indeed, until now ChlP cloning of formaldehyde crosslinked nucleoprotein complexes was scarcely employed. ChIP cloning was, however, used to study E2F (9 targets) (74), E2A (8 targets) (23), Egr1 (1 target) (11), EWS/ATF-1 (6 targets) (32), RUNX1 (1 target) (30), BARX2 (21 targets) (69), Smad4 (1 target) (64), STAT5a/b (9 targets) (50) and RUNX2 (4 targets) (2). Aii gene candidates were confirmed by independent ChIP experiments and subsequent PCR analyses using clone-specific primers. Due to the fact that it is very difficult to completely avoid nonspecific immunoprecipitated DNA, validation of findings is extremely important (74). Some investigators reported similar or modified approaches to isolate genomic DNA, but did not apply independent ChIP experiments to confirm *in vivo* binding (37;56;60;62). This limits the value of findings as no proof of *in vivo* binding to immunoprecipitated DNA is presented. In strong contrast numerous independent experiments and thoroughly validated HNF4α gene targets were employed. Specifically, three independent ChIP experiments to annotate targets were requested. Prior to cloning it was further searched for known targets in immunoprecipitated DNA to proof specificity. The number of clones identified is in line with findings reported for other transcription factors based on the ChIP-cloning procedure (see above). Specifically, none of these studies cloned already known targets due to low amount of immunoprecipitated DNA and when compared with the expected number of genes targeted by a transcription factor. A further point of consideration is the even distribution of gene targets amongst different chromosomes. This demonstrates the utility of ChIP-cloning procedure in identifying genome wide targets. Also being identified were several targets for HNF4α within a chromosome and half of the cloned fragments were annotated to regions of 5 to 50kb upstream or downstream of coding DNA, whereas the other half of the cloned fragments were sequences derived from intron 1 or intron 2. Notably, Weinmann et al (74) identified promoter regions of three novel E2F targets from ChIP-cloned fragments, but most investigators detected rarely proximal promoter binding sites from ChIP-derived clones. For instance, Jishage et al (32) described the cloning of six confirmed EWS/ATF-1 targets, whereby only one target contained a promoter binding site, whereas three were located far away from transcription initiation site. In the study of Martone et al (46) NFkB binding sites were investigated by the "CHIP on a chip" assay. Specifically, the authors revealed binding of NFkB proximal to 5' ends, but observed binding with high frequency at many other sites, including introns (in total 40% of the sites) as well as sites distal to 5' prime end. Therefore, the distribution of binding sites for NFkB for human chromosome 20 is strikingly similar to the results with HNF4α. Indeed, approximately 90% of the well-known HNF4α binding sites are located within 3000bp upstream of the predicted start site of transcription (TSS) and 60% are located within 500bp upstream of the TSS. However, binding of specific transcription factors is not restricted to proximal promoter regions alone. It is clear by now that a typical animal gene may contain several enhancers located in 5' and 3' regulatory regions over distances of 100kb, in addition to binding sites within introns (43). Intronic enhancers have become well known in recent years and are located predominantly in intron 1 and intron 2 (42). In the past, searches for transcription factor binding sites were focused on proximal promoter region even though binding-sites for HNF4α were also described for the 5' enhancer regions of OTC (52), ApoB (1), and CYP3A4 (71) and the 3' enhancer regions of EPO (21) and ApoAl (3). Likewise, HNF4α contacts intronic enhancers in the case of aldolase B (24), apolipoprotein B (1) and adenosine deaminase (16). Specifically, proximal promoters (-1 to -3500bp) of HNF4α ChIP-clones with Swiss Prot entry for putative binding sites and designed primer pairs for cloned fragments and studied predicted promoter binding sites in independent CHIP experiments were analyzed. *In vivo* HNF4α binding to EPS15R, KIAA0774 and TRPC1 was CHIP-verified for recognition sequences in the first intron and *in vivo* binding to PLCB1, UGTREL1, C20orf13, FMR2, NEB and PRPF3 was verified for promoter binding sites. Furthermore, it was CHIP verified *in vivo* HNF4α binding to a region around 7.5 kb upstream of KCNQ4 and around 4.7 kb downstream of NFYC. Once again, *in vitro* binding of HNF4α to promoter recognition elements of these targets was confirmed as well (see Fig. 7D and Table 7)

Noteworthy, several approaches were reported for identification of HNF4α gene targets and this included a bioinformatic approach (17), HNF4α overexpression (49;70) and ChIP-assays combined with microarrays (53). Indeed, Ellrot et al (17) developed an algorithm based on the Markow chain optimization method to scan the human genome for HNF4α binding sites. 71% of the resulting sites were confirmed by *in vitro* binding assays. Surprisingly, in this report the genes were not specified and *in vitro* binding alone is insufficient evidence and disregards (a) binding based upon synergy with other factors and (b) ignores *in vivo* accessibility to high order chromatin structure. For example, it was analyzed *in vitro* binding of 43 computational predicted HNF4α binding sites based on the CHIP cloned and sequenced DNA. Notably, it was able to confirm 15 binding sites only, based on EMSA. Indeed, algorithms developed by Transfac and Genomatix are weight matrix-based. Several matrixes were applied for the detection of binding sites and carried out independent experiments to confirm both *in vivo* and *in vitro* binding. However, the applied algorithm produced conflicting results with one recognition site being detected by the Transfac matrix only whereas an other site was detected by the Genomatix matrix only. By applying several algorithm false positive and false negative results were obtained. Therefore experimental confirmation is a must for target site validation. As shown here and by other investigators for c-myc (19) and E2F (36) in-silico approaches will be improved when the modular organization of regulatory regions into promoter models is incorporated (20). The most basic forms of regulatory modules are composite elements consisting of pairs of functional transcription factor binding sites that act synergistically (35). These composite modules were successfully used for database searches that were independent of direct sequence similarity (39). Additionally, experimentally verified motifs enable the development of improved computer algorithms.

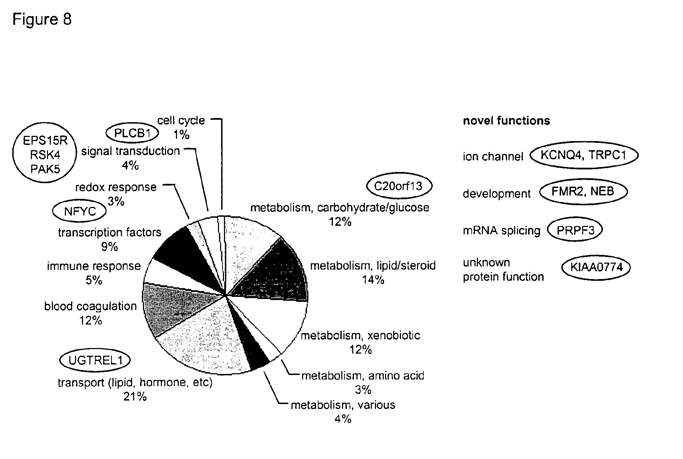

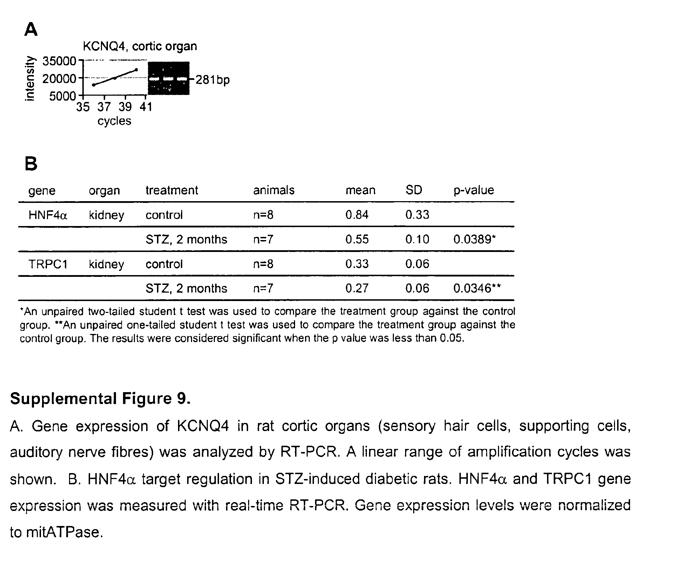

Next to ChlP-cloning of novel HNF4α gene targets identification may be achieved by studying gene expression after HNF4α overexpression. This approach yielded 62 novel gene candidates in a human hepatoma cell line (49) and 338 probe-sets (whereas approx. 50% of the probe-sets were annotated to specific genes) in a rat insulinoma cell line (70). In both studies HNF4α affects predominantly targets involved in metabolic processes, but targets involved in cell communication, cell cycle and development were also reported. However, no bioinformatic analyses or EMSA assays were applied to confirm findings. Moreover, the newly identified genes may not necessarily function as direct targets, because changes in gene expression might be due to indirect effects resulting from altered signal-transduction cascades. Furthermore, HNF4α could influence gene expression by controlling HNF1α (44), PXR (pregnane X receptor) (34;44), PPARα (peroxisome proliferator-activated receptor α) (57), HNF6 (59) or other as yet unknown cooperating transcription factors. Therefore, regulated genes could, in part, be indirect targets, unless its binding was validated *in vivo.*
Finally, Odom et al (53) reported gene target identification for HNF1α, HNF4α, and HNF6 based on chromatin immunoprecipitation combined with DNA-DNA hybridization on a 13000 human promoter sequences containing microarray. In the case of HNF4α the number of contacted promoters was unexpected high, i.e. 1575 potential HNF4α target genes in hepatocytes were identified, corresponding to 12% of the genes represented on the array. Further, 42% of the genes occupied by RNA-polymerase II were also occupied by HNF4α. It was used the same antibody as reported by Odom et al (53). This antibody suffers from significant drawbacks in ChIP-experiments (see results) and yielded only low amounts of immunoprecipitated DNA. As reported above two consecutive rounds of immunoprecipitation in addition to n=3 independent experiments to confirm novel HNF4α candidate gene are performed. Additionally, it is considered a comparison of findings to the no antibody control as a must. Though it is undisputed that the ChIP-chip assay will be invaluable there is a definitive need for thorough evaluation of potential targets. Only 48 (= 3%) of 1575 putative HNF4α targets were verified in separate gene-specific ChIP experiments, though a 16% frequency of false positives was reported for the assay. Additionally, HNF4α DNA-binding was not distinguished from protein-protein interactions, as in vitro binding was not analyzed. The potential targets remain speculative until they are carefully validated. When the data described by Naiki et al (49) were compared with data derived from the ChIP-chip assay only 17 genes were common. Likewise, when the novel HNF4α candidate genes from this report were compared with findings reported by Odom et al (53) only C20orf13 and UGTREL1 were in common, but none of the gene targets being identified were observed in the HNF4α overexpression studies reported by Naiki et al (49) and by Thomas et al (70). In conclusion, this invention discloses the successful identification and validation of n = 13 HNF4α gene targets and suggest novel roles for this factor in cell cycle regulation, signal transduction, metabolism, nutrient transport, ion channel, development and mRNA splicing (see Fig 8). Therefore, HNF4α is versatile and functions beyond the control of metabolic processes. In the future, identification of genome wide targets regulated by a specific transcription factor may be feasible by coupling well-controlled ChIP-chip experiments with improved bioinformatic approaches, which allows for the development of composite modules that take combinatorial and synergistic action of several transcription factors into account.

### Methods

Experiments were performed using the following methods

### Caco-2 cell culture

Caco-2 cells were obtained from and cultivated as recommended by DSMZ. Essentially, cells were cultured in DMEM supplemented with 10% FCS and 200 µg/ml penicillin, 200 µg/ml streptomycin and 615 µg/ml L-glutamic acid. Cells were used between the 5. and the 20. passage and were checked for purity and morphological abnormalities by phase contrast microscopy. Caco-2 cells were seeded with a density of 4 x 10⁶ cells per 75 cm² flask and harvested after 11 days.

### Isolation of nuclear extracts

The use of animals was approved by the local government of Hannover with project license 02-548. Sprague Dawley rats (n=3) were treated with a single i.p. dose of 100 mg of Aroclor 1254 per kg bodyweight and killed 72 h later. Nuclear extracts from rat liver were prepared as described by Gorski et al (22), whereas nuclear extracts from Caco-2 cells were isolated by a modified method of Dignam et al (13). Eleven days after seeding cells were washed twice with ice-cold PBS, scraped into microcentrifuge tubes and centrifuged for 5 min at 2000 x g, 4°C. Cell pellets were resuspended in hypotonic buffer (10 mM Tris pH 7.4, 2 mM MgCl₂, 140 mM NaCl, 1 mM DTT, 4 mM Pefabloc, 1% Aprotinin, 40 mM β-glycerophosphate, 1 mM sodiumorthovanadate and 0.5% TX100) at 4°C for 10 min (300 µl for 1 x 10⁷ cells), transferred onto one volume of 50% sucrose in hypotonic buffer and centrifuged at 14000 x g and 4°C for 10 min. Nuclei were resuspended in Dignam C buffer (20 mM Hepes pH 7.9, 25% glycerol, 420 mM NaCl, 1.5 mM MgCl₂, 0.2 mM EDTA, 1 mM DTT, 4 mM Pefabloc, 1% Aprotinin, 40 mM β-glycerophosphate, 1 mM sodiumorthovanadate, 30 µl for 1x10⁷ cells) and gently shaked at 4°C for 30 min. Nuclear debris was removed by centrifugation at 14000 x g at 4°C for 10 min. The extracts were aliquoted and stored at -70°C. Protein concentrations were determined according to the method of Smith et al (67).

### Western blot analysis

Nuclear extracts were separated on a 12% SDS-polyacrylamide gel and blotted onto a PVDF membrane in 25 mM Tris and 190 mM Glycin at 4°C for 3 h at 350 mA. The antibody directed against HNF4α was purchased from Santa Cruz Biotechnology (sc-8987x). The antigen-antibody complexes were visualized using the ECL detection system as recommended by the manufacturer NEN Life Science Products and chemiluminescence was recorded with Kodak IS 440 CF.

### Electrophoretic mobility shift assays

Nuclear extracts were used as described in the figure legends. Binding buffer consisted of 25 mM HEPES, pH 7.6, 5 mM MgCl₂, 34 mM KCI, 2 mM DTT, 2 mM Pefabloc, 2% Aprotinin, 40 ng of poly (di-dC)/µl and 100 ng of bovine serum albumin/µl. Oligonucleotides and nuclear proteins were incubated for 20 minutes on ice. Free DNA and DNA-protein complexes were resolved on a 6% polyacrylamide gel. The oligonucleotides were purchased from MWG Biotech and used as ³²P-labeled probes, for sequence information see **Table 1.** Super shift experiments were done with antibodies (Santa Cruz Biotechnology) against HNF4α (sc-6556x), HNF1α (sc-6547x) or COUP-TF (sc-6578x).

### Crosslinking and Chromatin immunoprecipitation (ChIP)

All CHIP procedures were carried out as described by Weinmann et al (74) with some modifications. Caco-2 cells were treated with 1% formaldehyde at room temperature for 10 min under constant agitation. The reaction was stopped by the addition of glycine to obtain a final concentration of 125 mM. Cells were washed twice with ice-cold PBS, detached with trypsin and collected by centrifugation. Cells were resuspended in lysis buffer (5 mM PIPES, 85 mM KCI, 0.5% NP40 and 1x complete protease inhibitor, Roche) and incubated on ice for 20 min. The nuclei were collected by microcentrifugation and then resuspended in nuclei lysis buffer (1% SDS, 10 mM EDTA pH 8.0, 50 mM Tris-HCl pH 8.1 and 1x complete) and incubated on ice for 10 min. The samples were sonicated on ice until crosslinked chromatin was fragmented to approximately 0.2-1.6 kbp. Protein A-Sepharose CLB4 (Pharmacia) was blocked with 1 mg ml⁻¹ BSA and 1 mg ml⁻¹ herring sperm DNA (Promega) and washed extensively before use. Chromatin preparations were precleared by incubation with 'blocked' Protein A-Sepharose for 1 h at 4°C. The Protein A-Sepharose was removed by centrifugation and the precleared chromatin was diluted 1:3 with immunoprecipitation (IP) dilution buffer (0.01% SDS, 1.2 mM EDTA pH 8.0, 1.1% Triton X100 and 1x complete). Precleared chromatin from 2.5x10⁷ cells was incubated with 1 µg goat polyclonal HNF4α antibody (sc-6556, Santa Cruz) or no antibody and rotated at 4°C overnight. Immunoprecipitates were recovered by incubation with a secondary antibody (rabbit anti-goat) and 'blocked' Protein A-Sepharose, washed twice with dialysis buffer (50 mM Tris-HCl pH 8.0, 2 mM EDTA, 0.2% sarkosyl) and four times with IP wash buffer (100 mM Tris-HCl pH 9.0, 500 mM LiCl, 1% NP40, 1% deoxycholic acid). Prior to the first wash, the supernatant from the reaction with no antibody was saved as total input chromatin and was processed with the eluted immunoprecipitates at the beginning of the cross-link reversal step. Elution was done with 30 µl elution buffer (1% SDS, 50 mM NaHCO₃) and samples were diluted 1:10 with IP dilution buffer. Two samples were pooled for a second immunoprecipitation step with the HNF4α antibody. After further recovering and washing steps, elution was done two times with 150 µl elution buffer each. Crosslinks were then reversed by the addition of NaCl to a final concentration of 300mM, and RNA was removed by the addition of 10 µg of RNase A per sample followed by incubation at 65°C for 4-5 h. The samples were then precipitated at -20°C overnight by addition of 2.5 volumes of ethanol and then pelleted by microcentrifugation. The samples were resuspended in 100 µl Tris-EDTA (pH 7.6), 25 µl 5x proteinase K buffer (1.25% SDS, 50 mM Tris-HCl pH 7.5, 25 mM EDTA pH 8.0) and 125 µg proteinase K (Roth) and incubated at 55°C for 2h. DNA purification was done by extraction with phenol-chloroform-isoamyl alcohol (25:24:1) and subsequent ethanol precipitation. The pellets were resuspended in 30 µl H₂O and analyzed by PCR. A mock probe, containing buffer without chromatin, was treated categorically throughout the whole immunoprecipitation procedure and throughout DNA isolation and purification to control for external DNA contamination.

PCR was done in a mixture containing 2 µl of purified DNA or 2 µl of a 1:200 dilution of the total input sample, 1 µM of each primer, 0.25 mM dNTP mixture, 0.625 U Thermostart-Taq (Abgene) and 1x PCR-buffer (Abgene, with 1.5 mM MgCl₂) in a total volume of 20 µl. PCRs were carried out with a T3 Thermocycler (Biometra, Göttingen, Germany) with the following conditions: initial denaturation at 95°C for 15 min (Thermostart activation), denaturation at 94°C for 30 sec, annealing at different temperatures for 45 sec **(Table 2),** extension at 72°C for 45 sec, final extension at 74°C for 10 min, 45 cycles. A detailed account of PCR primers to analyze immunoprecipitated target genes is given in **Table 2.** The PCR-amplification products were run on a 2.0% agarose gel and analyzed by ethidium bromide staining.

### Cloning of immunoprecipitated DNA

The immunoprecipitated DNA was treated with T4 DNA polymerase (New England Biolabs) to create blunt ends, purified, and cloned into the zero-blunt vector (Invitrogen) using the zero-blunt PCR cloning kit (Invitrogen) according to the manufacturers recommendations. Colonies having inserts were identified by restriction enzyme digestion using enzymes in the polylinker.

### Sequence Analysis

Plasmid-DNA was purified with QlAquick PCR Purification Kit (Qiagen), subjected to cycle sequencing with vector-specific primers using BigDyeTerminator v3.1 Kit and injected into ABI 3100 Genetic Analyzer (Applied Biosystems). Sequences were identified by database searches (GenBank version Build 35.1, maintained by NCBI) for human genomic matches. Detailed sequence information is given in Table 3.

### Bioinformatic searching for HNF4α binding-sites

The transcription start site (TSS, +1) of the NCBl mRNA reference sequence (RefSeq) was aligned using the UCSC Genome Browser (http://genome.ucsc.edu/) for promoter annotation of the respective clones. Cloned fragments and respective proximal promoters of gene targets (-1 to -3000 bp) were checked for putative HNF4α binding-sites with two different weight matrix-based tools, i.e. V$HNF4_01 with cut-off core similarity 0.75 and matrix similarity 0.78, Transfac matrix (Biobase, www.biobase.de) and V$HNF4_01 with cut-off core similarity 0.75 and matrix similarity 0.82 or V$HNF4_02 with cut-off core similarity 0.75 and matrix similarity 0.76, Genomatix matrix (Genomatix, www.genomatox.de).

### RT-PCR

Total RNA was isolated using the nucleospin RNA Isolation Kit (Macherey-Nagel) according to the manufacturers recommendations. 4 µg total RNA from each sample was used for reverse transcription (Omniscript Reverse Transcriptase, Qiagen). PCR was done in a mixture containing a cDNA equivalent to 25 ng of total RNA, 1 µM of each primer, 0.25 mM dNTP mixture, 0.625 U Thermostart-Taq (Abgene) and 1x PCR-buffer (Abgene, with 1.5 mM MgCl₂) in a total volume of 20 µl. PCR-reactions were carried out with a thermocycler (T3, Biometra) with the following conditions: initial denaturation at 95°C for 15 min (Thermostart activation), denaturation at 94°C for 30 sec, annealing at different temperatures for 45 sec **(Table 4),** extension at 72°C for 45 sec, final extension at 74°C for 10 min. Various cycle numbers were used to demonstrate linearity and amplification products were separated using a 1.5 % agarose gel and stained with ethidium bromide. A detailed oligonucleotide sequence information is given in **Table 4.**

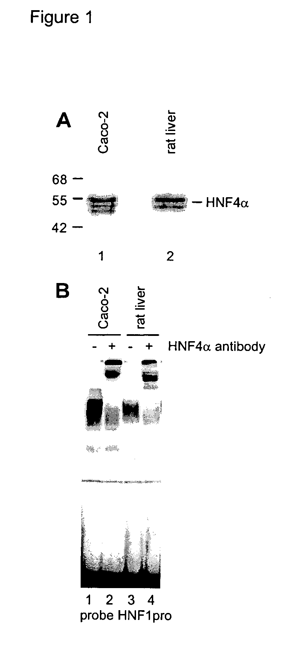

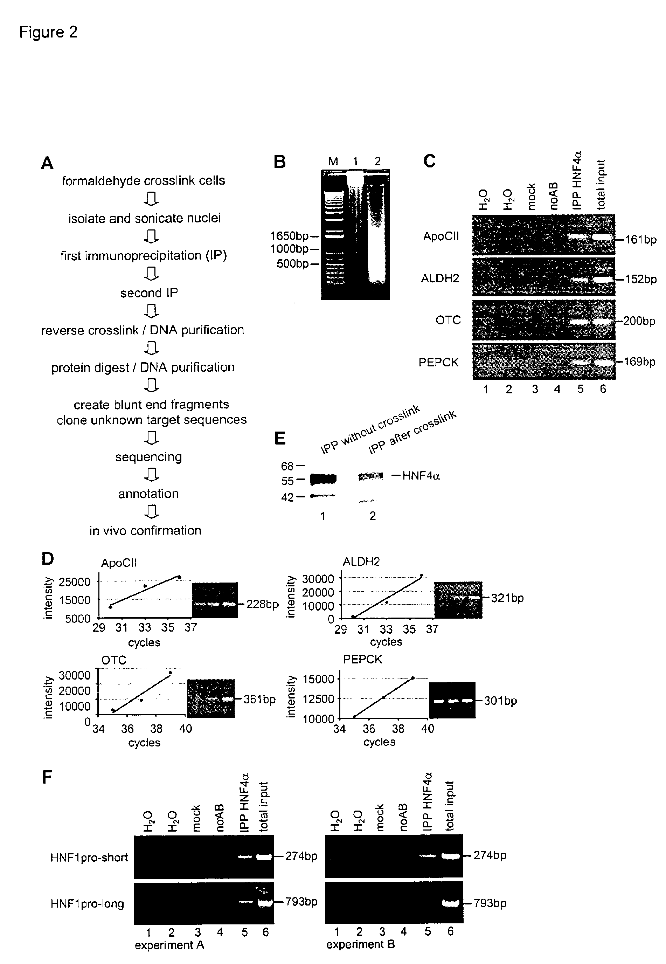

Based on formaldehyde crosslinking of nuclear proteins and cloning of immunoprecipitated DNA novel HNF4α gene targets were searched in the human intestinal cell line Caco-2, which differentiates spontaneously from cryptlike to villuslike enterocytes upon reaching confluence (29;55). At this stage, HNF4α protein expression is comparable to its expression in rat liver (Fig. 1A). Further, EMSA-studies provided additional evidence for abundant HNF4α DNA-binding to the A-site of the HNF1α-promoter (HNF1pro) (Fig. 1 B), which is an established recognition site for HNF4α (63;66). The invention therefore discloses the ChIP-cloning (Fig. 2A) of novel HNF4α gene targets from total input DNA of highly differentiated Caco-2 cell cultures. After brief formaldehyde crosslinking nuclei were isolated and subsequently extracted as described in the Material and Methods section. Soluble chromatin was fragmented by mechanical shearing. The sonication of DNA was optimized to obtain DNA fragments between 200bp and 1600bp (Fig. 2B). The HNF4α immunoprecipitated DNA was screened for enrichment of promoter regions of well-known HNF4α target-genes and HNF4α recognition sites herein (63;66). PCR-assays of immunoprecipitated DNA enabled identification of apolipoprotein-CII (ApoCII), aldehyde-dehydrogenase-2 (ALDH2), ornithine-transcarbamylase (OTC) and phosphoenolpyruvate-carboxykinase (PEPCK), all of which are well known targets for HNF4α (Fig. 2C). To control for unspecific binding DNA was also prepared in the absence of the HNF4α antibody. As shown in Fig 2C, none of the requested and well-known HNF4α target sequences could be amplified. A "mock" sample containing buffer without chromatin was used throughout the immunoprecipitation procedure and throughout the DNA isolation to control for external DNA contamination derived from buffer and wash solutions. Finally, expression of the HNF4α targeted genes ApoCll, ALDH2, OTC and PEPCK was further confirmed by RT-PCR (Fig. 2D). The invention therefore provides strong evidence for immunoprecipitated DNA to be enriched for HNF4α binding sites and it demonstrates selectivity of the adopted procedure based on a well-controlled experimental approach. Noteworthy, two rounds were employed of consecutive chromatin immunoprecipitations to decrease the amount of nonspecific DNA. The price for high specificity was, however, iow yield of DNA after the second immunoprecipitation step. Therefore, several immunoprecipitations were carried out in parallel and immunoprecipitated DNA was pooled following the DNA purification step (74;75). It is of considerable importance that single immunoprecipitations followed by PCR amplification of target sequences do not provide conclusive information for immunoprecipitates to contain target DNA. Indeed, signal enrichment of target sequences is insufficient when compared to the no antibody control. The procedure therefore requested two consecutive rounds of immunoprecipitation. Due to the second immunoprecipitation step, immunoprecipitated DNA yield was limited. Further, antibody recognition of the HNF4α epitope is partially hampered, when DNA is crosslinked (Fig. 1 E). Presumably, masking of the epitope after formaldehyde crosslinking is a plausible reason for immunoprecipitated DNA not to contain the full complement of known HNF4α targets. The immunoprecipitated DNA was screened for established HNF4α targets by PCR and the assay was optimized to investigate the A-site within the HNF1α promoter. Different PCR strategies were used for smaller (274bp) and larger (793bp) amplification products. Strikingly, the long fragment (Fig. 1 F) from HNF4α immunoprecipitated DNA could rarely be amplified, though immunoprecipitated DNA was positive for the short fragment. We assume DNA sonification to fragment DNA unfavorably and experimental conditions were standardized to obtain DNA fragments between 200bp and 1600bp and PCR assays were therefore optimized to allow for DNA template sizes between 150 and 300bp.

### ChIP cloning of novel HNF4α gene targets

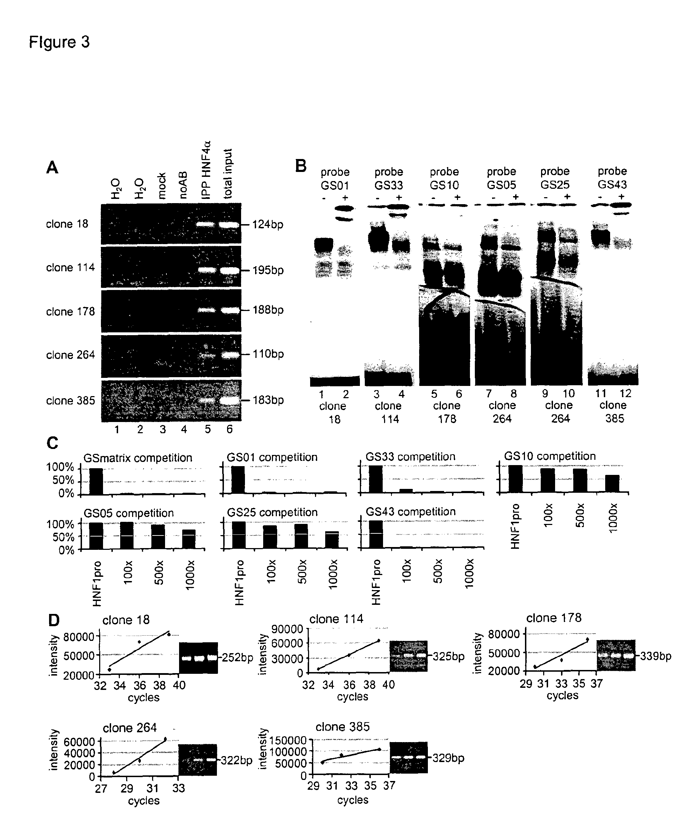

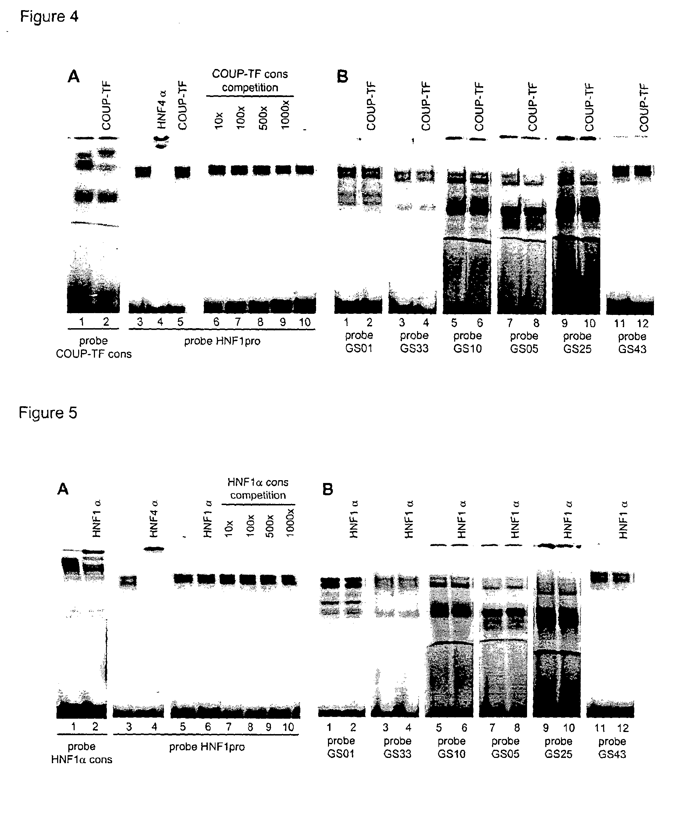

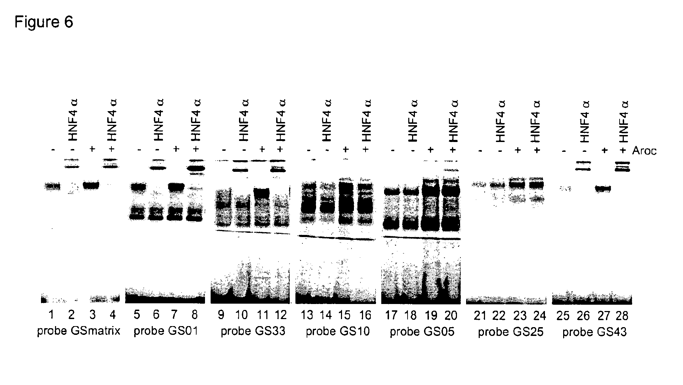

ChIP-assays of immunoprecipitated DNA yielded clones with inserts up to 1800bp. The inserts were sequenced by capillary electrophoresis and amplification with vector-specific primers and the genomic sequences were identified by database searches (GenBank, maintained by NCBI) for human genomic matches (Table 5). Approximately 50% of sequenced clones represented clearly annotated human sequences. Thereof, one half could be annotated within genes with established or predicted functions or were mapped to EST's, whereas the other half of clones could be traced back to known chromosomal localization but are of uncertain gene ID's. Nonetheless, these clones did harbor regulatory regions for HNF4α. In addition, some of the cloned fragments were within intronic regions and this agrees well with findings reported by others (23;46;68). Therefore proximal promoter sequences were analyzed. Cloned fragments as well as promoter sequences were interrogated for putative HNF4α binding-sites with two different bioinformatic matrixes. Accordingly, primer pairs were designed to confirm predicted sites experimentally and independent ChIP-experiments followed by PCR-analyses with clone-specific and/or promoter-specific primers enabled robust identification of novel HNF4α target genes. The invention discloses the identification of 13 novel HNF4α gene targets in some detail. HNF4α *in vivo* binding was confirmed with clone114 and clone178 (Fig. 3A). Additionally, predicted binding-sites in the promoter of clone18, clone264 and clone385 were bound specifically by HNF4α *in vivo* (Fig. 3A). The ability of HNF4α to bind to cognate recognition sites was studied by EMSA with ³²P-labeled probes specifically designed to encompass the predicted HNF4α-sites located in clone114 (GS33), clone178 (GS10), in the promoter of clone18 (GS01), clone264 (GS05, GS25) and clone385 (GS43) (Fig. 3B). Supershift experiments with a specific HNF4α antibody evidenced strong binding of HNF4α with the probes GS01, GS33 and GS43 but weaker binding with the probes GS05, GS10 and GS25. HNF4α displays different *in vitro* binding affinities for novel gene targets. To estimate binding affinity of HNF4α, competition experiments were carried out (Fig. 3C). The HNF1 pro-site served as labeled probe to capture HNF4α nuclear protein and competition was first analyzed with a specifically designed probe based on a weighted bioinformatic matrix. This probe (GSmatrix) competed successfully for HNF4α binding (100x, reduction to 3,2%). Likewise, competitive EMSA with probes GS01, GS33 and GS43 provided evidence for strong binding with 100-fold excess of probes to result in 2,6%, 12,7% and 3% reduction in HNF4α binding, respectively, whereas competition with probe GS05, GS10 and GS25 was minimal, i.e. no change to approximately 85% at 100-fold excess. Competition experiments were complemented by supershift experiments, and in vivo and in vitro binding of HNF4α for clone114, clone178 and binding sites within the promoter of clone18, clone264 and clone385 were confirmed. The invention further confirmed transcript expression of the new HNF4α gene targets (clone18, clone114, clone178, clone264 and clone385) in cultures of Caco-2 cells (Fig. 3D). This provided additional evidence for a role for HNF4α in the transcriptional regulation of these genes.
Further, the newly identified HNF4α binding sites were studied for interaction with COUP-TF or HNF1α. Initially it was assayed for COUP-TF (Fig. 4A, lane 1 and 2) and HNF1α (Fig. 5A, lane 1 and 2) and binding of these proteins was confirmed. Neither COUP-TF (Fig. 4A, lane 5) nor HNF1α (Fig. 5A, lane 4) contacted, however, the A-site of the HNF1α promoter (HNF1pro). Similar, COUP-TF (Fig. 4B) and HNF1α (Fig. 5B) did not bind to the newly identified HNF4α recognition sites.
As gene expression of a broad range of CYP isozymes is regulated by HNF4α (33) and treatment of hepatocytes with Aroclor 1254 led to simultaneous induction of HNF4α and several detoxifying enzymes (4), HNF4α-binding in liver nuclear extracts of control and Aroclor treated rats was investigated by EMSA. Binding of HNF4α to an optimized binding sites (GSmatrix) as well as to the newly identified binding sites (GS01, GS33, GS10, GS05 and GS43) was increased after Aroclor treatment (Fig. 6), thus providing additional evidence for the newly identified gene targets to be striktly regulated by HNF4α.
A summary of the cloned HNF4α targets is given in Table 6. Clone18 contained a ChlP-verified HNF4α binding site in the promoter region (around -2539) and was identified as C20orf13, a gene coding for a protein with predicted asparaginase activity. Clone114 was ChIP-verified from the first intron and identified as KlAA0774. The function of the coded protein product is so far unknown. Clone178 was ChlP-verified from the first intron and identified as epidermal growth factor receptor substrate (EPS15R). Clone264 contained a ChlP-verified HNF4α binding site in the promoter region (around -928) and was identified as phospholipase C, beta 1 (PLCB1). Clone385 contained a ChIP-verified HNF4α binding site in the promoter region (around -3578) and was identified as UDP-galactose transporter related protein (UGTREL1). Additionally, two kinases, RSK4 (clone23) and PAK5 (clone113) were cloned and confirmed as novel HNF4α gene targets (data reported in detail elsewhere (51)). Table 7 gives an account of HNF4α gene targets with confirmed *in vivo* binding (Fig. 7A) but lack HNF4α *in vitro* binding to the ChIP confirmed fragments as studied by EMSA (Fig. 7B). Further, transcript expression of these clones in Caco-2 cells was confirmed (Fig. 7C). Clone84 contained a ChIP-verified HNF4α binding site in the promoter region (around -575) and was identified as fragile X mental retardation protein 2 (FMR2). Clone177 contained a ChIP-verified HNF4α binding sites in the promoter region (around -355) and was identified as nebulin, i.e. a giant muscle protein. Clone261 was ChIP-verified from a region located around 7.5 kb upstream of potassium voltage-gated channel, KQT-like subfamily, member 4 (KCNQ4) and around 4.7 kb downstream of the C-subunit of nuclear factor Y (NFYC). Clone310 contained a ChIP-verified HNF4α binding site in the promoter region (around -5) and was identified as pre-mRNA processing factor 3 (PRPF3). Clone460 was ChIP-verified from the first intron and identified as transient receptor potential cation channel, subfamily C, member 1 (TRPC1). In addition, in vitro binding of HNF4α to further putative promoter binding sites was analyzed and HNF4α binding to FMR2, NFYC, KCNQ4 and PRPF3 was observed (Fig. 7D).

The discovery of the above described target genes of HNF4α and their function comprises an enormous potential for the treatment of metabolic diseases including diabetes and diabetic caused diseases and tumor growth. By cooperation with pharmaceutic companies this knowledge can be excellently used to develop new straightforward strategies and outstanding agents for the simple treatment of metabolic disorders. Such agents can be, for example, drugs which affect the genes or their gene products being dysregulated due to the disease.

### Reference List

1. Antes, T. J., S. A. Goodart, W. Chen, and B. Levy-Wilson. 2001. Human apolipoprotein B gene intestinal control region. Biochemistry 40:6720-6730.
2. Barski, A. and B. Frenkel. 2004. CHIP Display: novel method for identification of genomic targets of transcription factors. Nucleic Acids Res 32:e104.
3. Bisaha, J. G., T. C. Simon, J. I. Gordon, and J. L. Breslow. 1995. Characterization of an Enhancer Element in the Human Apolipoprotein C-III Gene That Regulates Human Apolipoprotein A-I Gene Expression in the Intestinal Epithelium. J.Biol.Chem. 270:19979-19988.
4. Borlak, J. and T. Thum. 2001. Induction of nuclear transcription factors, cytochrome P450 monooxygenases, and glutathione S-transferase alpha gene expression in Aroclor 1254-treated rat hepatocyte cultures. Biochemical Pharmacology 61:145-153.
5. Caricasole, A., C. Sala, R. Roncarati, E. Formenti, and G. C. Terstappen. 2000. Cloning and characterization of the human phosphoinositide-specific phospholipase C-beta 1 (PLC beta 1). Biochim.Biophys.Acta 1517:63-72.
6. Chakarova, C. F., M. M. Hims, H. Bolz, L. Abu-Safieh, R. J. Patel, M. G. Papaioannou, C. F. Inglehearn, T. J. Keen, C. Willis, A. T. Moore, T. Rosenberg, A. R. Webster, A. C. Bird, A. Gal, D. Hunt, E. N. Vithana, and S. S. Bhattacharya. 2002. Mutations in HPRP3, a third member of pre-mRNA splicing factor genes, implicated in autosomal dominant retinitis pigmentosa. Hum Mol Genet 11:87-92.
7. Chen, W. S., K. Manova, D. C. Weinstein, S. A. Duncan, A. S. Plump, V. R. Prezioso, R. F. Bachvarova, and J. E. Darnell, Jr. 1994. Disruption of the HNF-4 gene, expressed in visceral endoderm, leads to cell death in embryonic ectoderm and impaired gastrulation of mouse embryos. Genes Dev 8:2466-2477.
8. Chiba, H., T. Gotoh, T. Kojima, S. Satohisa, K. Kikuchi, M. Osanal, and N. Sawada. 2003. Hepatocyte nuclear factor (HNF)-4alpha triggers formation of functional tight junctions and establishment of polarized epithelial morphology in F9 embryonal carcinoma cells. Exp Cell Res 286:288-297.
9. Chiba, H., T. Itoh, S. Satohisa, N. Sakai, H. Noguchi, M. Osanai, T. Kojima, and N. Sawada. 2005. Activation of p21(CIP1/WAF1) gene expression and inhibition of cell proliferation by overexpression of hepatocyte nuclear factor-4alpha. Exp Cell Res 302:11-21.
10. Clapham, D. E. 2003. TRP channels as cellular sensors. Nature 426:517-524.
11. DeBelle, I., J. X. Wu, S. Sperandio, D. Mercola, and E. D. Adamson. 2003. In vivo cloning and characterization of a new growth suppressor protein TOE1 as a direct target gene of Egr1. J Biol Chem 278:14306-14312.
12. Deloukas, P., L. H. Matthews, J. Ashurst, J. Burton, J. G. Gilbert, M. Jones, G. Stavrides, J. P. Almeida, A. K. Babbage, C. L. Bagguley, J. Bailey, K. F. Barlow, K. N. Bates, L. M. Beard, D. M. Beare, O. P. Beasley, C. P. Bird, S. E. Blakey, A. M. Bridgeman, A. J. Brown, D. Buck, W. Burrill, A. P. Butler, C. Carder, N. P. Carter, J. C. Chapman, M. Clamp, G. Clark, L. N. Clark, S. Y. Clark, C. M. Clee, S. Clegg, V. E. Cobley, R. E. Collier, R. Connor, N. R. Corby, A. Coulson, G. J. Coville, R. Deadman, P. Dhami, M. Dunn, A. G. Ellington, J. A. Frankland, A. Fraser, L. French, P. Garner, D. V. Grafham, C. Griffiths, M. N. Griffiths, R. Gwilliam, R. E. Hall, S. Hammond, J. L. Harley, P. D. Heath, S. Ho, J. L. Holden, P. J. Howden, E. Huckle, A. R. Hunt, S. E. Hunt, K. Jekosch, C. M. Johnson, D. Johnson, M. P. Kay, A. M. Kimberley, A. King, A. Knights, G. K. Laird, S. Lawlor, M. H. Lehvaslaiho, M. Leversha, C. Lloyd, D. M. Lloyd, J. D. Lovell, V. L. Marsh, S. L. Martin, L. J. McConnachie, K. McLay, A. A. McMurray, S. Milne, D. Mistry, M. J. Moore, J. C. Mullikin, T. Nickerson, K. Oliver, A. Parker, R. Patel, T. A. Pearce, A. I. Peck, B. J. Phillimore, S. R. Prathalingam, R. W. Plumb, H. Ramsay, C. M. Rice, M. T. Ross, C. E. Scott, H. K. Sehra, R. Shownkeen, S. Sims, C. D. Skuce, M. L. Smith, C. Soderlund, C. A. Steward, J. E. Sulston, M. Swann, N. Sycamore, R. Taylor, L. Tee, D. W. Thomas, A. Thorpe, A. Tracey, A. C. Tromans, M. Vaudin, M. Wall, J. M. Wallis, S. L. Whitehead, P. Whittaker, D. L. Willey, L. Williams, S. A. Williams, L. Wilming, P. W. Wray, T. Hubbard, R. M. Durbin, D. R. Bentley, S. Beck, and J. Rogers. 2001. The DNA sequence and comparative analysis of human chromosome 20. Nature 414:865-871.
13. Dignam, J. D., R. M. Lebovitz, and R. G. Roeder. 1983. Accurate transcription initiation by RNA polymerase II in a soluble extract from isolated mammalian nuclei. Nucleic Acids Res 11:1475-1489.
14. Donner, K., M. Sandbacka, V. L. Lehtokari, C. Wallgren-Pettersson, and K. Pelin. 2004. Complete genomic structure of the human nebulin gene and identification of alternatively spliced transcripts. Eur.J Hum Genet 12:744-751.
15. Durmus, C., S. Yetiser, and O. Durmus. 2004. Auditory brainstem evoked responses in insulin-dependent (ID) and non-insulin-dependent (NID) diabetic subjects with normal hearing. Int J Audiol. 43:29-33.
16. Dusing, M. R., A. G. Brickner, S. Y. Lowe, M. B. Cohen, and D. A. Wiginton. 2000. A duodenum-specific enhancer regulates expression along three axes in the small intestine. Am J Physiol Gastrointest.Liver Physiol 279:G1080-G1093.
17. Ellrott, K., C. Yang, F. M. Sladek, and T. Jiang. 2002. Identifying transcription factor binding sites through Markov chain optimization. Bioinformatics. 18 Suppl 2 :S100-S109.
18. Faenza, I., A. Bavelloni, R. Fiume, G. Lattanzi, N. M. Maraldi, R. S. Gilmour, A. M. Martelli, P. G. Suh, A. M. Billi, and L. Cocco. 2003. Up-regulation of nuclear PLCbeta1 in myogenic differentiation. J Cell Physiol 195:446-452.
19. Fernandez, P. C., S. R. Frank, L. Wang, M. Schroeder, S. Liu, J. Greene, A. Cocito, and B. Amati. 2003. Genomic targets of the human c-Myc protein. Genes Dev 17:1115-1129.
20. Gailus-Durner, V., M. Scherf, and T. Werner. 2001. Experimental data of a single promoter can be used for in silico detection of genes with related regulation in the absence of sequence similarity. Mamm.Genome 12:67-72.
21. Galson, D. L., T. Tsuchiya, D. S. Tendler, L. E. Huang, Y. Ren, T. Ogura, and H. F. Bunn. 1995. The orphan receptor hepatic nuclear factor 4 functions as a transcriptional activator for tissue-specific and hypoxia-specific erythropoietin gene expression and is antagonized by EAR3/COUP-TF1. Mol.Cell.Biol. 15:2135-2144.
22. Gorski, K., M. Carneiro, and U. Schibler. 1986. Tissue-specific in vitro transcription from the mouse albumin promoter. Cell 47:767-776.
23. Greenbaum, S. and Y. Zhuang. 2002. Identification of E2A target genes in B lymphocyte development by using a gene tagging-based chromatin immunoprecipitation system. Proc Natl Acad Sci U.S.A 99:15030-15035.
24. Gregori, C., A. Porteu, C. Mitchell, A. Kahn, and A. L. Pichard. 2002. In vivo functional characterization of the aldolase B gene enhancer. J Biol Chem 277:28618-28623.
**25.** Gu, Y., K. L. Mcllwain, E. J. Weeber, T. Yamagata, B. Xu, B. A. Antalffy, C. Reyes, L. Yuva-Paylor, D. Armstrong, H. Zoghbi, J. D. Sweatt, R. Paylor, and D. L. Nelson. 2002. Impaired conditioned fear and enhanced long-term potentiation in Fmr2 knock-out mice. J Neurosci. 22:2753-2763.
26. Guo, H., J. Wei, Y. Inoue, F. J. Gonzalez, and P. C. Kuo. 2003. Serine/threonine phosphorylation regulates HNF-4alpha-dependent redox-mediated iNOS expression in hepatocytes. Am J Physiol Cell Physiol 284:C1090-C1099.
27. Hatzis, P. and I. Talianidis. 2002. Dynamics of enhancer-promoter communication during differentiation-induced gene activation. Mol Cell 10:1467-1477.
28. Hayhurst, G. P., Y. H. Lee, G. Lambert, J. M. Ward, and F. J. Gonzalez. 2001. Hepatocyte nuclear factor 4alpha (nuclear receptor 2A1) is essential for maintenance of hepatic gene expression and lipid homeostasis. Mol Cell Biol 21:1393-1403.
29. Hu, C. and D. H. Perlmutter. 1999. Regulation of alpha1-antitrypsin gene expression in human intestinal epithelial cell line caco-2 by HNF-1alpha and HNF-4. Am J Physiol 276:G1181-G1194.
30. Hug, B. A., N. Ahmed, J. A. Robbins, and M. A. Lazar. 2004. A chromatin immunoprecipitation screen reveals protein kinase Cbeta as a direct RUNX1 target gene. J Biol Chem. 279:825-830.
31. Ishida, N., N. Miura, S. Yoshioka, and M. Kawakita. 1996. Molecular cloning and characterization of a novel isoform of the human UDP-galactose transporter, and of related complementary DNAs belonging to the nucleotide-sugar transporter gene family. J Biochem.(Tokyo) 120:1074-1078.
**32.** Jishage, M., T. Fujino, Y. Yamazaki, H. Kuroda, and T. Nakamura. 2003. Identification of target genes for EWS/ATF-1 chimeric transcription factor. Oncogene 22:41-49.
33. Jover, R., R. Bort, M. J. Gomez-Lechon, and J. V. Castell. 2001. Cytochrome P450 regulation by hepatocyte nuclear factor 4 in human hepatocytes: a study using adenovirus-mediated antisense targeting. Hepatology 33:668-675.
34. Kamiya, A., Y. Inoue, and F. J. Gonzalez. 2003. Role of the hepatocyte nuclear factor 4[alpha] in control of the pregnane X receptor during fetal liver development. Hepatology 37:1375-1384.
35. Kel, A., O. Kel-Margoulis, V. Babenko, and E. Wingender. 1999. Recognition of NFATp/AP-1 composite elements within genes induced upon the activation of immune cells. J Mol Biol 288:353-376.
36. Kel, A. E., O. V. Kel-Margoulis, P. J. Farnham, S. M. Bartley, E. Wingender, and M. Q. Zhang. 2001. Computer-assisted identification of cell cycle-related genes: new targets for E2F transcription factors. J Mol Biol 309:99-120.
37. Kim, J. H., P. Hui, D. Yue, J. Aycock, C. Leclerc, A. R. Bjoring, and A. S. Perkins. 1998. Identification of candidate target genes for EVI-1, a zinc finger oncoprotein, using a novel selection strategy. Oncogene 17:1527-1538.
38. Klapisz, E., I. Sorokina, S. Lemeer, M. Pijnenburg, A. J. Verkleij, and P. van Bergen en Henegouwen. 2002. A Ubiquitin-interacting Motif (UIM) Is Essential for Eps15 and Eps15R Ubiquitination. J.Biol.Chem. 277:30746-30753.
39. Klingenhoff, A., K. Frech, K. Quandt, and T. Werner. 1999. Functional promoter modules can be detected by formal models independent of overall nucleotide sequence similarity. Bioinformatics. 15:180-186.
40. Ktistaki, E. and I. Talianidis . 1997. Chicken ovalbumin upstream promoter transcription factors act as auxiliary cofactors for hepatocyte nuclear factor 4 and enhance hepatic gene expression. Mol Cell Biol 17:2790-2797.
41. Ktistaki, E. and I. Talianidis . 1997. Modulation of hepatic gene expression by hepatocyte nuclear factor 1. Science 277:109-112.
42. Le Hir, H., A. Nott, and M. J. Moore. 2003. How introns influence and enhance eukaryotic gene expression. Trends Biochem Sci 28:215-220.
43. Levine, M. and R. Tjian. 2003. Transcription regulation and animal diversity. Nature 424:147-151.
44. Li, J., G. Ning, and S. A. Duncan. 2000. Mammalian hepatocyte differentiation requires the transcription factor HNF-4alpha. Genes Dev 14:464-474.
45. Love-Gregory, L. D., J. Wasson, J. Ma, C. H. Jin, B. Glaser, B. K. Suarez, and M. A. Permutt. 2004. A Common Polymorphism in the Upstream Promoter Region of the Hepatocyte Nuclear Factor-4alpha Gene on Chromosome 20q Is Associated With Type 2 Diabetes and Appears to Contribute to the Evidence for Linkage in an Ashkenazi Jewish Population. Diabetes 53:1134-1140.
46. Martone, R., G. Euskirchen, P. Bertone, S. Hartman, T. E. Royce, N. M. Luscombe, J. L. Rinn, F. K. Nelson, P. Miller, M. Gerstein, S. Weissman, and M. Snyder. 2003. Distribution of NF-{kappa}B-binding sites across human chromosome 22. PNAS 100:12247-12252.
47. Matuoka, K. and C. K. Yu. 1999. Nuclear factor Y (NF-Y) and cellular senescence. Exp Cell Res 253:365-371.
48. Nagase, T., K. Ishikawa, M. Suyama, R. Kikuno, N. Miyajima, A. Tanaka, H. Kotani, N. Nomura, and O. Ohara. 1998. Prediction of the coding sequences of unidentified human genes. XI. The complete sequences of 100 new cDNA clones from brain which code for large proteins in vitro. DNA Res. 5:277-286.
**49.** Naiki, T., M. Nagaki, Y. Shidoji, H. Kojima, M. lmose, T. Kato, N. Ohishi, K. Yagi, and H. Moriwaki. 2002. Analysis of gene expression profile induced by hepatocyte nuclear factor 4alpha in hepatoma cells using an oligonucleotide microarray. J Biol Chem 277:14011-14019.
50. Nelson, E. A., S. R. Walker, J. V. Alvarez, and D. A. Frank. 2004. Isolation of unique STAT5 targets by chromatin immunoprecipitation-based gene identification. J Biol Chem.
51. Niehof, M. and J. Borlak. 2005. RSK4 and PAK5 are novel candidate genes in diabetic rat kidney and brain. Mol Pharmacol, Mol. Pharmacol. 67 604-611.
52. Nishiyori, A., H. Tashiro, A. Kimura, K. Akagi, K. Yamamura, M. Mori, and M. Takiguchi. 1994. Determination of tissue specificity of the enhancer by combinatorial operation of tissue-enriched transcription factors. Both HNF-4 and C/EBP beta are required for liver-specific activity of the ornithine transcarbamylase enhancer. J.Biol.Chem. 269:1323-1331.
53. Odom, D. T., N. Zizlsperger, D. B. Gordon, G. W. Bell, N. J. Rinaldi, H. L. Murray, T. L. Volkert, J. Schreiber, P. A. Rolfe, D. K. Gifford, E. Fraenkel, G. I. Bell, and R. A. Young. 2004. Control of Pancreas and Liver Gene Expression by HNF Transcription Factors. Science 303:1378-1381.
54. Parviz, F., C. Matullo, W. D. Garrison, L. Savatski, J. W. Adamson, G. Ning, K. H. Kaestner, J. M. Rossi, K. S. Zaret, and S. A. Duncan. 2003. Hepatocyte nuclear factor 4alpha controls the development of a hepatic epithelium and liver morphogenesis. Nat Genet 34:292-296.
55. Perlmutter, D. H., J. D. Daniels, H. S. Auerbach, K. Schryver-Kecskemeti, H. S. Winter, and D. H. Alpers. 1989. The alpha 1-antitrypsin gene is expressed in a human intestinal epithelial cell line. J.Biol.Chem. 264:9485-9490.
56. Phelps, D. E. and G. R. Dressier. 1996. Identification of novel Pax-2 binding sites by chromatin precipitation. J Biol Chem 271:7978-7985.
57. Pineda, T., I, Y. Jamshidi, D. M. Flavell, J. C. Fruchart, and B. Staels. 2002. Characterization of the human PPARalpha promoter: identification of a functional nuclear receptor response element. Mol Endocrinol. 16:1013-1028.
58. Qian, F., P. Huang, L. Ma, A. Kuznetsov, N. Tamarina, and L. H. Philipson. 2002. TRP genes: candidates for nonselective cation channels and store-operated channels in insulin-secreting cells. Diabetes 51 Suppl 1:S183-S189.
59. Rastegar, M., G. G. Rousseau, and F. P. Lemaigre. 2000. CCAAT/enhancer-binding protein-alpha is a component of the growth hormone-regulated network of liver transcription factors. Endocrinology 141:1686-1692.
60. Robinson, L., A. Panayiotakis, T. S. Papas, I. Kola, and A. Seth. 1997. ETS target genes: identification of egr1 as a target by RNA differential display and whole genome PCR techniques. Proc Natl Acad Sci U.S.A 94:7170-7175.
61. Romier, C., F. Cocchiarella, R. Mantovani, and D. Moras. 2003. The NF-YB/NF-YC structure gives insight into DNA binding and transcription regulation by CCAAT factor NF-Y. J Biol Chem. 278:1336-1345.
62. Santoro, R., S. Wolfl, and H. P. Saluz. 1999. UV-Laser induced protein/DNA crosslinking reveals sequence variations of DNA elements bound by c-Jun in vivo. Biochem Biophys Res Commun 256:68-74.
63. Schrem, H., J. Klempnauer, and J. Borlak. 2002. Liver-enriched transcription factors in liver function and development. Part I: the hepatocyte nuclear factor network and liver-specific gene expression. PharmacoLRev 54:129-158.
64. Seki, K. and A. Hata. 2004. Indian hedgehog gene is a target of the bone morphogenetic protein signaling pathway. J.BioLChem. 279:18544-18549.
65. Silander, K., K. L. Mohlke, L. J. Scott, E. C. Peck, P. Hollstein, A. D. Skol, A. U. Jackson, P. Deloukas, S. Hunt, G. Stavrides, P. S. Chines, M. R. Erdos, N. Narisu, K. N. Conneely, C. Li, T. E. Fingerlin, S. K. Dhanjal, T. T. Valle, R. N. Bergman, J. Tuomilehto, R. M. Watanabe, M. Boehnke, and F. S. Collins. 2004. Genetic Variation Near the Hepatocyte Nuclear Factor-4alpha Gene Predicts Susceptibility to Type 2 Diabetes. Diabetes 53:1141-1149.
66. Sladek, F. M. and S. D. Seidel. 2001. Hepatocyte nuclear factor 4alpha, p. 309-361. In T. Burris and E. R. B. McCabe (ed.), Nuclear Receptors and Disease. Academic Press, London.
67. Smith, P. K., R. I. Krohn, G. T. Hermanson, A. K. Mallia, F. H. Gartner, M. D. Provenzano, E. K. Fujimoto, N. M. Goeke, B. J. Olson, and D. C. Klenk. 1985. Measurement of protein using bicinchoninic acid. Anal.Biochem. 150:76-85.
68. Solano, P. J., B. Mugat, D. Martin, F. Girard, J. M. Huibant, C. Ferraz, B. Jacq, J. Demaille, and F. Maschat. 2003. Genome-wide identification of in vivo Drosophila Engrailed-binding DNA fragments and related target genes. Development 130:1243-1254.
69. Stevens, T. A., J. S. lacovoni, D. B. Edelman, and R. Meech. 2004. Identification of novel binding elements and gene targets for the homeodomain protein BARX2. J Biol Chem. 279:14520-14530.
70. Thomas, H., S. Senkel, S. Erdmann, T. Arndt, G. Turan, L. Klein-Hitpass, and G. U. Ryffel. 2004. Pattern of genes influenced by conditional expression of the transcription factors HNF6, HNF4alpha and HNF1beta in a pancreatic beta-cell line. Nucleic Acids Res 32:e150.
71. Tirona, R. G., W. Lee, B. F. Leake, L. B. Lan, C. B. Cline, V. Lamba, F. Parviz, S. A. Duncan, Y. Inoue, F. J. Gonzalez, E. G. Schuetz, and R. B. Kim. 2003. The orphan nuclear receptor HNF4alpha determines PXR- and CAR-mediated xenobiotic induction of CYP3A4. Nat Med 9:220-224.
72. Tsuchiya, T., Y. Kominato, and M. Ueda. 2002. Human hypoxic signal transduction through a signature motif in hepatocyte nuclear factor 4. J Biochem (Tokyo) 132:37-44.
73. Van Camp, G., P. J. Coucke, J. Akita, E. Fransen, S. Abe, E. M. De Leenheer, P. L. Huygen, C. W. Cremers, and S. Usami. 2002. A mutational hot spot in the KCNQ4 gene responsible for autosomal dominant hearing impairment. Hum Mutat 20:15-19.
74. Weinmann, A. S., S. M. Bartley, T. Zhang, M. Q. Zhang, and P. J. Farnham. 2001. Use of chromatin immunoprecipitation to clone novel E2F target promoters. Mol Cell Biol 21:6820-6832.
75. Weinmann, A. S. and P. J. Farnham. 2002. Identification of unknown target genes of human transcription factors using chromatin immunoprecipitation. Methods 26:37-47.

### Tables 1-7

**Table 1. Shift-probes sequences.**

| gene / clone | oligo-name | sequence |
|---|---|---|
| HNF1α | HNF1pro | AAGGCTGAAGTCCAAAGTTCAGTCCCTTC |
| - | GSmatrix | AGGGGGGGTCAAAGGTCACGGTC |
| - | HNF1cons | CCAGTTAATGATTAACCACTGGC |
| - | COUP-TF cons | TGAGCCCTTGACCCCT |
| clone 18, pro-site | GS01 | TTAGAGTACAAAGATCAAGATGC |
| clone 23, pro-site a | GS26 | AATGGAGGGCATAGGTCAACAGC |
| clone 23, pro-site b | GS27 | CCAGCGCTCAAAAGGTTGGCAGT |
| clone 84, pro-site a | GS28 | TCCAGGGCCTATAGCTCGCTGAC |
| clone 84, pro-site b | GS65 | CTGAAGGCATAAAGGTCGGGGGC |
| clone 113, site a | GS09 | TGTTGGGTACAATGTTCAATATT |
| clone 113, site b | GS16 | AAAGCTGACTAAGGTACATGTGC |
| clone 113, pro-site b | GS46 | TTAAGTGATTAAAGTTCAATATT |
| clone 114 | GS33 | AAATAGATACAAAGTCCTCCTTC |
| clone 177, pro-site a | GS24 | CGGAAGGGTTAAAGCCTCTCAAA |
| clone 177, pro-site b | GS03 | TGACTGGACAAATGACATGAAGA |
| clone 178 | GS10 | AACTGTGGTCCAAGCACACCATG |
| clone 261 | GS36 | CCTAGCTTTTGTCCCACAGCTCC |
| clone 261.1, pro-site | GS76 | GGGCGGGGTCAAAACTCAGATCT |
| clone 261.2, pro-site | GS82 | TGTGGTGGGGAAAGTCCGTCCTC |
| clone 264, pro-site a | GS05 | AAACCCGGGCAAACGGTTACGTT |
| clone 264, pro-site b | GS25 | CCCGGGTTTCAAGATCAGCGCGC |
| clone 310, pro-site a | GS23 | TAGGGCGGTCAGAAGGTTTCCGG |
| clone 310, pro-site b | GS70 | CCTGAGGTCCAGAGTTCGAGACG |
| clone 385, pro-site | GS43 | TGACAAGAACAAAGTCCATTTCA |
| clone 460, site a | GS34 | CTCACTGTGCAATGGTTATTTTA |
| clone 460, site b | GS35 | TCTTCCCTCAAAAGTTCTGAGAG |

**Table 2. ChlP-PCR primer sequences.**

| gene / clone | primer name | primer sequence | fragment length | annealing |
|---|---|---|---|---|
| HNF1α | Ni16 | fwd: CATGATGCCCCTACAAGGTT | 274bp | 60°C |
| | | rev: ATTGGAGCTGGGGAAATTCT | | |
| HNF1α | Ni18 | fwd: CAGCACTGTTCTTGGCACAT | 793bp | 60°C |
| | | rev: CAGCACTGTTCTTGGCACAT | | |
| ApoCll | Ni57 | fwd: GTTCCCTGTGACGTGACCTT | 161bp | 60°C |
| | | rev: ACGGGCACAGAGAGGATTTA | | |
| ALDH2 | Ni58 | fwd: CATCTCCTTCACCTCCGAAA | 152bp | 60°C |
| | | rev: CAGCTCGCCTTGGTTGAG | | |
| OTC | Ni118 | fwd: AGGAGGCCAGGCAATAAAAG | 200bp | 60°C |
| | | rev: GGGGGCCACCTAAAAACTAA | | |
| PEPCK | Ni119 | fwd: GGCACAGAGCAGACAATCAA | 169bp | 60°C |
| | | rev: TTGGCAAAACACCACAGCTA | | |
| clone 18, pro-site | Ni3 | fwd: TCTTCCTGTTCCCACCTCTC | 124bp | 60°C |
| | | rev: AGGACAGAGGGGGCTTACTT | | |
| clone 23, pro-site a | Ni8 | fwd: CCAACTCAGGACCTTGGAGA | 141bp | 60°C |
| | | rev: GGCCAGCTTTGCTTCATTAG | | |
| clone 23, pro-site b | Ni13 | fwd: GAGCTGCTGTGCCTGGTACT | 148bp | 60°C |
| | | rev: TTTTTGCTGACGGGAGAGAT | | |
| clone 84, pro-site a | Ni2 | fwd: CAAGGGCAGTCATTTGTTCC | 148bp | 60°C |
| | | rev: GAAGGCGGTCACCTTCAC | | |
| clone 113, site a | Ni61 | fwd: TCATCACGGACATAAAGATGGA | 160bp | 60°C |
| | | rev: GCATAGTGGTGGGGGTTCT | | |
| clone 113, site b | Ni73 | fwd: AGCAGAACCCCCACCACTAT | 187bp | 60°C |
| | | rev: TCACCCAGAAAGTTCCCTTG | | |
| clone 113, pro-site a | Ni5 | fwd: CCGGTCAAGTCTGAACCAGT | 126bp | 60°C |
| | | rev: TTAACTAGGCAAGCCCAAGC | | |
| clone 113, pro-site c | N1116 | fwd: TAGTCCCTGTGGCTGCAGTA | 232bp | 60°C |
| | | rev: TCTCAATGGCTGATTACAGGTT | | |
| clone 114 | Ni62 | fwd: AAAGGCCTAATCTTTTGTTTCTACA | 195bp | 60°C |
| | | rev: TCACAGCACATTTTATGTGTCAA | | |
| clone 177, pro-site a and b | Ni115 | fwd: ACTTCCAGCCTGTGCAGTTC | 196bp | 60°C |
| | | rev: CTGGTGAACCTTACCAGAGTGA | | |
| clone 178 | Ni64 | fwd: TGTCACTGCTCCAAACTGGT | 188bp | 60°C |
| | | rev: ACCTTTGAGGTTTGGCCTTT | | |
| clone 261 | Ni88 | fwd: CCCTTCCCACCAACTCTTG | 190bp | 60°C |
| | | rev: GAAGACACCAGCAGCCTAGC | | |
| clone 264, pro-site a and b | Ni7 | fwd: TCTGTCTCGAAAGCACAACG | 110bp | 60°C |
| | | rev: AGAAGAGCGCAGTTGAGAGG | | |
| clone 310, pro-site | Ni9 | fwd: CTCCTTGGTCACGTGTTGG | 131bp | 60°C |
| | | rev: CAAACTTCAGCCCCTGAGAC | | |
| clone 385, pro-site | Ni112 | fwd: AAGAGGGGCTTCATCAGGTT | 183bp | 60°C |
| | | rev: CTCACCCTCTCTCGCTGTCT | | |
| clone 460, site a and b | Ni82 | fwd: TGGGTTGCCTGAGTTCTCTT | 182bp | 60°C |
| | | rev: TCTGTCCTCAGTTTGACAGGAA | | |

**Table 3. Sequence information of clones.**

| clone | gene name | Acc Number | chromosome | bp relative to transcription start site NCBI GenBank Version Build 35.1 |
|---|---|---|---|---|
| clone 18 | C20orf13 | NM_017714 | 20 | +198113 bis +198612 (500 bp) |
| clone 23 | RSK4 / RPS6KA6 | NM_014496 | X | +13766 to +14366 (601 bp) |
| clone 84 | FMR2 | NM_002025 | X | +8271 bis +8440 (170 bp) |
| clone 113 | PAK5 / PAK7 | NM_045653 | 20 | +246492 to +246837 (346 bp) |
| clone114 | KIAA0774 | XM_166270 | 13 | +7954 bis +8111 (158 bp) |
| clone177 | NEB | NM_004543 | 2 | +3957 bis +4396 (440 bp) |
| clone178 | EPS15R (EPS15L1) | NM_021235 | 19 | -7250 bis -7612 (363 bp) |
| | | | | UCSC_hg17* +22121 bis +22483 (363 bp) |
| clone 261 | NFYC / KCNQ4 KCNQ4 | NM_0142237 NM_004700 NM_004700 | 1 | NFYC: +84624 bis +84750 (127bp) |
| | | | | KCNQ4: -7694 bis -7568 (127bp) |
| clone 264 | PLCB1 | NM_015192 | 20 | +106006 bis +106181 (176 bp) |
| clone 310 | PRPF3 (HPRP3, PRP3) | NM_004698 | 1 | +16434 bis +16480 (47 bp) |
| clone 385 | UGTREL1 (SLC35B1) | NM_005827 | 17 | +6820 bis +6924 (105 bp) |
| clone 460 | TRPC1 | NM_003304 | 3 | +1632 bis +1965 (334bp) |
| *UCSC Genome Browser, Version hg17 (http://genome.ucsc.edu/) | | | | |

**Table 4. RT-PCR primer sequences.**

| gene /clone | primer name | accession number | primer | fragment length | annealing |
|---|---|---|---|---|---|
| ApoCli | Ni95 | NM_000483 | fwd: CCTCCCAGCTCTGTTTCTTG | 228bp | 60°C |
| | | | rev: GCTGCTGTGCTTTTGCTGTA | | |
| ALDH2 | Ni25 | NM_000690 | fwd: TGAAGGGGACAAGGAAGATG | 321bp | 58°C |
| | | | rev: ACAGGTTCATGGCGTGTGTA | | |
| OTC | Ni120 | NM_000531 | fwd: CATGGCAGATGCAGTATTGG | 261bp | 60°C |
| | | | rev: GGAGTAGCTGCCTGAAGGTG | | |
| PEPCK | Ni121 | NM_002591 | fwd: TCAGGCGGCTGAAGAAGTAT | 301bp | 60°C |
| | | | rev: ACGTAGGGTGAATCCGTCAG | | |
| clone18 | Ni29 | NM_017714 | fwd: CAAATGCAGGAATGGGATCT | 252bp | 58°C |
| | | | rev: GGCAAGAGGGTATTCCATGA | | |
| clone 23 | Ni69 | NM_014496 | fwd: GGATTTTCTCAGGGGAGGAG | 311bp | 60°C |
| | | | rev: AATCAGCACTCTGGGAATGG | | |
| clone 84 | Ni96 | NM_002025 | fwd: CACAAGGAGACTGCCACAAA | 328 bp | 50°C |
| | | | rev: AGGGACCATTATTGCCACTG | | |
| clone 113 | Ni72 | NM_020341 | fwd: GAATCAGACAAGCCCTCAGC | 309bp | 55°C |
| | | | rev: CCAGACGGGTACTGGTGACT | | |
| clone114 | Ni122 | XM_166270 | fwd: CTGACCTTCCAGAGCCAGTC | 325bp | 50°C |
| | | | rev: TAGCATTTTCCTCCGACAGC | | |
| clone 177 | Ni51 | NM_004543 | fwd: CCCAGAGGCTACACCACAAT | 332 bp | 55°C |
| | | | rev: GAAAGCTTGCAACCCTTGAG | | |
| clone178 | Ni71 | NM_021235 | fwd: GCAGACAAGATGCGATTTGA | 339bp | 60°C |
| | | | rev: AAGCTCCTTCACGCCAGTAA | | |
| clone 261.1 | Ni102 | NFYC NM_0142237 | fwd: AAAGACTTCCGAGTGCAGGA | 316bp | 60°C |
| | | | rev: GCTCGGCAGGAGTTACAGAC | | |
| clone 261.2 | Ni103 | KCNQ4 NM_004700 | fwd: AGGAACTTGCCAACGAGTGT | 331bp | 60°C |
| | | | rev: CTATGCGCGTAGACCACTGA | | |
| clone264 | Ni52 | NM_015192 | fwd: GTTTTCAGCAGATCGGAAGC | 322bp | 55°C |
| | | | rev: GAGGCTGTTGTTGGGTTCAT | | |
| clone 310 | Ni53 | NM_004698 | fwd: CCCCAATGGCTTTGATCTTA | 321bp | 55°C |
| | | | rev: GCTCTGACGTGGGCTTCTAC | | |
| clone385 | Ni105 | NM_005827 | fwd: CTATCTGGGTGCCATGGTCT | 329bp | 60°C |
| | | | rev: GGTTGGAGCCTGTTTGGTAA | | |
| clone 460 | Ni106 | NM_003304 | fwd: TGGATGTTGCACCTGTCATT | 325bp | 54°C |
| | | | rev: TTACATTGCCGGGCTAGTTC | | |

**Table 5. Summary of ChIP clone annotations after sequencing.**

| | % of clones |
|---|---|
| total | 100 |
| cloning artefacts | 22.7 |
| total human sequences | 77.3 |
| total human sequences without DNA homology | 68.0 |
| not clearly identified | 14.7 |
| gene products with established or predicted function | 17.3 |
| ESTs, computer prediction, etc | 12.0 |
| DNA of known chromosomal location but uncertain gene ID and/or function, in part putative regulatory regions | 24.0 |

**Table 6. Summary of clone information with HNF4α in vitro binding to ChIP confirmed fragments.**

| clone | gene name | localization | | Swiss-Prot / mRNA NCBI | mRNA expression in Caco-2 cells | HNF4α promoter binding sites | | molecular function | biological process |
|---|---|---|---|---|---|---|---|---|---|
| 18 | C20orf13 | chromosome 20, 11. intron | | Q9H6P5 / | yes | -2520 | ChIP confirmed | asparaginase | metabolism / |
| | | | | NM_01771 | | | EMSA binding | activity | glycoprotein |
| | | | | 4 | | | | (prediction) | catabolism |
| | | | | | | | | | (prediction) |
| | | | | | | | | | |
| 114 | KIAA077 | chromosome 13, 1. intron | | O94872 / | yes | | | hypothetical protein | unknown |
| | 4 | | Chip confirmed | XM_16627 | | | | | |
| | | | EMSA binding | 0 | | | | | |
| | | | | | | | | | |
| 178 | EPS15R | chromosome 19,1. intron* | | Q9UBC2 / | yes | | | receptor activity | endocytosis |
| | (EPS15L1 | | ChIP confirmed | NM_02123 | | | | | signal transduction |
| | ) | | EMSA binding | 5 | | | | | |
| | | | | | | | | | |
| 264 | PLCB1 | chromosome 20, 2. intron | | Q9NQ66/ | yes | -919/-906 | ChIP | phospholipase | cell communication / |
| | | | | NM_01519 | | confirmed | | activity | signal transduction |
| | | | | 2 | | -919 | EMSA binding site a | | regulation of cell |
| | | | | | | -906 | EMSA binding site b | | cycle |
| | | | | | | | | | |
| 385 | UGTREL | chromosome 17, 3'UTR | | P78383 / | yes | -3579 | ChIP confirmed | UDP-galactose | transport |
| | 1 | | | NM_00582 | | | EMSA binding | transporter activity | |
| | (SLC35B | | | 7 | | | | | |
| | 1) | | | | | | | | |
| | | | | | | | | | |
| reported in detail in (51) | | | | | | | | | |
| 23 | RSK4 | chromosome X, 1.intron | | Q9UK32 / | yes | -1430 | ChIP confirmed | protein kinase | cell communication / |
| | (RPS6KA | | | NM_01444 | | | EMSA binding | activity | signal transduction |
| | 6) | | | 96 | | -2053 | ChIP confirmed | | |
| | | | | | | | EMSA binding | | |
| | | | | | | | | | |
| 113 | PAK5 | chromosome 20, 3. intron | | Q9P286/ | below the limit of | -951 | ChIP confirmed | protein kinase | cell communication / |
| | (PAK7) | | ChIp confirmed | NM_04565 | detection | -1766 | EMSA binding | activity | signal transduction |
| | | | EMSA binding | 3 | | -2181 | ChIP confirmed | | |

| | | | | | | | | | |
|---|---|---|---|---|---|---|---|---|---|
| *UCSB Genome Browser, Version hg17 (http://genome.ucsc.edu/). UCSC_hg17 = 1. intron / NCBI Version Build 35.1, NCBI_b35.1 = -7.5kb upstream TSS (see Supplement Table 3) | | | | | | | | | |

**Table 7. Summary of clone information without HNF4α in vitro binding to ChIP confirmed fragments.**

| clone | gene name | localization | | Swiss-Prot / mRNA NCBI | mRNA expression in Caco-2 cells | HNF4α promoter binding sites | | molecular function | biological process |
|---|---|---|---|---|---|---|---|---|---|
| 84 | FMR2 | chromosome X, 1. intron | | P51816 / | yes | -576 | ChIP confirmed | transcription regulator | development |
| | | | | NM_002025 | | -1893 | EMSA binding | activity | |
| | | | | | | | | (prediction) | |
| | | | | | | | | | |
| 177 | NEB | chromosome 2, 4. intron | | P20929 / | yes | -374/-341 | ChIP confirmed | structural constituent of | development |
| | | | | NM_004543 | | | | muscle | |
| | | | | | | | | | |
| 261 | | chromosome 1 | | | | | | | |
| | | | ChIP confirmed | | | | | | |
| | | | | | | | | | |
| 261.1 | NFYC | 4.7 kb downstream | | Q13952 / | yes | -163 | EMSA binding | transcription regulator | transcription / |
| | | | | NM_014223 | | | | activity | redox response |
| | | | | | | | | | |
| 261.2 | KCNQ4 | 7,5 kb upstream | | P56696 / | yes | -2518 | | ion channel activity | ion transport / |
| | | | | NM_004700 | | | | | ion channel |
| | | | | | | | | | |
| 310 | PRPF3 | chromosome 1, 9. intron | | O43395 / | yes | -28 | ChIP confirmed | mRNA splicing factor | nuclear mRNA |
| | (HPRP3, | | | NM_004698 | | -3119 | EMSA binding | activity | splicing |
| | PRP3) | | | | | | | | |
| | | | | | | | | | |
| 460 | TRPC1 | chromosome 3, 1.intron | | P48995 / | yes | | | ion channel activity | ion transport / |
| | | | ChIP confirmed | NM_003304 | | | | | ion channel |

### Figure Legends and Figures

### Figure Legends

### Figure 1

### HNF4α protein expression in differentiated Caco-2 cells.

**(A)** HNF4α western blotting analysis of 30 µg Caco-2 cell nuclear extract (lane 1) or 30 µg rat liver nuclear extract (lane 2). **(B)** Electrophoretic mobility shift experiment with 2,5 µg Caco-2 cell nuclear extract (lane 1 and 2) or 2,5 µg rat liver nuclear extract (lane 3 and 4) and an oligonucleotide corresponding to the A-site of the HNF1α promoter (HNF1 pro) as ³²P labeled probe. For supershift analysis an antibody directed against HNF4α was added (lane 2, lane 4).

### Figure 2

### HNF4α chromatin immunoprecipitation assay with HNF4α target genes.

**(A)** Flow chart of HNF4α chromatin immunoprecipitation assay (ChIP-assay). **(B)** Fragmentation of total input DNA prior to (lane 1) and after sonification (lane 2) under standard conditions. **(C)** CHIP experiments were performed with cultures of Caco-2 cells and an antibody against HNF4α (IPP HNF4α, lane 5) or no antibody (noAB, lane 4). Following DNA purification samples were subjected to PCR with primers designed to amplify promoters of different HNF4α positive targets. The primers annealed proximal to the HNF4α binding-sites of the apolipoprotein Cll (ApoCll) promoter, the aldehyde dehydrogenase 2 (ALDH2) promoter, the ornithine transcarbamylase (OTC) promoter, and the phosphoenolpyruvate carboxykinase (PEPCK) promoter, all of which are well-known HNF4α targets. A mock probe (mock, lane 3) and an aliquot of the total input sample (total input, lane 6) were also examined by PCR. Routinely, two reactions containing H₂O instead of template were included in each PCR as negative control (lane 1 and 2). **(D)** Gene expression of ApoCll, ALDH2, OTC and PEPCK in cultures of Caco-2 cells was analyzed by RT-PCR. A linear range of amplification cycles was shown. **(E)** HNF4α immunoprecipitation with and without crosslink. HNF4α western blot analysis of HNF4α immunoprecipitated complexes of Caco-2 cells without crosslink (lane 1) or after crosslink (lane 2) with 1% formaldehyde. Prior to SDS-PAGE, crosslink samples were heated to 95 °C in conventional SDS-polyacrylamide gel electrophoresis gel-loading buffer in the presence of 0.5 M 2-mercaptoethanol for 1 h to reverse protein-protein crosslinks. **(F)** Comparison of short- and long-fragment ChIP-PCR. CHIP experiments were performed with cultures of Caco-2 cells and an antibody against HNF4α (IPP HNF4α, lane 5) or no antibody (noAB, lane 4). Following DNA purification samples were subjected to PCR with primers designed to amplify short (274bp) or long fragments (793bp) containing the A-site within the HNF1α promoter as HNF4α positive target. A mock probe (mock, lane 3) and an aliquot of the total input sample (total input, lane 6) were also examined by PCR. Routinely two reactions containing H₂O instead of template were included in each PCR as negative control (lane 1 and 2). Two experiments were shown exemplarily.

### Figure 3

### Confirmation of ChIP clones by examination of HNF4α binding in vivo und in vitro, corresponding to Table 6.

**(A)** Independent ChlP experiments were performed with cultures of Caco-2 cells and an antibody against HNF4α (IPP HNF4α, lane 5) or no antibody (noAB, lane 4). Following DNA purification samples were subjected to PCR with primers designed for putative HNF4α binding-sites of clones and their promoters (clone18, clone114, clone178, clone264, clone385). A mock probe (lane 3) and an aliquot of the total input sample (lane 6) were also examined by PCR. Routinely two reactions containing H₂O instead of template were included in each PCR as negative control (lane 1 and 2). **(B)** Electrophoretic mobility shift assays with 2,5 µg Caco-2 cell nuclear extract and oligonucleotides (GS01, lane 1 and 2; GS33, lane 3 and 4; GS10, lane 5 and 6; GS05, lane 7 and 8; GS25, lane 9 and 10; GS43, lane 11 and 12) corresponding to putative HNF4α binding-sites within the identified clones and promoters as ³²P labeled probe. In supershift assays an antibody directed against HNF4α (+) was added (lane 2, 4, 6, 8, 10, 12). **(C)** Competition experiments. Electrophoretic mobility shift experiments were carried out with 2,5 µg Caco-2 cell nuclear extracts and an oligonucleotide corresponding to the A-site of the HNF1α promoter as ³²P labeled probe. An optimized HNF4α binding-site (GSmatrix) and the putative HNF4α binding-sites (GS01, GS33, GS10, GS05, GS25, GS43) were added as 100 fold, 500 fold and 1000 fold molar excess. Dried gels were analysed with a Molecular Imager (BioRad) using the Quantity One software (BioRad). HNF4α binding to the A-site of the HNF1α promoter as ³²P labeled probe was set to 100% and competition was quantified for each oligonucleotide. **(D)** Gene expression of clone18, clone114, clone178, clone264 and clone385 in cultures of Caco-2 cells was analyzed by RT-PCR. A linear range of amplification cycles was shown.

### Figure 4

### Analysis of COUP-TF interference in HNF4α DNA binding.

**(A)** Electrophoretic mobility shift assays with 10 µg Caco-2 cell nuclear extract and an oligonucleotide corresponding to a consensus COUP-TF binding-site (COUP-TF cons, lane 1 and 2) or 2,5 µg Caco-2 cell nuclear extract and an oligonucleotide corresponding to the A-site of the HNF1α promoter (HNF1pro, lane 3 to 10) as ³²P labeled probe. For supershift analysis antibodies directed against COUP-TF (lane 2 and 5) and against HNF4α (lane 4) were added. **(B)** Electrophoretic mobility shift assays with 10 µg Caco-2 cell nuclear extract and oligonucleotides corresponding to the putative HNF4α binding-sites (GS01, lane 1 and 2; GS33, lane 3 and 4; GS10, lane 5 and 6; GS05, lane 7 and 8; GS25, lane 9 and 10; GS43, lane 11 and 12) as ³²P labeled probe. For supershift analysis an antibody directed against COUP-TF (lane 2, 4, 6, 8, 10, 12) was added.

### Figure 5

### Analysis of HNF1α interference in HNF4α DNA binding.

(A) Electrophoretic mobility shift assays with 10 µg Caco-2 cell nuclear extract and an oligonucleotide corresponding to a consensus HNF1α binding-site (HNF1α cons, lane 1 and 2) or 2,5 µg Caco-2 cell nuclear extract and an oligonucleotide corresponding to the A-site of the HNF1α promoter (HNF1pro, lane 3 to 10) as ³²P labeled probe. For supershift analysis antibodies directed against HNF1α (lane 2 and 6) and against HNF4α (lane 4) were added. **(B)** Electrophoretic mobility shift assays with 10 µg Caco-2 cell nuclear extract and oligonucleotides corresponding to the putative HNF4α binding-sites (GS01, lane 1 and 2; GS33, lane 3 and 4; GS10, lane 5 and 6; GS05, lane 7 and 8; GS25, lane 9 and 10; GS43, lane 11 and 12) as ³²P labeled probe. For supershift analysis an antibody directed against HNF1α (lane 2, 4, 6, 8, 10, 12) was added.

### Figure 6

### Aroclor treatment induces HNF4α in rat liver: in vitro binding to new targets

Electrophoretic mobility shift assay with 2,5 µg rat liver nuclear extract of control (lane 1, 2, 5, 6, 9, 10, 13, 14, 17, 18, 21, 22, 25, 26) or Aroclor treated animals (lane 3, 4, 7, 8, 11, 12, 15, 16, 19, 20, 23, 24, 27, 28) and oligonucleotides corresponding to putative HNF4α binding-sites (GSmatrix, lane 1-4; GS01, lane 5-8; GS33, lane 9-12; GS10, lane 13-16; GS05, lane 17-20; GS25, lane 21-24; GS43, lane 25-28) as ³²P labeled probe. For supershift analysis an antibody directed against HNF4α was added (lane 2, 4, 6, 8, 10, 12, 14, 16, 18, 20, 22, 24, 26, 28).

### Figure 7

### Confirmation of ChIP clones by examination of HNF4α binding in vivo und in vitro, corresponding to Table 7.

**(A)** CHIP experiments were performed with cultures of Caco-2 cells and an antibody against HNF4α (IPP HNF4α, lane 5) or no antibody (noAB, lane 4). Following DNA purification samples were subjected to PCR with primers designed for putative HNF4α binding-sites of clones and their promoters (clone84, clone177, clone261, clone310, clone460). A mock probe (lane 3) and an aliquot of the total input sample (lane 6) were also examined by PCR. Routinely two reactions containing H₂O instead of template were included in each PCR as negative control (lane 1 and 2). **(B)** Analysis of HNF4α *in vitro* binding to *in vivo* confirmed fragments. Electrophoretic mobility shift assays with 2,5 µg Caco-2 cell nuclear extract and oligonucleotides (GS28, lane 1 and 2; GS03, lane 3 and 4; GS24, lane 5 and 6; GS36, lane 7 and 8; GS23, lane 9 and 10; GS34, lane 11 and 12; GS35, lane 13 and 14) corresponding to putative HNF4α binding-sites as ³²P labeled probe. In supershift assays an antibody directed against HNF4α (+) was added (lane 2, 4, 6, 8, 10, 12, 14). **(C)** Gene expression of clone84, clone177, clone261.1, clone261.2, clone310 and clone460 in cultures of Caco-2 cells was analyzed by RT-PCR. A linear range of amplification was shown. **(D)** Analysis of HNF4α *in vitro* binding to further putative binding sites in promoters of CHIP clones. Electrophoretic mobility shift assays with 2,5 µg Caco-2 cell nuclear extract and oligonucleotides (GS65, lane 1 and 2; GS76, lane 3 and 4; GS82, lane 5 and 6; GS70, lane 7 and 8) corresponding to putative HNF4α binding-sites within the promoters of CHIP clones as ³²P labeled probe. In supershift assays an antibody directed against HNF4α (+) was added (lane 2, 4, 6, 8).

### Fig 8. Biological functions of HNF4α gene targets.

76 well-known HNF4α gene targets are assigned to functions in percent. The 13 novel HNF4α gene targets described in this study are assigned to functions with their gene names.

## Claims

1. Use of one or more genes selected from the group of C20orf13, KIAA0774, EPS15R, PLCB1, UGTREL1, RSK4, PAK5, FMR2, NEB, NFYC, KCNQ4, PRPF3, TRPC1, and/or their mutants and/or variations and/or parts thereof to screen for and to identify drugs against metabolic diseases, including type 1 and/or type 2 diabetes mellitus, and/or diabetes-caused diseases, including diabetic nephropathy, hearing dysfunction, diabetic neuropathy, cardiovascular diseases or diseases of the retina, and/or tumor growth.

2. Use of one or more gene products being coded by genes selected from the group of C20orf13, KIAA0774, EPS15R, PLCB1, UGTREL1, RSK4, PAK5, FMR2, NEB, NFYC, KCNQ4, PRPF3, TRPC1, and/or their mutants and/or variations and/or parts thereof to screen for and to identify drugs against metabolic diseases, including type 1 and/or type 2 diabetes mellitus, and/or diabetes-caused diseases, including diabetic nephropathy, hearing dysfunction, diabetic neuropathy, cardiovascular diseases or diseases of the retina, and/or tumor growth.

3. Use of the gene TRPC1 and/or gene products being coded by the gene TRPC1, and/or its mutants and/or variations and/or parts thereof to screen for and to identify drugs against type 1 and/or type 2 diabetes mellitus and/or diabetic nephropathy.

4. Use of the gene KCNQ4 and/or gene products being coded by the gene KCNQ4 and/or its mutants and/or variations and/or parts thereof to screen for and to identify drugs against hearing dysfunction.

5. Use of the gene EPS15R and/or gene products being coded by the gene EPS15R and/or its mutants and/or variations and/or parts thereof to screen for and to identify drugs against tumor growth.

6. Use of the gene PLCB1 and/or gene products being coded by the gene PLCB1 and/or its mutants and/or variations and/or parts thereof to screen for and to identify drugs against tumor growth.

7. Use of the gene C20orf13 and/or gene products being coded by the gene C20orf13 and/or its mutants and/or variations and/or parts thereof to screen for and to identify drugs against tumor growth.

8. Use of the gene UGTREL1 and/or gene products being coded by the gene UGTREL1 and/or its mutants and/or variations and/or parts thereof to screen for and to identify drugs against tumor growth.

9. Use as claimed in claims 1-2 and claim 5, wherein the function of the gene EPS15R and/or gene products being coded by the gene EPS15R and/or its mutants and/or variations and/or parts thereof
- in binding to EGF-receptor and/or
- in endocytosis and proteosomal degradation of EGF-receptor and/or
- in inhibition of EGF-receptor and/or
- in EGF-receptor mediated signal transduction and/or
- in regulation of tumor growth
is determined.

10. Use as claimed in claims 1-2 and claim 6, wherein the function of the gene PLCB1 and/or gene products being coded by the gene PLCB1 and/or its mutants and/or variations and/or parts thereof
- in activation through G-proteins and/or
- in stimulation of PKC and/or
- in nuclear activation through ERK1 and ERK2 and/or
- in regulation of differentiation and/or
- in regulation of proliferation and/or
- in regulation of cell cycle and/or
- in regulation of tumor growth
is determined.

11. Use as claimed in claims 1-2 and claim 7, wherein the function of the gene c20orf13 and/or gene products being coded by the gene c20orf13 and/or its mutants and/or variations and/or parts thereof
- in tapase function and/or
- in catalysing glycoprotein metabolism and/or
- in decrease of deamidation of asparagine and/or
- in regulation of tumor growth and/or
- in cellular differentiation and/or
- in regulation of HOX genes and/or
in organogenesis / development
is determined.

12. Use as claimed in claims 1-2 and claim 8, wherein the function of the gene UGTREL1 and/or gene products being coded by the gene UGTREL1 and/or its mutants and/or variations and/or parts thereof
- in glycoconjugate synthesis and/or
- in effects on cell adhesion and tumor growth
is determined.

13. Use as claimed in claims 1-2 , wherein the function of the gene RSK4 and/or gene products being coded by the gene RSK4 and/or its mutants and/or variations and/or parts thereof
- in mediating MAP/ERK signal transduction and/or
- in regulation of gene expression by phosphorylation of transcription factors (namely CREB, CBP/p300, ERα, IKBα/NFkB, c-Fos) and/or
- in regulation of cell cycle and/or
- in regulation of cell proliferation and/or
- in regulation of cell differentiation and/or
- in the treatment of nephro- and neuropathies
is determined.

14. Use as claimed in claims 1-2, wherein the function of the gene PAK5 and/or gene products being coded by the gene PAK5 and/or its mutants and/or variations and/or parts thereof
- in mediating effects of rho-proteins (such as cdc42 or p21-rac1) and/or
- in regulation of MAPK signaling pathways
- in regulation of cytoskeletal dynamics and/or
- in regulation of cell cycle and/or
- in regulation of cell proliferation and/or
- in regulation of cell differentiation and/or
- in the treatment of nephro- and neuropathies
is determined.

15. Use as claimed in claims 1-2, wherein the function of the gene FMR2 and/or gene products being coded by the gene FMR2 and/or its mutants and/or variations and/or parts thereof
- in transcriptional activation of genes and/or
- in regulation of cell differentiation of for instance neuronal cells
is determined.

16. Use as claimed in claims 1-2, wherein the function of the gene NEB and/or gene products being coded by the gene NEB and/or its mutants and/or variations and/or parts thereof
- in maintaining structural integrity of cardiac and skeletal muscle
is determined.

17. Use as claimed in claims 1-2, wherein the function of the gene NFYC and/or gene products being coded by the gene NFYC and/or its mutants and/or variations and/or parts thereof
- in transcriptional activation of genes and/or
- in regulation through cellular redox potential and/or
- in regulation based on redox response
is determined.

18. Use as claimed in claims 1-2 and claim 4, wherein the function of the gene KCNQ4 and/or gene products being coded by the gene KCNQ4 and/or its mutants and/or variations and/or parts thereof
- in hearing impairment and hearing lost including late stage complications of metabolic diseases and/or
- in generating potassium currents and membrane potentials in brain to transduce signals and/or
- in generating faultless potassium currents and membrane potentials in other organs, e.g. heart, to transduce signals
is determined.

19. Use as claimed in claims 1-2, wherein the function of the gene PRPF3 and/or gene products being coded by the gene PRPF3 and/or its mutants and/or variations and/or parts thereof
- in participating in pre-mRNA splicing as component of the spliceosome and/or
- as part of faultless gene expression and/or
- in treatment of disorders linked to RNA-spliceosome
is determined.

20. Use as claimed in claims 1-3, wherein the function of the gene TRPC1 and/or gene products being coded by the gene TRPC1 and/or its mutants and/or variations and/or parts thereof
- as part of heterodimers with other TRP proteins and/or
- in primary mode of Ca²⁺ entry after receptor activation or after store-dependent activation and/or
- in calcium homeostasis including kidney and pancreas and/or
- as non-selective cation channels in beta-cells and/or
- in insulin secretion by regulating pancreatic beta-cell plasma membrane potential in a K_{ATP} channel independent manner and/or
- in glucose-signalling and/or
- in the treatment of metabolic disorders including diabetes and nephropathy
is determined.

21. Use as claimed in claims 1-20, wherein drugs regulate the expression of one or more of said genes and/or the function of one or more of said gene products and/or their derived molecules and are used for the (production of means for) treatment of metabolic diseases, including type 1 and/or type 2 diabetes mellitus, and/or diabetes-caused diseases, including diabetic nephropathy, hearing dysfunction, diabetic neuropathy, cardiovascular diseases or diseases of the retina, and/or tumor growth.

22. Use as claimed in claims 1-21, wherein DNA and/or or related molecules encoding one or more of said gene products and/or derived structures are used.

23. Use as claimed in claims 1-21, wherein one or more polypeptides and/or derived molecules having the function of one or more of said gene products, are used.

24. Use of one or more genes selected from the group of C20orf13, KIAA0774, EPS15R, PLCB1, UGTREL1, RSK4, PAK5, FMR2, NEB, NFYC, KCNQ4, PRPF3, TRPC1, and/or their mutants and/or variations and/or parts thereof and/or their gene products and/or related molecules of said genes and/or derived molecules of said gene products for preparing a medicament for the treatment of metabolic diseases, including type 1 and/or type 2 diabetes mellitus, and/or diabetes-caused diseases, including diabetic nephropathy, hearing dysfunction, diabetic neuropathy, cardiovascular diseases or diseases of the retina, and/or tumor growth.

25. A method of identifying compounds directed against metabolic diseases, including type 1 and/or type 2 diabetes mellitus, and/or diabetes-caused diseases, including diabetic nephropathy, hearing dysfunction, diabetic neuropathy, cardiovascular diseases or diseases of the retina, and/or tumor growth, wherein one or more genes selected from the group of C20orf13, KIAA0774, EPS15R, PLCB1, UGTREL1, RSK4, PAK5, FMR2, NEB, NFYC, KCNQ4, PRPF3, TRPC1 and/or their mutants and/or variations and/or parts thereof and/or related molecules and/or their gene products and/or derived structures are incubated with a compound to be tested and changes in the expression of said genes and/or derived sequences and/or the function of said gene products and/or derived structures are determined.

26. A method for treating metabolic diseases, including type 1 and/or type 2 diabetes mellitus, and/or diabetes-caused diseases, including diabetic nephropathy, hearing dysfunction, diabetic neuropathy, cardiovascular diseases or diseases of the retina, and/or tumor growth in a subject, comprising administering to the subject, a therapeutically effective amount of a compound that has affinity to one or more gene sequences selected of the group of C20orf13, KIAA0774, EPS15R, PLCB1, UGTREL1, RSK4, PAK5, FMR2, NEB, NFYC, KCNQ4, PRPF3, TRPC1 and/or their mutants and/or variations and/or parts thereof and/or their regulatory elements and/or their mRNA and/or to one or more of their gene products and/or derived structures.

27. A method of claim 26, wherein the compound comprises a nucleic acid and/or a peptide chain and/or a related molecule influencing the expression of one or more genes selected of the group of C20orf13, KIAA0774, EPS15R, PLCB1, UGTREL1, RSK4, PAK5, FMR2, NEB, NFYC, KCNQ4, PRPF3, TRPC1 and/or their mutants and/or variations and/or parts thereof and/or their regulatory elements and/or their mRNA and/or the function of one or more of their gene products and/or derived structures.

28. A method for treating metabolic diseases, including type 1 and/or type 2 diabetes mellitus, and/or diabetes-caused diseases, including diabetic nephropathy, hearing dysfunction, diabetic neuropathy, cardiovascular diseases or diseases of the retina, and/or tumor growth in a subject, comprising administering to the subject, a therapeutically effective amount of a compound agonizing the function of one or more gene products encoded by genes selected from the group of C20orf13, KIAA0774, EPS15R, PLCB1, UGTREL1, RSK4, PAK5, FMR2, NEB, NFYC, KCNQ4, PRPF3, TRPC1.

29. A method as claimed in claims 26-28, wherein the compound comprises a nucleic acid encoding a gene product being coded by a gene selected from the group of C20orf13, KIAA0774, EPS15R, PLCB1, UGTREL1, RSK4, PAK5, FMR2, NEB, NFYC, KCNQ4, PRPF3, TRPC1 and/or their mutants and/or variations and/or parts thereof.

30. A method as claimed in claims 26-28, wherein the compound comprises a nucleic acid and/or a peptide chain and/or a related molecule.

31. A method of claim 30, wherein the compound additionally comprises an expression vector.

32. A method as claimed in claims 28 and 29, wherein the compound comprises a functional gene product.

33. A method for treating metabolic diseases, including type 1 and/or type 2 diabetes mellitus, and/or diabetes-caused diseases, including diabetic nephropathy, hearing dysfunction, diabetic neuropathy, cardiovascular diseases or diseases of the retina, and/or tumor growth in a subject, comprising administering to the subject, a therapeutically effective amount of a compound that decreases bioactivity and/or prevents expression of a mutant being coded by a mutated gene selected from the group of C20orf13, KIAA0774, EPS15R, PLCB1, UGTREL1, RSK4, PAK5, FMR2, NEB, NFYC, KCNQ4, PRPF3, TRPC1.

34. A method of claim 33, wherein the compound is selected from the group consisting of an anti sense molecule, ribozyme or triple helix molecule.

35. A method for treating metabolic diseases, including type 1 and/or type 2 diabetes mellitus, and/or diabetes-caused diseases, including diabetic nephropathy, hearing dysfunction, diabetic neuropathy, cardiovascular diseases or diseases of the retina, and/or tumor growth in a subject, comprising administering to the subject, a therapeutically effective amount of a compound that reduces the overexpression of a normal gene selected from the group of C20orf13, KIAA0774, EPS15R, PLCB1, UGTREL1, RSK4, PAK5, FMR2, NEB, NFYC, KCNQ4, PRPF3, TRPC1.

36. A method of claim 35, wherein the compound is selected from the group consisting of an anti sense molecule, ribozyme or triple helix molecule.

37. Substances which regulate the gene products being coded by one or more genes selected from the group of C20orf13, KIAA0774, EPS15R, PLCB1, UGTREL1, RSK4, PAK5, FMR2, NEB, NFYC, KCNQ4, PRPF3, TRPC1.

38. Substances according to claim 37 which regulate the gene products being coded by one or more genes selected from the group of C20orf13, KIAA0774, EPS15R, PLCB1, UGTREL1, RSK4, PAK5, FMR2, NEB, NFYC, KCNQ4, PRPF3, TRPC1 to a normal (functional) level.


SEQUENCE LISTING

<110> Fraunhofer-Gesellschaft zur Förderung der angewandten Forschung e.V.

<120> Use of novel HNF4alpha target genes and their gene products.

<130> Fra08/EP

<160> 39

<170> PatentIn version 3.1

<210> 1
<211> 1263
<212> DNA
<213> homo sapiens

<220>
<221> misc_feature
<223> C20orf13 gene sequence made up of non-contigous segments of gene
map locus 20p12.1

<400> 1
atgaccatgg agaaggggat gagttctgga gaagggctgc cttccagatc atctcaggtt 60
tcggctggta aaataacagc caaagagttg gaaacaaagc agtcctataa agagaaacga 120
ggaggctttg tgttggtgca tgcaggtgca ggttatcatt ctgaatccaa agccaaggag 180
tataaacatg tatgcaaacg agcttgtcag aaggcaattg aaaagctgca ggccggtgct 240
cttgcaactg acgcagtcac tgcagcactg gtggaacttg aggattctcc ttttacaaat 300
gcaggaatgg gatctaatct aaatctgtta ggtgaaattg agtgtgatgc cagcataatg 360
gatggaaaat ccttaaattt tggagcagtt ggagcactga gtggaatcaa gaacccggtc 420
tcggttgcca acagactctt atgtgaaggg cagaagggca agctctcggc tggcagaatt 480
cctccctgct ttttagttgg agaaggagcc tacagatggg cagtagatca tggaataccc 540
tcttgccctc ctaacatcat gaccacaaga ttcagtttag ctgcatttaa aagaaacaag 600
aggaaactag agctggcaga aagggtggac acagatttta tgcaactaaa gaaaagaaga 660
caatcaagtg agaaggaaaa tgactcaggc actttggaca cggtaggcgc tgtggttgtg 720

gaccacgaag ggaatgttgc tgctgctgtc tccagtggag gcttggcctt gaaacatccg 780
gggagagttg ggcaggctgc tctttatgga tgtggctgct gggctgaaaa tactggagct 840
cataacccct actccacagc tgtgagtacc tcaggatgtg gagagcatct tgtgcgcacc 900
atactggcta gagaatgttc acatgcttta caagctgagg atgctcacca agccctgttg 960
gagactatgc aaaacaagtt tatcagttca cctttccttg ccagtgaaga tggcgtgctt 1020
ggcggagtga ttgtcctccg ttcatgcaga tgttctgccg agcctgactt ctcccaaaat 1080
aagcagacac ttctagtgga atttctgtgg agccacacga cggagagcat gtgtgtcgga 1140
tatatgtcag cccaggatgg gaaagccaag actcacattt caagacttcc tcctggtgcg 1200
gtggcaggac agtctgtggc aatcgaaggt ggggtgtgcc gcctggagag cccagtgaac 1260
tga 1263

<210> 2
<211> 2347
<212> DNA
<213> homo sapiens

<220>
<221> misc_feature
<223> C20orf13 mRNA

<400> 2
gctgaagcgg ggtaattcct ctcctgcaat tacttttgga tggaagtatg cccctttctc 60
agtagaagat ggtaatcttg gagaatgacc atggagaagg ggatgagttc tggagaaggg 120
ctgccttcca gatcatctca ggtttcggct ggtaaaataa cagccaaaga gttggaaaca 180
aagcagtcct ataaagagaa acgaggaggc tttgtgttgg tgcatgcagg tgcaggttat 240
cattctgaat ccaaagccaa ggagtataaa catgtatgca aacgagcttg tcagaaggca 300
attgaaaagc tgcaggccgg tgctcttgca actgacgcag tcactgcagc actggtggaa 360
cttgaggatt ctccttttac aaatgcagga atgggatcta atctaaatct gttaggtgaa 420
attgagtgtg atgccagcat aatggatgga aaatccttaa attttggagc agttggagca 480
ctgagtggaa tcaagaaccc ggtctcggtt gccaacagac tcttatgtga agggcagaag 540
ggcaagctct cggctggcag aattcctccc tgctttttag ttggagaagg agcctacaga 600
tgggcagtag atcatggaat accctcttgc cctcctaaca tcatgaccac aagattcagt 660
ttagctgcat ttaaaagaaa caagaggaaa ctagagctgg cagaaagggt ggacacagat 720
tttatgcaac taaagaaaag aagacaatca agtgagaagg aaaatgactc aggcactttg 780
gacacggtag gcgctgtggt tgtggaccac gaagggaatg ttgctgctgc tgtctccagt 840

ggaggcttgg ccttgaaaca tccggggaga gttgggcagg ctgctcttta tggatgtggc 900
tgctgggctg aaaatactgg agctcataac ccctactcca cagctgtgag tacctcagga 960
tgtggagagc atcttgtgcg caccatactg gctagagaat gttcacatgc tttacaagct 1020
gaggatgctc accaagccct gttggagact atgcaaaaca agtttatcag ttcacctttc 1080
cttgccagtg aagatggcgt gcttggcgga gtgattgtcc tccgttcatg cagatgttct 1140
gccgagcctg acttctccca aaataagcag acacttctag tggaatttct gtggagccac 1200
acgacggaga gcatgtgtgt cggatatatg tcagcccagg atgggaaagc caagactcac 1260
atttcaagac ttcctcctgg tgcggtggca ggacagtctg tggcaatcga aggtggggtg 1320
tgccgcctgg agagcccagt gaactgaccc ttcaggctga gtgtgaagcg tctcagaggc 1380
atttcagaac ctgagctttt gggggttttt aactgaagtt ggttgtttta tctttcttgt 1440
tttataattc ctattgcaac ctcgtgcact gctcgagaca caagtgctgc tgtagttagc 1500
gcttagtgac acgcgggcct ttggtgggtg agcgggactg tgtgtgagtg tgtgcgcgta 1560
tgtgcgcaca tatgtgtatg tgtggagtat gtgtgtttgc ttctccgtgg atgaaataga 1620
aactcctcat tgtgtgacca ggaatggtta aatcatcttt acaaaatgtg tgctttaact 1680
gtttacaagt aaaacctaaa gttgcaggaa acatttttta tttcgtaaag aggtaccaac 1740
tgtcgctgat gtgatatgtc agaactgaag agtaaatcta cttgtttaaa tgacttgaca 1800
gtggtagtgc tccatttaat aacagtaata agtaataaag tgtttttatt tgttaaccag 1860
tttaagtgga tcctgtggta acttaaactg ttgttctcat cccttatatg ggcatttttt 1920
ctttaacaaa gaatggtttc agtgaaacaa tctagcagag aattaatgtc agaacctttt 1980
taaataatag tctgattgat acagtttgta cttatttcat caagcttttc taagcttaaa 2040
tattgcatag cttcgagctg tatggactat attatgaaag aatatgtaaa gagaacatac 2100
agtaatgcac agtccttaat ttgtgtataa tggaaagtta tttacaatat aacactgtaa 2160
ataagaaagc aaagtttatg ggaaaattca atattatctt tgtttttgtt taaatatatt 2220
tttaagataa aggcacaaaa ataaaagaag cgtattactg ggtatagtat gtgactcctc 2280
ttctcagact aataaattat cttttgaatc cttggaaaaa aaaaaaaaaa aaaaaaaaaa 2340
aaaaaaa 2347

<210> 3
<211> 420
<212> PRT
<213> homo sapiens

<220>
<221> misc_feature
<223> C20orf13 gene product

<400> 3

Met Thr Met Glu Lys Gly Met Ser Ser Gly Glu Gly Leu Pro Ser Arg
1               5                   10                  15

Ser Ser Gln Val Ser Ala Gly Lys Ile Thr Ala Lys Glu Leu Glu Thr
            20                  25                  30

Lys Gln Ser Tyr Lys Glu Lys Arg Gly Gly Phe Val Leu Val His Ala
        35                  40                  45

Gly Ala Gly Tyr His Ser Glu Ser Lys Ala Lys Glu Tyr Lys His Val
    50                  55                  60

Cys Lys Arg Ala Cys Gln Lys Ala Ile Glu Lys Leu Gln Ala Gly Ala
65                  70                  75                  80

Leu Ala Thr Asp Ala Val Thr Ala Ala Leu Val Glu Leu Glu Asp Ser
                85                  90                  95

Pro Phe Thr Asn Ala Gly Met Gly Ser Asn Leu Asn Leu Leu Gly Glu
            100                 105                 110

Ile Glu Cys Asp Ala Ser Ile Met Asp Gly Lys Ser Leu Asn Phe Gly
        115                 120                 125

Ala Val Gly Ala Leu Ser Gly Ile Lys Asn Pro Val Ser Val Ala Asn
    130                 135                 140

Arg Leu Leu Cys Glu Gly Gln Lys Gly Lys Leu Ser Ala Gly Arg Ile
145                 150                 155                 160

Pro Pro Cys Phe Leu Val Gly Glu Gly Ala Tyr Arg Trp Ala Val Asp
                165                 170                 175

His Gly Ile Pro Ser Cys Pro Pro Asn Ile Met Thr Thr Arg Phe Ser
            180                 185                 190

Leu Ala Ala Phe Lys Arg Asn Lys Arg Lys Leu Glu Leu Ala Glu Arg
        195                 200                 205

Val Asp Thr Asp Phe Met Gln Leu Lys Lys Arg Arg Gln Ser Ser Glu
    210                 215                 220

Lys Glu Asn Asp Ser Gly Thr Leu Asp Thr Val Gly Ala Val Val Val
225                 230                 235                 240

Asp His Glu Gly Asn Val Ala Ala Ala Val Ser Ser Gly Gly Leu Ala
                245                 250                 255

Leu Lys His Pro Gly Arg Val Gly Gln Ala Ala Leu Tyr Gly Cys Gly
            260                 265                 270

Cys Trp Ala Glu Asn Thr Gly Ala His Asn Pro Tyr Ser Thr Ala Val
        275                 280                 285

Ser Thr Ser Gly Cys Gly Glu His Leu Val Arg Thr Ile Leu Ala Arg
    290                 295                 300

Glu Cys Ser His Ala Leu Gln Ala Glu Asp Ala His Gln Ala Leu Leu
305                 310                 315                 320

Glu Thr Met Gln Asn Lys Phe Ile Ser Ser Pro Phe Leu Ala Ser Glu
                325                 330                 335

Asp Gly Val Leu Gly Gly Val Ile Val Leu Arg Ser Cys Arg Cys Ser
            340                 345                 350

Ala Glu Pro Asp Ser Ser Gln Asn Lys Gln Thr Leu Leu Val Glu Phe
        355                 360                 365

Leu Trp Ser His Thr Thr Glu Ser Met Cys Val Gly Tyr Met Ser Ala
    370                 375                 380

Gln Asp Gly Lys Ala Lys Thr His Ile Ser Arg Leu Pro Pro Gly Ala
385                 390                 395                 400

Val Ala Gly Gln Ser Val Ala Ile Glu Gly Gly Val Cys Arg Leu Glu
                405                 410                 415

Ser Pro Val Asn
            420

<210>  4

<211>  1047

<212>  DNA

<213>  homo sapiens


<220>

<221>  misc_feature

<223>  KIAA0774 gene sequence made up of non-contigous segments of gene
       map locus 13q12.3


<400>  4
atgggccatt gctgctgcaa gccttataac tgccttcagt gcctggacaa gacgaatgaa      60

agtgcccttg tgaaagaaaa agagctgtca atcgaacttg caaacatcag ggatgaagtt     120

gccttccata cagcaaagtg cgagaaacta caaaaggaga aggaggagct ggagaggcgg 180
ttcgaggacg aggtgaagag gctgggctgg cagcagcagg ccgagctcca ggagctggag 240
gagcggctgc agctgcaatt cgaggcggaa atggcgcgcc tgcaggagga gcacggtgac 300
cagctgctga gcatccggtg tcaacaccag gagcaggtgg aagatctcac cgccagccat 360
gatgctgctc tcctagagat ggaaaataac cacacagttg ccatcacaat cctgcaggat 420
gaccacgacc acaaagtcca agaattgatg tccactcatg agcttgaaaa gaaagaattg 480
gaagaaaatt ttgaaaaact gcggctgtca ttgcaggacc aggtggacac gctgaccttc 540
cagagccagt ctctgcggga cagagcccgc cgcttcgaag aggccttgag gaagaacaca 600
gaggagcagc tggagattgc attggctcct tatcagcact tggaagaaga catgaagagt 660
ctgaagcagg tattagaaat gaagaatcag caaatacacg agcaagaaaa gaagattctt 720
gagctggaaa agctggcaga aaagaacatt atcctagaag aaaagatcca ggttctccaa 780
cagcagaacg aagacctcaa agcaaggatt gaccaaaaca cagttgtcac cagacagctg 840
tcggaggaaa atgctaacct ccaggaatat gttgagaagg aaacccagga gaagaagaga 900
ttgagccgaa ccaatgaaga gctgctttgg aagctccaaa ctggggaccc gaccagtccg 960
attaaactct cgcccacatc tcccgtttac cgcggctcct cctcggggcc ctcctctccg 1020
gccagagtca gcacaacacc cagatga 1047

<210> 5
<211> 4636
<212> DNA
<213> homo sapiens

<220>
<221> misc_feature
<223> KIAA0774 mRNA

<400> 5
atgagcgtcc cagtggctcc taagaaatca tgttacactc agttgcggga caacagaaat 60
gcagcaagaa ataataatga aagcatctta agtctgggag atacgaatgc caatcaaatc 120
atgttggagg tcagctcctc tcatgacgag tccaagacat gtgacctggg agatgaaatt 180
ggaaatacaa attcaagtga gccagaaaac cgtacccatt tccataagga atttcaccaa 240
cttcagggct ttgggaaagg ctctcaggct ggctctgcca gcctgaaaga ttttagactt 300
tcttcaacca ttcagaggga actcaatgaa gagcacacag tggagagagg cacagatagc 360
ctgcagacca cgcggagtat tcagggacca agtctgtcga gttggaggaa tgtgatgagt 420
gaggccagtc tagacgtttt ggctaaaagg gatgctgaaa ttccccggca tgttcccaag 480

gataaactgg caaagaccct tgacaatgag gaactgagga ggcattcttt ggaaagagca 540
agcagctctg tagctgcagt cgggagcctg actccgcagc atccacagcc tctatccctc 600
gactcccggg aagcacgggg tcagatacct gggggtgggg aggggccaca gaagacattg 660
ccagaccacg ctgtcccggc agctttccct gcaactgaca gtacctcaga gggaaagagt 720
gtgcgtcatc ctaaaccatc tacctcagaa agcaagcaga gcactccctc agagacccaa 780
acagtggggg cacatgtact gcaggtgtgc agtgagcaca catcacattc cgcccatcca 840
gagcctgctc tgaatttgac tttggcatcg aaggaaatcc caagtaaact ggaagcacaa 900
ttaggtcagg gaaagggaga ggccaagctg gatctgaaat atgttcctcc caggagagtt 960
gaacaggagg gaaaggcagc ccaggaaggg tatctgggat gccacaagga agagaatctg 1020
tcagccttgg agggaaggga tccatgtggg gaagcacacc cggaagccac cgatgcactt 1080
ggccatctgc tgaacagtga cctccaccac cttggggtgg gaagaggcaa ctgtgaagag 1140
aagagaggag tcaacccagg ggagcaggat tctctccaca ccacccccaa acagggctct 1200
gcttccttag gaggggctga taatcagccc actggcaaaa tttcaccatg tgcaggtgag 1260
aagttgggtg aaaggacatc cagcagcttt tcaccaggtg acagtcatgt ggcttttatt 1320
cctaataatc tgactgacag caagcccttg gatgtcattg aggaggaaag gcggttgggc 1380
agtgggaata aggacagtgt tatggttttg gtgttcaatc cttctgttgg agagaacaag 1440
acggaggtgc ctgagcccct ggaccctcaa agtggccgct cagaagcacg ggaaagcaaa 1500
gaggtcacca catctgttgc tgaaaacagg aaccttctag agaatgcaga taagattgaa 1560
agcacctcag caagagcaga ttcagttctc aatattccag cacccctcca cccagagaca 1620
actgtgaaca tgacctacca gcctacaaca cccagtagca gttttcagga tgttagcgtg 1680
ttcggtatgg atgcggggtc ccccttggta gttccacccc ctactgatag tgcacgcttg 1740
ttgaacacgt cccccaaagt gcctgacaag aacacttgcc ccagtgggat ccccaagcct 1800
gtcttcacac attccaagga cacaccttcc tcgcaggagg gaatggagaa ctatcaggtt 1860
gaaaaaacag aggagaggac agaaactaag cccatcatta tgcccaagcc caagcatgtg 1920
aggcccaaga tcatcaccta catcaggagg aatccccagg ccctgggcca ggtggacgcc 1980
tcgctggttc cagtggggct tccatatgcc ccgcccacat gtaccatgcc tcttccccac 2040
gaagagaagg cagcaggtgg tgacctgaag ccatctgcca acctctatga gaaattcaag 2100
ccagacctgc agaagccaag ggtcttcagt tccggattga tggtgtctgg aatcaagccc 2160
ccgggacatc ctttcagtca aatgagtgaa aagtttttgc aggaggttac agaccaccct 2220
ggaaaagaag agttttgttc tcctccctat gctcattatg aagtccctcc aactttctat 2280
cggtcagcca tgctccttaa gccccagcta ggattgggtg caatgtcccg tttaccatct 2340
gcaaagagca ggattctgat tgcaagtcag aggtcttcag cgagcgccat ccacccacca 2400
ggacccataa caacagccac cagtctctac agttccgatc cttcagattt aaagaaagct 2460
tccagttcaa atgctgcaaa atccaatctc ccgaaatctg gtctccgtcc tcccggatac 2520

tcacgtctcc cggcagccaa actggcggca tttggctttg tccggagctc cagcgtctcc 2580
tcagtctcca gcacccagtc cggggacagt gcacagccag agcagggccg gccagccacc 2640
cgttcaacct ttgggaatga agaacagcca gttctgaagg catctctgcc ttctaaggac 2700
acacccaagg gggccggccg ggtggcccct ccagcatcct ccagtgtgac agcaccccgc 2760
aggagtttac ttccagcgcc aaaatccact tccacacccg ctggaacaaa gaaagatgct 2820
cagaaagatc aagatacgaa taaacctgct gtttcatctc ctaagagagt agcagcttca 2880
accaccaagc ttcattcacc aggataccca aagcagagga ctgcggcagc tcgaaatggg 2940
tttccgccca agccggaccc gcaggcccgt gaggctgagc ggcagctggt gctgcggctg 3000
aaggagcggt gtgagcagca gaccagacag ctgggcgttg cgcaagggga gctgaagagg 3060
gccatctgcg gctttgatgc cctcgccgtg gccacgcagc atttctttag aaagaatgaa 3120
agtgcccttg tgaaagaaaa agagctgtca atcgaacttg caaacatcag ggatgaagtt 3180
gccttccata cagcaaagtg cgagaaacta caaaaggaga aggaggagct ggagaggcgg 3240
ttcgaggacg aggtgaagag gctgggctgg cagcagcagg ccgagctcca ggagctggag 3300
gagcggctgc agctgcaatt cgaggcggaa atggcgcgcc tgcaggagga gcacggtgac 3360
cagctgctga gcatccggtg tcaacaccag gagcaggtgg aagatctcac cgccagccat 3420
gatgctgctc tcctagagat ggaaaataac cacacagttg ccatcacaat cctgcaggat 3480
gaccacgacc acaaagtcca agaattgatg tccactcatg agcttgaaaa gaaagaattg 3540
gaagaaaatt ttgaaaaact gcggctgtca ttgcaggacc aggtggacac gctgaccttc 3600
cagagccagt ctctgcggga cagagcccgc cgcttcgaag aggccttgag gaagaacaca 3660
gaggagcagc tggagattgc attggctcct tatcagcact tggaagaaga catgaagagt 3720
ctgaagcagg tattagaaat gaagaatcag caaatacacg agcaagaaaa gaagattctt 3780
gagctggaaa agctggcaga aaagaacatt atcctagaag aaaagatcca ggttctccaa 3840
cagcagaacg aagacctcaa agcaaggatt gaccaaaaca cagttgtcac cagacagctg 3900
tcggaggaaa atgctaacct ccaggaatat gttgagaagg aaacccagga gaagaagaga 3960
ttgagccgaa ccaatgaaga gctgctttgg aagctccaaa ctggggaccc gaccagtccg 4020
attaaactct cgcccacatc tcccgtttac cgcggctcct cctcggggcc ctcctctccg 4080
gccagagtca gcacaacacc cagatgacgc cactacacgg cctgcgggag ctccggcttc 4140
tcgtcctccg gtctccaccc tgagggagca ccgacccggt gccgccggag ctggccctgt 4200
gcgcatgctc agtagctgcg aatgcatcct aggcgcgtcc tcctctgatc cccgtgtaag 4260
actgccctgg tgtcggcact taggaatgtg taaatggtaa agtctgatgt gcaaacgttt 4320
taccatagtt agagccaaaa gaaagacact tgcaattgtt cttgagcaat gaactttcac 4380
tgcagaattt caggttagtt acaaaaagct cagttttcaa tatacattga ataatcattg 4440
tgtactgcac cgatatgtgt gtatatttag atatacgtat atacacatgc tgcggttctg 4500
aatttcattt tttataacat gaagtgctga catattttag tgaaggtcag cagttttcta 4560

acttgtgcct aagaattatt gggaaatgaa aatgcatttc tatctagctt cccaggaata 4620
tttctaccca aaatag 4636

<210> 6
<211> 1381
<212> PRT
<213> homo sapiens

<220>
<221> misc_feature
<223> KIAA0774 gene product

<400> 6
Asp Cys Met Ala Ser Ser Pro Thr Lys Gly Leu Thr Met Ser Val Pro
1               5                   10                  15

Val Ala Pro Lys Lys Ser Cys Tyr Thr Gln Leu Arg Asp Asn Arg Asn
            20                  25                  30

Ala Ala Arg Asn Asn Asn Glu Ser Ile Leu Ser Leu Gly Asp Thr Asn
        35                  40                  45

Ala Asn Gln Ile Met Leu Glu Val Ser Ser Ser His Asp Glu Ser Lys
    50                  55                  60

Thr Cys Asp Leu Gly Asp Glu Ile Gly Asn Thr Asn Ser Ser Glu Pro
65                  70                  75                  80

Glu Asn Arg Thr His Phe His Lys Glu Phe His Gln Leu Gln Gly Phe
                85                  90                  95

Gly Lys Gly Ser Gln Ala Gly Ser Ala Ser Leu Lys Asp Phe Arg Leu
            100                 105                 110

Ser Ser Thr Ile Gln Arg Glu Leu Asn Glu Glu His Thr Val Glu Arg
        115                 120                 125

Gly Thr Asp Ser Leu Gln Thr Thr Arg Ser Ile Gln Gly Pro Ser Leu
    130                 135                 140

Ser Ser Trp Arg Asn Val Met Ser Glu Ala Ser Leu Asp Val Leu Ala
145                 150                 155                 160

Lys Arg Asp Ala Glu Ile Pro Arg His Val Pro Lys Asp Lys Leu Ala
                165                 170                 175

Lys Thr Leu Asp Asn Glu Glu Leu Arg Arg His Ser Leu Glu Arg Ala
            180                 185                 190

Ser Ser Ser Val Ala Ala Val Gly Ser Leu Thr Pro Gln His Pro Gln
        195                 200                 205

Pro Leu Ser Leu Asp Ser Arg Glu Ala Arg Gly Gln Ile Pro Gly Gly
    210                 215                 220

Gly Glu Gly Pro Gln Lys Thr Leu Pro Asp His Ala Val Pro Ala Ala
225                 230                 235                 240

Phe Pro Ala Thr Asp Ser Thr Ser Glu Gly Lys Ser Val Arg His Pro
                245                 250                 255

Lys Pro Ser Thr Ser Glu Ser Lys Gln Ser Thr Pro Ser Glu Thr Gln
            260                 265                 270

Thr Val Gly Ala His Val Leu Gln Val Cys Ser Glu His Thr Ser His
        275                 280                 285

Ser Ala His Pro Glu Pro Ala Leu Asn Leu Thr Leu Ala Ser Lys Glu
    290                 295                 300

Ile Pro Ser Lys Leu Glu Ala Gln Leu Gly Gln Gly Lys Gly Glu Ala
305                 310                 315                 320

Lys Leu Asp Leu Lys Tyr Val Pro Pro Arg Arg Val Glu Gln Glu Gly
                325                 330                 335

Lys Ala Ala Gln Glu Gly Tyr Leu Gly Cys His Lys Glu Glu Asn Leu
            340                 345                 350

Ser Ala Leu Glu Gly Arg Asp Pro Cys Gly Glu Ala His Pro Glu Ala
        355                 360                 365

Thr Asp Ala Leu Gly His Leu Leu Asn Ser Asp Leu His His Leu Gly
    370                 375                 380

Val Gly Arg Gly Asn Cys Glu Glu Lys Arg Gly Val Asn Pro Gly Glu
385                 390                 395                 400

Gln Asp Ser Leu His Thr Thr Pro Lys Gln Gly Ser Ala Ser Leu Gly
                405                 410                 415

Gly Ala Asp Asn Gln Pro Thr Gly Lys Ile Ser Pro Cys Ala Gly Glu
            420                 425                 430

Lys Leu Gly Glu Arg Thr Ser Ser Ser Phe Ser Pro Gly Asp Ser His
        435                 440                 445

Val Ala Phe Ile Pro Asn Asn Leu Thr Asp Ser Lys Pro Leu Asp Val
    450                 455                 460

Ile Glu Glu Glu Arg Arg Leu Gly Ser Gly Asn Lys Asp Ser Val Met
465                 470                 475                 480

Val Leu Val Phe Asn Pro Ser Val Gly Glu Asn Lys Thr Glu Val Pro
                485                 490                 495

Glu Pro Leu Asp Pro Gln Ser Gly Arg Ser Glu Ala Arg Glu Ser Lys
            500                 505                 510

Glu Val Thr Thr Ser Val Ala Glu Asn Arg Asn Leu Leu Glu Asn Ala
        515                 520                 525

Asp Lys Ile Glu Ser Thr Ser Ala Arg Ala Asp Ser Val Leu Asn Ile
    530                 535                 540

Pro Ala Pro Leu His Pro Glu Thr Thr Val Asn Met Thr Tyr Gln Pro
545                 550                 555                 560

Thr Thr Pro Ser Ser Ser Phe Gln Asp Val Ser Val Phe Gly Met Asp
                565                 570                 575

Ala Gly Ser Pro Leu Val Val Pro Pro Pro Thr Asp Ser Ala Arg Leu
            580                 585                 590

Leu Asn Thr Ser Pro Lys Val Pro Asp Lys Asn Thr Cys Pro Ser Gly
        595                 600                 605

Ile Pro Lys Pro Val Phe Thr His Ser Lys Asp Thr Pro Ser Ser Gln
    610                 615                 620

Glu Gly Met Glu Asn Tyr Gln Val Glu Lys Thr Glu Glu Arg Thr Glu
625                 630                 635                 640

Thr Lys Pro Ile Ile Met Pro Lys Pro Lys His Val Arg Pro Lys Ile
                645                 650                 655

Ile Thr Tyr Ile Arg Arg Asn Pro Gln Ala Leu Gly Gln Val Asp Ala
            660                 665                 670

Ser Leu Val Pro Val Gly Leu Pro Tyr Ala Pro Pro Thr Cys Thr Met
        675                 680                 685

Pro Leu Pro His Glu Glu Lys Ala Ala Gly Gly Asp Leu Lys Pro Ser
    690                 695                 700

Ala Asn Leu Tyr Glu Lys Phe Lys Pro Asp Leu Gln Lys Pro Arg Val
705                 710                 715                 720

Phe Ser Ser Gly Leu Met Val Ser Gly Ile Lys Pro Pro Gly His Pro
                725                 730                 735

Phe Ser Gln Met Ser Glu Lys Phe Leu Gln Glu Val Thr Asp His Pro
            740                 745                 750

Gly Lys Glu Glu Phe Cys Ser Pro Pro Tyr Ala His Tyr Glu Val Pro
        755                 760                 765

Pro Thr Phe Tyr Arg Ser Ala Met Leu Leu Lys Pro Gln Leu Gly Leu
    770                 775                 780

Gly Ala Met Ser Arg Leu Pro Ser Ala Lys Ser Arg Ile Leu Ile Ala
785                 790                 795                 800

Ser Gln Arg Ser Ser Ala Ser Ala Ile His Pro Pro Gly Pro Ile Thr
                805                 810                 815

Thr Ala Thr Ser Leu Tyr Ser Ser Asp Pro Ser Ala Asp Leu Lys Lys
            820                 825                 830

Ala Ser Ser Ser Asn Ala Ala Lys Ser Asn Leu Pro Lys Ser Gly Leu
        835                 840                 845

Arg Pro Pro Gly Tyr Ser Arg Leu Pro Ala Ala Lys Leu Ala Ala Phe
    850                 855                 860

Gly Phe Val Arg Ser Ser Ser Val Ser Ser Val Ser Ser Thr Gln Ser
865                 870                 875                 880

Gly Asp Ser Ala Gln Pro Glu Gln Gly Arg Pro Ala Thr Arg Ser Thr
                885                 890                 895

Phe Gly Asn Glu Glu Gln Pro Val Leu Lys Ala Ser Leu Pro Ser Lys
            900                 905                 910

Asp Thr Pro Lys Gly Ala Gly Arg Val Ala Pro Pro Ala Ser Ser Ser
        915                 920                 925

Val Thr Ala Pro Arg Arg Ser Leu Leu Pro Ala Pro Lys Ser Thr Ser
    930                 935                 940

Thr Pro Ala Gly Thr Lys Lys Asp Ala Pro Lys Asp Gln Asp Thr Asn
945                 950                 955                 960

Lys Pro Ala Val Ser Ser Pro Lys Arg Val Ala Ala Ser Thr Thr Lys
                965                 970                 975

Leu His Ser Pro Gly Tyr Pro Lys Gln Arg Thr Ala Ala Ala Arg Asn
            980                 985                 990

Gly Phe Pro Pro Lys Pro Asp Pro Gln Ala Arg Glu Ala Glu Arg Gln
        995                 1000                1005

Leu Val Leu Arg Leu Lys Glu Arg Cys Glu Gln Gln Thr Arg Gln
    1010                1015                1020

Leu Gly Val Ala Gln Gly Glu Leu Lys Arg Ala Ile Cys Gly Phe
    1025                1030                1035

Asp Ala Leu Ala Val Ala Thr Gln His Phe Phe Arg Lys Asn Glu
    1040                1045                1050

Ser Ala Leu Val Lys Glu Lys Glu Leu Ser Ile Glu Leu Ala Asn
    1055                1060                1065

Ile Arg Asp Glu Val Ala Phe His Thr Ala Lys Cys Glu Lys Leu
    1070                1075                1080

Gln Lys Glu Lys Glu Glu Leu Glu Arg Arg Phe Glu Asp Glu Val
    1085                1090                1095

Lys Arg Leu Gly Trp Gln Gln Gln Ala Glu Leu Gln Glu Leu Glu
    1100                1105                1110

Glu Arg Leu Gln Leu Gln Phe Glu Ala Glu Met Ala Arg Leu Gln
    1115                1120                1125

Glu Glu His Gly Asp Gln Leu Leu Ser Ile Arg Cys Gln His Gln
    1130                1135                1140

Glu Gln Val Glu Asp Leu Thr Ala Ser His Asp Ala Ala Leu Leu
    1145                1150                1155

Glu Met Glu Asn Asn His Thr Val Ala Ile Thr Ile Leu Gln Asp
    1160                1165                1170

Asp His Asp His Lys Val Gln Glu Leu Met Ser Thr His Glu Leu
    1175                1180                1185

Glu Lys Lys Glu Leu Glu Glu Asn Phe Glu Lys Leu Arg Leu Ser
    1190                1195                1200

Leu Gln Asp Gln Val Asp Thr Leu Thr Phe Gln Ser Gln Ser Leu
    1205                1210                1215

Arg Asp Arg Ala Arg Arg Phe Glu Glu Ala Leu Arg Lys Asn Thr
    1220                1225                1230

Glu Glu Gln Leu Glu Ile Ala Leu Ala Pro Tyr Gln His Leu Glu
    1235                1240                1245

Glu Asp Met Lys Ser Leu Lys Gln Val Leu Glu Met Lys Asn Gln
    1250                1255                1260

Gln Ile His Glu Gln Glu Lys Lys Ile Leu Glu Leu Glu Lys Leu
    1265                1270                1275

Ala Glu Lys Asn Ile Ile Leu Glu Glu Lys Ile Gln Val Leu Gln
    1280                1285                1290

Gln Gln Asn Glu Asp Leu Lys Ala Arg Ile Asp Gln Asn Thr Val
    1295                1300                1305

Val Thr Arg Gln Leu Ser Glu Glu Asn Ala Asn Leu Gln Glu Tyr
    1310                1315                1320

Val Glu Lys Glu Thr Gln Glu Lys Lys Arg Leu Ser Arg Thr Asn
    1325                1330                1335

Glu Glu Leu Leu Trp Lys Leu Gln Thr Gly Asp Pro Thr Ser Pro
    1340                1345                1350

Ile Lys Leu Ser Pro Thr Ser Pro Val Tyr Arg Gly Ser Ser Ser
    1355                1360                1365

Gly Pro Ser Ser Pro Ala Arg Val Ser Thr Thr Pro Arg
    1370                1375                1380

<210>  7

<211>  2595

<212>  DNA

<213>  homo sapiens

<220>

<221>  misc_feature

<223>  EPS15R gene sequence made up of non-contigous segments of gene ma
       p locus 19p13.11

<400>  7
atggcggcgc cgctcatccc cctctcccag cagattccca ctggaaattc gttgtatgaa      60

tcttattaca agcaggtcga tccggcatac acagggaggg tgggggcgag tgaagctgcg     120

ctttttctaa agaagtctgg cctctcggac attatccttg ggaagatatg ggacttggcc     180

gatccagaag gtaaagggtt cttggacaaa cagggtttct atgttgcact gagactggtg     240

gcctgtgcac agagtggcca tgaagttacc ttgagcaatc tgaatttgag catgccaccg     300

cctaaatttc acgacaccag cagccctctg atggtcacac cgccctctgc agaggcccac 360
tgggctgtga gggtggaaga aaaggccaaa tttgatggga tttttgaaag cctcttgccc 420
atcaatggtt tgctctctgg agacaaagtc aagccagtcc tcatgaactc aaagctgcct 480
cttgatgtcc tgggcagggt ctgggacctc agtgacattg acaaggatgg gcacttggat 540
cgagatgagt tcgctgtggc catgcacttg gtgtaccgag ccctggagaa ggagcccgtg 600
ccctccgccc tgcccccgtc cctcatccca ccctccaaga gaaagaagac tgtgttccct 660
ggcgccgtcc ccgtcctgcc tgccagcccc ccaccaaaag acagcctccg ctccacgccg 720
tcccacggca gcgtcagcag cctcaacagc acagggagcc tgtcccccaa gcacagcctc 780
aagcaaacac agccaacagt gaactgggtg gtgcccgtgg cagacaagat gcgatttgat 840
gagatattcc tgaagaccga cctggacctg gatggctacg tgagtggcca ggaggtgaag 900
gagatcttca tgcactcggg cctcacccag aaccttctag cacacatatg ggccctggcc 960
gatacgaggc aaacggggaa gttaagcaaa gaccaattcg cgttagctat gtatttcatt 1020
cagcagaagg tcagtaaagg catcgaccct cctcaagtcc tctcgccgga catggtcccg 1080
ccttcggaga gaggcacgcc cggcccggac agttcaggct ctctcggctc cggggagttt 1140
actggcgtga aggagcttga tgacatcagt caagagattg cccagttaca aagagagaaa 1200
tattcactgg aacaagacat tcgagaaaag gaagaggcaa tcagacagaa aaccagcgag 1260
gtgcaggaat tacaaaatga cctagaccgg gaaacaagca gtttgcagga gctcgaggct 1320
cagaaacagg atgctcaaga ccgcctggac gagatggacc agcagaaggc caagctccga 1380
gacatgctga gcgacgtccg gcagaagtgc caggatgaga ctcagatgat ctcatcactg 1440
aaaacgcaaa tccaatctca ggaatctgac ttaaagtccc aggaagacga tctgaaccga 1500
gccaagtcgg agctgaaccg attgcagcag gaggaaaccc agctggagca gagcattcag 1560
gctgggcgag tccagctgga aaccatcatc aagtccctga agtcaacgca agacgaaatc 1620
aaccaggcaa ggagcaaact ttcccagctg catgaaagcc gccaggaggc ccacaggagc 1680
ctggagcagt atgaccaggt gctcgatgga gcccatggtg ccagcctgac cgacctggcc 1740
aacctgagcg aaggcgtctc cctggcagag aggggcagtt ttggagccat ggatgatcct 1800
ttcaaaaata aagccttgtt atttagcaac aacacgcaag agttgcatcc ggatcctttc 1860
cagacagaag accccttcaa atctgaccca tttaaaggag ctgacccctt caaaggcgac 1920
ccgttccaga atgacccctt tgcagaacag cagacaactt caacagatcc atttggaggg 1980
gaccctttca aagaaagtga cccattccgt ggctctgcca ctgacgactt cttcaagaaa 2040
cagacaaaga atgacccatt tacctcggat ccattcacga aaaacccttc cttaccttcg 2100
aagctcgacc cctttgaatc cagtgatccc ttttcatcct ccagtgtctc ctcaaaagga 2160
tcagatccct ttggaacctt agatcccttc ggaagtgggt ccttcaatag tgctgaaggc 2220
tttgccgact tcagccagat gtccaagccc ccaccttctg ggcctttcac ctcctccttg 2280
ggaggggcag gattctcaga tgaccccttt aaaagtaaac aggacactcc tgctctgcct 2340

ccgaagaaac ctgctcctcc acggcctaaa ccgcccagcg gtaaaagtac acctgtaagc 2400
cagcttggtt ccgcagactt tcccgaggcc cccgatccat tccagccact cggggctgac 2460
agcggcgacc cgttccaaag taaaaagggg tttggggacc cgtttagtgg aaaagaccca 2520
tttgtcccct cctctgcagc taaaccttct aaggcctctg cctcgggctt tgcagacttc 2580
acctctgtaa gttga 2595

<210> 8
<211> 2774
<212> DNA
<213> homo sapiens

<220>
<221> misc_feature
<223> EPS15R mRNA

<400> 8
gggaagatgg cggcgccgct catccccctc tcccagcaga ttcccactgg aaattcgttg 60
tatgaatctt attacaagca ggtcgatccg gcatacacag ggagggtggg ggcgagtgaa 120
gctgcgcttt ttctaaagaa gtctggcctc tcggacatta tccttgggaa gatatgggac 180
ttggccgatc cagaaggtaa agggttcttg gacaaacagg gtttctatgt tgcactgaga 240
ctggtggcct gtgcacagag tggccatgaa gttaccttga gcaatctgaa tttgagcatg 300
ccaccgccta aatttcacga caccagcagc cctctgatgg tcacaccgcc ctctgcagag 360
gcccactggg ctgtgagggt ggaagaaaag gccaaatttg atgggatttt tgaaagcctc 420
ttgcccatca atggtttgct ctctggagac aaagtcaagc cagtcctcat gaactcaaag 480
ctgcctcttg atgtcctggg cagggtctgg gacctcagtg acattgacaa ggatgggcac 540
ttggatcgag atgagttcgc tgtggccatg cacttggtgt accgagccct ggagaaggag 600
cccgtgccct ccgccctgcc cccgtccctc atcccaccct ccaagagaaa gaagactgtg 660
ttccctggcg ccgtccccgt cctgcctgcc agccccccac caaaagacag cctccgctcc 720
acgccgtccc acggcagcgt cagcagcctc aacagcacag ggagcctgtc ccccaagcac 780
agcctcaagc aaacacagcc aacagtgaac tgggtggtgc ccgtggcaga caagatgcga 840
tttgatgaga tattcctgaa gaccgacctg gacctggatg gctacgtgag tggccaggag 900
gtgaaggaga tcttcatgca ctcgggcctc acccagaacc ttctagcaca catatgggcc 960
ctggccgata cgaggcaaac ggggaagtta agcaaagacc aattcgcgtt agctatgtat 1020
ttcattcagc agaaggtcag taaaggcatc gaccctcctc aagtcctctc gccggacatg 1080
gtcccgcctt cggagagagg cacgcccggc ccggacagtt caggctctct cggctccggg 1140

gagtttactg gcgtgaagga gcttgatgac atcagtcaag agattgccca gttacaaaga   1200
gagaaatatt cactggaaca agacattcga gaaaaggaag aggcaatcag acagaaaacc   1260
agcgaggtgc aggaattaca aaatgaccta gaccgggaaa caagcagttt gcaggagctc   1320
gaggctcaga aacaggatgc tcaagaccgc ctggacgaga tggaccagca gaaggccaag   1380
ctccgagaca tgctgagcga cgtccggcag aagtgccagg atgagactca gatgatctca   1440
tcactgaaaa cgcaaatcca atctcaggaa tctgacttaa agtcccagga agacgatctg   1500
aaccgagcca agtcggagct gaaccgattg cagcaggagg aaacccagct ggagcagagc   1560
attcaggctg ggcgagtcca gctggaaacc atcatcaagt ccctgaagtc aacgcaagac   1620
gaaatcaacc aggcaaggag caaactttcc cagctgcatg aaagccgcca ggaggcccac   1680
aggagcctgg agcagtatga ccaggtgctc gatggagccc atggtgccag cctgaccgac   1740
ctggccaacc tgagcgaagg cgtctccctg gcagagaggg gcagttttgg agccatggat   1800
gatcctttca aaaataaagc cttgttattt agcaacaaca cgcaagagtt gcatccggat   1860
cctttccaga cagaagaccc cttcaaatct gacccattta aaggagctga ccccttcaaa   1920
ggcgacccgt tccagaatga cccctttgca gaacagcaga caacttcaac agatccattt   1980
ggaggggacc ctttcaaaga aagtgaccca ttccgtggct ctgccactga cgacttcttc   2040
aagaaacaga caaagaatga cccatttacc tcggatccat tcacgaaaaa cccttcctta   2100
ccttcgaagc tcgacccctt tgaatccagt gatccctttt catcctccag tgtctcctca   2160
aaaggatcag atccctttgg aaccttagat cccttcggaa gtgggtcctt caatagtgct   2220
gaaggctttg ccgacttcag ccagatgtcc aagcccccac cttctgggcc tttcacctcc   2280
tccttgggag gggcaggatt ctcagatgac ccctttaaaa gtaaacagga cactcctgct   2340
ctgcctccga agaaacctgc tcctccacgg cctaaaccgc ccagcggtaa aagtacacct   2400
gtaagccagc ttggttccgc agactttccc gaggcccccg atccattcca gccactcggg   2460
gctgacagcg gcgacccgtt ccaaagtaaa aaggggtttg gggacccgtt tagtggaaaa   2520
gacccatttg tcccctcctc tgcagctaaa ccttctaagg cctctgcctc gggctttgca   2580
gacttcacct ctgtaagttg agtcctccgc ctccgggcca ccccactccc ttccgcttgc   2640
agcttccctg ggatttttgt ctccttttaa aggcaaacct cccagcttct ttagcctctt   2700
ggtacctcac actctctgtc cctcgcgtta tttattctac actgccactt ctgtaagaaa   2760
aacagtttct caat                                                     2774

<210>  9

<211>  864

<212>  PRT

<213>  homo sapiens


<220>

<221>  misc_feature

<223>  EPS15R gene product

<400>  9

Met Ala Ala Pro Leu Ile Pro Leu Ser Gln Gln Ile Pro Thr Gly Asn
1               5                   10                  15

Ser Leu Tyr Glu Ser Tyr Tyr Lys Gln Val Asp Pro Ala Tyr Thr Gly
            20                  25                  30

Arg Val Gly Ala Ser Glu Ala Ala Leu Phe Leu Lys Lys Ser Gly Leu
        35                  40                  45

Ser Asp Ile Ile Leu Gly Lys Ile Trp Asp Leu Ala Asp Pro Glu Gly
    50                  55                  60

Lys Gly Phe Leu Asp Lys Gln Gly Phe Tyr Val Ala Leu Arg Leu Val
65                  70                  75                  80

Ala Cys Ala Gln Ser Gly His Glu Val Thr Leu Ser Asn Leu Asn Leu
                85                  90                  95

Ser Met Pro Pro Pro Lys Phe His Asp Thr Ser Ser Pro Leu Met Val
            100                 105                 110

Thr Pro Pro Ser Ala Glu Ala His Trp Ala Val Arg Val Glu Glu Lys
        115                 120                 125

Ala Lys Phe Asp Gly Ile Phe Glu Ser Leu Leu Pro Ile Asn Gly Leu
    130                 135                 140

Leu Ser Gly Asp Lys Val Lys Pro Val Leu Met Asn Ser Lys Leu Pro
145                 150                 155                 160

Leu Asp Val Leu Gly Arg Val Trp Asp Leu Ser Asp Ile Asp Lys Asp
                165                 170                 175

Gly His Leu Asp Arg Asp Glu Phe Ala Val Ala Met His Leu Val Tyr
            180                 185                 190

Arg Ala Leu Glu Lys Glu Pro Val Pro Ser Ala Leu Pro Pro Ser Leu
        195                 200                 205

Ile Pro Pro Ser Lys Arg Lys Lys Thr Val Phe Pro Gly Ala Val Pro
    210                 215                 220

Val Leu Pro Ala Ser Pro Pro Pro Lys Asp Ser Leu Arg Ser Thr Pro
225                 230                 235                 240

Ser His Gly Ser Val Ser Ser Leu Asn Ser Thr Gly Ser Leu Ser Pro
                245                 250                 255

Lys His Ser Leu Lys Gln Thr Gln Pro Thr Val Asn Trp Val Val Pro
            260                 265                 270

Val Ala Asp Lys Met Arg Phe Asp Glu Ile Phe Leu Lys Thr Asp Leu
        275                 280                 285

Asp Leu Asp Gly Tyr Val Ser Gly Gln Glu Val Lys Glu Ile Phe Met
    290                 295                 300

His Ser Gly Leu Thr Gln Asn Leu Leu Ala His Ile Trp Ala Leu Ala
305                 310                 315                 320

Asp Thr Arg Gln Thr Gly Lys Leu Ser Lys Asp Gln Phe Ala Leu Ala
                325                 330                 335

Met Tyr Phe Ile Gln Gln Lys Val Ser Lys Gly Ile Asp Pro Pro Gln
            340                 345                 350

Val Leu Ser Pro Asp Met Val Pro Pro Ser Glu Arg Gly Thr Pro Gly
        355                 360                 365

Pro Asp Ser Ser Gly Ser Leu Gly Ser Gly Glu Phe Thr Gly Val Lys
    370                 375                 380

Glu Leu Asp Asp Ile Ser Gln Glu Ile Ala Gln Leu Gln Arg Glu Lys
385                 390                 395                 400

Tyr Ser Leu Glu Gln Asp Ile Arg Glu Lys Glu Glu Ala Ile Arg Gln
                405                 410                 415

Lys Thr Ser Glu Val Gln Glu Leu Gln Asn Asp Leu Asp Arg Glu Thr
            420                 425                 430

Ser Ser Leu Gln Glu Leu Glu Ala Gln Lys Gln Asp Ala Gln Asp Arg
        435                 440                 445

Leu Asp Glu Met Asp Gln Gln Lys Ala Lys Leu Arg Asp Met Leu Ser
    450                 455                 460

Asp Val Arg Gln Lys Cys Gln Asp Glu Thr Gln Met Ile Ser Ser Leu
465                 470                 475                 480

Lys Thr Gln Ile Gln Ser Gln Glu Ser Asp Leu Lys Ser Gln Glu Asp
                485                 490                 495

Asp Leu Asn Arg Ala Lys Ser Glu Leu Asn Arg Leu Gln Gln Glu Glu
            500                 505                 510

Thr Gln Leu Glu Gln Ser Ile Gln Ala Gly Arg Val Gln Leu Glu Thr
        515                 520                 525

Ile Ile Lys Ser Leu Lys Ser Thr Gln Asp Glu Ile Asn Gln Ala Arg
    530                 535                 540

Ser Lys Leu Ser Gln Leu His Glu Ser Arg Gln Glu Ala His Arg Ser
545                 550                 555                 560

Leu Glu Gln Tyr Asp Gln Val Leu Asp Gly Ala His Gly Ala Ser Leu
                565                 570                 575

Thr Asp Leu Ala Asn Leu Ser Glu Gly Val Ser Leu Ala Glu Arg Gly
            580                 585                 590

Ser Phe Gly Ala Met Asp Asp Pro Phe Lys Asn Lys Ala Leu Leu Phe
        595                 600                 605

Ser Asn Asn Thr Gln Glu Leu His Pro Asp Pro Phe Gln Thr Glu Asp
    610                 615                 620

Pro Phe Lys Ser Asp Pro Phe Lys Gly Ala Asp Pro Phe Lys Gly Asp
625                 630                 635                 640

Pro Phe Gln Asn Asp Pro Phe Ala Glu Gln Gln Thr Thr Ser Thr Asp
                645                 650                 655

Pro Phe Gly Gly Asp Pro Phe Lys Glu Ser Asp Pro Phe Arg Gly Ser
            660                 665                 670

Ala Thr Asp Asp Phe Phe Lys Lys Gln Thr Lys Asn Asp Pro Phe Thr
        675                 680                 685

Ser Asp Pro Phe Thr Lys Asn Pro Ser Leu Pro Ser Lys Leu Asp Pro
    690                 695                 700

Phe Glu Ser Ser Asp Pro Phe Ser Ser Ser Ser Val Ser Ser Lys Gly
705                 710                 715                 720

Ser Asp Pro Phe Gly Thr Leu Asp Pro Phe Gly Ser Gly Ser Phe Asn
                725                 730                 735

Ser Ala Glu Gly Phe Ala Asp Phe Ser Gln Met Ser Lys Pro Pro Pro
            740                 745                 750

Ser Gly Pro Phe Thr Ser Ser Leu Gly Gly Ala Gly Phe Ser Asp Asp
        755                 760                 765

Pro Phe Lys Ser Lys Gln Asp Thr Pro Ala Leu Pro Pro Lys Lys Pro
    770                 775                 780

Ala Pro Pro Arg Pro Lys Pro Pro Ser Gly Lys Ser Thr Pro Val Ser
785                 790                 795                 800

Gln Leu Gly Ser Ala Asp Phe Pro Glu Ala Pro Asp Pro Phe Gln Pro
                805                 810                 815

Leu Gly Ala Asp Ser Gly Asp Pro Phe Gln Ser Lys Lys Gly Phe Gly
            820                 825                 830

Asp Pro Phe Ser Gly Lys Asp Pro Phe Val Pro Ser Ser Ala Ala Lys
        835                 840                 845

Pro Ser Lys Ala Ser Ala Ser Gly Phe Ala Asp Phe Thr Ser Val Ser
    850                 855                 860

<210>  10

<211>  3651

<212>  DNA

<213>  homo sapiens

<220>

<221>  misc_feature

<223>  PLCB1 gene sequence made up of non-contigous segments of gene map
       locus 20p12

<400>  10
atggccgggg ctcaacccgg agtgcacgcc ttgcaactca agcccgtgtg cgtgtccgac      60

agcctcaaga agggcaccaa attcgtcaag tgggatgatg actcaactat tgttactcca     120

attattttga ggactgaccc tcagggattt ttcttttact ggacagatca aaacaaggag     180

acagagctac tggatctcag ccttgtcaaa gatgccagat gtgggagaca cgccaaagct     240

cccaaggacc ccaaattacg tgaacttttg gatgtgggga acatcgggcg cctggagcag     300

cgcatgatca cagtggtgta tgggcctgac ctcgtgaaca tctcccattt gaatctcgtg     360

gctttccaag aagaagtggc caaggaatgg acaaatgagg ttttcagttt ggcaacaaac     420

ctgctggccc aaaacatgtc cagggatgca tttctggaaa aagcctatac taaacttaag     480

ctgcaagtca ctccagaagg gcgtattcct ctcaaaaaca tatatcgctt gttttcagca     540

gatcggaagc gagttgaaac tgctttagag gcttgtagtc ttccatcttc aaggaatgat     600

tcaatacctc aagaagattt cactccagaa gtgtacagag ttttcctcaa caacctttgc     660

cctcgacctg aaattgataa catcttttca gaatttggtg caaaaagcaa accatatctt     720

accgttgatc agatgatgga ttttatcaac cttaagcagc gagatcctcg gcttaatgaa     780

atactttatc cacctctaaa acaagagcaa gtccaagtat tgattgagaa gtatgaaccc     840

aacaacagcc tcgccagaaa aggacaaata tcagtggatg ggttcatgcg ctatctgagt 900
ggagaagaaa acggagtcgt ttcacctgag aaactggatt tgaatgaaga catgtctcag 960
cccctttctc actatttcat taattcctcg cacaacacct acctcacagc tggccaactg 1020
gctggaaact cctctgttga gatgtatcgc caagtgctcc tgtctggttg tcgctgtgtg 1080
gagctggact gctggaaggg acggactgca gaagaggaac ctgtcatcac ccatggcttc 1140
accatgacaa ctgaaatatc tttcaaggaa gtgatagaag caattgcgga gtgtgcattt 1200
aagacttcac cttttccaat tctcctttcg tttgagaacc atgtggattc cccaaagcag 1260
caagccaaga tggcggagta ctgccgactg atctttgggg atgcccttct catggagccc 1320
ctggaaaaat atccactgga atctggagtt cctcttccaa gccctatgga tttaatgtat 1380
aaaattttgg tgaaaaataa gaagaaatca cacaagtcat cagaaggaag cggcaaaaag 1440
aagctctcag aacaagcctc caacacctac agtgactcct ccagcatgtt cgagccctca 1500
tccccaggag ccggagaagc tgatacggaa agtgacgacg acgatgatga tgatgactgt 1560
aaaaaatctt caatggatga ggggactgct ggaagtgagg ctatggccac agaagaaatg 1620
tctaatctgg tgaactatat tcagccagtc aagtttgagt catttgaaat ttcaaaaaaa 1680
agaaataaaa gttttgaaat gtcttccttc gtggaaacca aaggacttga acaactcacc 1740
aagtctccag tggaatttgt agaatataac aaaatgcagc ttagcaggat atatccaaaa 1800
ggaacacgtg tggattcatc caactatatg cctcagctct tctggaatgc aggttgtcag 1860
atggtggcac ttaatttcca gacaatggac ctggctatgc aaataaatat ggggatgtat 1920
gaatacaacg ggaagagtgg ctacagattg aagccagagt tcatgaggag gcctgacaag 1980
cattttgatc catttactga aggcatcgta gatgggatag tggcaaacac tttgtctgtt 2040
aagattattt caggtcagtt tctttctgat aagaaagttg ggacttacgt ggaagtagat 2100
atgtttggtt tgcctgtgga tacaaggagg aaggcattta agaccaaaac atcccaagga 2160
aatgctgtga atcctgtctg ggaagaagaa cctattgtgt tcaaaaaggt ggttcttcct 2220
actctggcct gtttgagaat agcagtttat gaagaaggag gtaaattcat tggccaccgt 2280
atcttgccag tgcaagccat tcggccaggc tatcactata tctgtctaag gaatgaaagg 2340
aaccagcctc tgacgctgcc tgctgtcttt gtctacatag aagtgaaaga ctatgtgcca 2400
gacacatatg cagatgtcat cgaagcttta tcaaacccaa tccgatatgt gaacctgatg 2460
gaacagagag ctaagcaatt ggctgctttg acactggaag atgaagaaga agtaaagaaa 2520
gaggctgatc ctggagaaac accatcagag gctccaagtg aagcgagaac gactccagca 2580
gaaaatgggg tgaatcacac tacaaccctg acacccaagc caccctccca ggctctccac 2640
agccagccag ctccaggttc tgtaaaggca cctgccaaaa cagaagatct tattcagagt 2700
gtcttaacag aagtggaagc acagaccatc gaagaactaa agcaacagaa atcgtttgtg 2760
aaacttcaaa agaaacacta caaagaaatg aaagacctgg ttaagagaca ccacaagaaa 2820
accactgacc ttatcaaaga acacactacc aagtataatg aaattcagaa tgactacttg 2880

agaaggagag ccgctttgga aaagtccgcc aaaaaggaca gtaagaaaaa atcggaaccc 2940
agcagccctg atcatggttc atcaacgatt gagcaagacc tcgctgctct ggatgctgaa 3000
atgacccaaa agttaataga cttgaaggac aaacaacagc agcagctgct taatcttcgg 3060
caagaacagt attatagtga aaaataccag aagcgagaac atattaaact gcttattcaa 3120
aagttgacgg atgtcgcaga agagtgtcag aacaatcagt taaagaagct caaagaaatc 3180
tgtgagaaag aaaagaaaga attaaagaag aaaatggata aaaagaggca ggagaagata 3240
acagaagcta aatccaaaga caaaagtcag atggaagagg agaagacaga gatgatccgg 3300
tcatatatcc aggaagtggt gcagtatatc aagaggctag aagaagcgca aagtaaacgg 3360
caagaaaaac tcgtagagaa acacaaggaa atacgtcagc agatcctgga tgaaaagccc 3420
aagctgcagg tggagctgga gcaagaatac caagacaaat tcaaaagact gcccctcgag 3480
attttggaat tcgtgcagga agccatgaaa ggaaagatca gtgaagacag caatcacggt 3540
tctgcccctc tctccctgtc ctcagaccct ggaaaagtga accacaagac tccctccagt 3600
gaggagctgg gaggagacat cccaggaaaa gaatttgata ctcctctgtg a 3651

<210> 11
<211> 6705
<212> DNA
<213> homo sapiens

<220>
<221> misc_feature
<223> PLCB1 mRNA

<400> 11
   cagatggccg gggctcaacc cggagtgcac gccttgcaac tcaagcccgt gtgcgtgtcc 60
   gacagcctca agaagggcac caaattcgtc aagtgggatg atgactcaac tattgttact 120
   ccaattattt tgaggactga ccctcaggga tttttctttt actggacaga tcaaaacaag 180
   gagacagagc tactggatct cagccttgtc aaagatgcca gatgtgggag acacgccaaa 240
   gctcccaagg accccaaatt acgtgaactt ttggatgtgg ggaacatcgg gcgcctggag 300
   cagcgcatga tcacagtggt gtatgggcct gacctcgtga acatctccca tttgaatctc 360
   gtggctttcc aagaagaagt ggccaaggaa tggacaaatg aggttttcag tttggcaaca 420
   aacctgctgg cccaaaacat gtccagggat gcatttctgg aaaaagccta tactaaactt 480
   aagctgcaag tcactccaga agggcgtatt cctctcaaaa acatatatcg cttgttttca 540
   gcagatcgga agcgagttga aactgcttta gaggcttgta gtcttccatc ttcaaggaat 600
   gattcaatac ctcaagaaga tttcactcca gaagtgtaca gagttttcct caacaacctt 660
   tgccctcgac ctgaaattga taacatcttt tcagaatttg gtgcaaaaag caaaccatat 720
   cttaccgttg atcagatgat ggattttatc aaccttaagc agcgagatcc tcggcttaat 780
   gaaatacttt atccacctct aaaacaagag caagtccaag tattgattga gaagtatgaa 840
   cccaacaaca gcctcgccag aaaaggacaa atatcagtgg atgggttcat gcgctatctg 900
   agtggagaag aaaacggagt cgtttcacct gagaaactgg atttgaatga agacatgtct 960
   cagccccttt ctcactattt cattaattcc tcgcacaaca cctacctcac agctggccaa 1020
   ctggctggaa actcctctgt tgagatgtat cgccaagtgc tcctgtctgg ttgtcgctgt 1080
   gtggagctgg actgctggaa gggacggact gcagaagagg aacctgtcat cacccatggc 1140
   ttcaccatga caactgaaat atctttcaag gaagtgatag aagcaattgc ggagtgtgca 1200
   tttaagactt caccttttcc aattctcctt tcgtttgaga accatgtgga ttccccaaag 1260
   cagcaagcca agatggcgga gtactgccga ctgatctttg gggatgccct tctcatggag 1320
   cccctggaaa aatatccact ggaatctgga gttcctcttc caagccctat ggatttaatg 1380
   tataaaattt tggtgaaaaa taagaagaaa tcacacaagt catcagaagg aagcggcaaa 1440
   aagaagctct cagaacaagc ctccaacacc tacagtgact cctccagcat gttcgagccc 1500
   tcatccccag gagccggaga agctgatacg gaaagtgacg acgacgatga tgatgatgac 1560
   tgtaaaaaat cttcaatgga tgaggggact gctggaagtg aggctatggc cacagaagaa 1620
   atgtctaatc tggtgaacta tattcagcca gtcaagtttg agtcatttga aatttcaaaa 1680
   aaaagaaata aaagttttga aatgtcttcc ttcgtggaaa ccaaaggact tgaacaactc 1740
   accaagtctc cagtggaatt tgtagaatat aacaaaatgc agcttagcag gatatatcca 1800
   aaaggaacac gtgtggattc atccaactat atgcctcagc tcttctggaa tgcaggttgt 1860
   cagatggtgg cacttaattt ccagacaatg gacctggcta tgcaaataaa tatggggatg 1920
   tatgaataca acgggaagag tggctacaga ttgaagccag agttcatgag gaggcctgac 1980
   aagcattttg atccatttac tgaaggcatc gtagatggga tagtggcaaa cactttgtct 2040
   gttaagatta tttcaggtca gtttctttct gataagaaag ttgggactta cgtggaagta 2100
   gatatgtttg gtttgcctgt ggatacaagg aggaaggcat ttaagaccaa aacatcccaa 2160
   ggaaatgctg tgaatcctgt ctgggaagaa gaacctattg tgttcaaaaa ggtggttctt 2220
   cctactctgg cctgtttgag aatagcagtt tatgaagaag gaggtaaatt cattggccac 2280
   cgtatcttgc cagtgcaagc cattcggcca ggctatcact atatctgtct aaggaatgaa 2340
   aggaaccagc ctctgacgct gcctgctgtc tttgtctaca tagaagtgaa agactatgtg 2400
   ccagacacat atgcagatgt catcgaagct ttatcaaacc caatccgata tgtgaacctg 2460
   atggaacaga gagctaagca attggctgct ttgacactgg aagatgaaga agaagtaaag 2520
   aaagaggctg atcctggaga aacaccatca gaggctccaa gtgaagcgag aacgactcca 2580
   gcagaaaatg gggtgaatca cactacaacc ctgacaccca agccaccctc ccaggctctc 2640
   cacagccagc cagctccagg ttctgtaaag gcacctgcca aaacagaaga tcttattcag 2700
   agtgtcttaa cagaagtgga agcacagacc atcgaagaac taaagcaaca gaaatcgttt 2760
   gtgaaacttc aaaagaaaca ctacaaagaa atgaaagacc tggttaagag acaccacaag 2820
   aaaaccactg accttatcaa agaacacact accaagtata atgaaattca gaatgactac 2880
   ttgagaagga gagccgcttt ggaaaagtcc gccaaaaagg acagtaagaa aaaatcggaa 2940
   cccagcagcc ctgatcatgg ttcatcaacg attgagcaag acctcgctgc tctggatgct 3000
   gaaatgaccc aaaagttaat agacttgaag gacaaacaac agcagcagct gcttaatctt 3060
   cggcaagaac agtattatag tgaaaaatac cagaagcgag aacatattaa actgcttatt 3120
   caaaagttga cggatgtcgc agaagagtgt cagaacaatc agttaaagaa gctcaaagaa 3180
   atctgtgaga aagaaaagaa agaattaaag aagaaaatgg ataaaaagag gcaggagaag 3240
   ataacagaag ctaaatccaa agacaaaagt cagatggaag aggagaagac agagatgatc 3300
   cggtcatata tccaggaagt ggtgcagtat atcaagaggc tagaagaagc gcaaagtaaa 3360
   cggcaagaaa aactcgtaga gaaacacaag gaaatacgtc agcagatcct ggatgaaaag 3420
   cccaagctgc aggtggagct ggagcaagaa taccaagaca aattcaaaag actgcccctc 3480
   gagattttgg aattcgtgca ggaagccatg aaaggaaaga tcagtgaaga cagcaatcac 3540
   ggttctgccc ctctctccct gtcctcagac cctggaaaag tgaaccacaa gactccctcc 3600
   agtgaggagc tgggaggaga catcccagga aaagaatttg atactcctct gtgaatgctc 3660
   ctgccaggcc ttcagaaatt gcatggccac tccagcgtca tcggactctc tcttattaca 3720
   aagatcactg cccaggacca tcttcccgag aagcatccct tagcctaaaa tccacaccaa 3780
   agggagagtt ccagaggaat ccatgaagaa ttcccatgcc caggctccat gtgtcatgtg 3840
   gaaacctcca caggtctgct agtgaagaat gcatgtatgt gagatttttg ttttctttcc 3900
   aatagcaaat tcaaagcaag caacttgcag gctccatgga acttttaatg aaggacagtg 3960
   tcttctttga agaaaatcaa gctcgtgttt ttattcgaag ctctggtgta aaatatttca 4020
   aagtcataga aatagtttga gaaatgcata gcattattta acactattga acagccgact 4080
   ttgagcattg tttcttctaa ctgcccctca actaccatta tcttcaagtc aacatgcata 4140
   ttacattttc atcctttgct ttgcaagcac tggtggcttg cagtttgcta atttatttat 4200
   catagagtca tcaatgtatt tgttgctgac atggttttat tagatactgt agtgattcaa 4260
   ataagttttc tatttgaaaa aaaaaatcac ttgattgtat ccttgcccag tgaagccatc 4320
   ctaagactta gcaatatgga ttgtacattt ggctgcatga gcaagtcggc cgcacacttc 4380
   cagacagtgt gctgtttgaa ttgactattt gcactcaaag tctgggtatt cattggttat 4440
   tggcctgaaa tgatcaaata actacaaatg atctgttgaa taaaaatagt tgagctgata 4500
   tatgttaagc agatattcaa tcagaatgaa caggttccgg tggttatttt gccgtttgac 4560
   attttttatg gttcatttat ttttaatata gagaggaaga ttgaatattt atctagagaa 4620
   tacaaagacc cacatgtaaa tgataggtat tatctccatg tatatatgta cccacttagt 4680
   catgtaagtg catatacaca tacacacaca tgagtgtaga catgtgttta ttaattgaca 4740
   atgacccaaa tctcttccac aagacttaaa accaaattca gggacaaatg gatagagaag 4800
   aaaagggtca aacatcgaga ttacatggat gttaaattat atggagacgc taagaaataa 4860
   ttgatggagc cattgatgca aaccgaagta gatttagaac ttatatgaat ttgatttata 4920
   ttttgcaaga tcaaaaatta gatgttaagt atcagatttt aagcttgttt taatggtcaa 4980
   aaaattagga cagaaataat atggacattt attagtatct tccataattt ttaagtctga 5040
   cacatttcta ttttattcta acataaaaaa acttccatta tatatttact aagtatattt 5100
   aattcactta actctgtctt tataagttcc tattttagat ggaataagag aaacaaatta 5160
   tatcaaggta aaactgatca aaagcataat tgaaagttct gaaaaaagaa aaataataat 5220
   atgtagaaaa atgtaactta gagagtaaca catgaacatt gaagttaaaa cccagaagcc 5280
   agatgctcac agttttattt tactttaaaa taaacctgtc tgactgtagc tttgtgaaat 5340
   atcttaaaac gcaaaaacca attgtgtcct gaaaattgtt tcaagaattt aatattttta 5400
   tgaaaattat tttatttaac tttaagcaat aactagagat tacaattaaa ttttaatcaa 5460
   aatgaaggct tagttcaaac ataaggaaac agtgtttgat taaaaaaaac acatctagta 5520
   agacgtaagg ggaaaatcac atcctctttg gagatgatta tattttgatc tgaagggttt 5580
   ggggtgttca ttagacactt aataaaactt aacttccaat gaaaaagaaa tcttttgtaa 5640
   atcattctac ttttgcactt tgaaagaaag gcgttaatca taaagaagca agaatggtca 5700
   aaatcgaatg ctgcattttt ataaacaaaa ttacagactg tctgaaattg aagaagaatg 5760
   aactaataat agcattcata accaaacaca atgatgatta ttgcagaaca ttgtatcaca 5820
   ttttagtcca gagatagaat aaagttgaat aaccttgact tacacaaaac tgttttggta 5880
   gttggatttc attatcttag tgaatttagt cattttacaa tatgtttgta tttggccatt 5940
   tactgtaatc acatttttat atctgtacaa tgacactttt tgcagttgtg gggtagtgtg 6000
   taacactgtc catcttgcat cattgaaact actacaatga tactatcatt taataatatt 6060
   aatattactt gaaatagact aagataaaga aaaggggtct gtatgatgtg cagttttgtg 6120
   cctttatgta tttgccttgt tctttgtcga atgtgtgaaa ttccgtactg tggtttttcc 6180
   tataatagaa agtagagctg tgtattaaat tagactgtgt ctctctgata cctttacact 6240
   actgagaata gcatggtttt ggccatgtaa accaattttc aaagttctaa tgacatagcc 6300
   atgtgttttt ggttttttat atttcatttt aaaatttgag tatcaccata cattaattaa 6360
   tactcctgta gtagataagc tgtcattaag taattcccaa aaaaagggcc atttgcttgc 6420
   attactttga atttaatgtt gcgcttgtgc actgtgttaa tattgtttgt gatggattgg 6480
   acgttgtgac tcttgccttt taagaagaaa aaaaagatag gacaaagtat ttgaagctct 6540
   taaaatgtac atattttggt tcttctatct caaattattt aaaatgcata attcacattt 6600
   ttgtaataat tctatgcaat tttgtggcat gatgtttctt ccacttgtaa ttttatgtgc 6660
   tttcatcaca aatccaaagg aaagaataaa aatttcttaa cacaa 6705

<210>  12

<211>  1216

<212>  PRT

<213>  homo sapiens

<220>

<221>  misc_feature

<223>  PLCB1 gene product

<400>  12

<210>  13

<211>  969

<212>  DNA

<213>  homo sapiens


<220>

<221>  misc_feature

<223>  UGTREL1 gene sequence made up of non-contigous segments of gene m
      ap locus 17q21.33

<400>  13
atggcctcta gcagctccct ggtgcccgac cggctgcgcc tgccgctctg cttcctgggt      60

gtctttgtct gctattttta ctatgggatc ctgcaggaaa agataacaag aggaaagtat     120

ggggaaggag ccaagcagga gacgttcacc tttgccttaa ctttggtctt cattcaatgt     180

gtgatcaatg ctgtgtttgc caagatcttg atccagtttt ttgacactgc cagggtggat     240

cgtacccgga gctggctcta tgctgcctgt tctatctcct atctgggtgc catggtctcc     300

agcaattcag cactacagtt tgtcaactac ccaactcagg tccttggtaa atcctgcaag     360

ccaatcccag tcatgctcct tggggtgacc ctcttgaaga agaagtaccc gttggccaag     420

tacctgtgtg tgctgttaat tgtggctgga gtggcccttt tcatgtacaa acccaagaaa     480

gttgttggga tagaagaaca cacagtcggc tatggagagc tactcttgct attatcgctg     540

accctggatg gactgactgg tgtttcccag gaccacatgc gggctcatta ccaaacaggc     600

tccaaccaca tgatgctgaa catcaacctt tggtcgacat tgctgctggg aatgggaatc     660

ctgttcactg gggagctctg ggagttcttg agctttgctg aaaggtaccc tgccatcatc     720

tataacatcc tgctctttgg gctgaccagt gccctgggtc agagcttcat ctttatgacg     780

gttgtgtatt ttggtcccct gacctgctcc atcatcacta caactcgaaa gttcttcaca     840

attttggcct ctgtgatcct cttcgccaat cccatcagcc ccatgcagtg ggtgggcact     900

gtgcttgtgt tcctgggtct tggtcttgat gccaagtttg ggaaaggagc taagaagaca     960

tcccactag                                                             969

<210>  14

<211>  1186

<212>  DNA

<213>  homo sapiens


<220>

<221>  misc_feature

<223>  UGTREL1 mRNA


<400>  14
gatgtccggc tggagctgtc gcctccgccg ccgctgctgc cggtgccggt tgtgagcggg      60

tctccagtcg gctcctctgg gcgtctcatg gcctctagca gctccctggt gcccgaccgg     120

ctgcgcctgc cgctctgctt cctgggtgtc tttgtctgct atttttacta tgggatcctg     180

caggaaaaga taacaagagg aaagtatggg gaaggagcca agcaggagac gttcaccttt    240
gccttaactt tggtcttcat tcaatgtgtg atcaatgctg tgtttgccaa gatcttgatc    300
cagttttttg acactgccag ggtggatcgt acccggagct ggctctatgc tgcctgttct    360
atctcctatc tgggtgccat ggtctccagc aattcagcac tacagtttgt caactaccca    420
actcaggtcc ttggtaaatc ctgcaagcca atcccagtca tgctccttgg ggtgaccctc    480
ttgaagaaga agtacccgtt ggccaagtac ctgtgtgtgc tgttaattgt ggctggagtg    540
gcccttttca tgtacaaacc caagaaagtt gttgggatag aagaacacac agtcggctat    600
ggagagctac tcttgctatt atcgctgacc ctggatggac tgactggtgt ttcccaggac    660
cacatgcggg ctcattacca aacaggctcc aaccacatga tgctgaacat caacctttgg    720
tcgacattgc tgctgggaat gggaatcctg ttcactgggg agctctggga gttcttgagc    780
tttgctgaaa ggtaccctgc catcatctat aacatcctgc tctttgggct gaccagtgcc    840
ctgggtcaga gcttcatctt tatgacggtt gtgtatttgg gtcccctgac ctgctccatc    900
atcactacaa ctcgaaagtt cttcacaatt ttggcctctg tgatcctctt cgccaatccc    960
atcagcccca tgcagtgggt gggcactgtg cttgtgttcc tgggtcttgg tcttgatgcc    1020
aagtttggga aaggagctaa gaagacatcc cactaggaag agagagacta cctccacatc    1080
aagaatattt aagttattat ctcaaacagt gacatctctt gggaaaatgg acttaatagg    1140
aatatgggac tgagttccag tcttttttaa taaaataaaa tcaagc                  1186

<210>  15

<211>  322

<212>  PRT

<213>  homo sapiens


<220>

<221>  misc_feature

<223>  UGTREL1 gene product


<400>  15

Met Ala Ser Ser Ser Ser Leu Val Pro Asp Arg Leu Arg Leu Pro Leu
1               5                   10                  15


Cys Phe Leu Gly Val Phe Val Cys Tyr Phe Tyr Tyr Gly Ile Leu Gln
            20                  25                  30


Glu Lys Ile Thr Arg Gly Lys Tyr Gly Glu Gly Ala Lys Gln Glu Thr
        35                  40                  45


Phe Thr Phe Ala Leu Thr Leu Val Phe Ile Gln Cys Val Ile Asn Ala

50                  55                  60

Val Phe Ala Lys Ile Leu Ile Gln Phe Phe Asp Thr Ala Arg Val Asp
65                  70                  75                  80

Arg Thr Arg Ser Trp Leu Tyr Ala Ala Cys Ser Ile Ser Tyr Leu Gly
                85                  90                  95

Ala Met Val Ser Ser Asn Ser Ala Leu Gln Phe Val Asn Tyr Pro Thr
            100                 105                 110

Gln Val Leu Gly Lys Ser Cys Lys Pro Ile Pro Val Met Leu Leu Gly
        115                 120                 125

Val Thr Leu Leu Lys Lys Lys Tyr Pro Leu Ala Lys Tyr Leu Cys Val
    130                 135                 140

Leu Leu Ile Val Ala Gly Val Ala Leu Phe Met Tyr Lys Pro Lys Lys
145                 150                 155                 160

Val Val Gly Ile Glu Glu His Thr Val Gly Tyr Gly Glu Leu Leu Leu
                165                 170                 175

Leu Leu Ser Leu Thr Leu Asp Gly Leu Thr Gly Val Ser Gln Asp His
            180                 185                 190

Met Arg Ala His Tyr Gln Thr Gly Ser Asn His Met Met Leu Asn Ile
        195                 200                 205

Asn Leu Trp Ser Thr Leu Leu Leu Gly Met Gly Ile Leu Phe Thr Gly
    210                 215                 220

Glu Leu Trp Glu Phe Leu Ser Phe Ala Glu Arg Tyr Pro Ala Ile Ile
225                 230                 235                 240

Tyr Asn Ile Leu Leu Phe Gly Leu Thr Ser Ala Leu Gly Gln Ser Phe
                245                 250                 255

Ile Phe Met Thr Val Val Tyr Phe Gly Pro Leu Thr Cys Ser Ile Ile
            260                 265                 270

Thr Thr Thr Arg Lys Phe Phe Thr Ile Leu Ala Ser Val Ile Leu Phe
        275                 280                 285

Ala Asn Pro Ile Ser Pro Met Gln Trp Val Gly Thr Val Leu Val Phe
    290                 295                 300

Leu Gly Leu Gly Leu Asp Ala Lys Phe Gly Lys Gly Ala Lys Lys Thr
305                 310                 315                 320

Ser His

<210>  16

<211>  2238

<212>  DNA

<213>  homo sapiens



<220>

<221>  misc_feature

<223>  RSK4 gene sequence made up of non-contigous segments of gene map
       locus Xq21



<400>  16
atgctaccat tcgctcctca ggacgagccc tgggaccgag aaatggaagt gttcagcggc      60

ggcggcgcga gcagcggcga ggtaaatggt cttaaaatgg ttgatgagcc aatggaagag     120

ggagaagcag attcttgtca tgatgaagga gttgttaaag aaatccctat tactcatcat     180

gttaaggaag gctatgagaa agcagatcct gcacagtttg agttgctcaa ggttcttggt     240

caggggtcat ttggaaaggt ttttcttgtt agaaagaaga ccggtcctga tgctgggcag     300

ctctatgcaa tgaaggtgtt aaaaaaagcc tctttaaaag ttcgagacag agttcggaca     360

aagatggaga gggatatact ggtggaagta aatcatccat ttattgtcaa attgcactat     420

gcctttcaga ctgaagggaa actgtactta atactggatt ttctcagggg aggagatgtt     480

ttcacaagat tatccaaaga ggttctgttt acagaggaag atgtgaaatt ctacctcgca     540

gaactggccc ttgctttgga tcatctgcac caattaggaa ttgtttatag agacctgaag     600

ccagaaaaca ttttgcttga tgaaatagga catatcaaat taacagattt tggactcagc     660

aaggagtcag tagatcaaga aaagaaggct tactcatttt gtggtacagt agagtatatg     720

gctcctgaag tagtaaatag gagaggccat tcccagagtg ctgattggtg gtcatatggt     780

gttcttatgt ttgaaatgct tactggtact ctgccatttc aaggtaaaga cagaaatgag     840

accatgaata tgatattaaa agcaaaactt ggaatgcctc aatttcttag tgctgaagca     900

caaagtcttc taaggatgtt attcaaaagg aatccagcaa atagattggg atcagaagga     960

gttgaagaaa tcaaaagaca tctgtttttt gcaaatattg actgggataa attatataaa    1020

agagaagttc aacctccttt caaacctgct tctggaaaac cagatgatac tttttgtttt    1080

gatcctgaat ttactgcaaa aacacctaaa gattctcccg gtttgccagc cagtgcaaat    1140

gctcatcagc tcttcaaagg attcagcttt gttgcaactt ctattgcaga agaatataaa    1200

atcactccta tcacaagtgc aaatgtatta ccaattgttc agataaatgg aaatgctgca    1260

caatttggtg aagtatatga attgaaggag gatattggtg ttggctccta ctctgtttgc    1320

aagcgatgca tacatgcaac taccaacatg gaatttgcag tgaagatcat tgacaaaagt    1380

aagcgagacc cttcagaaga gattgaaata ttgatgcgct atggacaaca tcccaacatt 1440
attactttga aggatgtctt tgatgatggt agatatgttt accttgttac ggatttaatg 1500
aaaggaggag agttacttga ccgtattctc aaacaaaaat gtttctcgga acgggaggct 1560
agtgatatac tatatgtaat aagtaagaca gttgactatc ttcattgtca aggagttgtt 1620
catcgtgatc ttaaacctag taatatttta tacatggatg aatcagccag tgcagattca 1680
atcaggatat gtgattttgg gtttgcaaaa caacttcgag gagaaaatgg acttctctta 1740
actccatgct acactgcaaa ctttgttgca cctgaggttc ttatgcaaca gggatatgat 1800
gctgcttgtg atatctggag tttaggagtc cttttttaca caatgttggc tggctacact 1860
ccatttgcta atggccccaa tgatactcct gaagagatac tgctgcgtat aggcaatgga 1920
aaattctctt tgagtggtgg aaactgggac aatatttcag acggagcaaa ggatttgctt 1980
tcccatatgc ttcatatgga cccacatcag cggtatactg ctgaacaaat attaaagcac 2040
tcatggataa ctcacagaga ccagttgcca aatgatcagc caaagagaaa tgatgtgtca 2100
catgttgtta agggagcaat ggttgcaaca tactctgccc tgactcacaa gacctttcaa 2160
ccagtcctag agcctgtagc tgcttcaagc ttagcccagc gacggagcat gaaaaagcga 2220
acatcaactg gcctgtaa 2238

<210> 17
<211> 2640
<212> DNA
<213> homo sapiens

<220>
<221> misc_feature
<223> RSK4 mRNA

<400> 17
   acggtttttt tttttttttt tttttttttt tttttttttt tttttttttt ttttataaaa 60
   ttattagtat aaaaggggaa atgctaccat tcgctcctca ggacgagccc tgggaccgag 120
   aaatggaagt gttcagcggc ggcggcgcga gcagcggcga ggtaaatggt cttaaaatgg 180
   ttgatgagcc aatggaagag ggagaagcag attcttgtca tgatgaagga gttgttaaag 240
   aaatccctat tactcatcat gttaaggaag gctatgagaa agcagatcct gcacagtttg 300
   agttgctcaa ggttcttggt caggggtcat ttggaaaggt ttttcttgtt agaaagaaga 360
   ccggtcctga tgctgggcag ctctatgcaa tgaaggtgtt aaaaaaagcc tctttaaaag 420
   ttcgagacag agttcggaca aagatggaga gggatatact ggtggaagta aatcatccat 480
   ttattgtcaa attgcactat gcctttcaga ctgaagggaa actgtactta atactggatt 540
   ttctcagggg aggagatgtt ttcacaagat tatccaaaga ggttctgttt acagaggaag 600
   atgtgaaatt ctacctcgca gaactggccc ttgctttgga tcatctgcac caattaggaa 660
   ttgtttatag agacctgaag ccagaaaaca ttttgcttga tgaaatagga catatcaaat 720
   taacagattt tggactcagc aaggagtcag tagatcaaga aaagaaggct tactcatttt 780
   gtggtacagt agagtatatg gctcctgaag tagtaaatag gagaggccat tcccagagtg 840
   ctgattggtg gtcatatggt gttcttatgt ttgaaatgct tactggtact ctgccatttc 900
   aaggtaaaga cagaaatgag accatgaata tgatattaaa agcaaaactt ggaatgcctc 960
   aatttcttag tgctgaagca caaagtcttc taaggatgtt attcaaaagg aatccagcaa 1020
   atagattggg atcagaagga gttgaagaaa tcaaaagaca tctgtttttt gcaaatattg 1080
   actgggataa attatataaa agagaagttc aacctccttt caaacctgct tctggaaaac 1140
   cagatgatac tttttgtttt gatcctgaat ttactgcaaa aacacctaaa gattctcccg 1200
   gtttgccagc cagtgcaaat gctcatcagc tcttcaaagg attcagcttt gttgcaactt 1260
   ctattgcaga agaatataaa atcactccta tcacaagtgc aaatgtatta ccaattgttc 1320
   agataaatgg aaatgctgca caatttggtg aagtatatga attgaaggag gatattggtg 1380
   ttggctccta ctctgtttgc aagcgatgca tacatgcaac taccaacatg gaatttgcag 1440
   tgaagatcat tgacaaaagt aagcgagacc cttcagaaga gattgaaata ttgatgcgct 1500
   atggacaaca tcccaacatt attactttga aggatgtctt tgatgatggt agatatgttt 1560
   accttgttac ggatttaatg aaaggaggag agttacttga ccgtattctc aaacaaaaat 1620
   gtttctcgga acgggaggct agtgatatac tatatgtaat aagtaagaca gttgactatc 1680
   ttcattgtca aggagttgtt catcgtgatc ttaaacctag taatatttta tacatggatg 1740
   aatcagccag tgcagattca atcaggatat gtgattttgg gtttgcaaaa caacttcgag 1800
   gagaaaatgg acttctctta actccatgct acactgcaaa ctttgttgca cctgaggttc 1860
   ttatgcaaca gggatatgat gctgcttgtg atatctggag tttaggagtc cttttttaca 1920
   caatgttggc tggctacact ccatttgcta atggccccaa tgatactcct gaagagatac 1980
   tgctgcgtat aggcaatgga aaattctctt tgagtggtgg aaactgggac aatatttcag 2040
   acggagcaaa ggatttgctt tcccatatgc ttcatatgga cccacatcag cggtatactg 2100
   ctgaacaaat attaaagcac tcatggataa ctcacagaga ccagttgcca aatgatcagc 2160
   caaagagaaa tgatgtgtca catgttgtta agggagcaat ggttgcaaca tactctgccc 2220
   tgactcacaa gacctttcaa ccagtcctag agcctgtagc tgcttcaagc ttagcccagc 2280
   gacggagcat gaaaaagcga acatcaactg gcctgtaaga tttgtggtgt tcctaggcca 2340
   aactggatga agatgaaatt aaatgtgtgg ctttttccct attcttatca aaggcatcgt 2400
   tgtctgctaa attacttgaa tattaagtaa tattaaatcc ccatttttag gggaagtgag 2460
   atttaaaaaa ccattcacag gtccacaata ttcatactat gtgtttgcag tagtgttcaa 2520
   gtgtttattt aagcatataa ttggtgtcca ccaggtcctc acaacttctc tgcacacaag 2580
   cttctaaaat tcctttcaaa taaagttact ttaatattta aaaaaaaaaa aaaaaaaaaa 2640

<210> 18

<211> 745

<212> PRT

<213> homo sapiens

<220>

<221> misc_feature

<223> RSK4 gene product

<400> 18

Met Leu Pro Phe Ala Pro Gln Asp Glu Pro Trp Asp Arg Glu Met Glu
1               5                   10                  15

Val Phe Ser Gly Gly Gly Ala Ser Ser Gly Glu Val Asn Gly Leu Lys
            20                  25                  30

Met Val Asp Glu Pro Met Glu Glu Gly Glu Ala Asp Ser Cys His Asp
        35                  40                  45

Glu Gly Val Val Lys Glu Ile Pro Ile Thr His His Val Lys Glu Gly
    50                  55                  60

Tyr Glu Lys Ala Asp Pro Ala Gln Phe Glu Leu Leu Lys Val Leu Gly
65                  70                  75                  80

Gln Gly Ser Phe Gly Lys Val Phe Leu Val Arg Lys Lys Thr Gly Pro
                85                  90                  95

Asp Ala Gly Gln Leu Tyr Ala Met Lys Val Leu Lys Lys Ala Ser Leu
            100                 105                 110

Lys Val Arg Asp Arg Val Arg Thr Lys Met Glu Arg Asp Ile Leu Val
        115                 120                 125

Glu Val Asn His Pro Phe Ile Val Lys Leu His Tyr Ala Phe Gln Thr
    130                 135                 140

Glu Gly Lys Leu Tyr Leu Ile Leu Asp Phe Leu Arg Gly Gly Asp Val
145                 150                 155                 160

Phe Thr Arg Leu Ser Lys Glu Val Leu Phe Thr Glu Glu Asp Val Lys
                165                 170                 175

Phe Tyr Leu Ala Glu Leu Ala Leu Ala Leu Asp His Leu His Gln Leu
            180                 185                 190

Gly Ile Val Tyr Arg Asp Leu Lys Pro Glu Asn Ile Leu Leu Asp Glu
        195                 200                 205

Ile Gly His Ile Lys Leu Thr Asp Phe Gly Leu Ser Lys Glu Ser Val
    210                 215                 220

Asp Gln Glu Lys Lys Ala Tyr Ser Phe Cys Gly Thr Val Glu Tyr Met
225                 230                 235                 240

Ala Pro Glu Val Val Asn Arg Arg Gly His Ser Gln Ser Ala Asp Trp
                245                 250                 255

Trp Ser Tyr Gly Val Leu Met Phe Glu Met Leu Thr Gly Thr Leu Pro
            260                 265                 270

Phe Gln Gly Lys Asp Arg Asn Glu Thr Met Asn Met Ile Leu Lys Ala
        275                 280                 285

Lys Leu Gly Met Pro Gln Phe Leu Ser Ala Glu Ala Gln Ser Leu Leu
    290                 295                 300

Arg Met Leu Phe Lys Arg Asn Pro Ala Asn Arg Leu Gly Ser Glu Gly
305                 310                 315                 320

Val Glu Glu Ile Lys Arg His Leu Phe Phe Ala Asn Ile Asp Trp Asp
                325                 330                 335

Lys Leu Tyr Lys Arg Glu Val Gln Pro Pro Phe Lys Pro Ala Ser Gly
            340                 345                 350

Lys Pro Asp Asp Thr Phe Cys Phe Asp Pro Glu Phe Thr Ala Lys Thr
        355                 360                 365

Pro Lys Asp Ser Pro Gly Leu Pro Ala Ser Ala Asn Ala His Gln Leu
    370                 375                 380

Phe Lys Gly Phe Ser Phe Val Ala Thr Ser Ile Ala Glu Glu Tyr Lys
385                 390                 395                 400

Ile Thr Pro Ile Thr Ser Ala Asn Val Leu Pro Ile Val Gln Ile Asn
                405                 410                 415

Gly Asn Ala Ala Gln Phe Gly Glu Val Tyr Glu Leu Lys Glu Asp Ile
            420                 425                 430

Gly Val Gly Ser Tyr Ser Val Cys Lys Arg Cys Ile His Ala Thr Thr
        435                 440                 445

Asn Met Glu Phe Ala Val Lys Ile Ile Asp Lys Ser Lys Arg Asp Pro
    450                 455                 460

Ser Glu Glu Ile Glu Ile Leu Met Arg Tyr Gly Gln His Pro Asn Ile
465                 470                 475                 480

Ile Thr Leu Lys Asp Val Phe Asp Asp Gly Arg Tyr Val Tyr Leu Val
                485                 490                 495

Thr Asp Leu Met Lys Gly Gly Glu Leu Leu Asp Arg Ile Leu Lys Gln
            500                 505                 510

Lys Cys Phe Ser Glu Arg Glu Ala Ser Asp Ile Leu Tyr Val Ile Ser
        515                 520                 525

Lys Thr Val Asp Tyr Leu His Cys Gln Gly Val Val His Arg Asp Leu
    530                 535                 540

Lys Pro Ser Asn Ile Leu Tyr Met Asp Glu Ser Ala Ser Ala Asp Ser
545                 550                 555                 560

Ile Arg Ile Cys Asp Phe Gly Phe Ala Lys Gln Leu Arg Gly Glu Asn
                565                 570                 575

Gly Leu Leu Leu Thr Pro Cys Tyr Thr Ala Asn Phe Val Ala Pro Glu
            580                 585                 590

Val Leu Met Gln Gln Gly Tyr Asp Ala Ala Cys Asp Ile Trp Ser Leu
        595                 600                 605

Gly Val Leu Phe Tyr Thr Met Leu Ala Gly Tyr Thr Pro Phe Ala Asn
    610                 615                 620

Gly Pro Asn Asp Thr Pro Glu Glu Ile Leu Leu Arg Ile Gly Asn Gly
625                 630                 635                 640

Lys Phe Ser Leu Ser Gly Gly Asn Trp Asp Asn Ile Ser Asp Gly Ala
                645                 650                 655

Lys Asp Leu Leu Ser His Met Leu His Met Asp Pro His Gln Arg Tyr
            660                 665                 670

Thr Ala Glu Gln Ile Leu Lys His Ser Trp Ile Thr His Arg Asp Gln
        675                 680                 685

Leu Pro Asn Asp Gln Pro Lys Arg Asn Asp Val Ser His Val Val Lys
    690                 695                 700

Gly Ala Met Val Ala Thr Tyr Ser Ala Leu Thr His Lys Thr Phe Gln
705                 710                 715                 720

Pro Val Leu Glu Pro Val Ala Ala Ser Ser Leu Ala Gln Arg Arg Ser
                725                 730                 735

Met Lys Lys Arg Thr Ser Thr Gly Leu
            740                 745

<210>  19

<211>  2160

<212>  DNA

<213>  homo sapiens

<220>

<221>  misc_feature

<223>  PAK5 gene sequence made up of non-contigous segments of gene map
       locus 20p12

<400>  19
atgtttggga agaaaaagaa aaagattgaa atatctggcc cgtccaactt tgaacacagg      60

gttcatactg ggtttgatcc acaagagcag aagtttaccg gccttcccca gcagtggcac     120

agcctgttag cagatacggc caacaggcca aagcctatgg tggacccttc atgcatcaca     180

cccatccagc tggctcctat gaagacaatc gttagaggaa acaaaccctg caaggaaacc     240

tccatcaacg gcctgctaga ggattttgac aacatctcgg tgactcgctc caactcccta     300

aggaaagaaa gcccacccac cccagatcag ggagcctcca gccacggtcc aggccacgcg     360

gaagaaaatg gcttcatcac cttctcccag tattccagcg aatccgatac tactgctgac     420

tacacgaccg aaaagtacag ggagaagagt ctctatggag atgatctgga tccgtattat     480

agaggcagcc acgcagccaa gcaaaatggg cacgtaatga aaatgaagca cggggaggcc     540

tactattctg aggtgaagcc tttgaaatcc gattttgcca gattttctgc cgattatcac     600

tcacatttgg actcactgag caaaccaagt gaatacagtg acctcaagtg ggagtatcag     660

agagcctcga gtagctcccc tctggattat tcattccaat tcacaccttc tagaactgca     720

gggaccagcg ggtgctccaa ggagagcctg gcgtacagtg aaagtgaatg gggacccagc     780

ctggatgact atgacaggag gccaaagtct tcgtacctga atcagacaag ccctcagccc     840

accatgcggc agaggtccag gtcaggctcg ggactccagg aaccgatgat gccatttgga     900

gcaagtgcat ttaaaaccca tccccaagga cactcctaca actcctacac ctaccctcgc     960

ttgtccgagc ccacaatgtg cattccaaag gtggattacg atcgagcaca gatggtcctc    1020

agccctccac tgtcagggtc tgacacctac cccaggggcc ctgccaaact acctcaaagt    1080

caaagcaaat cgggctattc ctcaagcagt caccagtacc cgtctgggta ccacaaagcc    1140

accttgtacc atcacccctc cctgcagagc agttcgcagt acatctccac ggcttcctac    1200

ctgagctccc tcagcctctc atccagcacc tacccgccgc ccagctgggg ctcctcctcc 1260
gaccagcagc cctccagggt gtcccatgaa cagtttcggg cggccctgca gctggtggtc 1320
agcccaggag accccaggga atacttggcc aactttatca aaatcgggga aggctcaacc 1380
ggcatcgtat gcatcgccac cgagaaacac acagggaaac aagttgcagt gaagaaaatg 1440
gacctccgga agcaacagag acgagaactg cttttcaatg aggtcgtgat catgcgggat 1500
taccaccatg acaatgtggt tgacatgtac agcagctacc ttgtcggcga tgagctctgg 1560
gtggtcatgg agtttctaga aggtggtgcc ttgacagaca ttgtgactca caccagaatg 1620
aatgaagaac agatagctac tgtctgcctg tcagttctga gagctctctc ctaccttcat 1680
aaccaaggag tgattcacag ggacataaaa agtgactcca tcctcctgac aagcgatggc 1740
cggataaagt tgtctgattt tggtttctgt gctcaagttt ccaaagaggt gccgaagagg 1800
aaatcattgg ttggcactcc ctactggatg gcccctgagg tgatttctag gctaccttat 1860
gggacagagg tggacatctg gtccctcggg atcatggtga tagaaatgat tgatggcgag 1920
cccccctact tcaatgagcc tcccctccag gcgatgcgga ggatccggga cagtttacct 1980
ccaagagtga aggacctaca caaggtttct tcagtgctcc ggggattcct agacttgatg 2040
ttggtgaggg agccctctca gagagcaaca gcccaggaac tcctcggaca tccattctta 2100
aaactagcag gtccaccgtc ttgcatcgtc cccctcatga gacaatacag gcatcactga 2160

<210> 20
<211> 4655
<212> DNA
<213> homo sapiens

<220>
<221> misc_feature
<223> PAK5 mRNA

<400> 20
   cagtagtagt tccccagcgt gcgcccgggg agaccgggaa catggcgctg ggagcgctgt 60
   agcagctgag aaggggctga ggcaccgccg cttcgctgac agccggccac caggtttctg 120
   tctcccagta taatcatatc cagccccatg gctcttactg gggcctctga aatgtttatc 180
   accaaatctg tgtctgcagt tccaacctct tccctgagca tcaaagctgt atttccaact 240
   ttcgctggat gcttctatct ggaaatacac tgtggtgaaa tgcttccacc tcttgctaaa 300
   atgaacactg aggaaaaatg aagaagactg acaagcacca gcgaaaagtt gcagaataga 360
   aatagccaca ctcctctgga gtctttaatt catccacagc catcatataa aggttttggc 420
   atcatgtttg ggaagaaaaa gaaaaagatt gaaatatctg gcccgtccaa ctttgaacac 480
   agggttcata ctgggtttga tccacaagag cagaagttta ccggccttcc ccagcagtgg 540
   cacagcctgt tagcagatac ggccaacagg ccaaagccta tggtggaccc ttcatgcatc 600
   acacccatcc agctggctcc tatgaagaca atcgttagag gaaacaaacc ctgcaaggaa 660
   acctccatca acggcctgct agaggatttt gacaacatct cggtgactcg ctccaactcc 720
   ctaaggaaag aaagcccacc caccccagat cagggagcct ccagccacgg tccaggccac 780
   gcggaagaaa atggcttcat caccttctcc cagtattcca gcgaatccga tactactgct 840
   gactacacga ccgaaaagta cagggagaag agtctctatg gagatgatct ggatccgtat 900
   tatagaggca gccacgcagc caagcaaaat gggcacgtaa tgaaaatgaa gcacggggag 960
   gcctactatt ctgaggtgaa gcctttgaaa tccgattttg ccagattttc tgccgattat 1020
   cactcacatt tggactcact gagcaaacca agtgaataca gtgacctcaa gtgggagtat 1080
   cagagagcct cgagtagctc ccctctggat tattcattcc aattcacacc ttctagaact 1140
   gcagggacca gcgggtgctc caaggagagc ctggcgtaca gtgaaagtga atggggaccc 1200
   agcctggatg actatgacag gaggccaaag tcttcgtacc tgaatcagac aagccctcag 1260
   cccaccatgc ggcagaggtc caggtcaggc tcgggactcc aggaaccgat gatgccattt 1320
   ggagcaagtg catttaaaac ccatccccaa ggacactcct acaactccta cacctaccct 1380
   cgcttgtccg agcccacaat gtgcattcca aaggtggatt acgatcgagc acagatggtc 1440
   ctcagccctc cactgtcagg gtctgacacc taccccaggg gccctgccaa actacctcaa 1500
   agtcaaagca aatcgggcta ttcctcaagc agtcaccagt acccgtctgg gtaccacaaa 1560
   gccaccttgt accatcaccc ctccctgcag agcagttcgc agtacatctc cacggcttcc 1620
   tacctgagct ccctcagcct ctcatccagc acctacccgc cgcccagctg gggctcctcc 1680
   tccgaccagc agccctccag ggtgtcccat gaacagtttc gggcggccct gcagctggtg 1740
   gtcagcccag gagaccccag ggaatacttg gccaacttta tcaaaatcgg ggaaggctca 1800
   accggcatcg tatgcatcgc caccgagaaa cacacaggga aacaagttgc agtgaagaaa 1860
   atggacctcc ggaagcaaca gagacgagaa ctgcttttca atgaggtcgt gatcatgcgg 1920
   gattaccacc atgacaatgt ggttgacatg tacagcagct accttgtcgg cgatgagctc 1980
   tgggtggtca tggagtttct agaaggtggt gccttgacag acattgtgac tcacaccaga 2040
   atgaatgaag aacagatagc tactgtctgc ctgtcagttc tgagagctct ctcctacctt 2100
   cataaccaag gagtgattca cagggacata aaaagtgact ccatcctcct gacaagcgat 2160
   ggccggataa agttgtctga ttttggtttc tgtgctcaag tttccaaaga ggtgccgaag 2220
   aggaaatcat tggttggcac tccctactgg atggcccctg aggtgatttc taggctacct 2280
   tatgggacag aggtggacat ctggtccctc gggatcatgg tgatagaaat gattgatggc 2340
   gagcccccct acttcaatga gcctcccctc caggcgatgc ggaggatccg ggacagttta 2400
   cctccaagag tgaaggacct acacaaggtt tcttcagtgc tccggggatt cctagacttg 2460
   atgttggtga gggagccctc tcagagagca acagcccagg aactcctcgg acatccattc 2520
   ttaaaactag caggtccacc gtcttgcatc gtccccctca tgagacaata caggcatcac 2580
   tgagcagagg attcgtgtag gtggcaaagc tagatgagga catgagaata attcaggaga 2640
   acaaaaggaa acacagaaca tgcaaaaggc ctgtgcattc tagaccagcc aattggtggg 2700
   acagcgtgat gaccggcagg gttcaacaga ccagggcatc ttcttgtgtc ttaaacaggc 2760
   atctctccac tgacagccgg tgtggtcact tggagcacgg ctttaataag tcattattat 2820
   atttttcagc ccttcatcca gcaaatcaga aggactcagt acaaactccg ttatgatata 2880
   tcctagccac atgcagggta acatgtagga ttttctatat tgaaagaata cttttctggc 2940
   aaaaaaaaaa aaaaaagaaa gaaaggaaaa caaaaagcac tttttcttta atggtagcag 3000
   tataatgtat tttgcaacga atttgtaatt tttctgtacg atagttttga taatttatag 3060
   tactttgatg tcatgtagcc attgtatcag ttgaagtaat acttgtttac tagaggagtt 3120
   tgaacaaagc ctttcctact tttttatccc tttaagagaa ccaatgattc tttaggaact 3180
   ttgaatactg aatgactctc aatcaccgtc agctttagta aaatctcttt cttatcctaa 3240
   caagtgtctt atttggtgga agaagaatta agagtgatgg tgatggtgtg cacgtttcat 3300
   taatccaacc aaaaataatg aaataaaatt tgagccacag tataccactc cttgggataa 3360
   agttaaatat ttttaaagat cacattttcc atgaacgcct ctagtagcaa accattcttt 3420
   tgcacaccac aatgtttccc tcagtgccct ttctcaaatg ggtacaatgt tcccttgtgg 3480
   ccaaatttcc ctcccaggga gcaatttcag tgctaggatc attggattca gttcccaaaa 3540
   tagaatgttt cagtgagacc atgagaattc caggctcaca aagggagagg agagaacagg 3600
   gcaagacgtt tggtttcatt tgtcaccatt tttaaaactc tgtatgctag cacaccaaac 3660
   tcttgtctat atttaccttt gtaccacagt attaatcgct attgttcatg tatcgtgctg 3720
   gaagtctgaa ctgactctag aggatgaatt agcaagaggg tattttacca ggtatgatct 3780
   gacttcagtt gtgcccatgt tataatgtgt ttccgacata ggagagtcgt gctgctgtct 3840
   agatcttctt gaatgttgat aaaaatgaat gactactaca atacattttg tgttgcttgt 3900
   tggatgaatt tgcatgttaa ctgtaggcca atatagattt gcctttaaaa ctctggaaga 3960
   gctacatagt catcattagt ttctattaat tatgcatcag acaaaagcca tttgttacca 4020
   aactgggaaa acagaggctt ttcttaacta tttcacatac tgtaacaaat atgaatttaa 4080
   atttgtgata gcgctctggt tgctctaagc ataattaaga atttttgtaa ttaataggtt 4140
   gctaattatt tatcactgct aaaaaggaaa aaaggcataa aatgaccttc tactgattag 4200
   attttcagtt ttctttcaaa ctggaaatgc ctccataaat atgatctatg attttgcttc 4260
   ataaaacagc aaatcaatgt tttatgtaaa atattaaagc attaatataa atatgtgaga 4320
   ataaaaacaa tctaaatcca gaaaatggca gtcctaaatg ttcatgagac agattgtatt 4380
   aatttaacca ggactatgta gaagtagaaa gaaaagaaaa agaaaatctt ttttaaacca 4440
   gaataaacat taaaaactat tgcagaaaat agtggatttt ggattccaaa cattttcgac 4500
   agtgtaatgg aaattttctt gtaattttct taccatcggg tattttttaa agtattcatt 4560
   gagtttacca aaagttactg tagcttaaaa ggttttgtga gcactaacta ttggcagaaa    4620
   ctgcatttgc aaataaaaat aaatgtttgc ctttt                               4655

<210>  21

<211>  719

<212>  PRT

<213>  homo sapiens

<220>

<221>  misc_feature

<223>  PAK5 gene product

<400>  21

Met Phe Gly Lys Lys Lys Lys Lys Ile Glu Ile Ser Gly Pro Ser Asn
1               5                   10                  15

Phe Glu His Arg Val His Thr Gly Phe Asp Pro Gln Glu Gln Lys Phe
            20                  25                  30

Thr Gly Leu Pro Gln Gln Trp His Ser Leu Leu Ala Asp Thr Ala Asn
        35                  40                  45

Arg Pro Lys Pro Met Val Asp Pro Ser Cys Ile Thr Pro Ile Gln Leu
    50                  55                  60

Ala Pro Met Lys Thr Ile Val Arg Gly Asn Lys Pro Cys Lys Glu Thr
65                  70                  75                  80

Ser Ile Asn Gly Leu Leu Glu Asp Phe Asp Asn Ile Ser Val Thr Arg
                85                  90                  95

Ser Asn Ser Leu Arg Lys Glu Ser Pro Pro Thr Pro Asp Gln Gly Ala
            100                 105                 110

Ser Ser His Gly Pro Gly His Ala Glu Glu Asn Gly Phe Ile Thr Phe
        115                 120                 125

Ser Gln Tyr Ser Ser Glu Ser Asp Thr Thr Ala Asp Tyr Thr Thr Glu
    130                 135                 140

Lys Tyr Arg Glu Lys Ser Leu Tyr Gly Asp Asp Leu Asp Pro Tyr Tyr
145                 150                 155                 160

Arg Gly Ser His Ala Ala Lys Gln Asn Gly His Val Met Lys Met Lys
                165                 170                 175

His Gly Glu Ala Tyr Tyr Ser Glu Val Lys Pro Leu Lys Ser Asp Phe
            180                 185                 190

Ala Arg Phe Ser Ala Asp Tyr His Ser His Leu Asp Ser Leu Ser Lys
        195                 200                 205

Pro Ser Glu Tyr Ser Asp Leu Lys Trp Glu Tyr Gln Arg Ala Ser Ser
    210                 215                 220

Ser Ser Pro Leu Asp Tyr Ser Phe Gln Phe Thr Pro Ser Arg Thr Ala
225                 230                 235                 240

Gly Thr Ser Gly Cys Ser Lys Glu Ser Leu Ala Tyr Ser Glu Ser Glu
                245                 250                 255

Trp Gly Pro Ser Leu Asp Asp Tyr Asp Arg Arg Pro Lys Ser Ser Tyr
            260                 265                 270

Leu Asn Gln Thr Ser Pro Gln Pro Thr Met Arg Gln Arg Ser Arg Ser
        275                 280                 285

Gly Ser Gly Leu Gln Glu Pro Met Met Pro Phe Gly Ala Ser Ala Phe
    290                 295                 300

Lys Thr His Pro Gln Gly His Ser Tyr Asn Ser Tyr Thr Tyr Pro Arg
305                 310                 315                 320

Leu Ser Glu Pro Thr Met Cys Ile Pro Lys Val Asp Tyr Asp Arg Ala
                325                 330                 335

Gln Met Val Leu Ser Pro Pro Leu Ser Gly Ser Asp Thr Tyr Pro Arg
            340                 345                 350

Gly Pro Ala Lys Leu Pro Gln Ser Gln Ser Lys Ser Gly Tyr Ser Ser
        355                 360                 365

Ser Ser His Gln Tyr Pro Ser Gly Tyr His Lys Ala Thr Leu Tyr His
    370                 375                 380

His Pro Ser Leu Gln Ser Ser Ser Gln Tyr Ile Ser Thr Ala Ser Tyr
385                 390                 395                 400

Leu Ser Ser Leu Ser Leu Ser Ser Ser Thr Tyr Pro Pro Pro Ser Trp
                405                 410                 415

Gly Ser Ser Ser Asp Gln Gln Pro Ser Arg Val Ser His Glu Gln Phe
            420                 425                 430

Arg Ala Ala Leu Gln Leu Val Val Ser Pro Gly Asp Pro Arg Glu Tyr
        435                 440                 445

Leu Ala Asn Phe Ile Lys Ile Gly Glu Gly Ser Thr Gly Ile Val Cys
    450                 455                 460

Ile Ala Thr Glu Lys His Thr Gly Lys Gln Val Ala Val Lys Lys Met
465                 470                 475                 480

Asp Leu Arg Lys Gln Gln Arg Arg Glu Leu Leu Phe Asn Glu Val Val
                485                 490                 495

Ile Met Arg Asp Tyr His His Asp Asn Val Val Asp Met Tyr Ser Ser
            500                 505                 510

Tyr Leu Val Gly Asp Glu Leu Trp Val Val Met Glu Phe Leu Glu Gly
        515                 520                 525

Gly Ala Leu Thr Asp Ile Val Thr His Thr Arg Met Asn Glu Glu Gln
    530                 535                 540

Ile Ala Thr Val Cys Leu Ser Val Leu Arg Ala Leu Ser Tyr Leu His
545                 550                 555                 560

Asn Gln Gly Val Ile His Arg Asp Ile Lys Ser Asp Ser Ile Leu Leu
                565                 570                 575

Thr Ser Asp Gly Arg Ile Lys Leu Ser Asp Phe Gly Phe Cys Ala Gln
            580                 585                 590

Val Ser Lys Glu Val Pro Lys Arg Lys Ser Leu Val Gly Thr Pro Tyr
        595                 600                 605

Trp Met Ala Pro Glu Val Ile Ser Arg Leu Pro Tyr Gly Thr Glu Val
    610                 615                 620

Asp Ile Trp Ser Leu Gly Ile Met Val Ile Glu Met Ile Asp Gly Glu
625                 630                 635                 640

Pro Pro Tyr Phe Asn Glu Pro Pro Leu Gln Ala Met Arg Arg Ile Arg
                645                 650                 655

Asp Ser Leu Pro Pro Arg Val Lys Asp Leu His Lys Val Ser Ser Val
            660                 665                 670

Leu Arg Gly Phe Leu Asp Leu Met Leu Val Arg Glu Pro Ser Gln Arg
        675                 680                 685

Ala Thr Ala Gln Glu Leu Leu Gly His Pro Phe Leu Lys Leu Ala Gly
    690                 695                 700

Pro Pro Ser Cys Ile Val Pro Leu Met Arg Gln Tyr Arg His His
705                 710                 715

<210> 22

<211> 3936

<212> DNA

<213> homo sapiens

<220>

<221> misc_feature

<223> FMR2 gene sequence made up of non-contigous segments of gene map
      locus Xq28

<400> 22
atggatctat tcgacttttt cagagactgg gacttggagc agcagtgtca ctatgaacaa      60

gaccgtagtg cacttaaaaa aagggaatgg gagcggagga atcaagaagt ccagcaagaa     120

gacgatctct tttcttcagg ctttgatctt tttggggagc catacaaggt agctgaatat     180

acaaacaaag gtgatgcact tgccaaccga gtccagaaca cgcttggaaa ctatgatgaa     240

atgaagaatt tgctaactaa ccattctaat cagaatcacc tagtgggaat tccaaagaat     300

tctgtgcccc agaatcccaa caacaaaaat gaaccaagct tttttccaga acaaaagaac     360

agaataattc cacctcacca ggataatacc catccttcag caccaatgcc tccaccttct     420

gttgtgatac tgaattcaac tctaatacac agcaacagaa aatcaaaacc tgagtggtca     480

cgtgatagtc ataaccctag cactgtactg gcaagccagg ccagtggtca gccaaacaag     540

atgcagactt tgacacagga ccagtctcaa gccaaactgg aagacttctt tgtctaccca     600

gctgaacagc cccagattgg agaagttgaa gagtcaaacc catctgcaaa ggaagacagt     660

aaccctaatt ctagtggaga agatgctttc aaagaaatct ttcaatccaa ttcaccggaa     720

gaatctgaat tcgccgtgca agcgcctggg tctcccctag tggcttcctc tttattagct     780

cctagcagtg gcctttcagt tcaaaacttc ccaccagggc tttactgcaa aacaagcatg     840

gggcagcaaa agccaactgc atacgtcaga cccatggatg gccaggacca ggcaccggac     900

atctcaccaa cactgaaacc ttcaattgaa tttgagaaca gctttgggaa tctgtcattt     960

ggaacactct tggatggaaa acccagtgca gccagttcaa agactaaact gccaaagttc    1020

accatcctcc aaacaagtga agtaagcctt cccagtgatc caagctgtgt tgaagaaatc    1080

ttgcgggaga tgacccattc ctggcctact cctctcactt ccatgcatac tgctggacac    1140

tctgagcaga gcaccttttc catcccagga caggaatcgc agcatctgac cccaggattc    1200

accttacaaa agtggaatga cccaaccacc agagcttcta caaagtcagt gtctttcaaa    1260

tcgatgcttg aggatgacct gaagctgagc agtgatgaag atgaccttga gcctgtgaag    1320

accttgacca ctcagtgcac tgccactgag ctctaccagg ctgttgaaaa ggcaaaacct    1380

aggaataatc ctgtgaaccc acccttggcc actccccagc ccccacctgc agtgcaagcc    1440

agcgggggtt ctggcagctc cagcgaatcg gagagcagct ctgagtcgga ttcagacact 1500
gaaagtagca ccactgacag cgaatctaat gaggcacctc gtgtggcaac tccagagcct 1560
gagccaccct caaccaacaa gtggcaactg gataaatggc ttaacaaagt gacatcccag 1620
aacaagtctt ttatttgtgg cccaaatgaa acacccatgg agactatttc tctgcctcct 1680
ccaatcatcc aaccaatgga agtccagatg aaagtgaaga cgaatgccag tcaggtccca 1740
gctgaaccca aagaaaggcc tctcctcagt ctcattaggg agaaagcccg tccacggccc 1800
actcagaaaa ttccagaaac aaaggctttg aagcataagt tgtcaacaac tagtgagaca 1860
gtgtctcaaa ggacaattgg gaaaaaacag cccaaaaaag ttgagaagaa caccagcact 1920
gacgagttta cctggcccaa accaaatatt accagcagca ctcccaaaga aaaagaaagt 1980
gtggagcttc atgacccacc aagaggccgc aacaaagcca ctgcccacaa accagcccct 2040
aggaaagaac caagacctaa catccctttg gctcccgaga agaagaagta cagagggcct 2100
ggcaagattg tgccaaagtc tcgggaattc attgaaacag attcatctac atctgactcc 2160
aacacagatc aggaagagac cctgcaaatc aaagtcctgc ctccgtgcat tatttctgga 2220
ggtaatactg ccaaatccaa ggaaatctgt ggtgccagcc tgaccctcag caccttaatg 2280
agtagcagtg gcagcaacaa caacttatcc atcagtaatg aagagccaac attttcacct 2340
attcctgtca tgcaaactga aatcctgtcc cctctgcgag atcatgagaa cctgaaaaac 2400
ctctgggtga agattgacct tgacttactc tctagagtac ctggccacag ctcactccat 2460
gcagcacctg ccaagccaga ccacaaggag actgccacaa aacccaagcg tcagacagct 2520
gtcacagctg tggagaaacc agcccctaag ggcaaacgta agcacaagcc aatagaagtt 2580
gcagagaaga tccctgagaa gaagcagcgc ctggaggagg ccacaactat ctgcttgctc 2640
cctccttgca tctcaccagc cccaccccac aagcctccca acactagaga aaataattca 2700
tccaggagag caaatagaag aaaggaagaa aaactatttc ctcctccact ttccccactg 2760
ccagaggacc ctccacgccg cagaaatgtc agtggcaata atggtccctt tggtcaagac 2820
aaaaacatcg ccatgactgg acaaatcaca tctaccaaac ctaagagaac tgaaggcaaa 2880
ttctgtgcta ctttcaaagg gatatcggta aatgagggag acactccaaa aaaggcatcc 2940
tctgccacca tcactgtcac caatactgct attgccactg ctactgtcac tgctactgcc 3000
attgtcacca ccactgtcac agctactgcc accgccacgg ccaccaccac aactactacc 3060
actaccattt ccaccatcac ctctaccatc actactggcc tcatggatag cagtcacctg 3120
gagatgacgt cctgggcggc tctgcccctt ctatccagca gcagcactaa tgtccggaga 3180
cccaagctca cttttgatga ctcggttcac aatgctgatt attacatgca agaagctaag 3240
aagctgaagc acaaagctga tgcactgttc gagaaatttg gcaaagctgt gaattatgct 3300
gatgccgccc tctccttcac tgaatgtggc aatgccatgg aacgcgaccc tctggaagca 3360
aagtccccat acaccatgta ctctgagact gtggagctcc tcaggtatgc aatgaggctg 3420
aagaactttg caagtccctt ggcttcggat ggggacaaaa agctagcagt actatgctac 3480

cgatgtttat cactcctcta tttgagaatg tttaagctga agaaggacca tgctatgaag 3540
tactccagat cactgatgga atattttaag caaaatgctt caaaagtcgc acagataccc 3600
tctccatggg taagcaatgg aaagaacact ccatccccag tgtctctcaa caacgtctcc 3660
cccatcaacg caatggggaa ctgtaacaat ggcccagtca ccattcccca gcgcattcac 3720
cacatggctg ccagccacgt caacatcact agcaatgtgt tacggggcta tgaacactgg 3780
gatatggccg acaaactgac aagagaaaac aaagaattct ttggtgatct ggacacgctg 3840
atggggcctc tgacccagca cagcagcatg accaatcttg tccgctacgt tcgccaagga 3900
ctgtgttggc tgcgcatcga tgcccacttg ttgtag 3936

<210> 23
<211> 13695
<212> DNA
<213> homo sapiens

<220>
<221> misc_feature
<223> FMR2 mRNA

<400> 23
cgccgcctgt gcagccgctg ccgccgccgc cgccgccgcc gccgccgccg ccgccgccgc 60
cgccgctgcc gccccggctg ccgcgccgcg ccgctgcctc tgccccggcc gcccccgccg 120
ccgctgccgc cgccggcccg cagccagcca ggcgggcggc ccagcccgcc tgagcccgca 180
gcggctgccg ccgcagcgtc gggtcgctgg gtgcgcgggc taccgcggac cgagcggacc 240
cgagtgggcg accaggcgct tgcccgccca gtgccactgc cgccgcttcc tcgccggagc 300
acaggaccag acacctccag cgcccgctgc tgctgccgat gcggcccgga cacttttagc 360
tgggcgggag ggctggagag ccgggggccg ccgagaaccg ccagcgagct gtgccgagag 420
ccgcgccgac ccgctgcgat cagggacagg cgcccgcccg ccgccgccgc ctggccgcta 480
tggatctatt cgactttttc agagactggg acttggagca gcagtgtcac tatgaacaag 540
accgtagtgc acttaaaaaa agggaatggg agcggaggaa tcaagaagtc cagcaagaag 600
acgatctctt ttcttcaggc tttgatcttt ttggggagcc atacaaggta gctgaatata 660
caaacaaagg tgatgcactt gccaaccgag tccagaacac gcttggaaac tatgatgaaa 720
tgaagaattt gctaactaac cattctaatc agaatcacct agtgggaatt ccaaagaatt 780
ctgtgcccca gaatcccaac aacaaaaatg aaccaagctt ttttccagaa caaaagaaca 840
gaataattcc acctcaccag gataataccc atccttcagc accaatgcct ccaccttctg 900
ttgtgatact gaattcaact ctaatacaca gcaacagaaa atcaaaacct gagtggtcac 960

gtgatagtca taaccctagc actgtactgg caagccaggc cagtggtcag ccaaacaaga 1020
tgcagacttt gacacaggac cagtctcaag ccaaactgga agacttcttt gtctacccag 1080
ctgaacagcc ccagattgga gaagttgaag agtcaaaccc atctgcaaag gaagacagta 1140
accctaattc tagtggagaa gatgctttca aagaaatctt tcaatccaat tcaccggaag 1200
aatctgaatt cgccgtgcaa gcgcctgggt ctcccctagt ggcttcctct ttattagctc 1260
ctagcagtgg cctttcagtt caaaacttcc caccagggct ttactgcaaa acaagcatgg 1320
ggcagcaaaa gccaactgca tacgtcagac ccatggatgg ccaggaccag gcaccggaca 1380
tctcaccaac actgaaacct tcaattgaat ttgagaacag ctttgggaat ctgtcatttg 1440
gaacactctt ggatggaaaa cccagtgcag ccagttcaaa gactaaactg ccaaagttca 1500
ccatcctcca aacaagtgaa gtaagccttc ccagtgatcc aagctgtgtt gaagaaatct 1560
tgcgggagat gacccattcc tggcctactc ctctcacttc catgcatact gctggacact 1620
ctgagcagag caccttttcc atcccaggac aggaatcgca gcatctgacc ccaggattca 1680
ccttacaaaa gtggaatgac ccaaccacca gagcttctac aaagtcagtg tctttcaaat 1740
cgatgcttga ggatgacctg aagctgagca gtgatgaaga tgaccttgag cctgtgaaga 1800
ccttgaccac tcagtgcact gccactgagc tctaccaggc tgttgaaaag gcaaaaccta 1860
ggaataatcc tgtgaaccca cccttggcca ctccccagcc cccacctgca gtgcaagcca 1920
gcgggggttc tggcagctcc agcgaatcgg agagcagctc tgagtcggat tcagacactg 1980
aaagtagcac cactgacagc gaatctaatg aggcacctcg tgtggcaact ccagagcctg 2040
agccaccctc aaccaacaag tggcaactgg ataaatggct taacaaagtg acatcccaga 2100
acaagtcttt tatttgtggc ccaaatgaaa cacccatgga gactatttct ctgcctcctc 2160
caatcatcca accaatggaa gtccagatga aagtgaagac gaatgccagt caggtcccag 2220
ctgaacccaa agaaaggcct ctcctcagtc tcattaggga gaaagcccgt ccacggccca 2280
ctcagaaaat tccagaaaca aaggctttga agcataagtt gtcaacaact agtgagacag 2340
tgtctcaaag gacaattggg aaaaaacagc ccaaaaaagt tgagaagaac accagcactg 2400
acgagtttac ctggcccaaa ccaaatatta ccagcagcac tcccaaagaa aaagaaagtg 2460
tggagcttca tgacccacca agaggccgca acaaagccac tgcccacaaa ccagccccta 2520
ggaaagaacc aagacctaac atccctttgg ctcccgagaa gaagaagtac agagggcctg 2580
gcaagattgt gccaaagtct cgggaattca ttgaaacaga ttcatctaca tctgactcca 2640
acacagatca ggaagagacc ctgcaaatca aagtcctgcc tccgtgcatt atttctggag 2700
gtaatactgc caaatccaag gaaatctgtg gtgccagcct gaccctcagc accttaatga 2760
gtagcagtgg cagcaacaac aacttatcca tcagtaatga agagccaaca ttttcaccta 2820
ttcctgtcat gcaaactgaa atcctgtccc ctctgcgaga tcatgagaac ctgaaaaacc 2880
tctgggtgaa gattgacctt gacttactct ctagagtacc tggccacagc tcactccatg 2940
cagcacctgc caagccagac cacaaggaga ctgccacaaa acccaagcgt cagacagctg 3000

tcacagctgt ggagaaacca gcccctaagg gcaaacgtaa gcacaagcca atagaagttg 3060
cagagaagat ccctgagaag aagcagcgcc tggaggaggc cacaactatc tgcttgctcc 3120
ctccttgcat ctcaccagcc ccaccccaca agcctcccaa cactagagaa aataattcat 3180
ccaggagagc aaatagaaga aaggaagaaa aactatttcc tcctccactt tccccactgc 3240
cagaggaccc tccacgccgc agaaatgtca gtggcaataa tggtcccttt ggtcaagaca 3300
aaaacatcgc catgactgga caaatcacat ctaccaaacc taagagaact gaaggcaaat 3360
tctgtgctac tttcaaaggg atatcggtaa atgagggaga cactccaaaa aaggcatcct 3420
ctgccaccat cactgtcacc aatactgcta ttgccactgc tactgtcact gctactgcca 3480
ttgtcaccac cactgtcaca gctactgcca ccgccacggc caccaccaca actactacca 3540
ctaccatttc caccatcacc tctaccatca ctactggcct catggatagc agtcacctgg 3600
agatgacgtc ctgggcggct ctgccccttc tatccagcag cagcactaat gtccggagac 3660
ccaagctcac ttttgatgac tcggttcaca atgctgatta ttacatgcaa gaagctaaga 3720
agctgaagca caaagctgat gcactgttcg agaaatttgg caaagctgtg aattatgctg 3780
atgccgccct ctccttcact gaatgtggca atgccatgga acgcgaccct ctggaagcaa 3840
agtccccata caccatgtac tctgagactg tggagctcct caggtatgca atgaggctga 3900
agaactttgc aagtcccttg gcttcggatg gggacaaaaa gctagcagta ctatgctacc 3960
gatgtttatc actcctctat ttgagaatgt ttaagctgaa gaaggaccat gctatgaagt 4020
actccagatc actgatggaa tattttaagc aaaatgcttc aaaagtcgca cagataccct 4080
ctccatgggt aagcaatgga aagaacactc catccccagt gtctctcaac aacgtctccc 4140
ccatcaacgc aatggggaac tgtaacaatg gcccagtcac cattccccag cgcattcacc 4200
acatggctgc cagccacgtc aacatcacta gcaatgtgtt acggggctat gaacactggg 4260
atatggccga caaactgaca agagaaaaca aagaattctt tggtgatctg gacacgctga 4320
tggggcctct gacccagcac agcagcatga ccaatcttgt ccgctacgtt cgccaaggac 4380
tgtgttggct gcgcatcgat gcccacttgt tgtagtgggt gttctcagat ctctagcatc 4440
acgacccatc actctacctc taccagcgca ctgatggtca ctggtggaac tccactcact 4500
ggggaacgtt ctctttggtt atgtttgttt ttatgcttct tttgttatct gtaaaaaaca 4560
gaagtcattg taagttgaca ctacaactta agggcagtgt acgttttatt acttagtcat 4620
tttttttctt ttagcatttg atatgcattt ctcagattcc accatctttt tgtgctttat 4680
ggaatgacag tccctacaat attgttttaa gcccacacta cccaaaacaa agaatgggaa 4740
gcacttgtga taaagacagg ctcctgagaa atgcaacaag tggtcttaca tatacatgag 4800
aacttagaca caagggacca tcccccaaac tctactctta tacccagaaa agaacatatt 4860
tcagaatctg tcaaactttt gtgtatccca cagattcaat cttcaggtga gaattttcat 4920
tgtcaaaacc cactggttag atgttgtagc aacatcataa aatcaagagt atcaagaaaa 4980
taaatgagca tagcaatgct actcttaaaa agatgctatg ccacacaacc agaggacttt 5040

cttgttagca tccctttcct gattccctat tttgttaatt ttaatgataa gaagaaaggg 5100
tgacatttat tttgacaagt tttaggcatc agctggcatc agtgtttttc aactccatta 5160
tttgaagtgt aaatcctcac ctggggttct ctgtgtgcaa agctgtcctt ttgaagaaca 5220
gtttggttga tgcatgcctt agtagccaaa atgctacact ctagacttac aagtgggagt 5280
taagagaggt ctggaaagtg tccaacaagg aattcacacc tctgcctcct ttgcaacaac 5340
aacatttaca cagttggtaa gtgggtccat aactggcagg atttttaaat tgtattttgc 5400
tcaaatctat gggaacaaaa gtcaaggtat cactacctag aagtaatgat atacagtttt 5460
cttcctagtg gcttgaaaat ctggacttcc tcaattatta ttcacatttt ctctcttata 5520
ggttttctgt tttctacttt cttttttctc ttatctgtgt ttccctttcc tttgtttggc 5580
tcattaactt ttgactgaat tacaattact ccttttatta aagtccatat tattgtgaat 5640
catttccatg aaaatttcta agaaaactcc aaactctcta aatagtagct aacttttatt 5700
tttttaaaat gagtcgtggg gtagtgcttc accttgagat gctttgaaag agccctaaac 5760
attgggaacc attcacctaa tttggagaca tttctcactg gttgtgacta cccccttatg 5820
atccttcaca ttcattttat gtccctaaac atcacaatgt aaatatcatt tttgatgttc 5880
cagctcacca gaagattctt acacttgggg taaacactat ccatgcatta cttactggta 5940
attacctgct ggtatataat tccatgtagc ctttaatatg ctgggttatc aaattctgtt 6000
cactgagtta tgaccagata aataatagat atgcacatga aagatgcaaa cttgtgtgat 6060
tattaaagcc agccatgcag gtccatgata gaaacagcag gtgatgactc tgcactctca 6120
ttgtcaaggt tagctatatc cccagttgca aaacagccag acttgagctg tgctctggtc 6180
atctttgagt ttaaggcctt ttgttgtata aggctgtgga agttgtactc caatggctga 6240
agccatgttg ttaatatggc tgatgggagc atccctgcag ctgaacccag cactttttat 6300
gctcccactg tggttgagct ttatgtttac agtctcagca acaacactta tgcatccaaa 6360
cactcacaaa tgaaacctga aagaatcttt tctgagcctc ttaaaagagg aaaatgatga 6420
taacattaaa gactctgaac acccaaggtt ggtgtcacat ataaaaatta agctgatgac 6480
tttgcagtga ctcaagttgt ctctttatca tggtttacca ggtagagtgc ctggctatta 6540
ctatataatg aagcccactg gcttgacttg taagttcaac ctaaaccaca atcctagacc 6600
atcatggatt taggagtaga ttcttcttga aatcccacat ccagaaacta gacattagaa 6660
tgttgaggca gtttcccaga gaaacaagca tattgcctca tggatgaaag acttgtagtt 6720
ctagtttcag tgacttgtta tatctactta catacaacag ggaggcaaga ggattctctg 6780
tcatctctgg tgactgagtg taaaatatgt gccaagtctg cagcacagtg accaaatctg 6840
acaatcgagc tctggatcac cacttgatta tgtagtagac tcatttataa agcagcttag 6900
gaactaatta aacatggagg atgaattacc ttcctatccc ttgagataag acatctttca 6960
gtttcatgat taaggattgt tgctgtttta tagttactct gttcatcaca gtgtaaatgg 7020
tgatgcgtgt cgtaggtgtg cagctatttg agggactaag ggatggagat attctgtcaa 7080

atgaatctct tcagtatacc agtttgtggg agggatatga gacatgtgga tggcagtgag 7140
agatcgtgcc tctagatctt gatggaggct tggtgagaca cacttaaata agcacgtgga 7200
ggttagaata gagggcagag taaaaggaag ctccatctga gcaagtacac caaatgatct 7260
cagccctgca acttgaccca ggtagggcca ccactacgcc ttcacttgtc acccaagctc 7320
caaccacaga gagtttgaca agtttgtgtt atgatgttgg cttggctttg tatttttaat 7380
taactttgga tttttagtgg ttttgtcata taactgtctg agtttggtag gtaggattac 7440
tttgaaaagg gtttactagt gtggtcctcc gggtagaatt tagctgtaac atgttgttag 7500
ccagcctgta gactgttaat tacttaataa tctcattggg aaaatactag tagttttata 7560
tttggatgac ataattggaa aaagcagatt agctgctact acttttaaaa gacttaaggt 7620
cgggatgcct ttttttccat gtaaggaaat gaaaagaccc aaaatcttca ggcaaaaagc 7680
aagttgcaaa attagaaacc attggctaaa aatgtgtttt gttgagtttc caaatggatg 7740
aattttcatt tggacattac atcactaaat tcattagatt ttgtctgcat tggaaagata 7800
ctcttctagc atatctttcc caaagatatc taatttggat tctgtttcat gcaaatttgc 7860
atcccggagg ttgaagttgg agtttgaggt tggaaaatat ctttgaaggc agaatcagtt 7920
gagttgtgag ggtgaagcct cacatacttc tcaacagaca tgataaaatt cacctgcatg 7980
agttggcagg tgggagaacc aaactggatc actgggtaag actactcagt aaagcaatga 8040
actgcttgct tagagaagca tcactatccc cattgagaaa aatgtgtggc aagatgatac 8100
agctacacag tatcaaatga atgggtcaat tcagcacccc caaatttaat tctgtgggga 8160
aaaattattg agccagttgt cagtgttctg ttacatgact ggcagactaa attcttcatc 8220
gttgttgtta ttgttgttgt tgtttctcat tttcactcgc acggccttat tctcataatt 8280
aaaatctaat tcattttctc tttagtgtta gtagactcca acaacagaag tggcatctgt 8340
gtattcataa tcagcattta ccctggcagg agactaatca gataggccgg tctcagacat 8400
taatcctacc atctgatatt tttggtgaag gaaaaagtat taattctctt tccatcctcc 8460
tcctcagaaa tatagaagcc ctctttacca aaatcatcac attttactct gtaatctacc 8520
agctaaaaga aaattgcatt gaagccccac aaagccagat tgcagttctt gccccttttt 8580
gcgtctgaca tgagatgtta aagaattatt cattgtgctc acattgggtt aggggacact 8640
gaactgcttt ttagatccat gatcagtcat cattcttcta agagattgga gctttgctgt 8700
ttcattaact gtgcagtgta gactaatggt gtttaataaa aatcattcaa aatttcaaac 8760
tcttttgcca gtgacctcaa ttttgttggc tctgtgattt gtatcagact ttgaggaggg 8820
aagggggaag tgaaggaagc ctacgtccag gcccctgaca ggatgctgca gtagcaagct 8880
caagctcgcc tgcctgccag cagttgctgg tgagcagcag catgcagacc agctgtggga 8940
agcctcctga agaatgcccc agctgatgct ttcagctggg aatagtttgt tcctattggg 9000
gaactcattg ttctccagtc tctgcagcag gaagccagct gtcatattcg gagggaattt 9060
cagatgcttt acctttttgg ttttgtcctg catcactcat gtggctacga aagtgtctct 9120

gagaatagag cccaatgtgg tgacaatggg tagtcaaatg caccccagat gctcaagccc 9180
tgttgtggtt ctgcagtgtt tatgaaattg ggaggaagga gaccctggac agtaagcaaa 9240
attggagaca ctccaacgag gctaagttaa tgccgtgttg cccagaacaa gatctagctt 9300
ctcatttggt cagcctagca tgcaaccagt ggtgtgctgg taaaatgttt aacaaccagc 9360
tcgctgagaa tagaaagcac ctggtttgca ccatttgcca atttccatgg cataaatact 9420
accactttag atgattttaa gctaccaact gtgatgtcac tgaacacatg gttggaaaga 9480
gatgcacgca gttggctctt gcaagcctgg gcaaaaatgc ttcaacacgc cactggatgc 9540
agccagtcag agggttcata tttaatatat gtgttcatgt ggacacacac agacacacac 9600
acacaaactc acccttacac acacacttcg atgactaaaa caattacata gttttaagat 9660
atgaatcaat gtgtgaatgt agaaagctta tgataaggcc ctagaggtat gggttgccct 9720
ggaagcctag gttttaagca ggagaatagc tgagaagaat gaagccctcc tgagctgaaa 9780
ggagagatgg atcaatggag atggttccat catctccttc catatctcac aggtaaaatg 9840
ggcactcaga aaaccctcac gattgatttt ttaaaaagat aagtgagtgt tttttatttt 9900
attattattg tcatcattat tttgatttac aaatgctatt tgtaactttt acatgtaact 9960
aggataaagt atttacggga actctatgga gaatagcaca atccagaatt tactgtgttt 10020
ttcttttatg tgacgtggaa actcagtaat tctcccacct tcacattgtt gttcataaga 10080
attttacttt agttattagg gaatctaagt tttttgttaa catttgtttt tagttaaaag 10140
tatctactta ctgttttagc tctgaactca aaccagaata tctctgtatc aattgcatga 10200
ctattcagaa acaataatcc aaaccaaaat aattcttttt ccacccagta cgaagaaaac 10260
taagctcagt aacaagaagg cataaactaa agtatataat gaggctttca ttaaatacac 10320
acacacacac actcacacac acacacatac actttttaaa tttttaaatt aggcctccac 10380
acataaatca ttttgaaagt agaatagaaa atctcaaaga attcattctc ctggtcctgt 10440
gcatcttctg cagttaataa gaggtttgta tctggaaaga tggaagaact tgttctaaaa 10500
tcttattttt caaaaaaaaa tttccatttt ctctctgggc ctgtatccat ggttgaatgt 10560
tagccctgga ggagatccat gtcttactcg ctctttctgg cccttctgtc ttttgcctct 10620
gcaattcttt ttgtagctgg cacgatagca gggactgggg gtctatcctt tcatggtatt 10680
gctacaatat ttgtccttac tggaaaatgg taacatccgg gtctgattta attggcatta 10740
cacttacaca gggactctga gcacccccgt caccacacca gacagtggac cagttttcac 10800
agctacaaag agctagaaat gtgtttaaca tcatccagtg catcccctaa ttcaaaacca 10860
tcctcactaa tcaatcatat tcacccataa atattacaaa tgagattgat tccatctcaa 10920
gacaatttgt caaatactta attttcttcc tggatgattc tacttactgg atattttaga 10980
aagagaaatg tctgagataa aatccctcac atttactcaa tataacaaat tactgtttct 11040
actcctattc tgagtagtgc ttctgaagat tgtttgctgt agtgttgtct ttgataaaat 11100
gaatgtcagt agtgagcctt ttagagatac catgctcaga catcctcttt gggatcagaa 11160

gatacctaaa attctcccct tttgcccact tggttagatg agtgatatat tctttggatc 11220
ctgcaaagaa gagattggtt tcttttcttt tctggtggtg gtagtggttg tatctgtggc 11280
tgtgatggtt gttgttactt gtctctctct ctctctggct ctggcttttg ctttcctgct 11340
agtgttcttt ctctttccaa acaaatagtt aaattaaacg tgagcttctg aattgtactt 11400
gttcatactt tcaaaacata acagattaat aaaaatagat gtgtcctgat ttaaaacatg 11460
ccccctggaa aggcatgctg tattatgaaa tcgtgataat ataactgcat tattacatgg 11520
cagtataaat attagtctgt tgaattcatt tgtccaattg tataactttg tggagcagtg 11580
ttttgacctt tgatacataa ttctggagca agtggagtgg ttgcaggcag atgagacagt 11640
gttatatcag gatttttcaa tcaactttag ttggaggcct ggcaattaca aacatcttca 11700
gatgtttctg taaccattat aaatatgaaa aaaacctctt caaaaaattt cccatagtac 11760
ttcagtcaag actttttagg tttatctttt ttttttcatt tctccttttc cttttccatt 11820
atttttcgat gggggggttg ttatcattga ctgaagaaat attttgattg caatggtctc 11880
tctctctctc cccctctctc tctctctcct ctattctttc ctccttccct ctgtccatca 11940
cccctcatta aaatattgaa atctggagtc tttgataaat ctgcattaga ccaggctata 12000
tgctaggaat gaaatctggg caaatatcga tgggttttca aagaatgctc catgttcatt 12060
gggccctttc acaccccaca gtgataaatg aaaaggatag aggtagtttt ttcaaaagag 12120
cactttaata atatcctctg agacctaatg cagtttaaca aatgactcca cctatttttc 12180
cagtaggtaa attgactgag acttgcaaaa tacccctgag agttgtcagg ggtgtcttct 12240
gcctggtcta tagcgtgtgt gtttgctttg tatctaacag gcacattcac gtctcgtgta 12300
ctcatatgaa gtatttccta acattcccat tagcctgtat ataagaatca gaaagataat 12360
cccaacatgt tgtaaatgaa gatgtgactc tataaccttt ctcttcttcc tggaaaaaaa 12420
aggacatttt catgcatatt ttaaacagaa attttgtata tttaagtgtc atagaaaata 12480
tttattgagt aactgggaca caaatgggaa tttaattgtc atcatatgct ttgtgtgtgg 12540
ggatgcttac caacaccatg tcgctggacc attgtggcaa gccataactg cacaaagagt 12600
acacatcgtc agtgtgtgtg tgtgtgtgtg tgtgcgcgca cgcacgtgcg tgtgtgtgtc 12660
cctgcatgtg caacatgtct agcttgctgt ccttcatggg attttagctt tcccttcttg 12720
aaaaacatta ttttacagtt ccaggaggcc ctggttacat tactatatga aggcagtgat 12780
ttgaaatgaa aattcctttc ctcttggaag ctttggtcat aatatcatgg ttcaattaaa 12840
cggattccac cggactttgt gatgaaaaag gctctgttaa aatccaattg agtttccaag 12900
aggaaattgt agtaggtcaa gatgcatgag agggaagatg gaggccacct cagctggaga 12960
acatgagctg agttgagccc tcagtgttga agttgacttg ctccaagctg cagtctaaaa 13020
ccctggggcc cgtgcctggc ctatgctccc tcccaagtaa gtagaggagc agaaccatca 13080
ggaacagcct gcctggctcc tatgaagaaa acttcctgac gtcctgtccc caaaggaaga 13140
ccctttcccc aagggcaccc caggtggcca ttaaattgtg atgatcattc agaaagtgcc 13200

cccttggctt tatgagaatc caattagtct tctgaaccac cttttcttgg gtgcagattt 13260

ccaacattca tgctcattgc agatccacca actgtcactg ttcttaacaa gcatgctcgt 13320

cttgtcagaa tttcagtaag ttccaatttc ctgtacagac cagggtaaac tgttctaaaa 13380

tcaatcaatt aatgaaatgt tatctggttt ttaaaagctg gtttcatgtg ctttatgtgt 13440

ataaaactat atctgcctgt gtggctttgc atttcaaatg tgtggcgcac aagcgttttg 13500

ttggtgcttt gttctcagta cagtaactct gtgtacaaac attttaatgt ggttttgttg 13560

ttttccaaca agatgtctct gtaaaaatga tattggctga gctggtgcgt tggtttctct 13620

catagaggca ttaactatac tgccaatgca ttgaattatt taaaaatgca aaataaaatt 13680

tttatgaaaa tctca 13695

<210> 24

<211> 1311

<212> PRT

<213> homo sapiens

<220>

<221> misc_feature

<223> FMR2 gene product

<400> 24

Met Asp Leu Phe Asp Phe Phe Arg Asp Trp Asp Leu Glu Gln Gln Cys
1               5                   10                  15

His Tyr Glu Gln Asp Arg Ser Ala Leu Lys Lys Arg Glu Trp Glu Arg
            20                  25                  30

Arg Asn Gln Glu Val Gln Gln Glu Asp Asp Leu Phe Ser Ser Gly Phe
        35                  40                  45

Asp Leu Phe Gly Glu Pro Tyr Lys Val Ala Glu Tyr Thr Asn Lys Gly
    50                  55                  60

Asp Ala Leu Ala Asn Arg Val Gln Asn Thr Leu Gly Asn Tyr Asp Glu
65                  70                  75                  80

Met Lys Asn Leu Leu Thr Asn His Ser Asn Gln Asn His Leu Val Gly
                85                  90                  95

Ile Pro Lys Asn Ser Val Pro Gln Asn Pro Asn Asn Lys Asn Glu Pro
            100                 105                 110

Ser Phe Phe Pro Glu Gln Lys Asn Arg Ile Ile Pro Pro His Gln Asp
        115                 120                 125

Asn Thr His Pro Ser Ala Pro Met Pro Pro Pro Ser Val Val Ile Leu
    130                 135                 140

Asn Ser Thr Leu Ile His Ser Asn Arg Lys Ser Lys Pro Glu Trp Ser
145                 150                 155                 160

Arg Asp Ser His Asn Pro Ser Thr Val Leu Ala Ser Gln Ala Ser Gly
                165                 170                 175

Gln Pro Asn Lys Met Gln Thr Leu Thr Gln Asp Gln Ser Gln Ala Lys
            180                 185                 190

Leu Glu Asp Phe Phe Val Tyr Pro Ala Glu Gln Pro Gln Ile Gly Glu
        195                 200                 205

Val Glu Glu Ser Asn Pro Ser Ala Lys Glu Asp Ser Asn Pro Asn Ser
    210                 215                 220

Ser Gly Glu Asp Ala Phe Lys Glu Ile Phe Gln Ser Asn Ser Pro Glu
225                 230                 235                 240

Glu Ser Glu Phe Ala Val Gln Ala Pro Gly Ser Pro Leu Val Ala Ser
                245                 250                 255

Ser Leu Leu Ala Pro Ser Ser Gly Leu Ser Val Gln Asn Phe Pro Pro
            260                 265                 270

Gly Leu Tyr Cys Lys Thr Ser Met Gly Gln Gln Lys Pro Thr Ala Tyr
        275                 280                 285

Val Arg Pro Met Asp Gly Gln Asp Gln Ala Pro Asp Ile Ser Pro Thr
    290                 295                 300

Leu Lys Pro Ser Ile Glu Phe Glu Asn Ser Phe Gly Asn Leu Ser Phe
305                 310                 315                 320

Gly Thr Leu Leu Asp Gly Lys Pro Ser Ala Ala Ser Ser Lys Thr Lys
                325                 330                 335

Leu Pro Lys Phe Thr Ile Leu Gln Thr Ser Glu Val Ser Leu Pro Ser
            340                 345                 350

Asp Pro Ser Cys Val Glu Glu Ile Leu Arg Glu Met Thr His Ser Trp
        355                 360                 365

Pro Thr Pro Leu Thr Ser Met His Thr Ala Gly His Ser Glu Gln Ser
    370                 375                 380

Thr Phe Ser Ile Pro Gly Gln Glu Ser Gln His Leu Thr Pro Gly Phe
385                 390                 395                 400

Thr Leu Gln Lys Trp Asn Asp Pro Thr Thr Arg Ala Ser Thr Lys Ser
                405                 410                 415

Val Ser Phe Lys Ser Met Leu Glu Asp Asp Leu Lys Leu Ser Ser Asp
            420                 425                 430

Glu Asp Asp Leu Glu Pro Val Lys Thr Leu Thr Thr Gln Cys Thr Ala
        435                 440                 445

Thr Glu Leu Tyr Gln Ala Val Glu Lys Ala Lys Pro Arg Asn Asn Pro
    450                 455                 460

Val Asn Pro Pro Leu Ala Thr Pro Gln Pro Pro Pro Ala Val Gln Ala
465                 470                 475                 480

Ser Gly Gly Ser Gly Ser Ser Ser Glu Ser Glu Ser Ser Ser Glu Ser
                485                 490                 495

Asp Ser Asp Thr Glu Ser Ser Thr Thr Asp Ser Glu Ser Asn Glu Ala
            500                 505                 510

Pro Arg Val Ala Thr Pro Glu Pro Glu Pro Pro Ser Thr Asn Lys Trp
        515                 520                 525

Gln Leu Asp Lys Trp Leu Asn Lys Val Thr Ser Gln Asn Lys Ser Phe
    530                 535                 540

Ile Cys Gly Pro Asn Glu Thr Pro Met Glu Thr Ile Ser Leu Pro Pro
545                 550                 555                 560

Pro Ile Ile Gln Pro Met Glu Val Gln Met Lys Val Lys Thr Asn Ala
                565                 570                 575

Ser Gln Val Pro Ala Glu Pro Lys Glu Arg Pro Leu Leu Ser Leu Ile
            580                 585                 590

Arg Glu Lys Ala Arg Pro Arg Pro Thr Gln Lys Ile Pro Glu Thr Lys
        595                 600                 605

Ala Leu Lys His Lys Leu Ser Thr Thr Ser Glu Thr Val Ser Gln Arg
    610                 615                 620

Thr Ile Gly Lys Lys Gln Pro Lys Lys Val Glu Lys Asn Thr Ser Thr
625                 630                 635                 640

Asp Glu Phe Thr Trp Pro Lys Pro Asn Ile Thr Ser Ser Thr Pro Lys
                645                 650                 655

Glu Lys Glu Ser Val Glu Leu His Asp Pro Pro Arg Gly Arg Asn Lys
            660                 665                 670

Ala Thr Ala His Lys Pro Ala Pro Arg Lys Glu Pro Arg Pro Asn Ile
        675                 680                 685

Pro Leu Ala Pro Glu Lys Lys Lys Tyr Arg Gly Pro Gly Lys Ile Val
    690                 695                 700

Pro Lys Ser Arg Glu Phe Ile Glu Thr Asp Ser Ser Thr Ser Asp Ser
705                 710                 715                 720

Asn Thr Asp Gln Glu Glu Thr Leu Gln Ile Lys Val Leu Pro Pro Cys
                725                 730                 735

Ile Ile Ser Gly Gly Asn Thr Ala Lys Ser Lys Glu Ile Cys Gly Ala
            740                 745                 750

Ser Leu Thr Leu Ser Thr Leu Met Ser Ser Ser Gly Ser Asn Asn Asn
        755                 760                 765

Leu Ser Ile Ser Asn Glu Glu Pro Thr Phe Ser Pro Ile Pro Val Met
    770                 775                 780

Gln Thr Glu Ile Leu Ser Pro Leu Arg Asp His Glu Asn Leu Lys Asn
785                 790                 795                 800

Leu Trp Val Lys Ile Asp Leu Asp Leu Leu Ser Arg Val Pro Gly His
                805                 810                 815

Ser Ser Leu His Ala Ala Pro Ala Lys Pro Asp His Lys Glu Thr Ala
            820                 825                 830

Thr Lys Pro Lys Arg Gln Thr Ala Val Thr Ala Val Glu Lys Pro Ala
        835                 840                 845

Pro Lys Gly Lys Arg Lys His Lys Pro Ile Glu Val Ala Glu Lys Ile
    850                 855                 860

Pro Glu Lys Lys Gln Arg Leu Glu Glu Ala Thr Thr Ile Cys Leu Leu
865                 870                 875                 880

Pro Pro Cys Ile Ser Pro Ala Pro Pro His Lys Pro Pro Asn Thr Arg
                885                 890                 895

Glu Asn Asn Ser Ser Arg Arg Ala Asn Arg Arg Lys Glu Glu Lys Leu
            900                 905                 910

Phe Pro Pro Pro Leu Ser Pro Leu Pro Glu Asp Pro Pro Arg Arg Arg
        915                 920                 925

Asn Val Ser Gly Asn Asn Gly Pro Phe Gly Gln Asp Lys Asn Ile Ala
    930                 935                 940

Met Thr Gly Gln Ile Thr Ser Thr Lys Pro Lys Arg Thr Glu Gly Lys
945                 950                 955                 960

Phe Cys Ala Thr Phe Lys Gly Ile Ser Val Asn Glu Gly Asp Thr Pro
                965                 970                 975

Lys Lys Ala Ser Ser Ala Thr Ile Thr Val Thr Asn Thr Ala Ile Ala
            980                 985                 990

Thr Ala Thr Val Thr Ala Thr Ala  Ile Val Thr Thr Thr  Val Thr Ala
        995                 1000                 1005

Thr Ala  Thr Ala Thr Ala Thr  Thr Thr Thr Thr Thr  Thr Thr Ile
    1010                 1015                 1020

Ser Thr  Ile Thr Ser Thr Ile  Thr Thr Gly Leu Met  Asp Ser Ser
    1025                 1030                 1035

His Leu  Glu Met Thr Ser Trp  Ala Ala Leu Pro Leu  Leu Ser Ser
    1040                 1045                 1050

Ser Ser  Thr Asn Val Arg Arg  Pro Lys Leu Thr Phe  Asp Asp Ser
    1055                 1060                 1065

Val His  Asn Ala Asp Tyr Tyr  Met Gln Glu Ala Lys  Lys Leu Lys
    1070                 1075                 1080

His Lys  Ala Asp Ala Leu Phe  Glu Lys Phe Gly Lys  Ala Val Asn
    1085                 1090                 1095

Tyr Ala  Asp Ala Ala Leu Ser  Phe Thr Glu Cys Gly  Asn Ala Met
    1100                 1105                 1110

Glu Arg  Asp Pro Leu Glu Ala  Lys Ser Pro Tyr Thr  Met Tyr Ser
    1115                 1120                 1125

Glu Thr  Val Glu Leu Leu Arg  Tyr Ala Met Arg Leu  Lys Asn Phe
    1130                 1135                 1140

Ala Ser  Pro Leu Ala Ser Asp  Gly Asp Lys Lys Leu  Ala Val Leu
    1145                 1150                 1155

Cys Tyr  Arg Cys Leu Ser Leu  Leu Tyr Leu Arg Met  Phe Lys Leu
    1160                 1165                 1170

Lys Lys  Asp His Ala Met Lys  Tyr Ser Arg Ser Leu  Met Glu Tyr
    1175                 1180                 1185

Phe Lys Gln Asn Ala Ser Lys Val Ala Gln Ile Pro Ser Pro Trp
    1190                1195                1200

Val Ser Asn Gly Lys Asn Thr Pro Ser Pro Val Ser Leu Asn Asn
    1205                1210                1215

Val Ser Pro Ile Asn Ala Met Gly Asn Cys Asn Asn Gly Pro Val
    1220                1225                1230

Thr Ile Pro Gln Arg Ile His His Met Ala Ala Ser His Val Asn
    1235                1240                1245

Ile Thr Ser Asn Val Leu Arg Gly Tyr Glu His Trp Asp Met Ala
    1250                1255                1260

Asp Lys Leu Thr Arg Glu Asn Lys Glu Phe Phe Gly Asp Leu Asp
    1265                1270                1275

Thr Leu Met Gly Pro Leu Thr Gln His Ser Ser Met Thr Asn Leu
    1280                1285                1290

Val Arg Tyr Val Arg Gln Gly Leu Cys Trp Leu Arg Ile Asp Ala
    1295                1300                1305

His Leu Leu
    1310

<210>  25

<211>  20010

<212>  DNA

<213>  homo sapiens

<220>

<221>  misc_feature

<223>  NEB gene sequence made up of non-contigous segments of gene map l
       ocus 2q22

<400>  25
atggcagatg acgaagacta tgaggaggtg gtggagtact acacagaaga agtggtttac      60

gaagaggtgc cgggagagac aataacaaaa atttatgaga ctacgacaac aaggacatct     120

gactatgagc aatcagaaac ttccaaacca gctctggcac agccagcact ggcacagcca     180

gcatcagcaa agccggtgga gaggaggaag gtcatccgga agaaagtgga tccttcaaag     240

ttcatgaccc cctacattgc acacagtcag aaaatgcagg atctttttag cccaaataaa     300

tacaaggaga agtttgagaa aacaaaagga cagccatacg ccagcacaac agatactcca     360

gaacttcgca gaatcaaaaa agtacaagat caactcagtg aggttaagta tcgaatggat 420
ggtgatgttg ctaagactat atgtcacgta gatgaaaaag caaaggatat tgaacatgca 480
aagaaagtgt cgcagcaagt cagtaaggtt ttatacaagc agaactggga agacaccaag 540
gataagtacc tgcttcctcc tgatgcccct gaacttgtcc aggccgttaa gaacaccgcc 600
atgttcagca agaaactgta cactgaagac tgggaagcag acaaaagttt gttttacccc 660
tataatgata gcccggaact gaggagagtt gcccaggccc agaaagctct cagtgatgtt 720
gcctacaaaa aaggtctcgc tgaacagcaa gctcaattca cgcctctggc cgatcctcca 780
gatatagaat ttgccaagaa agtaaccaat caagtgagca agcaaaaata caaagaagac 840
tatgaaaata aaatcaaagg caaatggagt gagacacctt gctttgaagt tgcaaatgcc 900
agaatgaatg ctgataacat tagcacaagg aaataccagg aagattttga aaacatgaaa 960
gaccagatct acttcatgca gaccgaaaca ccagagtata aaatgaataa aaaagctggt 1020
gtggcagcta gcaaggtaaa atacaaagaa gactatgaaa agaataaagg aaaagcagat 1080
tataatgtgc ttcctgcttc agagaaccca cagcttaggc agctgaaggc agcaggagat 1140
gccctaagtg acaaactata caaggaaaac tatgaaaaga caaaagcaaa gagcataaat 1200
tactgcgaga cccccaaatt caagctcgat actgttctgc agaacttcag tagtgataaa 1260
aaatataaag attcctactt aaaagatatt ttgggacatt atgtaggcag cttcgaggat 1320
ccataccatt cacactgcat gaaagtcaca gctcaaaaca gtgataaaaa ctacaaagca 1380
gaatacgaag aagacagagg caaaggcttc ttccctcaga ccataactca agaatatgaa 1440
gcaattaaga aactagatca gtgtaaagac cacacctaca aagtccatcc agataagaca 1500
aaattcaccc aagttacaga ctctcctgtt ctgctacaag cccaagtcaa ttccaaacaa 1560
ctgagtgact taaattacaa agcaaaacat gaaagtgaaa agttcaagtg ccatatcccc 1620
cctgatactc ctgcttttat ccagcacaaa gtcaatgcct ataacttgag tgataatctt 1680
tataagcaag actgggagaa gagcaaagcc aaaaagtttg acattaaagt ggatgccatt 1740
cccctgctgg cagccaaagc caacaccaag aacaccagcg atgtgatgta caagaaagac 1800
tatgaaaaaa acaaagggaa aatgattgga gtcctcagca ttaatgacga tcccaagatg 1860
ctgcactcct tgaaggtggc caaaaaccag agtgatagat tatacaagga aaactatgag 1920
aagacaaagg caaagagtat gaattactgt gagaccccaa aatatcaact tgatactcag 1980
ctgaagaact tcagtgaggc tagatataaa gacttatatg taaaggatgt tttgggacat 2040
tatgtaggca gcatggagga cccatatcac acacactgca tgaaagttgc agctcaaaac 2100
agtgataaaa gttacaaagc agaatatgaa gaagataaag gaaaatgcta tttccctcag 2160
acaataacac aagaatatga cgcaatcaag aagctggacc agtgtaaaga tcatacctac 2220
aaagttcatc cagataagac caaattcacg gcagtcactg attctcctgt actgttgcaa 2280
gcccagctca acacgaaaca gcttagtgat ctgaattaca aagcaaaaca tgaaggtgag 2340
aggttcaagt gccatatacc agcagatgct ccacagttta tccaacacag agtcaatgcc 2400

tataatctga gtgataatgt ttataagcaa gactgggaga agagcaaagc caagaagttt 2460
gacattaaag tggacgccat tcccctgttg gcagccaaag ccaacaccaa gaacaccagc 2520
gatgtgatgt acaagaaaga ctatgaaaag agcaaaggga aaatgattgg agccctcagc 2580
attaatgacg atccaaagat gctgcactcc ttgaagacag ccaaaaacca gagtgatcgc 2640
gaatatcgaa aagattatga aaagtcaaaa actatctaca cggcacctct tgatatgctc 2700
caagtcactc aagctaagaa atctcaggca attgccagcg acgttgatta taagcacatc 2760
ttacacagtt acagctaccc ccctgatagc atcaatgtgg accttgccaa gaaggcatat 2820
gcgctgcaga gcgatgttga atacaaagct gactacaata gctggatgaa aggttgtggc 2880
tgggtgcctt ttgggtcctt agaaatggaa aaggcaaagc gagcttcaga catcctcaat 2940
gagaaaaaat atcgccaaca tccagacacc ctcaagttta cctcgattga agatgctcca 3000
attacagtac agtctaaaat taaccaggcc cagaggagtg atatcgctta caaagccaaa 3060
ggagaggaaa ttattcacaa ttacaacctg ccaccagacc tgccccagtt catccaggct 3120
aaagttaatg cctacaatat cagtgagaat atgtacaaag cagacttgaa agacttgagc 3180
aagaagggat atgacctgag aactgatgcg attcccatca gagctgccaa agctgccagg 3240
caggcggcga gtgacgttca gtacaaaaaa gactatgaaa aggctaaagg gaaaatggtt 3300
ggcttccaaa gtcttcaaga tgaccctaaa ctggttcatt atatgaacgt ggccaagata 3360
caatcagatc gggagtataa aaaagactat gagaagacaa agtccaaata caacacgccc 3420
catgatatgt tcaatgtcgt ggcggctaag aaagcccagg atgtggtcag caatgtcaac 3480
tataagcatt ctctccatca ttacacctac ttgcctgacg ccatggacct ggagctgtct 3540
aagaacatga tgcagataca gagtgataac gtctacaagg aagactacaa caactggatg 3600
aaaggcattg gctggattcc tattggcagt ctcgacgtcg aaaaagttaa aaaggccggt 3660
gatgctctga atgaaaagaa gtacaggcaa catccagaca ccctcaaatt taccagcatt 3720
gtggactccc cagttatggt ccaggcaaaa cagaacacga agcaagtcag tgatatctta 3780
tacaaggcta aaggagaaga tgtgaaacat aaatacacca tgagtcctga tcttcctcag 3840
tttctccagg ccaagtgcaa tgcttacagt ataagtgacg tctgttataa acgggattgg 3900
catgacttaa tacgcaaggg caacaatgtg ctgggcgatg ctattcccat cactgcagcc 3960
aaggcatcga gaaacattgc cagtgattat aaatacaagg aagcttatga gaagtcaaag 4020
ggaaagcatg tgggtttcag aagcctccag gatgatccca agctggtcca ctatatgaat 4080
gtggcaaagc tgcagtctga tcgtgaatac aagaagaact atgagaacac caaaaccagc 4140
taccataccc ctggggacat ggttacgatc acagctgcaa agatggccca ggatgtcgct 4200
accaatgtca actacaaaca gccattgcat cattacacat acctacctga cgccatgagt 4260
cttgagcata cgaggaatgt caatcaaatt cagagtgata atgtgtataa agacgagtat 4320
aacagcttct tgaagggcat cggatggatc cctattggtt ccctggaggt ggagaaggtc 4380
aagaaagcag gcgatgcatt aaatgagagg aagtatcgac agcacccaga taccgtcaag 4440

ttcacaagtg tgcctgattc catgggcatg atgttggctc agcataacac aaagcagcta 4500
agtgatttga actacaaggt agagggagag aaactgaagc acaagtatac tattgaccct 4560
gaattgcctc agtttattca agccaaagtc aacgccctca acatgagtga tgctcattat 4620
aaagcagatt ggaagaaaac cattcgcaag ggctatgatt tgagaccaga tgccatccca 4680
attgttgctg caaaaagttc aaggaatatt gctagtgatt gcaaatataa ggaggcctac 4740
gagaaagcca aaggcaagca agttggattt ctcagtcttc aggatgatcc taaactggtt 4800
cactacatga atgtggccaa aatccagtct gatcgtgagt acaaaaaggg ctatgaagcc 4860
agcaagacca agtaccacac acctctggat atggtcagtg tgacagctgc aaagaaatct 4920
caggaggttg ccaccaacgc caactacaga cagtcatacc accactacac tctcctgccc 4980
gatgccttga atgtggagca ctccaggaat gccatgcaga ttcagagtga taatctgtac 5040
aaatctgact tcaccaattg gatgaaaggg atcggctggg tgcccataga gtccctggag 5100
gtggagaagg caaagaaagc aggagagatt cttagtgaga agaagtatcg ccagcacccc 5160
gagaagctga agttcactta cgccatggac acaatggaac aggcacttaa caagagtaac 5220
aaactgaaca tggacaagag gctctacact gaaaaatgga acaaggacaa gaccaccatt 5280
catgtcatgc ctgacacacc ggatatttta ctctccagag taaaccaaat caccatgagt 5340
gataaactgt acaaagctgg ctgggaagag gaaaagaaga aaggatatga cctgaggcct 5400
gatgccattg caataaaggc tgcaagagcc tctagagaca ttgccagtga ttacaaatac 5460
aagaaagcct atgaacaagc caaagggaaa cacattggct tccggagcct ggaagatgac 5520
cccaagctgg tgcacttcat gcaagtggcc aagatgcagt cagaccggga atacaagaag 5580
ggatatgaga aatccaagac ctccttccac accccggtgg acatgctcag tgtggtggca 5640
gccaagaagt ctcaggaagt ggccaccaat gccaactaca ggaacgtgat ccatacctac 5700
aacatgcttc ctgatgccat gagctttgaa ttggccaaaa atatgatgca gattcaaagt 5760
gataatcagt acaaggctga ctatgctgac ttcatgaagg gcattggatg gctccctctg 5820
ggctccctgg aagcagagaa aaacaagaaa gccatggaga ttattagtga aaagaagtac 5880
cgccagcacc cagacacttt gaagtattcc acactcatgg actcgatgaa catggttttg 5940
gcccagaata atgcaaaaat tatgaacgaa catctctaca aacaagcatg ggaggctgac 6000
aaaaccaaag tccacatcat gcctgatatc ccccagatta ttttggcaaa ggcaaatgca 6060
attaatataa gtgataaact ctacaaactt tccttggaag agtctaaaaa gaaaggctat 6120
gatctcagac ctgatgcaat tcctatcaaa gctgccaagg cttccagaga tattgcaagt 6180
gattataaat acaagtacaa ttatgaaaaa gggaagggga aaatggttgg tttccgcagt 6240
ctcgaggatg atcccaaatt agtccattcc atgcaagtgg ctaagatgca atctgatcgg 6300
gagtacaaga aaaactatga gaacacaaag accagctacc acacccctgc cgacatgctc 6360
agtgtcacgg ctgcaaagga tgcccaagcc aacatcacca acactaacta caagcacctg 6420
attcacaagt acatcctcct tccagatgca atgaacattg agctgaccag gaatatgaat 6480

cgcatacaga gtgataatga atataagcaa gattacaatg aatggtacaa agggcttggc 6540
tggagtccag caggttctct ggaagtggag aaggccaaga aagcaactga atatgccagt 6600
gatcagaaat accgccagca cccgagcaac ttccagttta agaagctgac tgattccatg 6660
gacatggtgc ttgccaagca gaatgcacat accatgaaca agcatttata caccattgat 6720
tggaataaag ataagaccaa gattcatgtg atgcctgata caccagatat tttacaagcc 6780
aagcagaatc aaacactgta tagtcagaaa ctctataaac ttggatggga agaagctttg 6840
aagaaaggct atgatctccc agttgatgca atttctgtac agctagctaa agcttcaaga 6900
gacattgcta gtgattataa atacaaacaa ggctaccgaa agcaacttgg ccaccatgtt 6960
ggattccgga gtctgcaaga tgacccaaaa cttgtgttgt ccatgaatgt agccaaaatg 7020
cagagtgaaa gagaatacaa gaaggacttt gagaagtgga aaactaagtt ctccagccca 7080
gtggacatgt tgggagtggt actggccaag aagtgtcagg agttggttag tgacgtggac 7140
tacaagaact acctgcatca gtggacatgt ctgcctgatc agaacgatgt tgtgcaagct 7200
aagaaagttt atgaactgca aagtgagaat ctatataaat ctgaccttga gtggctgaga 7260
ggcataggat ggagtccctt gggttcttta gaggcagaaa agaacaagcg ggcttcggaa 7320
atcatcagtg agaagaaata tcgtcagcct ccagacagaa acaagttcac cagcattcct 7380
gatgccatgg atatagttct ggcaaagaca aatgccaaaa ataggagtga tagactttat 7440
agagaagctt gggacaaaga caagactcag atccacatca tgcctgatac acctgacatt 7500
gttctggcta aagcaaactt aatcaacaca agtgataaac tctaccgaat gggttatgag 7560
gagctgaaga gaaaaggtta cgatcttcct gttgatgcca taccaatcaa agcagcaaaa 7620
gcctcccggg aaattgccag tgaatacaag tacaaggaag gctttcgcaa gcagctcggc 7680
caccacattg gtgcccggaa cattgaagat gaccccaaga tgatgtggtc catgcatgtg 7740
gccaagatcc agagtgacag ggagtacaag aaggactttg agaagtggaa gaccaagttc 7800
agcagcccag tggacatgct gggggtggtg ttggcctata agtgccagac cttagtcagc 7860
gacgtggact acaagaacta cctgcaccag tggacatgcc tgcccgacca gagcgatgtc 7920
atccatgctc ggcaggccta tgacctccag agcgataatt tgtacaagtc agaccttcag 7980
tggctaaaag gcattggctg gatgactagt ggttctctcg aggatgagaa aaataaacga 8040
gccacccaga ttttgagtga ccatgtttac cgtcagcacc cagatcaatt taagttttcc 8100
agccttatgg attccatacc aatggttttg gcaaaaaaca atgctattac catgaatcat 8160
cgcctctata cagaagcttg ggataaagat aaaaccactg tccacattat gccagatacc 8220
cctgaagttt tattagctaa acaaaacaaa gtaaattaca gtgagaaatt gtataagctt 8280
ggcctagaag aagccaagag gaaaggttat gacatgcggg tagatgccat tcctatcaag 8340
gcagccaagg cctccagaga tattgcaagt gaattcaagt acaaagaagg ctatcgtaag 8400
cagctcggcc accacattgg tgcccgagct atacgtgatg accccaagat gatgtggtcc 8460
atgcacgtgg ccaagatcca gagtgacagg gagtacaaga aggactttga gaagtggaag 8520

accaagttca gcagcccagt ggacatgctg ggggtggtgc tggccaagaa gtgccagacc 8580
ttagtcagcg atgtggacta caagaactac ctgcaccagt ggacatgcct gcccgaccag 8640
agcgacgtca tccatgctcg gcaggcctat gacctccaga gcgataatat gtacaagtct 8700
gatctccagt ggatgagagg cattggctgg gtgtccattg gctctttgga tgtggaaaaa 8760
tgcaaaaggg caactgaaat tttgagtgat aaaatctatc gccagcctcc agacagattc 8820
aaatttacca gtgtgactga ctctctggaa caagtgctgg ccaagaacaa tgctctcaac 8880
atgaataagc gtttatacac agaggcctgg gacaaagaca agactcaaat tcacataatg 8940
cctgatacac cagagattat gttggcaagg cagaacaaaa tcaactacag tgagactcta 9000
tacaaacttg ccaatgaaga agcaaaaaag aaaggctacg acttgcgaag tgacgccatc 9060
cccatcgtgg ctgccaaggc ctccagggac gttatcagtg attacaaata caaagatggt 9120
taccgcaagc agctcggcca ccacattgga gcccggaaca ttgaagatga ccccaagatg 9180
atgtggtcca tgcatgtggc caagatccag agtgacaggg agtataagaa ggactttgag 9240
aagtggaaga ccaagttcag cagcccagtg gacatgctgg gagtggtgtt agccaagaag 9300
tgccagacct tagtcagcga tgtggactac aagaactacc tgcacgagtg gacgtgcctg 9360
cccgaccaga atgatgtcat ccatgctcgg caggcctatg acctccagag cgataacatt 9420
tacaaatctg atctccagtg gctgagaggc attggctggg tccccattgg gtctatggat 9480
gtggtcaagt gcaagagagc tgctgaaata ctgagtgata acatctaccg ccagcctccg 9540
gacaagctga aatttaccag tgtgactgac tctctagagc aggtgctggc caagaacaat 9600
gctctcaata tgaacaagcg cttatacaca gaagcctggg acaaagacaa gacccaagtc 9660
catattatgc ctgatacacc tgaaatcatg ttggcaagac aaaataaaat aaattatagt 9720
gagagcctct atcgtcaggc catggaagaa gccaagaaag aaggctatga cttgagaagt 9780
gatgccattc ccattgtggc tgccaaggcc tctcgggata ttgccagtga ttacaaatac 9840
aaagaagcat atcgtaagca gttgggtcac cacattggcg cccgagcagt acacgatgac 9900
cccaagataa tgtggtccct ccacattgcc aaagtgcaga gtgaccgtga gtacaagaaa 9960
gattttgaga aatacaagac aaggtacagc agcccagtgg acatgcttgg tatcgttttg 10020
gccaagaagt gtcagacctt ggtcagcgat gtggactata aacatcctct gcatgaatgc 10080
atctgcctgc ccgaccagaa tgacatcatt catgcacgga aagcctatga cctccagagt 10140
gacaatttgt ataagtcaga ccttgaatgg atgaaaggca ttggctgggt tccgattgat 10200
tccttggaag ttgttagggc caagagagct ggagaattac ttagtgatac tatctaccgt 10260
cagcgtccag aaacgctgaa atttaccagt ataacggaca ctccggagca ggtgctggca 10320
aaaaacaatg ctttaaacat gaataagcgc ttatatactg aagcctggga caatgacaag 10380
aaaactattc atgtcatgcc tgatacacca gaaatcatgt tagccaaact caaccgaata 10440
aactacagtg ataaactcta taaacttgct ttggaagagt ccaagaagga aggctatgac 10500
ttgcgtctgg atgccattcc aatccaagca gccaaggctt caagagatat tgctagtgat 10560

tacaagtaca aggaaggcta ccgcaaacag cttggccacc atattggggc ccggaacatt 10620
aaggatgacc cgaagatgat gtggtccatc catgtggcca agatccagag tgacagggag 10680
tacaagaagg agtttgagaa gtggaagacc aagttcagca gcccagtgga catgctgggg 10740
gtggtgctgg ccaagaagtg tcagatcctt gtaagcgaca tagactacaa gcatcccctg 10800
catgaatgga cctgcctgcc tgatcagaat gacgtcattc aggctcggaa ggcctatgac 10860
ctgcagagtg atgctattta caaatctgat cttgagtggc tgagaggcat aggatgggtt 10920
cccattggct ctgtagaggt cgagaaagtg aagagagctg gagaaatcct gagtgacagg 10980
aagtatcgcc agcctgcaga ccagctcaaa ttcacatgca ttaccgacac tccggaaatt 11040
gtcctagcaa agaataatgc cctgacaatg agcaagcatt tatacacaga agcttgggat 11100
gctgacaaaa cctccatcca cgtgatgcca gacaccccag atatcctgct ggccaagagt 11160
aattctgcca atatcagcca aaaactttac accaagggat gggatgaatc aaagatgaag 11220
gactatgatc tgagagcaga tgctatttcc atcaaaagtg ccaaggcctc cagggacatc 11280
gccagtgact acaaatacaa ggaagcctat gagaaacaga aaggccacca cattggagcc 11340
cagagcattg aagatgatcc caagattatg tgtgccatac atgcagaaaa aattcaaagt 11400
gaaagggagt acaagaagga attccaaaag tggaaaacca agttctctag cccagtggac 11460
atgttaagca tcttgctggc caagaaatgt cagactttgg tcactgacat ttattatcgc 11520
aattacctgc atgaatggac atgcatgccg gatcaaaacg acattatcca agcaaaaaag 11580
gcctatgacc tgcagagtga tgccctctac aaggctgact tggagtggtt gcgtggcatt 11640
ggctggatgc cccaagggtc tcctgaagtg ttgagagtca aaaacgccca gaatatcttt 11700
tgtgacagtg tctatcggac gcctgtggtg aaccttaagt acacaagcat tgttgacaca 11760
cctgaagtgg tccttgctaa atcaaatgct gaaaatatta gtattccaaa gtacagagag 11820
gtttgggaca aggataaaac ttcaatacac ataatgccag atactccaga aattaatctc 11880
gctagagcaa atgctcttaa tgtgagcaat aaactttacc gtgagggctg ggatgaaatg 11940
aaggcgggct gtgatgtccg gctggatgcc atccccatcc aggctgccaa ggcctccagg 12000
gagattgcca gtgactataa atataagctt gaccatgaga agcagaaggg acactacgtg 12060
ggcaccctca cagccaggga tgacaacaag atccgctggg ccctcatagc tgacaagctc 12120
cagaatgaac gagagtaccg gctggactgg gccaaatgga aggccaagat ccagagccct 12180
gtggacatgc tttccatcct gcactctaaa aattcccagg ctctggtcag tgacatggat 12240
taccgcaatt acctgcacca gtggacctgc atgcccgacc agaacgatgt gattcaggcc 12300
aagaaggcct acgaactgca gagcgataat gtttacaagg ctgacttgga atggttgcgt 12360
ggaattgggt ggatgccaaa tgactccgtg tccgtcaatc atgccaaaca tgccgcggac 12420
atcttcagtg agaaaaaata tcgcacaaaa atagaaactc tcaactttac gcctgtggat 12480
gacagagttg attatgtgac agcgaaacaa agtggcgaga tcctcgatga tattaaatac 12540
cggaaagact ggaatgccac caaatcaaag tacaccctca cagaaacccc cctgctgcac 12600

actgcccagg aggctgctag gatactggac cagtatctct acaaggaagg ctgggagaga 12660
caaaaagcca caggttacat tttgcctcca gatgctgtgc catttgttca tgcccatcac 12720
tgcaatgacg ttcagagtga gctgaaatac aaagctgaac atgtgaagca aaaaggtcat 12780
tatgttggtg tcccgacgat gagagatgat cctaagctgg tttggtttga gcatgcaggc 12840
cagattcaga atgagagact atacaaagag gactatcaca aaacaaaggc caaaatcaat 12900
atacctgctg atatggtgtc agtcttggcc gccaagcagg ggcagaccct tgtcagtgat 12960
attgattatc gtaattactt gcaccaatgg atgtgtcatc ctgaccagaa cgatgttatt 13020
caggcaagaa aggcctatga cctacagagt gataatgtct acagagctga cctggagtgg 13080
ctccgaggca ttggctggat cccactggat tctgtggacc atgtaagggt tactaagaac 13140
caggaaatga tgagtcagat caaatataag aaaaatgccc ttgaaaacta tcctaacttt 13200
acaagtgtgg tggatcctcc agagattgtt ttagccaaga ttaattctgt caatcaaagt 13260
gatgtaaaat ataaagaaac atttaataaa gcaaagggca aatatacgtt ttcaccagat 13320
acaccacata tctcccactc caaagacatg ggaaaactct acagtactat actgtataaa 13380
ggggcgtggg agggcaccaa ggcctatggc tacaccctgg atgagcgcta cattcccatt 13440
gttggagcca agcatgctga tctggtgaac agtgagctta aatacaaaga gacatatgag 13500
aagcagaaag gtcactacct ggctggaaaa gtgatcggtg aattccctgg tgtggttcac 13560
tgtctggatt tccaaaagat gaggagtgcg ttgaactaca gaaaacatta tgaggatacc 13620
aaagcaaatg ttcatatccc caatgacatg atgaatcacg tgctggctaa aaggtgccag 13680
tacatcctca gtgacctgga gtatcgacac tatttccacc agtggacgtc tcttctggaa 13740
gaacccaatg ttatacgcgt ccgaaacgcc caggagatct tgagtgataa tgtgtataaa 13800
gatgacctga attggttgaa aggcattggt tgctacgttt gggatacacc ccaaatcctc 13860
catgccaaga aatcatacga ccttcagagt cagctacaat atacagcagc aggtaaagaa 13920
aatctacaaa actataatct ggtcacagac acgcccctct atgtgactgc tgttcagagt 13980
ggcattaatg ccagtgaggt aaaatataaa gaaaattatc atcagattaa ggacaaatac 14040
acaacagttc tagaaacagt ggattatgac agaaccagaa acctgaagaa tctttacagc 14100
agtaacctgt acaaggaggc ctgggataga gtgaaagcca ccagctacat cctgccttcc 14160
agcaccttgt ccctgacaca cgccaagaac cagaagcatc tggccagcca tatcaaatat 14220
cgggaagaat atgaaaagtt caaagctctt tatacgttac caagaagtgt tgacgatgat 14280
ccgaacacag cacggtgcct ccgagttggc aagcttaaca tcgatcgcct gtacagatca 14340
gtttatgaaa agaacaagat gaaaatccac atcgtgcccg acatggtaga gatggttact 14400
gccaaggatt cccagaagaa agtcagtgag attgattacc gcctgcgcct ccacgaatgg 14460
atttgccacc ccgacttgca agtcaatgat cacgtcagga aagtcacaga tcagatcagc 14520
gatattgtat acaaggatga cctcaactgg ctgaaaggca ttggttgcta cgtctgggac 14580
actcctgaaa tcctccatgc caagcatgct tatgatctac gtgatgatat caagtataaa 14640

gctcacatgt tgaaaacaag gaatgactac aagcttgtca cagatacacc agtctacgtg 14700
caggctgtca aaagtgggaa acagctaagt gacgctgtct accactatga ctatgtgcac 14760
agtgtcagag gcaaagtggc tccaactacc aaaaccgtgg atctggaccg ggcccttcat 14820
gcatacaagc tccagagttc gaatctatac aaaaccagcc tgcgcaccct gcccactgga 14880
tatagacttc caggtgacac tcctcacttc aaacacatca aggacacccg ttacatgagc 14940
agttatttca agtacaaaga agcctatgaa cacaccaagg catatgggta tacacttggc 15000
cccaaagatg ttccatttgt ccacgtccgg agagtcaaca atgttaccag cgagagactg 15060
tatcgggaat tgtaccacaa actgaaagac aagatccata caactcccga tccccctgag 15120
atccgccaag tcaagaagac acaagaggct gtcagtgagt tgatctacaa atcagacttc 15180
ttcaagatgc agggccacat gatctctctg ccatacacac cccaagtgat ccattgccgc 15240
tatgtgggag acatcaccag tgatattaaa tacaaagagg acttgcaggt cctgaaggga 15300
tttggctgct tcctgtatga cactcctgac atggtccgct cccggcacct gcggaagctc 15360
tggtctaatt acctatacac tgataaggca agggagatgc gagacaaata caaagtggtg 15420
cttgacactc cagaatacag aaaagtgcaa gaactgaaga cacatctgag tgagctggtc 15480
tacagagctg caggcaagaa gcagaagtca atctttactt cagttcctga tactcctgat 15540
cttttaagag ccaagcgagg gcagaagctt cagagtcagt atctgtatgt tgaacttgcc 15600
accaaagaga gaccccatca tcacgctgga aaccagacca cagccttgaa gcatgctaaa 15660
gacgtgaagg acatggtcag tgagaaaaag tacaagattc aatatgaaaa gatgaaagac 15720
aagtacactc cggttccaga tacgccaatc ctcatcagag ccaagagggc ttactggaat 15780
gccagtgatc tacgctacaa agaaacattt caaaagacca aagggaaata ccacacggtg 15840
aaagatgccc tagacattgt ctatcatcgc aaagtcacag atgacatcag taaaataaaa 15900
tacaaggaga actacatgag ccagttgggt atctggaggt ccattcctga tcgtccagag 15960
catttccacc accgagcagt cactgacaca gtcagtgatg taaaatataa agaagacttg 16020
acttggctta aaggcattgg ttgctatgcc tatgataccc ctgatttcac tctggctgaa 16080
aagaacaaga ctctctacag caagtataag tataaagaag tatttgaaag gacaaagtca 16140
gatttcaagt atgttgccga ctctccgatc aataggcatt tcaagtatgc aactcaattg 16200
atgaatgaga aaaaatacag agctgattat gagcagcgga aagataaata ccacctggta 16260
gtcgatgagc ctagacatct gctggctaag acccgcagcg accagatcag tcagatcaaa 16320
tacaggaaaa actatgaaaa atcaaaggac aaatttacct caattgtgga tactccagaa 16380
cacctgcgta ctacaaaagt caacaaacaa atcagcgata tcctttataa attggaatac 16440
aacaaggcca aacccagagg ctacaccaca atccacgaca cgcccatgtt gctgcatgtc 16500
cgcaaggtta aagatgaagt cagtgatctg aaatacaaag aagtatacca aagaaataaa 16560
tccaactgca ccattgagcc agatgctgtt catatcaaag cagccaagga cgcctacaaa 16620
gtcaacacca atctggacta taagaaacag tacgaagcca acaaagccca ctggaagtgg 16680

actcctgacc gaccggactt cctccaggct gccaagtcat ccctgcagca aagcgatttt 16740
gaatataagc tggaccggga gttcctcaag ggttgcaagc tttctgtcac tgatgacaaa 16800
aacacggtgc tcgccctcag gaatacttta atagaaagtg atctgaaata caaagagaaa 16860
catgtcaagg aaagaggaac ctgccatgcc gtacctgaca cgcctcagat cctgctggcg 16920
aagactgtca gcaacctggt gtctgagaac aagtacaagg accatgtcaa gaagcacttg 16980
gcacagggct catacacaac actaccagag acccgggaca ctgttcacgt caaggaagtg 17040
accaagcatg tcagtgatac aaattacaaa aagaagtttg tcaaggagaa aggaaaatcc 17100
aactactcca tcatgctgga gccaccagag gtgaaacatg ctatggaagt ggccaagaag 17160
caaagtgatg tcgcttacag aaaagatgcc aaagagaacc tgcattacac cacagtggct 17220
gatcgaccag acatcaagaa ggccacacag gcagccaaac aggccagtga ggtggagtac 17280
agagccaagc accgcaagga aggcagccat ggcttaagca tgctcggtcg cccagacata 17340
gaaatggcca agaaggcagc caagctgagc agccaggtta aataccgaga aaatttcgat 17400
aaagaaaagg gcaagacacc aaaatacaat ccaaaagaca gccagctcta caaagtcatg 17460
aaagatgcta ataatcttgc aagtgaggtt aaatacaagg ctgacctgaa gaaacttcac 17520
aaacccgtga ctgacatgaa ggagtctctg atcatgaatc atgtcctgaa tacaagccaa 17580
cttgccagtt cttaccagta caagaagaag tatgagaaga gtaaaggcca ctaccacacc 17640
atacccgata atctggagca gcttcaccta aaagaggcca cagaattaca gagtatagtg 17700
aaatacaaag aaaagtatga aaaggaacga ggaaaaccca tgctggactt tgaaacacca 17760
acgtacatca ctgccaaaga gtctcagcag atgcagagtg ggaaagaata taggaaagat 17820
tatgaagagt ccattaaagg cagaaacctg actggcctgg aggtcacgcc agctttgtta 17880
catgtcaaat atgcaactaa aatagcaagc gagaaagagt acaggaaaga tctagaggaa 17940
agcatccgtg ggaagggcct cactgaaatg gaagatacac ctgacatgct aagagcaaag 18000
aatgccactc aaatcctcaa tgagaaagaa tataagcgag acctggaact ggaagtcaaa 18060
ggaagaggcc tgaatgccat ggccaatgaa actccggatt ttatgagggc caggaatgct 18120
actgatattg ccagtcagat taagtataag caatcagcag aaatggagaa agccaatttc 18180
acttctgtgg ttgatactcc agagatcatt catgcccaac aagtcaagaa tctttcaagc 18240
cagaaaaagt acaaggaaga tgctgagaag tccatgtcgt attatgagac tgttttggac 18300
accccagaga tacagagagt ccgggagaac caaaagaact tcagccttct ccaataccag 18360
tgtgacctta aaaacagtaa aggaaaaatt acagttgttc aagacacgcc agaaatactg 18420
cgtgtaaaag aaaatcagaa gaatttcagc tcggttttat ataaagagga tgtctcacca 18480
ggaacggcta tcggaaagac acctgagatg atgagagtga aacaaacaca ggaccacatt 18540
agctcggtga agtataagga agcaatagga caaggaactc caatccctga cctgcctgaa 18600
gtgaaacgtg tgaaggagac gcagaagcac attagctcgg ttatgtacaa agaaaacttg 18660
ggaacaggca ttccaaccac tgtgactcca gagattgaga gagtcaaacg caatcaagag 18720

aactttagct cggttttgta caaagaaaat ttggggaaag gaatcccaac acctatcact 18780

ccagagatgg agagagtcaa acgcaatcaa gagaacttta gctcggtgtt atacaaagaa 18840

aacatgggca agggaactcc tttacctgtc actcccgaga tggagcgagt caaacacaat 18900

caagaaaata ttagctcggt tttgtacaaa gaaaatgtgg ggaaagccac cgcaacccct 18960

gtcactcctg agatgcagag agtcaaacgc aatcaagaaa acattagctc ggtgttatac 19020

aaagagaacc tggggaaagc aacccccaca ccctttactc ctgagatgga aagagtgaaa 19080

cgcaatcaag aaaactttag ctcggtattg tacaaagaga acatgagaaa agcaactccg 19140

acacctgtta ctccagagat ggagagagct aagcgcaacc aagaaaacat tagctcggtt 19200

ctttattctg atagtttccg gaaacaaata caaggcaaag ctgcctatgt attggatacc 19260

cccgagatga gacgggtgag ggagacccaa cggcacatct caacggtgaa atatcatgaa 19320

gactttgaga aacacaaggg ttgcttcaca ccagtggtga cagatcctat cactgaacga 19380

gtaaagaaga acatgcagga cttcagtgac attaactacc gaggtattca gaggaaagtg 19440

gtagaaatgg aacaaaaacg gaatgaccaa gatcaggaga ctattacagg tttacgtgtc 19500

tggcgtacta atcctggttc ggtttttgac tatgatccag cagaagacaa catccagtcc 19560

cgaagcttac acatgattaa tgtccaagct cagcgccgga gccgggagca gtcacgatct 19620

gccagtgcac taagcgtcag tgggggtgag gagaagtctg agcattcaga agcaccagac 19680

caccaccttt cgacttacag cgacgggggt gtctttgcag tctcaacagc ttacaaacat 19740

gcaaaaacca cagagctccc acaacaacga tcatcttcag ttgctaccca acagacaacg 19800

gtatcttcca tcccatctca tccatctact gctggaaaaa tcttccgtgc catgtatgac 19860

tatatggctg ctgatgcaga tgaggtgtcc ttcaaggatg gagatgccat cataaatgtt 19920

caagcaattg atgaaggctg gatgtatggc actgtgcaga ggactggcag gaccggaatg 19980

ctcccagcca actacgttga agctatttag 20010

<210> 26

<211> 20839

<212> DNA

<213> homo sapiens

<220>

<221> misc_feature

<223> NEB mRNA

<400> 26
gtgataaaac tacaaagcag aatacgaaga agacagaggc aaaggcttct tccctcagac 60

cataactcaa gaatatgggg gtctcgcagt aatttatgct ctttgctttt gtcttttcat 120

agttttcctt gtatagtttg tcacttaggg catctcctgc tgccttcagc tgcctaagct 180
gtgggttctc tgaagcagga agcacattat aatctgcttt tcctttattc ttttcatagt 240
cttctttgta ttttgctgct gaggaaattt atttggtaga ttgaaggttt gaacgagagc 300
tacagaaacg aaagaaaaag tctgtataag ccaatggtgt tcgggaagaa aataacccca 360
ttgccttgag tttgtaggtg ccactactac tctgaaaaat ggcagatgac gaagactatg 420
aggaggtggt ggagtactac acagaagaag tggtttacga agaggtgccg ggagagacaa 480
taacaaaaat ttatgagact acgacaacaa ggacatctga ctatgagcaa tcagaaactt 540
ccaaaccagc tctggcacag ccagcactgg cacagccagc atcagcaaag ccggtggaga 600
ggaggaaggt catccggaag aaagtggatc cttcaaagtt catgaccccc tacattgcac 660
acagtcagaa aatgcaggat ctttttagcc caaataaata caaggagaag tttgagaaaa 720
caaaaggaca gccatacgcc agcacaacag atactccaga acttcgcaga atcaaaaaag 780
tacaagatca actcagtgag gttaagtatc gaatggatgg tgatgttgct aagactatat 840
gtcacgtaga tgaaaaagca aaggatattg aacatgcaaa gaaagtgtcg cagcaagtca 900
gtaaggtttt atacaagcag aactgggaag acaccaagga taagtacctg cttcctcctg 960
atgcccctga acttgtccag gccgttaaga acaccgccat gttcagcaag aaactgtaca 1020
ctgaagactg ggaagcagac aaaagtttgt tttaccccta taatgatagc ccggaactga 1080
ggagagttgc ccaggcccag aaagctctca gtgatgttgc ctacaaaaaa ggtctcgctg 1140
aacagcaagc tcaattcacg cctctggccg atcctccaga tatagaattt gccaagaaag 1200
taaccaatca agtgagcaag caaaaataca aagaagacta tgaaaataaa atcaaaggca 1260
aatggagtga gacaccttgc tttgaagttg caaatgccag aatgaatgct gataacatta 1320
gcacaaggaa ataccaggaa gattttgaaa acatgaaaga ccagatctac ttcatgcaga 1380
ccgaaacacc agagtataaa atgaataaaa aagctggtgt ggcagctagc aaggtaaaat 1440
acaaagaaga ctatgaaaag aataaaggaa aagcagatta taatgtgctt cctgcttcag 1500
agaacccaca gcttaggcag ctgaaggcag caggagatgc cctaagtgac aaactataca 1560
aggaaaacta tgaaaagaca aaagcaaaga gcataaatta ctgcgagacc cccaaattca 1620
agctcgatac tgttctgcag aacttcagta gtgataaaaa atataaagat tcctacttaa 1680
aagatatttt gggacattat gtaggcagct tcgaggatcc ataccattca cactgcatga 1740
aagtcacagc tcaaaacagt gataaaaact acaaagcaga atacgaagaa gacagaggca 1800
aaggcttctt ccctcagacc ataactcaag aatatgaagc aattaagaaa ctagatcagt 1860
gtaaagacca cacctacaaa gtccatccag ataagacaaa attcacccaa gttacagact 1920
ctcctgttct gctacaagcc caagtcaatt ccaaacaact gagtgactta aattacaaag 1980
caaaacatga aagtgaaaag ttcaagtgcc atatcccccc tgatactcct gcttttatcc 2040
agcacaaagt caatgcctat aacttgagtg ataatcttta taagcaagac tgggagaaga 2100
gcaaagccaa aaagtttgac attaaagtgg atgccattcc cctgctggca gccaaagcca 2160

acaccaagaa caccagcgat gtgatgtaca agaaagacta tgaaaaaaac aaagggaaaa 2220
tgattggagt cctcagcatt aatgacgatc ccaagatgct gcactccttg aaggtggcca 2280
aaaaccagag tgatagatta tacaaggaaa actatgagaa gacaaaggca aagagtatga 2340
attactgtga gaccccaaaa tatcaacttg atactcagct gaagaacttc agtgaggcta 2400
gatataaaga cttatatgta aaggatgttt tgggacatta tgtaggcagc atggaggacc 2460
catatcacac acactgcatg aaagttgcag ctcaaaacag tgataaaagt tacaaagcag 2520
aatatgaaga agataaagga aaatgctatt tccctcagac aataacacaa gaatatgacg 2580
caatcaagaa gctggaccag tgtaaagatc atacctacaa agttcatcca gataagacca 2640
aattcacggc agtcactgat tctcctgtac tgttgcaagc ccagctcaac acgaaacagc 2700
ttagtgatct gaattacaaa gcaaaacatg aaggtgagag gttcaagtgc catataccag 2760
cagatgctcc acagtttatc caacacagag tcaatgccta taatctgagt gataatgttt 2820
ataagcaaga ctgggagaag agcaaagcca agaagtttga cattaaagtg gacgccattc 2880
ccctgttggc agccaaagcc aacaccaaga acaccagcga tgtgatgtac aagaaagact 2940
atgaaaagag caaagggaaa atgattggag ccctcagcat taatgacgat ccaaagatgc 3000
tgcactcctt gaagacagcc aaaaaccaga gtgatcgcga atatcgaaaa gattatgaaa 3060
agtcaaaaac tatctacacg gcacctcttg atatgctcca agtcactcaa gctaagaaat 3120
ctcaggcaat tgccagcgac gttgattata agcacatctt acacagttac agctaccccc 3180
ctgatagcat caatgtggac cttgccaaga aggcatatgc gctgcagagc gatgttgaat 3240
acaaagctga ctacaatagc tggatgaaag gttgtggctg ggtgcctttt gggtccttag 3300
aaatggaaaa ggcaaagcga gcttcagaca tcctcaatga gaaaaaatat cgccaacatc 3360
cagacaccct caagtttacc tcgattgaag atgctccaat tacagtacag tctaaaatta 3420
accaggccca gaggagtgat atcgcttaca aagccaaagg agaggaaatt attcacaatt 3480
acaacctgcc accagacctg ccccagttca tccaggctaa agttaatgcc tacaatatca 3540
gtgagaatat gtacaaagca gacttgaaag acttgagcaa gaagggatat gacctgagaa 3600
ctgatgcgat tcccatcaga gctgccaaag ctgccaggca ggcggcgagt gacgttcagt 3660
acaaaaaaga ctatgaaaag gctaaaggga aaatggttgg cttccaaagt cttcaagatg 3720
accctaaact ggttcattat atgaacgtgg ccaagataca atcagatcgg gagtataaaa 3780
aagactatga gaagacaaag tccaaataca acacgcccca tgatatgttc aatgtcgtgg 3840
cggctaagaa agcccaggat gtggtcagca atgtcaacta taagcattct ctccatcatt 3900
acacctactt gcctgacgcc atggacctgg agctgtctaa gaacatgatg cagatacaga 3960
gtgataacgt ctacaaggaa gactacaaca actggatgaa aggcattggc tggattccta 4020
ttggcagtct cgacgtcgaa aaagttaaaa aggccggtga tgctctgaat gaaaagaagt 4080
acaggcaaca tccagacacc ctcaaattta ccagcattgt ggactcccca gttatggtcc 4140
aggcaaaaca gaacacgaag caagtcagtg atatcttata caaggctaaa ggagaagatg 4200

tgaaacataa atacaccatg agtcctgatc ttcctcagtt tctccaggcc aagtgcaatg 4260
cttacagtat aagtgacgtc tgttataaac gggattggca tgacttaata cgcaagggca 4320
acaatgtgct gggcgatgct attcccatca ctgcagccaa ggcatcgaga aacattgcca 4380
gtgattataa atacaaggaa gcttatgaga agtcaaaggg aaagcatgtg ggtttcagaa 4440
gcctccagga tgatcccaag ctggtccact atatgaatgt ggcaaagctg cagtctgatc 4500
gtgaatacaa gaagaactat gagaacacca aaaccagcta ccatacccct ggggacatgg 4560
ttacgatcac agctgcaaag atggcccagg atgtcgctac caatgtcaac tacaaacagc 4620
cattgcatca ttacacatac ctacctgacg ccatgagtct tgagcatacg aggaatgtca 4680
atcaaattca gagtgataat gtgtataaag acgagtataa cagcttcttg aagggcatcg 4740
gatggatccc tattggttcc ctggaggtgg agaaggtcaa gaaagcaggc gatgcattaa 4800
atgagaggaa gtatcgacag cacccagata ccgtcaagtt cacaagtgtg cctgattcca 4860
tgggcatgat gttggctcag cataacacaa agcagctaag tgatttgaac tacaaggtag 4920
agggagagaa actgaagcac aagtatacta ttgaccctga attgcctcag tttattcaag 4980
ccaaagtcaa cgccctcaac atgagtgatg ctcattataa agcagattgg aagaaaacca 5040
ttcgcaaggg ctatgatttg agaccagatg ccatcccaat tgttgctgca aaaagttcaa 5100
ggaatattgc tagtgattgc aaatataagg aggcctacga gaaagccaaa ggcaagcaag 5160
ttggatttct cagtcttcag gatgatccta aactggttca ctacatgaat gtggccaaaa 5220
tccagtctga tcgtgagtac aaaaagggct atgaagccag caagaccaag taccacacac 5280
ctctggatat ggtcagtgtg acagctgcaa agaaatctca ggaggttgcc accaacgcca 5340
actacagaca gtcataccac cactacactc tcctgcccga tgccttgaat gtggagcact 5400
ccaggaatgc catgcagatt cagagtgata atctgtacaa atctgacttc accaattgga 5460
tgaaagggat cggctgggtg cccatagagt ccctggaggt ggagaaggca aagaaagcag 5520
gagagattct tagtgagaag aagtatcgcc agcaccccga gaagctgaag ttcacttacg 5580
ccatggacac aatggaacag gcacttaaca agagtaacaa actgaacatg gacaagaggc 5640
tctacactga aaaatggaac aaggacaaga ccaccattca tgtcatgcct gacacaccgg 5700
atattttact ctccagagta aaccaaatca ccatgagtga taaactgtac aaagctggct 5760
gggaagagga aaagaagaaa ggatatgacc tgaggcctga tgccattgca ataaaggctg 5820
caagagcctc tagagacatt gccagtgatt acaaatacaa gaaagcctat gaacaagcca 5880
aggggaaaca cattggcttc cggagcctgg aagatgaccc caagctggtg cacttcatgc 5940
aagtggccaa gatgcagtca gaccgggaat acaagaaggg atatgagaaa tccaagacct 6000
ccttccacac cccggtggac atgctcagtg tggtggcagc caagaagtct caggaagtgg 6060
ccaccaatgc caactacagg aacgtgatcc atacctacaa catgcttcct gatgccatga 6120
gctttgaatt ggccaaaaat atgatgcaga ttcaaagtga taatcagtac aaggctgact 6180
atgctgactt catgaagggc attggatggc tccctctggg ctccctggaa gcagagaaaa 6240

acaagaaagc catggagatt attagtgaaa agaagtaccg ccagcaccca gacactttga 6300
agtattccac actcatggac tcgatgaaca tggttttggc ccagaataat gcaaaaatta 6360
tgaacgaaca tctctacaaa caagcatggg aggctgacaa aaccaaagtc cacatcatgc 6420
ctgatatccc ccagattatt ttggcaaagg caaatgcaat taatataagt gataaactct 6480
acaaactttc cttggaagag tctaaaaaga aaggctatga tctcagacct gatgcaattc 6540
ctatcaaagc tgccaaggct tccagagata ttgcaagtga ttataaatac aagtacaatt 6600
atgaaaaagg gaaggggaaa atggttggtt tccgcagtct cgaggatgat cccaaattag 6660
tccattccat gcaagtggct aagatgcaat ctgatcggga gtacaagaaa aactatgaga 6720
acacaaagac cagctaccac acccctgccg acatgctcag tgtcacggct gcaaaggatg 6780
cccaagccaa catcaccaac actaactaca agcacctgat tcacaagtac atcctccttc 6840
cagatgcaat gaacattgag ctgaccagga atatgaatcg catacagagt gataatgaat 6900
ataagcaaga ttacaatgaa tggtacaaag ggcttggctg gagtccagca ggttctctgg 6960
aagtggagaa ggccaagaaa gcaactgaat atgccagtga tcagaaatac cgccagcacc 7020
cgagcaactt ccagtttaag aagctgactg attccatgga catggtgctt gccaagcaga 7080
atgcacatac catgaacaag catttataca ccattgattg gaataaagat aagaccaaga 7140
ttcatgtgat gcctgataca ccagatattt tacaagccaa gcagaatcaa acactgtata 7200
gtcagaaact ctataaactt ggatgggaag aagctttgaa gaaaggctat gatctcccag 7260
ttgatgcaat ttctgtacag ctagctaaag cttcaagaga cattgctagt gattataaat 7320
acaaacaagg ctaccgaaag caacttggcc accatgttgg attccggagt ctgcaagatg 7380
acccaaaact tgtgttgtcc atgaatgtag ccaaaatgca gagtgaaaga gaatacaaga 7440
aggactttga gaagtggaaa actaagttct ccagcccagt ggacatgttg ggagtggtac 7500
tggccaagaa gtgtcaggag ttggttagtg acgtggacta caagaactac ctgcatcagt 7560
ggacatgtct gcctgatcag aacgatgttg tgcaagctaa gaaagtttat gaactgcaaa 7620
gtgagaatct atataaatct gaccttgagt ggctgagagg cataggatgg agtcccttgg 7680
gttctttaga ggcagaaaag aacaagcggg cttcggaaat catcagtgag aagaaatatc 7740
gtcagcctcc agacagaaac aagttcacca gcattcctga tgccatggat atagttctgg 7800
caaagacaaa tgccaaaaat aggagtgata gactttatag agaagcttgg gacaaagaca 7860
agactcagat ccacatcatg cctgatacac ctgacattgt tctggctaaa gcaaacttaa 7920
tcaacacaag tgataaactc taccgaatgg gttatgagga gctgaagaga aaaggttacg 7980
atcttcctgt tgatgccata ccaatcaaag cagcaaaagc ctcccgggaa attgccagtg 8040
aatacaagta caaggaaggc tttcgcaagc agctcggcca ccacattggt gcccggaaca 8100
ttgaagatga ccccaagatg atgtggtcca tgcatgtggc caagatccag agtgacaggg 8160
agtacaagaa ggactttgag aagtggaaga ccaagttcag cagcccagtg gacatgctgg 8220
gggtggtgtt ggcctataag tgccagacct tagtcagcga cgtggactac aagaactacc 8280

tgcaccagtg gacatgcctg cccgaccaga gcgatgtcat ccatgctcgg caggcctatg 8340
acctccagag cgataatttg tacaagtcag accttcagtg gctaaaaggc attggctgga 8400
tgactagtgg ttctctcgag gatgagaaaa ataaacgagc cacccagatt ttgagtgacc 8460
atgtttaccg tcagcaccca gatcaattta agttttccag ccttatggat tccataccaa 8520
tggttttggc aaaaaacaat gctattacca tgaatcatcg cctctataca gaagcttggg 8580
ataaagataa aaccactgtc cacattatgc cagatacccc tgaagtttta ttagctaaac 8640
aaaacaaagt aaattacagt gagaaattgt ataagcttgg cctagaagaa gccaagagga 8700
aaggttatga catgcgggta gatgccattc ctatcaaggc agccaaggcc tccagagata 8760
ttgcaagtga attcaagtac aaagaaggct atcgtaagca gctcggccac cacattggtg 8820
cccgagctat acgtgatgac cccaagatga tgtggtccat gcacgtggcc aagatccaga 8880
gtgacaggga gtacaagaag gactttgaga agtggaagac caagttcagc agcccagtgg 8940
acatgctggg ggtggtgctg gccaagaagt gccagacctt agtcagcgat gtggactaca 9000
agaactacct gcaccagtgg acatgcctgc ccgaccagag cgacgtcatc catgctcggc 9060
aggcctatga cctccagagc gataatatgt acaagtctga tctccagtgg atgagaggca 9120
ttggctgggt gtccattggc tctttggatg tggaaaaatg caaaagggca actgaaattt 9180
tgagtgataa aatctatcgc cagcctccag acagattcaa atttaccagt gtgactgact 9240
ctctggaaca agtgctggcc aagaacaatg ctctcaacat gaataagcgt ttatacacag 9300
aggcctggga caaagacaag actcaaattc acataatgcc tgatacacca gagattatgt 9360
tggcaaggca gaacaaaatc aactacagtg agactctata caaacttgcc aatgaagaag 9420
caaaaaagaa aggctacgac ttgcgaagtg acgccatccc catcgtggct gccaaggcct 9480
ccagggacgt tatcagtgat tacaaataca aagatggtta ccgcaagcag ctcggccacc 9540
acattggagc ccggaacatt gaagatgacc ccaagatgat gtggtccatg catgtggcca 9600
agatccagag tgacagggag tataagaagg actttgagaa gtggaagacc aagttcagca 9660
gcccagtgga catgctggga gtggtgttag ccaagaagtg ccagacctta gtcagcgatg 9720
tggactacaa gaactacctg cacgagtgga cgtgcctgcc cgaccagaat gatgtcatcc 9780
atgctcggca ggcctatgac ctccagagcg ataacattta caaatctgat ctccagtggc 9840
tgagaggcat tggctgggtc cccattgggt ctatggatgt ggtcaagtgc aagagagctg 9900
ctgaaatact gagtgataac atctaccgcc agcctccgga caagctgaaa tttaccagtg 9960
tgactgactc tctagagcag gtgctggcca agaacaatgc tctcaatatg aacaagcgct 10020
tatacacaga agcctgggac aaagacaaga cccaagtcca tattatgcct gatacacctg 10080
aaatcatgtt ggcaagacaa aataaaataa attatagtga gagcctctat cgtcaggcca 10140
tggaagaagc caagaaagaa ggctatgact tgagaagtga tgccattccc attgtggctg 10200
ccaaggcctc tcgggatatt gccagtgatt acaaatacaa agaagcatat cgtaagcagt 10260
tgggtcacca cattggcgcc cgagcagtac acgatgaccc caagataatg tggtccctcc 10320

acattgccaa agtgcagagt gaccgtgagt acaagaaaga ttttgagaaa tacaagacaa 10380
ggtacagcag cccagtggac atgcttggta tcgttttggc caagaagtgt cagaccttgg 10440
tcagcgatgt ggactataaa catcctctgc atgaatgcat ctgcctgccc gaccagaatg 10500
acatcattca tgcacggaaa gcctatgacc tccagagtga caatttgtat aagtcagacc 10560
ttgaatggat gaaaggcatt ggctgggttc cgattgattc cttggaagtt gttagggcca 10620
agagagctgg agaattactt agtgatacta tctaccgtca gcgtccagaa acgctgaaat 10680
ttaccagtat aacggacact ccggagcagg tgctggcaaa aaacaatgct ttaaacatga 10740
ataagcgctt atatactgaa gcctgggaca atgacaagaa aactattcat gtcatgcctg 10800
atacaccaga aatcatgtta gccaaactca accgaataaa ctacagtgat aaactctata 10860
aacttgcttt ggaagagtcc aagaaggaag gctatgactt gcgtctggat gccattccaa 10920
tccaagcagc caaggcttca agagatattg ctagtgatta caagtacaag gaaggctacc 10980
gcaaacagct tggccaccat attggggccc ggaacattaa ggatgacccg aagatgatgt 11040
ggtccatcca tgtggccaag atccagagtg acagggagta caagaaggag tttgagaagt 11100
ggaagaccaa gttcagcagc ccagtggaca tgctgggggt ggtgctggcc aagaagtgtc 11160
agatccttgt aagcgacata gactacaagc atcccctgca tgaatggacc tgcctgcctg 11220
atcagaatga cgtcattcag gctcggaagg cctatgacct gcagagtgat gctatttaca 11280
aatctgatct tgagtggctg agaggcatag gatgggttcc cattggctct gtagaggtcg 11340
agaaagtgaa gagagctgga gaaatcctga gtgacaggaa gtatcgccag cctgcagacc 11400
agctcaaatt cacatgcatt accgacactc cggaaattgt cctagcaaag aataatgccc 11460
tgacaatgag caagcattta tacacagaag cttgggatgc tgacaaaacc tccatccacg 11520
tgatgccaga caccccagat atcctgctgg ccaagagtaa ttctgccaat atcagccaaa 11580
aactttacac caagggatgg gatgaatcaa agatgaagga ctatgatctg agagcagatg 11640
ctatttccat caaaagtgcc aaggcctcca gggacatcgc cagtgactac aaatacaagg 11700
aagcctatga gaaacagaaa ggccaccaca ttggagccca gagcattgaa gatgatccca 11760
agattatgtg tgccatacat gcagaaaaaa ttcaaagtga aagggagtac aagaaggaat 11820
tccaaaagtg gaaaaccaag ttctctagcc cagtggacat gttaagcatc ttgctggcca 11880
agaaatgtca gactttggtc actgacattt attatcgcaa ttacctgcat gaatggacat 11940
gcatgccgga tcaaaacgac attatccaag caaaaaaggc ctatgacctg cagagtgatg 12000
ccctctacaa ggctgacttg gagtggttgc gtggcattgg ctggatgccc caagggtctc 12060
ctgaagtgtt gagagtcaaa aacgcccaga atatcttttg tgacagtgtc tatcggacgc 12120
ctgtggtgaa ccttaagtac acaagcattg ttgacacacc tgaagtggtc cttgctaaat 12180
caaatgctga aaatattagt attccaaagt acagagaggt ttgggacaag gataaaactt 12240
caatacacat aatgccagat actccagaaa ttaatctcgc tagagcaaat gctcttaatg 12300
tgagcaataa actttaccgt gagggctggg atgaaatgaa ggcgggctgt gatgtccggc 12360

tggatgccat ccccatccag gctgccaagg cctccaggga gattgccagt gactataaat 12420
ataagcttga ccatgagaag cagaagggac actacgtggg caccctcaca gccagggatg 12480
acaacaagat ccgctgggcc ctcatagctg acaagctcca gaatgaacga gagtaccggc 12540
tggactgggc caaatggaag gccaagatcc agagccctgt ggacatgctt tccatcctgc 12600
actctaaaaa ttcccaggct ctggtcagtg acatggatta ccgcaattac ctgcaccagt 12660
ggacctgcat gcccgaccag aacgatgtga ttcaggccaa gaaggcctac gaactgcaga 12720
gcgataatgt ttacaaggct gacttggaat ggttgcgtgg aattgggtgg atgccaaatg 12780
actccgtgtc cgtcaatcat gccaaacatg ccgcggacat cttcagtgag aaaaaatatc 12840
gcacaaaaat agaaactctc aactttacgc ctgtggatga cagagttgat tatgtgacag 12900
cgaaacaaag tggcgagatc ctcgatgata ttaaataccg gaaagactgg aatgccacca 12960
aatcaaagta caccctcaca gaaacccccc tgctgcacac tgcccaggag gctgctagga 13020
tactggacca gtatctctac aaggaaggct gggagagaca aaaagccaca ggttacattt 13080
tgcctccaga tgctgtgcca tttgttcatg cccatcactg caatgacgtt cagagtgagc 13140
tgaaatacaa agctgaacat gtgaagcaaa aaggtcatta tgttggtgtc ccgacgatga 13200
gagatgatcc taagctggtt tggtttgagc atgcaggcca gattcagaat gagagactat 13260
acaaagagga ctatcacaaa acaaaggcca aaatcaatat acctgctgat atggtgtcag 13320
tcttggccgc caagcagggg cagacccttg tcagtgatat tgattatcgt aattacttgc 13380
accaatggat gtgtcatcct gaccagaacg atgttattca ggcaagaaag gcctatgacc 13440
tacagagtga taatgtctac agagctgacc tggagtggct ccgaggcatt ggctggatcc 13500
cactggattc tgtggaccat gtaagggtta ctaagaacca ggaaatgatg agtcagatca 13560
aatataagaa aaatgccctt gaaaactatc ctaactttac aagtgtggtg gatcctccag 13620
agattgtttt agccaagatt aattctgtca atcaaagtga tgtaaaatat aaagaaacat 13680
ttaataaagc aaagggcaaa tatacgtttt caccagatac accacatatc tcccactcca 13740
aagacatggg aaaactctac agtactatac tgtataaagg ggcgtgggag ggcaccaagg 13800
cctatggcta caccctggat gagcgctaca ttcccattgt tggagccaag catgctgatc 13860
tggtgaacag tgagcttaaa tacaaagaga catatgagaa gcagaaaggt cactacctgg 13920
ctggaaaagt gatcggtgaa ttccctggtg tggttcactg tctggatttc caaaagatga 13980
ggagtgcgtt gaactacaga aaacattatg aggataccaa agcaaatgtt catatcccca 14040
atgacatgat gaatcacgtg ctggctaaaa ggtgccagta catcctcagt gacctggagt 14100
atcgacacta tttccaccag tggacgtctc ttctggaaga acccaatgtt atacgcgtcc 14160
gaaacgccca ggagatcttg agtgataatg tgtataaaga tgacctgaat tggttgaaag 14220
gcattggttg ctacgtttgg gatacacccc aaatcctcca tgccaagaaa tcatacgacc 14280
ttcagagtca gctacaatat acagcagcag gtaaagaaaa tctacaaaac tataatctgg 14340
tcacagacac gcccctctat gtgactgctg ttcagagtgg cattaatgcc agtgaggtaa 14400

aatataaaga aaattatcat cagattaagg acaaatacac aacagttcta gaaacagtgg 14460
attatgacag aaccagaaac ctgaagaatc tttacagcag taacctgtac aaggaggcct 14520
gggatagagt gaaagccacc agctacatcc tgccttccag caccttgtcc ctgacacacg 14580
ccaagaacca gaagcatctg gccagccata tcaaatatcg ggaagaatat gaaaagttca 14640
aagctcttta tacgttacca agaagtgttg acgatgatcc gaacacagca cggtgcctcc 14700
gagttggcaa gcttaacatc gatcgcctgt acagatcagt ttatgaaaag aacaagatga 14760
aaatccacat cgtgcccgac atggtagaga tggttactgc caaggattcc cagaagaaag 14820
tcagtgagat tgattaccgc ctgcgcctcc acgaatggat ttgccacccc gacttgcaag 14880
tcaatgatca cgtcaggaaa gtcacagatc agatcagcga tattgtatac aaggatgacc 14940
tcaactggct gaaaggcatt ggttgctacg tctgggacac tcctgaaatc ctccatgcca 15000
agcatgctta tgatctacgt gatgatatca agtataaagc tcacatgttg aaaacaagga 15060
atgactacaa gcttgtcaca gatacaccag tctacgtgca ggctgtcaaa agtgggaaac 15120
agctaagtga cgctgtctac cactatgact atgtgcacag tgtcagaggc aaagtggctc 15180
caactaccaa aaccgtggat ctggaccggg cccttcatgc atacaagctc cagagttcga 15240
atctatacaa aaccagcctg cgcaccctgc ccactggata tagacttcca ggtgacactc 15300
ctcacttcaa acacatcaag gacacccgtt acatgagcag ttatttcaag tacaaagaag 15360
cctatgaaca caccaaggca tatgggtata cacttggccc caaagatgtt ccatttgtcc 15420
acgtccggag agtcaacaat gttaccagcg agagactgta tcgggaattg taccacaaac 15480
tgaaagacaa gatccataca actcccgatc cccctgagat ccgccaagtc aagaagacac 15540
aagaggctgt cagtgagttg atctacaaat cagacttctt caagatgcag ggccacatga 15600
tctctctgcc atacacaccc caagtgatcc attgccgcta tgtgggagac atcaccagtg 15660
atattaaata caaagaggac ttgcaggtcc tgaagggatt tggctgcttc ctgtatgaca 15720
ctcctgacat ggtccgctcc cggcacctgc ggaagctctg gtctaattac ctatacactg 15780
ataaggcaag ggagatgcga gacaaataca aagtggtgct tgacactcca gaatacagaa 15840
aagtgcaaga actgaagaca catctgagtg agctggtcta cagagctgca ggcaagaagc 15900
agaagtcaat ctttacttca gttcctgata ctcctgatct tttaagagcc aagcgagggc 15960
agaagcttca gagtcagtat ctgtatgttg aacttgccac caaagagaga ccccatcatc 16020
acgctggaaa ccagaccaca gccttgaagc atgctaaaga cgtgaaggac atggtcagtg 16080
agaaaaagta caagattcaa tatgaaaaga tgaaagacaa gtacactccg gttccagata 16140
cgccaatcct catcagagcc aagagggctt actggaatgc cagtgatcta cgctacaaag 16200
aaacatttca aaagaccaaa gggaaatacc acacggtgaa agatgcccta gacattgtct 16260
atcatcgcaa agtcacagat gacatcagta aaataaaata caaggagaac tacatgagcc 16320
agttgggtat ctggaggtcc attcctgatc gtccagagca tttccaccac cgagcagtca 16380
ctgacacagt cagtgatgta aaatataaag aagacttgac ttggcttaaa ggcattggtt 16440

gctatgccta tgatacccct gatttcactc tggctgaaaa gaacaagact ctctacagca 16500
agtataagta taaagaagta tttgaaagga caaagtcaga tttcaagtat gttgccgact 16560
ctccgatcaa taggcatttc aagtatgcaa ctcaattgat gaatgagaaa aaatacagag 16620
ctgattatga gcagcggaaa gataaatacc acctggtagt cgatgagcct agacatctgc 16680
tggctaagac ccgcagcgac cagatcagtc agatcaaata caggaaaaac tatgaaaaat 16740
caaaggacaa atttacctca attgtggata ctccagaaca cctgcgtact acaaaagtca 16800
acaaacaaat cagcgatatc ctttataaat tggaatacaa caaggccaaa cccagaggct 16860
acaccacaat ccacgacacg cccatgttgc tgcatgtccg caaggttaaa gatgaagtca 16920
gtgatctgaa atacaaagaa gtataccaaa gaaataaatc caactgcacc attgagccag 16980
atgctgttca tatcaaagca gccaaggacg cctacaaagt caacaccaat ctggactata 17040
agaaacagta cgaagccaac aaagcccact ggaagtggac tcctgaccga ccggacttcc 17100
tccaggctgc caagtcatcc ctgcagcaaa gcgattttga atataagctg gaccgggagt 17160
tcctcaaggg ttgcaagctt tctgtcactg atgacaaaaa cacggtgctc gccctcagga 17220
atactttaat agaaagtgat ctgaaataca aagagaaaca tgtcaaggaa agaggaacct 17280
gccatgccgt acctgacacg cctcagatcc tgctggcgaa gactgtcagc aacctggtgt 17340
ctgagaacaa gtacaaggac catgtcaaga agcacttggc acagggctca tacacaacac 17400
taccagagac ccgggacact gttcacgtca aggaagtgac caagcatgtc agtgatacaa 17460
attacaaaaa gaagtttgtc aaggagaaag gaaaatccaa ctactccatc atgctggagc 17520
caccagaggt gaaacatgct atggaagtgg ccaagaagca aagtgatgtc gcttacagaa 17580
aagatgccaa agagaacctg cattacacca cagtggctga tcgaccagac atcaagaagg 17640
ccacacaggc agccaaacag gccagtgagg tggagtacag agccaagcac cgcaaggaag 17700
gcagccatgg cttaagcatg ctcggtcgcc cagacataga aatggccaag aaggcagcca 17760
agctgagcag ccaggttaaa taccgagaaa atttcgataa agaaaagggc aagacaccaa 17820
aatacaatcc aaaagacagc cagctctaca aagtcatgaa agatgctaat aatcttgcaa 17880
gtgaggttaa atacaaggct gacctgaaga aacttcacaa acccgtgact gacatgaagg 17940
agtctctgat catgaatcat gtcctgaata caagccaact tgccagttct taccagtaca 18000
agaagaagta tgagaagagt aaaggccact accacaccat acccgataat ctggagcagc 18060
ttcacctaaa agaggccaca gaattacaga gtatagtgaa atacaaagaa aagtatgaaa 18120
aggaacgagg aaaacccatg ctggactttg aaacaccaac gtacatcact gccaaagagt 18180
ctcagcagat gcagagtggg aaagaatata ggaaagatta tgaagagtcc attaaaggca 18240
gaaacctgac tggcctggag gtcacgccag ctttgttaca tgtcaaatat gcaactaaaa 18300
tagcaagcga gaaagagtac aggaaagatc tagaggaaag catccgtggg aagggcctca 18360
ctgaaatgga agatacacct gacatgctaa gagcaaagaa tgccactcaa atcctcaatg 18420
agaaagaata taagcgagac ctggaactgg aagtcaaagg aagaggcctg aatgccatgg 18480

ccaatgaaac tccggatttt atgagggcca ggaatgctac tgatattgcc agtcagatta 18540
agtataagca atcagcagaa atggagaaag ccaatttcac ttctgtggtt gatactccag 18600
agatcattca tgcccaacaa gtcaagaatc tttcaagcca gaaaaagtac aaggaagatg 18660
ctgagaagtc catgtcgtat tatgagactg ttttggacac cccagagata cagagagtcc 18720
gggagaacca aaagaacttc agccttctcc aataccagtg tgaccttaaa aacagtaaag 18780
gaaaaattac agttgttcaa gacacgccag aaatactgcg tgtaaaagaa aatcagaaga 18840
atttcagctc ggttttatat aaagaggatg tctcaccagg aacggctatc ggaaagacac 18900
ctgagatgat gagagtgaaa caaacacagg accacattag ctcggtgaag tataaggaag 18960
caataggaca aggaactcca atccctgacc tgcctgaagt gaaacgtgtg aaggagacgc 19020
agaagcacat tagctcggtt atgtacaaag aaaacttggg aacaggcatt ccaaccactg 19080
tgactccaga gattgagaga gtcaaacgca atcaagagaa ctttagctcg gttttgtaca 19140
aagaaaattt ggggaaagga atcccaacac ctatcactcc agagatggag agagtcaaac 19200
gcaatcaaga gaactttagc tcggtgttat acaaagaaaa catgggcaag ggaactcctt 19260
tacctgtcac tcccgagatg gagcgagtca aacacaatca agaaaatatt agctcggttt 19320
tgtacaaaga aaatgtgggg aaagccaccg caacccctgt cactcctgag atgcagagag 19380
tcaaacgcaa tcaagaaaac attagctcgg tgttatacaa agagaacctg gggaaagcaa 19440
cccccacacc ctttactcct gagatggaaa gagtgaaacg caatcaagaa aactttagct 19500
cggtattgta caaagagaac atgagaaaag caactccgac acctgttact ccagagatgg 19560
agagagctaa gcgcaaccaa gaaaacatta gctcggttct ttattctgat agtttccgga 19620
aacaaataca aggcaaagct gcctatgtat tggatacccc cgagatgaga cgggtgaggg 19680
agacccaacg gcacatctca acggtgaaat atcatgaaga ctttgagaaa cacaagggtt 19740
gcttcacacc agtggtgaca gatcctatca ctgaacgagt aaagaagaac atgcaggact 19800
tcagtgacat taactaccga ggtattcaga ggaaagtggt agaaatggaa caaaaacgga 19860
atgaccaaga tcaggagact attacaggtt tacgtgtctg gcgtactaat cctggttcgg 19920
tttttgacta tgatccagca gaagacaaca tccagtcccg aagcttacac atgattaatg 19980
tccaagctca gcgccggagc cgggagcagt cacgatctgc cagtgcacta agcgtcagtg 20040
ggggtgagga gaagtctgag cattcagaag caccagacca ccacctttcg acttacagcg 20100
acgggggtgt ctttgcagtc tcaacagctt acaaacatgc aaaaaccaca gagctcccac 20160
aacaacgatc atcttcagtt gctacccaac agacaacggt atcttccatc ccatctcatc 20220
catctactgc tggaaaaatc ttccgtgcca tgtatgacta tatggctgct gatgcagatg 20280
aggtgtcctt caaggatgga gatgccatca taaatgttca agcaattgat gaaggctgga 20340
tgtatggcac tgtgcagagg actggcagga ccggaatgct cccagccaac tacgttgaag 20400
ctatttaggc atttcaaagc atcacacttg tctgcaggac ttacagatcc tgcagtcaat 20460
gtttcggttt agactctcca ctgttaccta agttctcaag ctgcctatgg tttttctgtg 20520

tcaatgtgat ttatggtagt accatccttt ctcctttggg ttttaaaata agttgcagaa 20580
cagacacttt aaaagcttct gcaatattat ttctgtgcct agagtctttc tccattataa 20640
acatgtttta acattatttc ttttctaaaa cagggatttt gaatatgcca aacacattaa 20700
aggaaaaata gcagagatgt tcaccttttc cttgctgatt gctaatgctt attatttcta 20760
attcagttct gaagttataa acttataatc aatacaaacc agcaactaat aaaacctcta 20820
attctgcaaa aaaaaaaaa 20839

<210> 27
<211> 6669
<212> PRT
<213> homo sapiens

<220>
<221> misc_feature
<223> NEB gene product

<400> 27
Met Ala Asp Asp Glu Asp Tyr Glu Glu Val Val Glu Tyr Tyr Thr Glu
1               5                   10                  15

Glu Val Val Tyr Glu Glu Val Pro Gly Glu Thr Ile Thr Lys Ile Tyr
            20                  25                  30

Glu Thr Thr Thr Thr Arg Thr Ser Asp Tyr Glu Gln Ser Glu Thr Ser
        35                  40                  45

Lys Pro Ala Leu Ala Gln Pro Ala Leu Ala Gln Pro Ala Ser Ala Lys
    50                  55                  60

Pro Val Glu Arg Arg Lys Val Ile Arg Lys Lys Val Asp Pro Ser Lys
65                  70                  75                  80

Phe Met Thr Pro Tyr Ile Ala His Ser Gln Lys Met Gln Asp Leu Phe
                85                  90                  95

Ser Pro Asn Lys Tyr Lys Glu Lys Phe Glu Lys Thr Lys Gly Gln Pro
            100                 105                 110

Tyr Ala Ser Thr Thr Asp Thr Pro Glu Leu Arg Arg Ile Lys Lys Val
        115                 120                 125

Gln Asp Gln Leu Ser Glu Val Lys Tyr Arg Met Asp Gly Asp Val Ala
    130                 135                 140

Lys Thr Ile Cys His Val Asp Glu Lys Ala Lys Asp Ile Glu His Ala
145                 150                 155                 160

Lys Lys Val Ser Gln Gln Val Ser Lys Val Leu Tyr Lys Gln Asn Trp
                165                 170                 175

Glu Asp Thr Lys Asp Lys Tyr Leu Leu Pro Pro Asp Ala Pro Glu Leu
            180                 185                 190

Val Gln Ala Val Lys Asn Thr Ala Met Phe Ser Lys Lys Leu Tyr Thr
        195                 200                 205

Glu Asp Trp Glu Ala Asp Lys Ser Leu Phe Tyr Pro Tyr Asn Asp Ser
    210                 215                 220

Pro Glu Leu Arg Arg Val Ala Gln Ala Gln Lys Ala Leu Ser Asp Val
225                 230                 235                 240

Ala Tyr Lys Lys Gly Leu Ala Glu Gln Gln Ala Gln Phe Thr Pro Leu
                245                 250                 255

Ala Asp Pro Pro Asp Ile Glu Phe Ala Lys Lys Val Thr Asn Gln Val
            260                 265                 270

Ser Lys Gln Lys Tyr Lys Glu Asp Tyr Glu Asn Lys Ile Lys Gly Lys
        275                 280                 285

Trp Ser Glu Thr Pro Cys Phe Glu Val Ala Asn Ala Arg Met Asn Ala
    290                 295                 300

Asp Asn Ile Ser Thr Arg Lys Tyr Gln Glu Asp Phe Glu Asn Met Lys
305                 310                 315                 320

Asp Gln Ile Tyr Phe Met Gln Thr Glu Thr Pro Glu Tyr Lys Met Asn
                325                 330                 335

Lys Lys Ala Gly Val Ala Ala Ser Lys Val Lys Tyr Lys Glu Asp Tyr
            340                 345                 350

Glu Lys Asn Lys Gly Lys Ala Asp Tyr Asn Val Leu Pro Ala Ser Glu
        355                 360                 365

Asn Pro Gln Leu Arg Gln Leu Lys Ala Ala Gly Asp Ala Leu Ser Asp
    370                 375                 380

Lys Leu Tyr Lys Glu Asn Tyr Glu Lys Thr Lys Ala Lys Ser Ile Asn
385                 390                 395                 400

Tyr Cys Glu Thr Pro Lys Phe Lys Leu Asp Thr Val Leu Gln Asn Phe
                405                 410                 415

Ser Ser Asp Lys Lys Tyr Lys Asp Ser Tyr Leu Lys Asp Ile Leu Gly
            420                 425                 430

His Tyr Val Gly Ser Phe Glu Asp Pro Tyr His Ser His Cys Met Lys
        435                 440                 445

Val Thr Ala Gln Asn Ser Asp Lys Asn Tyr Lys Ala Glu Tyr Glu Glu
    450                 455                 460

Asp Arg Gly Lys Gly Phe Phe Pro Gln Thr Ile Thr Gln Glu Tyr Glu
465                 470                 475                 480

Ala Ile Lys Lys Leu Asp Gln Cys Lys Asp His Thr Tyr Lys Val His
                485                 490                 495

Pro Asp Lys Thr Lys Phe Thr Gln Val Thr Asp Ser Pro Val Leu Leu
            500                 505                 510

Gln Ala Gln Val Asn Ser Lys Gln Leu Ser Asp Leu Asn Tyr Lys Ala
        515                 520                 525

Lys His Glu Ser Glu Lys Phe Lys Cys His Ile Pro Pro Asp Thr Pro
    530                 535                 540

Ala Phe Ile Gln His Lys Val Asn Ala Tyr Asn Leu Ser Asp Asn Leu
545                 550                 555                 560

Tyr Lys Gln Asp Trp Glu Lys Ser Lys Ala Lys Lys Phe Asp Ile Lys
                565                 570                 575

Val Asp Ala Ile Pro Leu Leu Ala Ala Lys Ala Asn Thr Lys Asn Thr
            580                 585                 590

Ser Asp Val Met Tyr Lys Lys Asp Tyr Glu Lys Asn Lys Gly Lys Met
        595                 600                 605

Ile Gly Val Leu Ser Ile Asn Asp Asp Pro Lys Met Leu His Ser Leu
    610                 615                 620

Lys Val Ala Lys Asn Gln Ser Asp Arg Leu Tyr Lys Glu Asn Tyr Glu
625                 630                 635                 640

Lys Thr Lys Ala Lys Ser Met Asn Tyr Cys Glu Thr Pro Lys Tyr Gln
                645                 650                 655

Leu Asp Thr Gln Leu Lys Asn Phe Ser Glu Ala Arg Tyr Lys Asp Leu
            660                 665                 670

Tyr Val Lys Asp Val Leu Gly His Tyr Val Gly Ser Met Glu Asp Pro
        675                 680                 685

Tyr His Thr His Cys Met Lys Val Ala Ala Gln Asn Ser Asp Lys Ser
    690                 695                 700

Tyr Lys Ala Glu Tyr Glu Glu Asp Lys Gly Lys Cys Tyr Phe Pro Gln
705                 710                 715                 720

Thr Ile Thr Gln Glu Tyr Asp Ala Ile Lys Lys Leu Asp Gln Cys Lys
                725                 730                 735

Asp His Thr Tyr Lys Val His Pro Asp Lys Thr Lys Phe Thr Ala Val
            740                 745                 750

Thr Asp Ser Pro Val Leu Leu Gln Ala Gln Leu Asn Thr Lys Gln Leu
        755                 760                 765

Ser Asp Leu Asn Tyr Lys Ala Lys His Glu Gly Glu Arg Phe Lys Cys
    770                 775                 780

His Ile Pro Ala Asp Ala Pro Gln Phe Ile Gln His Arg Val Asn Ala
785                 790                 795                 800

Tyr Asn Leu Ser Asp Asn Val Tyr Lys Gln Asp Trp Glu Lys Ser Lys
                805                 810                 815

Ala Lys Lys Phe Asp Ile Lys Val Asp Ala Ile Pro Leu Leu Ala Ala
            820                 825                 830

Lys Ala Asn Thr Lys Asn Thr Ser Asp Val Met Tyr Lys Lys Asp Tyr
        835                 840                 845

Glu Lys Ser Lys Gly Lys Met Ile Gly Ala Leu Ser Ile Asn Asp Asp
    850                 855                 860

Pro Lys Met Leu His Ser Leu Lys Thr Ala Lys Asn Gln Ser Asp Arg
865                 870                 875                 880

Glu Tyr Arg Lys Asp Tyr Glu Lys Ser Lys Thr Ile Tyr Thr Ala Pro
                885                 890                 895

Leu Asp Met Leu Gln Val Thr Gln Ala Lys Lys Ser Gln Ala Ile Ala
            900                 905                 910

Ser Asp Val Asp Tyr Lys His Ile Leu His Ser Tyr Ser Tyr Pro Pro
        915                 920                 925

Asp Ser Ile Asn Val Asp Leu Ala Lys Lys Ala Tyr Ala Leu Gln Ser
    930                 935                 940

Asp Val Glu Tyr Lys Ala Asp Tyr Asn Ser Trp Met Lys Gly Cys Gly
945                 950                 955                 960

Trp Val Pro Phe Gly Ser Leu Glu Met Glu Lys Ala Lys Arg Ala Ser
                965                 970                 975

Asp Ile Leu Asn Glu Lys Lys Tyr Arg Gln His Pro Asp Thr Leu Lys
            980                 985                 990

Phe Thr Ser Ile Glu Asp Ala Pro  Ile Thr Val Gln Ser  Lys Ile Asn
        995                 1000                 1005

Gln Ala  Gln Arg Ser Asp Ile  Ala Tyr Lys Ala Lys  Gly Glu Glu
    1010                 1015                 1020

Ile Ile  His Asn Tyr Asn Leu  Pro Pro Asp Leu Pro  Gln Phe Ile
    1025                 1030                 1035

Gln Ala  Lys Val Asn Ala Tyr  Asn Ile Ser Glu Asn  Met Tyr Lys
    1040                 1045                 1050

Ala Asp  Leu Lys Asp Leu Ser  Lys Lys Gly Tyr Asp  Leu Arg Thr
    1055                 1060                 1065

Asp Ala  Ile Pro Ile Arg Ala  Ala Lys Ala Ala Arg  Gln Ala Ala
    1070                 1075                 1080

Ser Asp  Val Gln Tyr Lys Lys  Asp Tyr Glu Lys Ala  Lys Gly Lys
    1085                 1090                 1095

Met Val  Gly Phe Gln Ser Leu  Gln Asp Asp Pro Lys  Leu Val His
    1100                 1105                 1110

Tyr Met  Asn Val Ala Lys Ile  Gln Ser Asp Arg Glu  Tyr Lys Lys
    1115                 1120                 1125

Asp Tyr  Glu Lys Thr Lys Ser  Lys Tyr Asn Thr Pro  His Asp Met
    1130                 1135                 1140

Phe Asn  Val Val Ala Ala Lys  Lys Ala Gln Asp Val  Val Ser Asn
    1145                 1150                 1155

Val Asn  Tyr Lys His Ser Leu  His His Tyr Thr Tyr  Leu Pro Asp
    1160                 1165                 1170

Ala Met  Asp Leu Glu Leu Ser  Lys Asn Met Met Gln  Ile Gln Ser
    1175                 1180                 1185

Asp Asn  Val Tyr Lys Glu Asp  Tyr Asn Asn Trp Met  Lys Gly Ile
    1190                 1195                 1200

Gly Trp  Ile Pro Ile Gly Ser  Leu Asp Val Glu Lys  Val Lys Lys
    1205                 1210                 1215

Ala Gly Asp Ala Leu Asn Glu Lys Lys Tyr Arg Gln His Pro Asp
    1220                    1225                    1230

Thr Leu Lys Phe Thr Ser Ile Val Asp Ser Pro Val Met Val Gln
    1235                    1240                    1245

Ala Lys Gln Asn Thr Lys Gln Val Ser Asp Ile Leu Tyr Lys Ala
    1250                    1255                    1260

Lys Gly Glu Asp Val Lys His Lys Tyr Thr Met Ser Pro Asp Leu
    1265                    1270                    1275

Pro Gln Phe Leu Gln Ala Lys Cys Asn Ala Tyr Ser Ile Ser Asp
    1280                    1285                    1290

Val Cys Tyr Lys Arg Asp Trp His Asp Leu Ile Arg Lys Gly Asn
    1295                    1300                    1305

Asn Val Leu Gly Asp Ala Ile Pro Ile Thr Ala Ala Lys Ala Ser
    1310                    1315                    1320

Arg Asn Ile Ala Ser Asp Tyr Lys Tyr Lys Glu Ala Tyr Glu Lys
    1325                    1330                    1335

Ser Lys Gly Lys His Val Gly Phe Arg Ser Leu Gln Asp Asp Pro
    1340                    1345                    1350

Lys Leu Val His Tyr Met Asn Val Ala Lys Leu Gln Ser Asp Arg
    1355                    1360                    1365

Glu Tyr Lys Lys Asn Tyr Glu Asn Thr Lys Thr Ser Tyr His Thr
    1370                    1375                    1380

Pro Gly Asp Met Val Thr Ile Thr Ala Ala Lys Met Ala Gln Asp
    1385                    1390                    1395

Val Ala Thr Asn Val Asn Tyr Lys Gln Pro Leu His His Tyr Thr
    1400                    1405                    1410

Tyr Leu Pro Asp Ala Met Ser Leu Glu His Thr Arg Asn Val Asn
    1415                    1420                    1425

Gln Ile Gln Ser Asp Asn Val Tyr Lys Asp Glu Tyr Asn Ser Phe
    1430                    1435                    1440

Leu Lys Gly Ile Gly Trp Ile Pro Ile Gly Ser Leu Glu Val Glu
    1445                    1450                    1455

Lys Val Lys Lys Ala Gly Asp Ala Leu Asn Glu Arg Lys Tyr Arg
    1460                    1465                    1470

Gln His Pro Asp Thr Val Lys Phe Thr Ser Val Pro Asp Ser Met
    1475                1480                1485

Gly Met Met Leu Ala Gln His Asn Thr Lys Gln Leu Ser Asp Leu
    1490                1495                1500

Asn Tyr Lys Val Glu Gly Glu Lys Leu Lys His Lys Tyr Thr Ile
    1505                1510                1515

Asp Pro Glu Leu Pro Gln Phe Ile Gln Ala Lys Val Asn Ala Leu
    1520                1525                1530

Asn Met Ser Asp Ala His Tyr Lys Ala Asp Trp Lys Lys Thr Ile
    1535                1540                1545

Arg Lys Gly Tyr Asp Leu Arg Pro Asp Ala Ile Pro Ile Val Ala
    1550                1555                1560

Ala Lys Ser Ser Arg Asn Ile Ala Ser Asp Cys Lys Tyr Lys Glu
    1565                1570                1575

Ala Tyr Glu Lys Ala Lys Gly Lys Gln Val Gly Phe Leu Ser Leu
    1580                1585                1590

Gln Asp Asp Pro Lys Leu Val His Tyr Met Asn Val Ala Lys Ile
    1595                1600                1605

Gln Ser Asp Arg Glu Tyr Lys Lys Gly Tyr Glu Ala Ser Lys Thr
    1610                1615                1620

Lys Tyr His Thr Pro Leu Asp Met Val Ser Val Thr Ala Ala Lys
    1625                1630                1635

Lys Ser Gln Glu Val Ala Thr Asn Ala Asn Tyr Arg Gln Ser Tyr
    1640                1645                1650

His His Tyr Thr Leu Leu Pro Asp Ala Leu Asn Val Glu His Ser
    1655                1660                1665

Arg Asn Ala Met Gln Ile Gln Ser Asp Asn Leu Tyr Lys Ser Asp
    1670                1675                1680

Phe Thr Asn Trp Met Lys Gly Ile Gly Trp Val Pro Ile Glu Ser
    1685                1690                1695

Leu Glu Val Glu Lys Ala Lys Lys Ala Gly Glu Ile Leu Ser Glu
    1700                1705                1710

Lys Lys Tyr Arg Gln His Pro Glu Lys Leu Lys Phe Thr Tyr Ala
    1715                1720                1725

Met Asp Thr Met Glu Gln Ala Leu Asn Lys Ser Asn Lys Leu Asn
    1730                1735                1740

Met Asp Lys Arg Leu Tyr Thr Glu Lys Trp Asn Lys Asp Lys Thr
    1745                1750                1755

Thr Ile His Val Met Pro Asp Thr Pro Asp Ile Leu Leu Ser Arg
    1760                1765                1770

Val Asn Gln Ile Thr Met Ser Asp Lys Leu Tyr Lys Ala Gly Trp
    1775                1780                1785

Glu Glu Glu Lys Lys Lys Gly Tyr Asp Leu Arg Pro Asp Ala Ile
    1790                1795                1800

Ala Ile Lys Ala Ala Arg Ala Ser Arg Asp Ile Ala Ser Asp Tyr
    1805                1810                1815

Lys Tyr Lys Lys Ala Tyr Glu Gln Ala Lys Gly Lys His Ile Gly
    1820                1825                1830

Phe Arg Ser Leu Glu Asp Asp Pro Lys Leu Val His Phe Met Gln
    1835                1840                1845

Val Ala Lys Met Gln Ser Asp Arg Glu Tyr Lys Lys Gly Tyr Glu
    1850                1855                1860

Lys Ser Lys Thr Ser Phe His Thr Pro Val Asp Met Leu Ser Val
    1865                1870                1875

Val Ala Ala Lys Lys Ser Gln Glu Val Ala Thr Asn Ala Asn Tyr
    1880                1885                1890

Arg Asn Val Ile His Thr Tyr Asn Met Leu Pro Asp Ala Met Ser
    1895                1900                1905

Phe Glu Leu Ala Lys Asn Met Met Gln Ile Gln Ser Asp Asn Gln
    1910                1915                1920

Tyr Lys Ala Asp Tyr Ala Asp Phe Met Lys Gly Ile Gly Trp Leu
    1925                1930                1935

Pro Leu Gly Ser Leu Glu Ala Glu Lys Asn Lys Lys Ala Met Glu
    1940                1945                1950

Ile Ile Ser Glu Lys Lys Tyr Arg Gln His Pro Asp Thr Leu Lys
    1955                1960                1965

Tyr Ser Thr Leu Met Asp Ser Met Asn Met Val Leu Ala Gln Asn
    1970                1975                1980

Asn Ala Lys Ile Met Asn Glu His Leu Tyr Lys Gln Ala Trp Glu
    1985                1990                1995

Ala Asp Lys Thr Lys Val His Ile Met Pro Asp Ile Pro Gln Ile
    2000                2005                2010

Ile Leu Ala Lys Ala Asn Ala Ile Asn Ile Ser Asp Lys Leu Tyr
    2015                2020                2025

Lys Leu Ser Leu Glu Glu Ser Lys Lys Lys Gly Tyr Asp Leu Arg
    2030                2035                2040

Pro Asp Ala Ile Pro Ile Lys Ala Ala Lys Ala Ser Arg Asp Ile
    2045                2050                2055

Ala Ser Asp Tyr Lys Tyr Lys Tyr Asn Tyr Glu Lys Gly Lys Gly
    2060                2065                2070

Lys Met Val Gly Phe Arg Ser Leu Glu Asp Asp Pro Lys Leu Val
    2075                2080                2085

His Ser Met Gln Val Ala Lys Met Gln Ser Asp Arg Glu Tyr Lys
    2090                2095                2100

Lys Asn Tyr Glu Asn Thr Lys Thr Ser Tyr His Thr Pro Ala Asp
    2105                2110                2115

Met Leu Ser Val Thr Ala Ala Lys Asp Ala Gln Ala Asn Ile Thr
    2120                2125                2130

Asn Thr Asn Tyr Lys His Leu Ile His Lys Tyr Ile Leu Leu Pro
    2135                2140                2145

Asp Ala Met Asn Ile Glu Leu Thr Arg Asn Met Asn Arg Ile Gln
    2150                2155                2160

Ser Asp Asn Glu Tyr Lys Gln Asp Tyr Asn Glu Trp Tyr Lys Gly
    2165                2170                2175

Leu Gly Trp Ser Pro Ala Gly Ser Leu Glu Val Glu Lys Ala Lys
    2180                2185                2190

Lys Ala Thr Glu Tyr Ala Ser Asp Gln Lys Tyr Arg Gln His Pro
    2195                2200                2205

Ser Asn Phe Gln Phe Lys Lys Leu Thr Asp Ser Met Asp Met Val
    2210                2215                2220

Leu Ala Lys Gln Asn Ala His Thr Met Asn Lys His Leu Tyr Thr
    2225                2230                2235

Ile Asp Trp Asn Lys Asp Lys Thr Lys Ile His Val Met Pro Asp
    2240                    2245                    2250

Thr Pro Asp Ile Leu Gln Ala Lys Gln Asn Gln Thr Leu Tyr Ser
    2255                    2260                    2265

Gln Lys Leu Tyr Lys Leu Gly Trp Glu Glu Ala Leu Lys Lys Gly
    2270                    2275                    2280

Tyr Asp Leu Pro Val Asp Ala Ile Ser Val Gln Leu Ala Lys Ala
    2285                    2290                    2295

Ser Arg Asp Ile Ala Ser Asp Tyr Lys Tyr Lys Gln Gly Tyr Arg
    2300                    2305                    2310

Lys Gln Leu Gly His His Val Gly Phe Arg Ser Leu Gln Asp Asp
    2315                    2320                    2325

Pro Lys Leu Val Leu Ser Met Asn Val Ala Lys Met Gln Ser Glu
    2330                    2335                    2340

Arg Glu Tyr Lys Lys Asp Phe Glu Lys Trp Lys Thr Lys Phe Ser
    2345                    2350                    2355

Ser Pro Val Asp Met Leu Gly Val Val Leu Ala Lys Lys Cys Gln
    2360                    2365                    2370

Glu Leu Val Ser Asp Val Asp Tyr Lys Asn Tyr Leu His Gln Trp
    2375                    2380                    2385

Thr Cys Leu Pro Asp Gln Asn Asp Val Val Gln Ala Lys Lys Val
    2390                    2395                    2400

Tyr Glu Leu Gln Ser Glu Asn Leu Tyr Lys Ser Asp Leu Glu Trp
    2405                    2410                    2415

Leu Arg Gly Ile Gly Trp Ser Pro Leu Gly Ser Leu Glu Ala Glu
    2420                    2425                    2430

Lys Asn Lys Arg Ala Ser Glu Ile Ile Ser Glu Lys Lys Tyr Arg
    2435                    2440                    2445

Gln Pro Pro Asp Arg Asn Lys Phe Thr Ser Ile Pro Asp Ala Met
    2450                    2455                    2460

Asp Ile Val Leu Ala Lys Thr Asn Ala Lys Asn Arg Ser Asp Arg
    2465                    2470                    2475

Leu Tyr Arg Glu Ala Trp Asp Lys Asp Lys Thr Gln Ile His Ile
    2480                    2485                    2490

Met Pro Asp Thr Pro Asp Ile Val Leu Ala Lys Ala Asn Leu Ile
    2495                2500                2505

Asn Thr Ser Asp Lys Leu Tyr Arg Met Gly Tyr Glu Glu Leu Lys
    2510                2515                2520

Arg Lys Gly Tyr Asp Leu Pro Val Asp Ala Ile Pro Ile Lys Ala
    2525                2530                2535

Ala Lys Ala Ser Arg Glu Ile Ala Ser Glu Tyr Lys Tyr Lys Glu
    2540                2545                2550

Gly Phe Arg Lys Gln Leu Gly His His Ile Gly Ala Arg Asn Ile
    2555                2560                2565

Glu Asp Asp Pro Lys Met Met Trp Ser Met His Val Ala Lys Ile
    2570                2575                2580

Gln Ser Asp Arg Glu Tyr Lys Lys Asp Phe Glu Lys Trp Lys Thr
    2585                2590                2595

Lys Phe Ser Ser Pro Val Asp Met Leu Gly Val Val Leu Ala Tyr
    2600                2605                2610

Lys Cys Gln Thr Leu Val Ser Asp Val Asp Tyr Lys Asn Tyr Leu
    2615                2620                2625

His Gln Trp Thr Cys Leu Pro Asp Gln Ser Asp Val Ile His Ala
    2630                2635                2640

Arg Gln Ala Tyr Asp Leu Gln Ser Asp Asn Leu Tyr Lys Ser Asp
    2645                2650                2655

Leu Gln Trp Leu Lys Gly Ile Gly Trp Met Thr Ser Gly Ser Leu
    2660                2665                2670

Glu Asp Glu Lys Asn Lys Arg Ala Thr Gln Ile Leu Ser Asp His
    2675                2680                2685

Val Tyr Arg Gln His Pro Asp Gln Phe Lys Phe Ser Ser Leu Met
    2690                2695                2700

Asp Ser Ile Pro Met Val Leu Ala Lys Asn Asn Ala Ile Thr Met
    2705                2710                2715

Asn His Arg Leu Tyr Thr Glu Ala Trp Asp Lys Asp Lys Thr Thr
    2720                2725                2730

Val His Ile Met Pro Asp Thr Pro Glu Val Leu Leu Ala Lys Gln
    2735                2740                2745

Asn Lys Val Asn Tyr Ser Glu Lys Leu Tyr Lys Leu Gly Leu Glu
    2750                2755                2760

Glu Ala Lys Arg Lys Gly Tyr Asp Met Arg Val Asp Ala Ile Pro
    2765                2770                2775

Ile Lys Ala Ala Lys Ala Ser Arg Asp Ile Ala Ser Glu Phe Lys
    2780                2785                2790

Tyr Lys Glu Gly Tyr Arg Lys Gln Leu Gly His His Ile Gly Ala
    2795                2800                2805

Arg Ala Ile Arg Asp Asp Pro Lys Met Met Trp Ser Met His Val
    2810                2815                2820

Ala Lys Ile Gln Ser Asp Arg Glu Tyr Lys Lys Asp Phe Glu Lys
    2825                2830                2835

Trp Lys Thr Lys Phe Ser Ser Pro Val Asp Met Leu Gly Val Val
    2840                2845                2850

Leu Ala Lys Lys Cys Gln Thr Leu Val Ser Asp Val Asp Tyr Lys
    2855                2860                2865

Asn Tyr Leu His Gln Trp Thr Cys Leu Pro Asp Gln Ser Asp Val
    2870                2875                2880

Ile His Ala Arg Gln Ala Tyr Asp Leu Gln Ser Asp Asn Met Tyr
    2885                2890                2895

Lys Ser Asp Leu Gln Trp Met Arg Gly Ile Gly Trp Val Ser Ile
    2900                2905                2910

Gly Ser Leu Asp Val Glu Lys Cys Lys Arg Ala Thr Glu Ile Leu
    2915                2920                2925

Ser Asp Lys Ile Tyr Arg Gln Pro Pro Asp Arg Phe Lys Phe Thr
    2930                2935                2940

Ser Val Thr Asp Ser Leu Glu Gln Val Leu Ala Lys Asn Asn Ala
    2945                2950                2955

Leu Asn Met Asn Lys Arg Leu Tyr Thr Glu Ala Trp Asp Lys Asp
    2960                2965                2970

Lys Thr Gln Ile His Ile Met Pro Asp Thr Pro Glu Ile Met Leu
    2975                2980                2985

Ala Arg Gln Asn Lys Ile Asn Tyr Ser Glu Thr Leu Tyr Lys Leu
    2990                2995                3000

Ala Asn Glu Glu Ala Lys Lys Lys Gly Tyr Asp Leu Arg Ser Asp
    3005                3010                3015

Ala Ile Pro Ile Val Ala Ala Lys Ala Ser Arg Asp Val Ile Ser
    3020                3025                3030

Asp Tyr Lys Tyr Lys Asp Gly Tyr Arg Lys Gln Leu Gly His His
    3035                3040                3045

Ile Gly Ala Arg Asn Ile Glu Asp Asp Pro Lys Met Met Trp Ser
    3050                3055                3060

Met His Val Ala Lys Ile Gln Ser Asp Arg Glu Tyr Lys Lys Asp
    3065                3070                3075

Phe Glu Lys Trp Lys Thr Lys Phe Ser Ser Pro Val Asp Met Leu
    3080                3085                3090

Gly Val Val Leu Ala Lys Lys Cys Gln Thr Leu Val Ser Asp Val
    3095                3100                3105

Asp Tyr Lys Asn Tyr Leu His Glu Trp Thr Cys Leu Pro Asp Gln
    3110                3115                3120

Asn Asp Val Ile His Ala Arg Gln Ala Tyr Asp Leu Gln Ser Asp
    3125                3130                3135

Asn Ile Tyr Lys Ser Asp Leu Gln Trp Leu Arg Gly Ile Gly Trp
    3140                3145                3150

Val Pro Ile Gly Ser Met Asp Val Val Lys Cys Lys Arg Ala Ala
    3155                3160                3165

Glu Ile Leu Ser Asp Asn Ile Tyr Arg Gln Pro Pro Asp Lys Leu
    3170                3175                3180

Lys Phe Thr Ser Val Thr Asp Ser Leu Glu Gln Val Leu Ala Lys
    3185                3190                3195

Asn Asn Ala Leu Asn Met Asn Lys Arg Leu Tyr Thr Glu Ala Trp
    3200                3205                3210

Asp Lys Asp Lys Thr Gln Val His Ile Met Pro Asp Thr Pro Glu
    3215                3220                3225

Ile Met Leu Ala Arg Gln Asn Lys Ile Asn Tyr Ser Glu Ser Leu
    3230                3235                3240

Tyr Arg Gln Ala Met Glu Glu Ala Lys Lys Glu Gly Tyr Asp Leu
    3245                3250                3255

Arg Ser Asp Ala Ile Pro Ile Val Ala Ala Lys Ala Ser Arg Asp
    3260                3265                3270

Ile Ala Ser Asp Tyr Lys Tyr Lys Glu Ala Tyr Arg Lys Gln Leu
    3275                3280                3285

Gly His His Ile Gly Ala Arg Ala Val His Asp Asp Pro Lys Ile
    3290                3295                3300

Met Trp Ser Leu His Ile Ala Lys Val Gln Ser Asp Arg Glu Tyr
    3305                3310                3315

Lys Lys Asp Phe Glu Lys Tyr Lys Thr Arg Tyr Ser Ser Pro Val
    3320                3325                3330

Asp Met Leu Gly Ile Val Leu Ala Lys Lys Cys Gln Thr Leu Val
    3335                3340                3345

Ser Asp Val Asp Tyr Lys His Pro Leu His Glu Cys Ile Cys Leu
    3350                3355                3360

Pro Asp Gln Asn Asp Ile Ile His Ala Arg Lys Ala Tyr Asp Leu
    3365                3370                3375

Gln Ser Asp Asn Leu Tyr Lys Ser Asp Leu Glu Trp Met Lys Gly
    3380                3385                3390

Ile Gly Trp Val Pro Ile Asp Ser Leu Glu Val Val Arg Ala Lys
    3395                3400                3405

Arg Ala Gly Glu Leu Leu Ser Asp Thr Ile Tyr Arg Gln Arg Pro
    3410                3415                3420

Glu Thr Leu Lys Phe Thr Ser Ile Thr Asp Thr Pro Glu Gln Val
    3425                3430                3435

Leu Ala Lys Asn Asn Ala Leu Asn Met Asn Lys Arg Leu Tyr Thr
    3440                3445                3450

Glu Ala Trp Asp Asn Asp Lys Lys Thr Ile His Val Met Pro Asp
    3455                3460                3465

Thr Pro Glu Ile Met Leu Ala Lys Leu Asn Arg Ile Asn Tyr Ser
    3470                3475                3480

Asp Lys Leu Tyr Lys Leu Ala Leu Glu Glu Ser Lys Lys Glu Gly
    3485                3490                3495

Tyr Asp Leu Arg Leu Asp Ala Ile Pro Ile Gln Ala Ala Lys Ala
    3500                3505                3510

Ser Arg Asp Ile Ala Ser Asp Tyr Lys Tyr Lys Glu Gly Tyr Arg
    3515                3520                3525

Lys Gln Leu Gly His His Ile Gly Ala Arg Asn Ile Lys Asp Asp
    3530                3535                3540

Pro Lys Met Met Trp Ser Ile His Val Ala Lys Ile Gln Ser Asp
    3545                3550                3555

Arg Glu Tyr Lys Lys Glu Phe Glu Lys Trp Lys Thr Lys Phe Ser
    3560                3565                3570

Ser Pro Val Asp Met Leu Gly Val Val Leu Ala Lys Lys Cys Gln
    3575                3580                3585

Ile Leu Val Ser Asp Ile Asp Tyr Lys His Pro Leu His Glu Trp
    3590                3595                3600

Thr Cys Leu Pro Asp Gln Asn Asp Val Ile Gln Ala Arg Lys Ala
    3605                3610                3615

Tyr Asp Leu Gln Ser Asp Ala Ile Tyr Lys Ser Asp Leu Glu Trp
    3620                3625                3630

Leu Arg Gly Ile Gly Trp Val Pro Ile Gly Ser Val Glu Val Glu
    3635                3640                3645

Lys Val Lys Arg Ala Gly Glu Ile Leu Ser Asp Arg Lys Tyr Arg
    3650                3655                3660

Gln Pro Ala Asp Gln Leu Lys Phe Thr Cys Ile Thr Asp Thr Pro
    3665                3670                3675

Glu Ile Val Leu Ala Lys Asn Asn Ala Leu Thr Met Ser Lys His
    3680                3685                3690

Leu Tyr Thr Glu Ala Trp Asp Ala Asp Lys Thr Ser Ile His Val
    3695                3700                3705

Met Pro Asp Thr Pro Asp Ile Leu Leu Ala Lys Ser Asn Ser Ala
    3710                3715                3720

Asn Ile Ser Gln Lys Leu Tyr Thr Lys Gly Trp Asp Glu Ser Lys
    3725                3730                3735

Met Lys Asp Tyr Asp Leu Arg Ala Asp Ala Ile Ser Ile Lys Ser
    3740                3745                3750

Ala Lys Ala Ser Arg Asp Ile Ala Ser Asp Tyr Lys Tyr Lys Glu
    3755                3760                3765

Ala Tyr Glu Lys Gln Lys Gly His His Ile Gly Ala Gln Ser Ile
    3770                3775                3780

Glu Asp Asp Pro Lys Ile Met Cys Ala Ile His Ala Glu Lys Ile
    3785                3790                3795

Gln Ser Glu Arg Glu Tyr Lys Lys Glu Phe Gln Lys Trp Lys Thr
    3800                3805                3810

Lys Phe Ser Ser Pro Val Asp Met Leu Ser Ile Leu Leu Ala Lys
    3815                3820                3825

Lys Cys Gln Thr Leu Val Thr Asp Ile Tyr Tyr Arg Asn Tyr Leu
    3830                3835                3840

His Glu Trp Thr Cys Met Pro Asp Gln Asn Asp Ile Ile Gln Ala
    3845                3850                3855

Lys Lys Ala Tyr Asp Leu Gln Ser Asp Ala Leu Tyr Lys Ala Asp
    3860                3865                3870

Leu Glu Trp Leu Arg Gly Ile Gly Trp Met Pro Gln Gly Ser Pro
    3875                3880                3885

Glu Val Leu Arg Val Lys Asn Ala Gln Asn Ile Phe Cys Asp Ser
    3890                3895                3900

Val Tyr Arg Thr Pro Val Val Asn Leu Lys Tyr Thr Ser Ile Val
    3905                3910                3915

Asp Thr Pro Glu Val Val Leu Ala Lys Ser Asn Ala Glu Asn Ile
    3920                3925                3930

Ser Ile Pro Lys Tyr Arg Glu Val Trp Asp Lys Asp Lys Thr Ser
    3935                3940                3945

Ile His Ile Met Pro Asp Thr Pro Glu Ile Asn Leu Ala Arg Ala
    3950                3955                3960

Asn Ala Leu Asn Val Ser Asn Lys Leu Tyr Arg Glu Gly Trp Asp
    3965                3970                3975

Glu Met Lys Ala Gly Cys Asp Val Arg Leu Asp Ala Ile Pro Ile
    3980                3985                3990

Gln Ala Ala Lys Ala Ser Arg Glu Ile Ala Ser Asp Tyr Lys Tyr
    3995                4000                4005

Lys Leu Asp His Glu Lys Gln Lys Gly His Tyr Val Gly Thr Leu
    4010                4015                4020

Thr Ala Arg Asp Asp Asn Lys Ile Arg Trp Ala Leu Ile Ala Asp
    4025                    4030                4035

Lys Leu Gln Asn Glu Arg Glu Tyr Arg Leu Asp Trp Ala Lys Trp
    4040                    4045                4050

Lys Ala Lys Ile Gln Ser Pro Val Asp Met Leu Ser Ile Leu His
    4055                    4060                4065

Ser Lys Asn Ser Gln Ala Leu Val Ser Asp Met Asp Tyr Arg Asn
    4070                    4075                4080

Tyr Leu His Gln Trp Thr Cys Met Pro Asp Gln Asn Asp Val Ile
    4085                    4090                4095

Gln Ala Lys Lys Ala Tyr Glu Leu Gln Ser Asp Asn Val Tyr Lys
    4100                    4105                4110

Ala Asp Leu Glu Trp Leu Arg Gly Ile Gly Trp Met Pro Asn Asp
    4115                    4120                4125

Ser Val Ser Val Asn His Ala Lys His Ala Ala Asp Ile Phe Ser
    4130                    4135                4140

Glu Lys Lys Tyr Arg Thr Lys Ile Glu Thr Leu Asn Phe Thr Pro
    4145                    4150                4155

Val Asp Asp Arg Val Asp Tyr Val Thr Ala Lys Gln Ser Gly Glu
    4160                    4165                4170

Ile Leu Asp Asp Ile Lys Tyr Arg Lys Asp Trp Asn Ala Thr Lys
    4175                    4180                4185

Ser Lys Tyr Thr Leu Thr Glu Thr Pro Leu Leu His Thr Ala Gln
    4190                    4195                4200

Glu Ala Ala Arg Ile Leu Asp Gln Tyr Leu Tyr Lys Glu Gly Trp
    4205                    4210                4215

Glu Arg Gln Lys Ala Thr Gly Tyr Ile Leu Pro Pro Asp Ala Val
    4220                    4225                4230

Pro Phe Val His Ala His His Cys Asn Asp Val Gln Ser Glu Leu
    4235                    4240                4245

Lys Tyr Lys Ala Glu His Val Lys Gln Lys Gly His Tyr Val Gly
    4250                    4255                4260

Val Pro Thr Met Arg Asp Asp Pro Lys Leu Val Trp Phe Glu His
    4265                    4270                4275

Ala Gly Gln Ile Gln Asn Glu Arg Leu Tyr Lys Glu Asp Tyr His
    4280                4285                4290

Lys Thr Lys Ala Lys Ile Asn Ile Pro Ala Asp Met Val Ser Val
    4295                4300                4305

Leu Ala Ala Lys Gln Gly Gln Thr Leu Val Ser Asp Ile Asp Tyr
    4310                4315                4320

Arg Asn Tyr Leu His Gln Trp Met Cys His Pro Asp Gln Asn Asp
    4325                4330                4335

Val Ile Gln Ala Arg Lys Ala Tyr Asp Leu Gln Ser Asp Asn Val
    4340                4345                4350

Tyr Arg Ala Asp Leu Glu Trp Leu Arg Gly Ile Gly Trp Ile Pro
    4355                4360                4365

Leu Asp Ser Val Asp His Val Arg Val Thr Lys Asn Gln Glu Met
    4370                4375                4380

Met Ser Gln Ile Lys Tyr Lys Lys Asn Ala Leu Glu Asn Tyr Pro
    4385                4390                4395

Asn Phe Thr Ser Val Val Asp Pro Pro Glu Ile Val Leu Ala Lys
    4400                4405                4410

Ile Asn Ser Val Asn Gln Ser Asp Val Lys Tyr Lys Glu Thr Phe
    4415                4420                4425

Asn Lys Ala Lys Gly Lys Tyr Thr Phe Ser Pro Asp Thr Pro His
    4430                4435                4440

Ile Ser His Ser Lys Asp Met Gly Lys Leu Tyr Ser Thr Ile Leu
    4445                4450                4455

Tyr Lys Gly Ala Trp Glu Gly Thr Lys Ala Tyr Gly Tyr Thr Leu
    4460                4465                4470

Asp Glu Arg Tyr Ile Pro Ile Val Gly Ala Lys His Ala Asp Leu
    4475                4480                4485

Val Asn Ser Glu Leu Lys Tyr Lys Glu Thr Tyr Glu Lys Gln Lys
    4490                4495                4500

Gly His Tyr Leu Ala Gly Lys Val Ile Gly Glu Phe Pro Gly Val
    4505                4510                4515

Val His Cys Leu Asp Phe Gln Lys Met Arg Ser Ala Leu Asn Tyr
    4520                4525                4530

Arg Lys His Tyr Glu Asp Thr Lys Ala Asn Val His Ile Pro Asn
    4535                4540                4545

Asp Met Met Asn His Val Leu Ala Lys Arg Cys Gln Tyr Ile Leu
    4550                4555                4560

Ser Asp Leu Glu Tyr Arg His Tyr Phe His Gln Trp Thr Ser Leu
    4565                4570                4575

Leu Glu Glu Pro Asn Val Ile Arg Val Arg Asn Ala Gln Glu Ile
    4580                4585                4590

Leu Ser Asp Asn Val Tyr Lys Asp Asp Leu Asn Trp Leu Lys Gly
    4595                4600                4605

Ile Gly Cys Tyr Val Trp Asp Thr Pro Gln Ile Leu His Ala Lys
    4610                4615                4620

Lys Ser Tyr Asp Leu Gln Ser Gln Leu Gln Tyr Thr Ala Ala Gly
    4625                4630                4635

Lys Glu Asn Leu Gln Asn Tyr Asn Leu Val Thr Asp Thr Pro Leu
    4640                4645                4650

Tyr Val Thr Ala Val Gln Ser Gly Ile Asn Ala Ser Glu Val Lys
    4655                4660                4665

Tyr Lys Glu Asn Tyr His Gln Ile Lys Asp Lys Tyr Thr Thr Val
    4670                4675                4680

Leu Glu Thr Val Asp Tyr Asp Arg Thr Arg Asn Leu Lys Asn Leu
    4685                4690                4695

Tyr Ser Ser Asn Leu Tyr Lys Glu Ala Trp Asp Arg Val Lys Ala
    4700                4705                4710

Thr Ser Tyr Ile Leu Pro Ser Ser Thr Leu Ser Leu Thr His Ala
    4715                4720                4725

Lys Asn Gln Lys His Leu Ala Ser His Ile Lys Tyr Arg Glu Glu
    4730                4735                4740

Tyr Glu Lys Phe Lys Ala Leu Tyr Thr Leu Pro Arg Ser Val Asp
    4745                4750                4755

Asp Asp Pro Asn Thr Ala Arg Cys Leu Arg Val Gly Lys Leu Asn
    4760                4765                4770

Ile Asp Arg Leu Tyr Arg Ser Val Tyr Glu Lys Asn Lys Met Lys
    4775                4780                4785

Ile His Ile Val Pro Asp Met Val Glu Met Val Thr Ala Lys Asp
    4790                4795                4800

Ser Gln Lys Lys Val Ser Glu Ile Asp Tyr Arg Leu Arg Leu His
    4805                4810                4815

Glu Trp Ile Cys His Pro Asp Leu Gln Val Asn Asp His Val Arg
    4820                4825                4830

Lys Val Thr Asp Gln Ile Ser Asp Ile Val Tyr Lys Asp Asp Leu
    4835                4840                4845

Asn Trp Leu Lys Gly Ile Gly Cys Tyr Val Trp Asp Thr Pro Glu
    4850                4855                4860

Ile Leu His Ala Lys His Ala Tyr Asp Leu Arg Asp Asp Ile Lys
    4865                4870                4875

Tyr Lys Ala His Met Leu Lys Thr Arg Asn Asp Tyr Lys Leu Val
    4880                4885                4890

Thr Asp Thr Pro Val Tyr Val Gln Ala Val Lys Ser Gly Lys Gln
    4895                4900                4905

Leu Ser Asp Ala Val Tyr His Tyr Asp Tyr Val His Ser Val Arg
    4910                4915                4920

Gly Lys Val Ala Pro Thr Thr Lys Thr Val Asp Leu Asp Arg Ala
    4925                4930                4935

Leu His Ala Tyr Lys Leu Gln Ser Ser Asn Leu Tyr Lys Thr Ser
    4940                4945                4950

Leu Arg Thr Leu Pro Thr Gly Tyr Arg Leu Pro Gly Asp Thr Pro
    4955                4960                4965

His Phe Lys His Ile Lys Asp Thr Arg Tyr Met Ser Ser Tyr Phe
    4970                4975                4980

Lys Tyr Lys Glu Ala Tyr Glu His Thr Lys Ala Tyr Gly Tyr Thr
    4985                4990                4995

Leu Gly Pro Lys Asp Val Pro Phe Val His Val Arg Arg Val Asn
    5000                5005                5010

Asn Val Thr Ser Glu Arg Leu Tyr Arg Glu Leu Tyr His Lys Leu
    5015                5020                5025

Lys Asp Lys Ile His Thr Thr Pro Asp Pro Pro Glu Ile Arg Gln
    5030                5035                5040

Val Lys Lys Thr Gln Glu Ala Val Ser Glu Leu Ile Tyr Lys Ser
    5045                5050                5055

Asp Phe Phe Lys Met Gln Gly His Met Ile Ser Leu Pro Tyr Thr
    5060                5065                5070

Pro Gln Val Ile His Cys Arg Tyr Val Gly Asp Ile Thr Ser Asp
    5075                5080                5085

Ile Lys Tyr Lys Glu Asp Leu Gln Val Leu Lys Gly Phe Gly Cys
    5090                5095                5100

Phe Leu Tyr Asp Thr Pro Asp Met Val Arg Ser Arg His Leu Arg
    5105                5110                5115

Lys Leu Trp Ser Asn Tyr Leu Tyr Thr Asp Lys Ala Arg Glu Met
    5120                5125                5130

Arg Asp Lys Tyr Lys Val Val Leu Asp Thr Pro Glu Tyr Arg Lys
    5135                5140                5145

Val Gln Glu Leu Lys Thr His Leu Ser Glu Leu Val Tyr Arg Ala
    5150                5155                5160

Ala Gly Lys Lys Gln Lys Ser Ile Phe Thr Ser Val Pro Asp Thr
    5165                5170                5175

Pro Asp Leu Leu Arg Ala Lys Arg Gly Gln Lys Leu Gln Ser Gln
    5180                5185                5190

Tyr Leu Tyr Val Glu Leu Ala Thr Lys Glu Arg Pro His His His
    5195                5200                5205

Ala Gly Asn Gln Thr Thr Ala Leu Lys His Ala Lys Asp Val Lys
    5210                5215                5220

Asp Met Val Ser Glu Lys Lys Tyr Lys Ile Gln Tyr Glu Lys Met
    5225                5230                5235

Lys Asp Lys Tyr Thr Pro Val Pro Asp Thr Pro Ile Leu Ile Arg
    5240                5245                5250

Ala Lys Arg Ala Tyr Trp Asn Ala Ser Asp Leu Arg Tyr Lys Glu
    5255                5260                5265

Thr Phe Gln Lys Thr Lys Gly Lys Tyr His Thr Val Lys Asp Ala
    5270                5275                5280

Leu Asp Ile Val Tyr His Arg Lys Val Thr Asp Asp Ile Ser Lys
    5285                5290                5295

Ile Lys Tyr Lys Glu Asn Tyr Met Ser Gln Leu Gly Ile Trp Arg
    5300                5305                5310

Ser Ile Pro Asp Arg Pro Glu His Phe His His Arg Ala Val Thr
    5315                5320                5325

Asp Thr Val Ser Asp Val Lys Tyr Lys Glu Asp Leu Thr Trp Leu
    5330                5335                5340

Lys Gly Ile Gly Cys Tyr Ala Tyr Asp Thr Pro Asp Phe Thr Leu
    5345                5350                5355

Ala Glu Lys Asn Lys Thr Leu Tyr Ser Lys Tyr Lys Tyr Lys Glu
    5360                5365                5370

Val Phe Glu Arg Thr Lys Ser Asp Phe Lys Tyr Val Ala Asp Ser
    5375                5380                5385

Pro Ile Asn Arg His Phe Lys Tyr Ala Thr Gln Leu Met Asn Glu
    5390                5395                5400

Lys Lys Tyr Arg Ala Asp Tyr Glu Gln Arg Lys Asp Lys Tyr His
    5405                5410                5415

Leu Val Val Asp Glu Pro Arg His Leu Leu Ala Lys Thr Arg Ser
    5420                5425                5430

Asp Gln Ile Ser Gln Ile Lys Tyr Arg Lys Asn Tyr Glu Lys Ser
    5435                5440                5445

Lys Asp Lys Phe Thr Ser Ile Val Asp Thr Pro Glu His Leu Arg
    5450                5455                5460

Thr Thr Lys Val Asn Lys Gln Ile Ser Asp Ile Leu Tyr Lys Leu
    5465                5470                5475

Glu Tyr Asn Lys Ala Lys Pro Arg Gly Tyr Thr Thr Ile His Asp
    5480                5485                5490

Thr Pro Met Leu Leu His Val Arg Lys Val Lys Asp Glu Val Ser
    5495                5500                5505

Asp Leu Lys Tyr Lys Glu Val Tyr Gln Arg Asn Lys Ser Asn Cys
    5510                5515                5520

Thr Ile Glu Pro Asp Ala Val His Ile Lys Ala Ala Lys Asp Ala
    5525                5530                5535

Tyr Lys Val Asn Thr Asn Leu Asp Tyr Lys Lys Gln Tyr Glu Ala
    5540                5545                5550

Asn Lys Ala His Trp Lys Trp Thr Pro Asp Arg Pro Asp Phe Leu
    5555                5560                5565

Gln Ala Ala Lys Ser Ser Leu Gln Gln Ser Asp Phe Glu Tyr Lys
    5570                5575                5580

Leu Asp Arg Glu Phe Leu Lys Gly Cys Lys Leu Ser Val Thr Asp
    5585                5590                5595

Asp Lys Asn Thr Val Leu Ala Leu Arg Asn Thr Leu Ile Glu Ser
    5600                5605                5610

Asp Leu Lys Tyr Lys Glu Lys His Val Lys Glu Arg Gly Thr Cys
    5615                5620                5625

His Ala Val Pro Asp Thr Pro Gln Ile Leu Leu Ala Lys Thr Val
    5630                5635                5640

Ser Asn Leu Val Ser Glu Asn Lys Tyr Lys Asp His Val Lys Lys
    5645                5650                5655

His Leu Ala Gln Gly Ser Tyr Thr Thr Leu Pro Glu Thr Arg Asp
    5660                5665                5670

Thr Val His Val Lys Glu Val Thr Lys His Val Ser Asp Thr Asn
    5675                5680                5685

Tyr Lys Lys Lys Phe Val Lys Glu Lys Gly Lys Ser Asn Tyr Ser
    5690                5695                5700

Ile Met Leu Glu Pro Pro Glu Val Lys His Ala Met Glu Val Ala
    5705                5710                5715

Lys Lys Gln Ser Asp Val Ala Tyr Arg Lys Asp Ala Lys Glu Asn
    5720                5725                5730

Leu His Tyr Thr Thr Val Ala Asp Arg Pro Asp Ile Lys Lys Ala
    5735                5740                5745

Thr Gln Ala Ala Lys Gln Ala Ser Glu Val Glu Tyr Arg Ala Lys
    5750                5755                5760

His Arg Lys Glu Gly Ser His Gly Leu Ser Met Leu Gly Arg Pro
    5765                5770                5775

Asp Ile Glu Met Ala Lys Lys Ala Ala Lys Leu Ser Ser Gln Val
    5780                5785                5790

Lys Tyr Arg Glu Asn Phe Asp Lys Glu Lys Gly Lys Thr Pro Lys
    5795                5800                5805

Tyr Asn Pro Lys Asp Ser Gln Leu Tyr Lys Val Met Lys Asp Ala
    5810                5815                5820

Asn Asn Leu Ala Ser Glu Val Lys Tyr Lys Ala Asp Leu Lys Lys
    5825                5830                5835

Leu His Lys Pro Val Thr Asp Met Lys Glu Ser Leu Ile Met Asn
    5840                5845                5850

His Val Leu Asn Thr Ser Gln Leu Ala Ser Ser Tyr Gln Tyr Lys
    5855                5860                5865

Lys Lys Tyr Glu Lys Ser Lys Gly His Tyr His Thr Ile Pro Asp
    5870                5875                5880

Asn Leu Glu Gln Leu His Leu Lys Glu Ala Thr Glu Leu Gln Ser
    5885                5890                5895

Ile Val Lys Tyr Lys Glu Lys Tyr Glu Lys Glu Arg Gly Lys Pro
    5900                5905                5910

Met Leu Asp Phe Glu Thr Pro Thr Tyr Ile Thr Ala Lys Glu Ser
    5915                5920                5925

Gln Gln Met Gln Ser Gly Lys Glu Tyr Arg Lys Asp Tyr Glu Glu
    5930                5935                5940

Ser Ile Lys Gly Arg Asn Leu Thr Gly Leu Glu Val Thr Pro Ala
    5945                5950                5955

Leu Leu His Val Lys Tyr Ala Thr Lys Ile Ala Ser Glu Lys Glu
    5960                5965                5970

Tyr Arg Lys Asp Leu Glu Glu Ser Ile Arg Gly Lys Gly Leu Thr
    5975                5980                5985

Glu Met Glu Asp Thr Pro Asp Met Leu Arg Ala Lys Asn Ala Thr
    5990                5995                6000

Gln Ile Leu Asn Glu Lys Glu Tyr Lys Arg Asp Leu Glu Leu Glu
    6005                6010                6015

Val Lys Gly Arg Gly Leu Asn Ala Met Ala Asn Glu Thr Pro Asp
    6020                6025                6030

Phe Met Arg Ala Arg Asn Ala Thr Asp Ile Ala Ser Gln Ile Lys
    6035                6040                6045

Tyr Lys Gln Ser Ala Glu Met Glu Lys Ala Asn Phe Thr Ser Val
    6050                6055                6060

Val Asp Thr Pro Glu Ile Ile His Ala Gln Gln Val Lys Asn Leu
    6065                6070                6075

Ser Ser Gln Lys Lys Tyr Lys Glu Asp Ala Glu Lys Ser Met Ser
    6080                6085                6090

Tyr Tyr Glu Thr Val Leu Asp Thr Pro Glu Ile Gln Arg Val Arg
    6095                6100                6105

Glu Asn Gln Lys Asn Phe Ser Leu Leu Gln Tyr Gln Cys Asp Leu
    6110                6115                6120

Lys Asn Ser Lys Gly Lys Ile Thr Val Val Gln Asp Thr Pro Glu
    6125                6130                6135

Ile Leu Arg Val Lys Glu Asn Gln Lys Asn Phe Ser Ser Val Leu
    6140                6145                6150

Tyr Lys Glu Asp Val Ser Pro Gly Thr Ala Ile Gly Lys Thr Pro
    6155                6160                6165

Glu Met Met Arg Val Lys Gln Thr Gln Asp His Ile Ser Ser Val
    6170                6175                6180

Lys Tyr Lys Glu Ala Ile Gly Gln Gly Thr Pro Ile Pro Asp Leu
    6185                6190                6195

Pro Glu Val Lys Arg Val Lys Glu Thr Gln Lys His Ile Ser Ser
    6200                6205                6210

Val Met Tyr Lys Glu Asn Leu Gly Thr Gly Ile Pro Thr Thr Val
    6215                6220                6225

Thr Pro Glu Ile Glu Arg Val Lys Arg Asn Gln Glu Asn Phe Ser
    6230                6235                6240

Ser Val Leu Tyr Lys Glu Asn Leu Gly Lys Gly Ile Pro Thr Pro
    6245                6250                6255

Ile Thr Pro Glu Met Glu Arg Val Lys Arg Asn Gln Glu Asn Phe
    6260                6265                6270

Ser Ser Val Leu Tyr Lys Glu Asn Met Gly Lys Gly Thr Pro Leu
    6275                6280                6285

Pro Val Thr Pro Glu Met Glu Arg Val Lys His Asn Gln Glu Asn
    6290                6295                6300

Ile Ser Ser Val Leu Tyr Lys Glu Asn Val Gly Lys Ala Thr Ala
    6305                6310                6315

Thr Pro Val Thr Pro Glu Met Gln Arg Val Lys Arg Asn Gln Glu
    6320                6325                6330

Asn Ile Ser Ser Val Leu Tyr Lys Glu Asn Leu Gly Lys Ala Thr
    6335                6340                6345

Pro Thr Pro Phe Thr Pro Glu Met Glu Arg Val Lys Arg Asn Gln
    6350                6355                6360

Glu Asn Phe Ser Ser Val Leu Tyr Lys Glu Asn Met Arg Lys Ala
    6365                6370                6375

Thr Pro Thr Pro Val Thr Pro Glu Met Glu Arg Ala Lys Arg Asn
    6380                6385                6390

Gln Glu Asn Ile Ser Ser Val Leu Tyr Ser Asp Ser Phe Arg Lys
    6395                6400                6405

Gln Ile Gln Gly Lys Ala Ala Tyr Val Leu Asp Thr Pro Glu Met
    6410                6415                6420

Arg Arg Val Arg Glu Thr Gln Arg His Ile Ser Thr Val Lys Tyr
    6425                6430                6435

His Glu Asp Phe Glu Lys His Lys Gly Cys Phe Thr Pro Val Val
    6440                6445                6450

Thr Asp Pro Ile Thr Glu Arg Val Lys Lys Asn Met Gln Asp Phe
    6455                6460                6465

Ser Asp Ile Asn Tyr Arg Gly Ile Gln Arg Lys Val Val Glu Met
    6470                6475                6480

Glu Gln Lys Arg Asn Asp Gln Asp Gln Glu Thr Ile Thr Gly Leu
    6485                6490                6495

Arg Val Trp Arg Thr Asn Pro Gly Ser Val Phe Asp Tyr Asp Pro
    6500                6505                6510

Ala Glu Asp Asn Ile Gln Ser Arg Ser Leu His Met Ile Asn Val
    6515                6520                6525

Gln Ala Gln Arg Arg Ser Arg Glu Gln Ser Arg Ser Ala Ser Ala
    6530                6535                6540

Leu Ser Val Ser Gly Gly Glu Glu Lys Ser Glu His Ser Glu Ala
    6545                6550                6555

Pro Asp His His Leu Ser Thr Tyr Ser Asp Gly Gly Val Phe Ala
    6560                6565                6570

Val Ser Thr Ala Tyr Lys His Ala Lys Thr Thr Glu Leu Pro Gln
    6575                6580                6585

Gln Arg Ser Ser Ser Val Ala Thr Gln Gln Thr Thr Val Ser Ser
    6590                6595                6600

Ile Pro Ser His Pro Ser Thr Ala Gly Lys Ile Phe Arg Ala Met
    6605                6610                6615

Tyr Asp Tyr Met Ala Ala Asp Ala Asp Glu Val Ser Phe Lys Asp
    6620                6625                6630

Gly Asp Ala Ile Ile Asn Val Gln Ala Ile Asp Glu Gly Trp Met
    6635                6640                6645

Tyr Gly Thr Val Gln Arg Thr Gly Arg Thr Gly Met Leu Pro Ala
    6650                6655                6660

Asn Tyr Val Glu Ala Ile
    6665

<210>  28

<211>  1008

<212>  DNA

<213>  homo sapiens

<220>

<221>  misc_feature

<223>  NFYC gene sequence made up of non-contigous segments of gene map
       locus 1p32

<400>  28
atgtccacag aaggaggatt tggtggtact agcagcagtg atgcccagca aagcctacag      60

tcgttctggc ctcgggtcat ggaagaaatc cggaatttaa cagtgaaaga cttccgagtg     120

caggaactcc cactggctcg tattaagaag attatgaaac tggatgaaga tgtgaagatg     180

atcagtgcag aagcgcctgt actctttgcc aaggcagccc agatttttat cacagagttg     240

actcttcgag cctggattca cacagaagat aacaagcgcc ggactctaca gagaaatgat     300

atcgccatgg caattacaaa atttgatcag tttgattttc tcatcgatat tgttccaaga     360

gatgaactga aacctccaaa gcgtcaggag gaggtgcgcc agtctgtaac tcctgccgag     420

ccagtccagt actatttcac gctggctcag caacccaccg ctgtccaagt ccagggccag     480

cagcaaggcc agcagaccac cagctccacg accaccatcc agcctgggca gatcatcatc     540

gcacagcctc agcagggcca gaccacacct gtgacaatgc aggttggaga aggtcagcag     600

gtgcagattg tccaggctca gccacagggt caagcccaac aggcccagag tggcactgga 660
cagaccatgc aggtgatgca gcagatcatc actaacacag gagagatcca gcagatcccg 720
gtgcagctga atgccggcca gctgcagtat atccgcttag cccagcctgt atcaggcact 780
caagttgtgc agggacagat ccagacactt gccaccaatg ctcaacagat tacacagaca 840
gaggtccagc aaggacagca gcagttcagc cagttcacag atggacagca gctctaccag 900
atccagcaag tcaccatgcc tgcgggccag gacctcgccc agcccatgtt catccagtca 960
gccaaccagc cctccgacgg gcaggccccc caggtgaccg gcgactga 1008

<210> 29
<211> 1965
<212> DNA
<213> homo sapiens

<220>
<221> misc_feature
<223> NFYC mRNA

<400> 29
   acgcgtccgg ggaaacggtg caaacggcgt ggccgccatc ttgcttgtgc ccccgcttcg 60
   cgcgcgctcc gtgacgcaca cttcccccct cccctccgcc gcgcctgggc ctctgcattg 120
   cccgactccg taggagcgcg ggggcggctc ctgctcttcc tggactcctg agcagagttg 180
   tcgagatgtc cacagaagga ggatttggtg gtactagcag cagtgatgcc cagcaaagcc 240
   tacagtcgtt ctggcctcgg gtcatggaag aaatccggaa tttaacagtg aaagacttcc 300
   gagtgcagga actcccactg gctcgtatta agaagattat gaaactggat gaagatgtga 360
   agatgatcag tgcagaagcg cctgtactct ttgccaaggc agcccagatt tttatcacag 420
   agttgactct tcgagcctgg attcacacag aagataacaa gcgccggact ctacagagaa 480
   atgatatcgc catggcaatt acaaaatttg atcagtttga ttttctcatc gatattgttc 540
   caagagatga actgaaacct ccaaagcgtc aggaggaggt gcgccagtct gtaactcctg 600
   ccgagccagt ccagtactat ttcacgctgg ctcagcaacc caccgctgtc caagtccagg 660
   gccagcagca aggccagcag accaccagct ccacgaccac catccagcct gggcagatca 720
   tcatcgcaca gcctcagcag ggccagacca cacctgtgac aatgcaggtt ggagaaggtc 780
   agcaggtgca gattgtccag gctcagccac agggtcaagc ccaacaggcc cagagtggca 840
   ctggacagac catgcaggtg atgcagcaga tcatcactaa cacaggagag atccagcaga 900
   tcccggtgca gctgaatgcc ggccagctgc agtatatccg cttagcccag cctgtatcag 960
   gcactcaagt tgtgcaggga cagatccaga cacttgccac caatgctcaa cagattacac 1020
   agacagaggt ccagcaagga cagcagcagt tcagccagtt cacagatgga cagcagctct 1080
   accagatcca gcaagtcacc atgcctgcgg gccaggacct cgcccagccc atgttcatcc 1140
   agtcagccaa ccagccctcc gacgggcagg ccccccaggt gaccggcgac tgagggcctg 1200
   agctggcaag gccaaggaca cccaacacaa tttttgccat acagccccag gcaatgggca 1260
   cagccttcct ccccagagga cccggccgac ctcagcgcct cctgcaggct aggacactgg 1320
   tgcactacac cccatgcctg ggggccgaga ttctccagca gaaagatgca atattttttg 1380
   tttccttttt ttccattttt ttctctaagg aatcaatatt tcaatatgtt gagtgtgtgt 1440
   ccaatgctat gaaattaaaa tattaaataa catatttatg gcattttctt gaagagtgtg 1500
   gttgaagaaa tatttctcct tttgtttttc tttttttttt gtttgttact gccacttctt 1560
   tttaggagca aatctcccca ggggtgtacg gtatttcttg actctgggaa cagctgctac 1620
   ccccaagact tgccacgttg ttctgccctc agatggaatt aggtgaatgt gtgtagctgc 1680
   tttttcactc gtggtcctct ccctatccct tgctctgacc ccagagctct gtgtatttgc 1740
   atccagaggc catggaaaca ttctttgcat ttaagagaca gatttatccc tgtggagagt 1800
   gggtggattc attgccacac tcttttctcc cagggaccca ggaaactagg actttgtgtg 1860
   tttgctgccc acctcccttt tattttttaa atgcattaaa aactgtgcta gtctcctttg 1920
   catggacttc aagctgcatg aaatgcaata aatctcattt tagat 1965

<210> 30

<211> 335

<212> PRT

<213> homo sapiens


<220>

<221> misc_feature

<223> NFYC gene product


<400> 30

Met Ser Thr Glu Gly Gly Phe Gly Gly Thr Ser Ser Ser Asp Ala Gln
1               5                   10                  15


Gln Ser Leu Gln Ser Phe Trp Pro Arg Val Met Glu Glu Ile Arg Asn
            20                  25                  30


Leu Thr Val Lys Asp Phe Arg Val Gln Glu Leu Pro Leu Ala Arg Ile
        35                  40                  45


Lys Lys Ile Met Lys Leu Asp Glu Asp Val Lys Met Ile Ser Ala Glu
    50                  55                  60

Ala Pro Val Leu Phe Ala Lys Ala Ala Gln Ile Phe Ile Thr Glu Leu
65                  70                  75                  80

Thr Leu Arg Ala Trp Ile His Thr Glu Asp Asn Lys Arg Arg Thr Leu
                85                  90                  95

Gln Arg Asn Asp Ile Ala Met Ala Ile Thr Lys Phe Asp Gln Phe Asp
            100                 105                 110

Phe Leu Ile Asp Ile Val Pro Arg Asp Glu Leu Lys Pro Pro Lys Arg
        115                 120                 125

Gln Glu Glu Val Arg Gln Ser Val Thr Pro Ala Glu Pro Val Gln Tyr
    130                 135                 140

Tyr Phe Thr Leu Ala Gln Gln Pro Thr Ala Val Gln Val Gln Gly Gln
145                 150                 155                 160

Gln Gln Gly Gln Gln Thr Thr Ser Ser Thr Thr Thr Ile Gln Pro Gly
                165                 170                 175

Gln Ile Ile Ile Ala Gln Pro Gln Gln Gly Gln Thr Thr Pro Val Thr
            180                 185                 190

Met Gln Val Gly Glu Gly Gln Gln Val Gln Ile Val Gln Ala Gln Pro
        195                 200                 205

Gln Gly Gln Ala Gln Gln Ala Gln Ser Gly Thr Gly Gln Thr Met Gln
    210                 215                 220

Val Met Gln Gln Ile Ile Thr Asn Thr Gly Glu Ile Gln Gln Ile Pro
225                 230                 235                 240

Val Gln Leu Asn Ala Gly Gln Leu Gln Tyr Ile Arg Leu Ala Gln Pro
                245                 250                 255

Val Ser Gly Thr Gln Val Val Gln Gly Gln Ile Gln Thr Leu Ala Thr
            260                 265                 270

Asn Ala Gln Gln Ile Thr Gln Thr Glu Val Gln Gln Gly Gln Gln Gln
        275                 280                 285

Phe Ser Gln Phe Thr Asp Gly Gln Gln Leu Tyr Gln Ile Gln Gln Val
    290                 295                 300

Thr Met Pro Ala Gly Gln Asp Leu Ala Gln Pro Met Phe Ile Gln Ser
305                 310                 315                 320

Ala Asn Gln Pro Ser Asp Gly Gln Ala Pro Gln Val Thr Gly Asp
                325                 330                 335

<210> 31

<211> 2088

<212> DNA

<213> homo sapiens

<220>

<221> misc_feature

<223> KCNQ4 gene sequence made up of non-contigous segments of gene map
 locus 1p34

<400> 31
atggccgagg cccccccgcg ccgcctcggc ctgggtcccc cgcccgggga cgccccccgc 60

gcggagctag tggcgctcac ggccgtgcag agcgaacagg gcgaggcggg cgggggcggc 120

tccccgcgcc gcctcggcct cctgggcagc cccctgccgc cgggcgcgcc cctccctggg 180

ccgggctccg gctcgggctc cgcctgcggc cagcgctcct cggccgcgca caagcgctac 240

cgccgcctgc agaactgggt ctacaacgtg ctggagcggc cccgcggctg ggccttcgtc 300

taccacgtct tcatattttt gctggtcttc agctgcctgg tgctgtctgt gctgtccact 360

atccaggagc accaggaact tgccaacgag tgtctcctca tcttggaatt cgtgatgatc 420

gtggttttcg gcttggagta catcgtccgg gtctggtccg ccggatgctg ctgccgctac 480

cgaggatggc agggtcgctt ccgctttgcc agaaagccct tctgtgtcat cgacttcatc 540

gtgttcgtgg cctcggtggc cgtcatcgcc gcgggtaccc agggcaacat cttcgccacg 600

tccgcgctgc gcagcatgcg cttcctgcag atcctgcgca tggtgcgcat ggaccgccgc 660

ggcggcacct ggaagctgct gggctcagtg gtctacgcgc atagcaagga gctgatcacc 720

gcctggtaca tcgggttcct ggtgctcatc ttcgcctcct tcctggtcta cctggctgag 780

aaggacgcca actccgactt ctcctcctac gccgactcgc tctggtgggg gacgattaca 840

ttgacaacca tcggctatgg tgacaagaca ccgcacacat ggctgggcag ggtcctggct 900

gctggcttcg ccttactggg catctctttc tttgccctgc ctgccggcat cctaggctcc 960

ggctttgccc tgaaggtcca ggagcagcac cggcagaagc acttcgagaa gcggaggatg 1020

ccggcagcca acctcatcca ggctgcctgg cgcctgtact ccaccgatat gagccgggcc 1080

tacctgacag ccacctggta ctactatgac agtatcctcc catccttcag agagctggcc 1140

ctcttgtttg agcacgtgca acgggcccgc aatgggggcc tacggcccct ggaggtgcgg 1200

cgggcgccgg tacccgacgg agcaccctcc cgttacccgc ccgttgccac ctgccaccgg 1260

ccgggcagca cctccttctg ccctggggaa agcagccgga tgggcatcaa agaccgcatc 1320

cgcatgggca gctcccagcg gcggacgggt ccttccaagc agcatctggc acctccaaca 1380

atgcccacct ccccaagcag cgagcaggtg ggtgaggcca ccagccccac caaggtgcaa 1440

aagagctgga gcttcaatga ccgcacccgc ttccgggcat ctctgagact caaaccccgc 1500

acctctgctg aggatgcccc ctcagaggaa gtagcagagg agaagagcta ccagtgtgag 1560

ctcacggtgg acgacatcat gcctgctgtg aagacagtca tccgctccat caggattctc 1620

aagttcctgg tggccaaaag gaaattcaag gagacactgc gaccgtacga cgtgaaggac 1680

gtcattgagc agtactcagc aggccacctg gacatgctgg gccggatcaa gagcctgcaa 1740

actcgggtgg accaaattgt gggtcggggg cccggggaca ggaaggcccg ggagaagggc 1800

gacaaggggc cctccgacgc ggaggtggtg gatgaaatca gcatgatggg acgcgtggtc 1860

aaggtggaga agcaggtgca gtccatcgag cacaagctgg acctgctgtt gggcttctat 1920

tcgcgctgcc tgcgctctgg cacctcggcc agcctgggcg ccgtgcaagt gccgctgttc 1980

gaccccgaca tcacctccga ctaccacagc cctgtggacc acgaggacat ctccgtctcc 2040

gcacagacgc tcagcatctc ccgctcggtc agcaccaaca tggactga 2088

<210> 32

<211> 2335

<212> DNA

<213> homo sapiens

<220>

<221> misc_feature

<223> KCNQ4 mRNA

<400> 32
agccatgcgt ctctgagcgc cccgagcgcg cccccgcccc ggaccgtgcc cgggccccgg 60

cgcccccagc ccggcgccgc ccatggccga ggcccccccg cgccgcctcg gcctgggtcc 120

cccgcccggg gacgcccccc gcgcggagct agtggcgctc acggccgtgc agagcgaaca 180

gggcgaggcg ggcgggggcg gctccccgcg ccgcctcggc ctcctgggca gccccctgcc 240

gccgggcgcg cccctccctg ggccgggctc cggctcgggc tccgcctgcg gccagcgctc 300

ctcggccgcg cacaagcgct accgccgcct gcagaactgg gtctacaacg tgctggagcg 360

gccccgcggc tgggccttcg tctaccacgt cttcatattt ttgctggtct tcagctgcct 420

ggtgctgtct gtgctgtcca ctatccagga gcaccaggaa cttgccaacg agtgtctcct 480

catcttggaa ttcgtgatga tcgtggtttt cggcttggag tacatcgtcc gggtctggtc 540

cgccggatgc tgctgccgct accgaggatg gcagggtcgc ttccgctttg ccagaaagcc 600

cttctgtgtc atcgacttca tcgtgttcgt ggcctcggtg gccgtcatcg ccgcgggtac 660

ccagggcaac atcttcgcca cgtccgcgct gcgcagcatg cgcttcctgc agatcctgcg 720

catggtgcgc atggaccgcc gcggcggcac ctggaagctg ctgggctcag tggtctacgc 780
gcatagcaag gagctgatca ccgcctggta catcgggttc ctggtgctca tcttcgcctc 900
cttcctggtc tacctggctg agaaggacgc caactccgac ttctcctcct acgccgactc 900
gctctggtgg gggacgatta cattgacaac catcggctat ggtgacaaga caccgcacac 960
atggctgggc agggtcctgg ctgctggctt cgccttactg ggcatctctt tctttgccct 1020
gcctgccggc atcctaggct ccggctttgc cctgaaggtc caggagcagc accggcagaa 1080
gcacttcgag aagcggagga tgccggcagc caacctcatc caggctgcct ggcgcctgta 1140
ctccaccgat atgagccggg cctacctgac agccacctgg tactactatg acagtatcct 1200
cccatccttc agagagctgg ccctcttgtt tgagcacgtg caacgggccc gcaatggggg 1260
cctacggccc ctggaggtgc ggcgggcgcc ggtacccgac ggagcaccct cccgttaccc 1320
gcccgttgcc acctgccacc ggccgggcag cacctccttc tgccctgggg aaagcagccg 1380
gatgggcatc aaagaccgca tccgcatggg cagctcccag cggcggacgg gtccttccaa 1440
gcagcatctg gcacctccaa caatgcccac ctccccaagc agcgagcagg tgggtgaggc 1500
caccagcccc accaaggtgc aaaagagctg gagcttcaat gaccgcaccc gcttccgggc 1560
atctctgaga ctcaaacccc gcacctctgc tgaggatgcc ccctcagagg aagtagcaga 1620
ggagaagagc taccagtgtg agctcacggt ggacgacatc atgcctgctg tgaagacagt 1680
catccgctcc atcaggattc tcaagttcct ggtggccaaa aggaaattca aggagacact 1740
gcgaccgtac gacgtgaagg acgtcattga gcagtactca gcaggccacc tggacatgct 1800
gggccggatc aagagcctgc aaactcgggt ggaccaaatt gtgggtcggg ggcccgggga 1860
caggaaggcc cgggagaagg gcgacaaggg gccctccgac gcggaggtgg tggatgaaat 1920
cagcatgatg ggacgcgtgg tcaaggtgga gaagcaggtg cagtccatcg agcacaagct 1980
ggacctgctg ttgggcttct attcgcgctg cctgcgctct ggcacctcgg ccagcctggg 2040
cgccgtgcaa gtgccgctgt tcgaccccga catcacctcc gactaccaca gccctgtgga 2100
ccacgaggac atctccgtct ccgcacagac gctcagcatc tcccgctcgg tcagcaccaa 2160
catggactga gggacttctc agaggcaggg cagcacacgg ccagccccgc ggcctggcgc 2220
tccgactgcc ctctgaggcc tccggactcc tctcgtactt gaactcactc cctcacgggg 2280
agagagacca cacgcagtat tgagctgcct gagtgggcgt ggtacctgct gtggg 2335

<210> 33

<211> 695

<212> PRT

<213> homo sapiens


<220>

<221> misc_feature

<223>  KCNQ4 gene product

<400>  33

<210>  34

<211>  2052

<212>  DNA

<213>  homo sapiens

<220>

<221>  misc_feature

<223>  PRPF3 gene sequence made up of non-contigous segments of gene map
       locus 1q21.1

<400>  34
atggcactgt caaagaggga gctggatgag ctgaaaccat ggatagagaa gacagtgaag      60

agggtcctgg gtttctcaga gcctacggtg gtcacagcag cattgaactg tgtggggaag 120
ggcatggaca agaagaaggc agccgatcat ctgaaacctt ttcttgatga ttctactctc 180
cgatttgtgg acaaactgtt tgaggctgtg gaggaaggcc gaagctctag gcattccaag 240
tctagcagtg acaggagcag aaaacgagag ctaaaggagg tgtttggtga tgactctgag 300
atctctaaag aatcatcagg agtaaagaag cgacgaatac cccgttttga ggaggtggaa 360
gaagagccag aggtgatccc tgggcctcca tcagagagcc ctggcatgct gactaagctc 420
cagatcaaac agatgatgga ggcagcaaca cgacaaatcg aggagaggaa aaaacagctg 480
agcttcatta gcccccctac acctcagcca aagactcctt cttcctccca accagaacga 540
cttcctattg gcaacactat tcagccctcc caggctgcca ctttcatgaa tgatgccatt 600
gagaaggcaa ggaaagcagc tgaactgcaa gctcgaatcc aagcccagct ggcactgaag 660
ccaggactca tcggcaatgc caacatggtg ggcctggcta atctccatgc catgggcatt 720
gctcccccga aggtggagtt aaaagaccaa acgaaaccta caccactgat cctggatgag 780
caagggcgca ctgtagatgc aacaggcaag gagattgagc tgacacaccg catgcctact 840
ctgaaagcca atattcgtgc tgtgaagagg gaacaattca agcaacaact aaaggaaaag 900
ccatcagaag acatggaatc caataccttt tttgaccccc gagtctccat tgccccttcc 960
cagcgccaga gacgcacttt taaattccat gacaagggca aatttgagaa gattgctcag 1020
cgattacgga caaaggctca actggagaag ctacaggcag agatttcaca agcagctcga 1080
aaaacaggca tccatacttc gactaggctt gccctcattg ctcctaagaa ggagctaaag 1140
gaaggagata ttcctgaaat tgagtggtgg gactcttaca taatccccaa tggctttgat 1200
cttacagagg aaaatcccaa gagagaagat tattttggaa tcacaaatct tgttgaacat 1260
ccagcccagc tcaatcctcc agttgacaat gacacaccag ttactctggg agtatatctt 1320
accaagaagg aacagaaaaa acttcggaga caaacaagga gggaagcaca gaaggaacta 1380
caagaaaaag tcaggctggg cctgatgcct cctccagaac ccaaagtgag aatttctaat 1440
ttgatgcgag tattaggaac agaagctgtt caagacccca cgaaggtaga agcccacgtc 1500
agagctcaga tggcaaaaag acagaaagcg catgaagagg ccaacgctgc ccgaaaactc 1560
acagcagaac agagaaaggt caagaaaatt aaaaagctta aagaagacat ttcacagggg 1620
gtacacatat ctgtatatag agttcgaaat ttgagcaacc cagccaagaa gttcaagatt 1680
gaagccaatg ctgggcaact gtacctgaca ggggtggtgg tactgcacaa ggatgtcaac 1740
gtggtagtag tggaaggggg ccccaaggcc cagaagaaat ttaagcgtct tatgctgcat 1800
cggataaagt gggatgaaca gacatctaac acaaagggag atgatgatga ggagtctgat 1860
gaggaagctg tgaagaaaac caacaaatgt gtactagtct gggagggtac agccaaagac 1920
cggagctttg gagagatgaa gtttaaacag tgtcctacag agaacatggc tcgtgagcat 1980
ttcaaaaagc atggggctga acactactgg gaccttgcgc tgagtgaatc tgtgttagag 2040
tccactgatt ga 2052

<210> 35

<211> 2344

<212> DNA

<213> homo sapiens

<220>

<221> misc_feature

<223> PRPF3 mRNA

<400> 35
gtctcagggg ctgaagtttg tgaggtgtag tattgagtcc tgtttgagct attgttctct 60

ttttcctgaa aaatggcact gtcaaagagg gagctggatg agctgaaacc atggatagag 120

aagacagtga agagggtcct gggtttctca gagcctacgg tggtcacagc agcattgaac 180

tgtgtgggga agggcatgga caagaagaag gcagccgatc atctgaaacc ttttcttgat 240

gattctactc tccgatttgt ggacaaactg tttgaggctg tggaggaagg ccgaagctct 300

aggcattcca agtctagcag tgacaggagc agaaaacgag agctaaagga ggtgtttggt 360

gatgactctg agatctctaa agaatcatca ggagtaaaga agcgacgaat accccgtttt 420

gaggaggtgg aagaagagcc agaggtgatc cctgggcctc catcagagag ccctggcatg 480

ctgactaagc tccagatcaa acagatgatg gaggcagcaa cacgacaaat cgaggagagg 540

aaaaaacagc tgagcttcat tagcccccct acacctcagc caaagactcc ttcttcctcc 600

caaccagaac gacttcctat tggcaacact attcagccct cccaggctgc cactttcatg 660

aatgatgcca ttgagaaggc aaggaaagca gctgaactgc aagctcgaat ccaagcccag 720

ctggcactga agccaggact catcggcaat gccaacatgg tgggcctggc taatctccat 780

gccatgggca ttgctccccc gaaggtggag ttaaaagacc aaacgaaacc tacaccactg 840

atcctggatg agcaagggcg cactgtagat gcaacaggca aggagattga gctgacacac 900

cgcatgccta ctctgaaagc caatattcgt gctgtgaaga gggaacaatt caagcaacaa 960

ctaaaggaaa agccatcaga agacatggaa tccaatacct tttttgaccc ccgagtctcc 1020

attgcccctt cccagcgcca gagacgcact tttaaattcc atgacaaggg caaatttgag 1080

aagattgctc agcgattacg gacaaaggct caactggaga agctacaggc agagatttca 1140

caagcagctc gaaaaacagg catccatact tcgactaggc ttgccctcat tgctcctaag 1200

aaggagctaa aggaaggaga tattcctgaa attgagtggt gggactctta cataatcccc 1260

aatggctttg atcttacaga ggaaaatccc aagagagaag attattttgg aatcacaaat 1320

cttgttgaac atccagccca gctcaatcct ccagttgaca atgacacacc agttactctg 1380

ggagtatatc ttaccaagaa ggaacagaaa aaacttcgga gacaaacaag gagggaagca 1440

cagaaggaac tacaagaaaa agtcaggctg ggcctgatgc ctcctccaga acccaaagtg 1500
agaatttcta atttgatgcg agtattagga acagaagctg ttcaagaccc cacgaaggta 1560
gaagcccacg tcagagctca gatggcaaaa agacagaaag cgcatgaaga ggccaacgct 1620
gcccgaaaac tcacagcaga acagagaaag gtcaagaaaa ttaaaaagct taaagaagac 1680
atttcacagg gggtacacat atctgtatat agagttcgaa atttgagcaa cccagccaag 1740
aagttcaaga ttgaagccaa tgctgggcaa ctgtacctga caggggtggt ggtactgcac 1800
aaggatgtca acgtggtagt agtggaaggg ggccccaagg cccagaagaa atttaagcgt 1860
cttatgctgc atcggataaa gtgggatgaa cagacatcta acacaaaggg agatgatgat 1920
gaggagtctg atgaggaagc tgtgaagaaa accaacaaat gtgtactagt ctgggagggt 1980
acagccaaag accggagctt tggagagatg aagtttaaac agtgtcctac agagaacatg 2040
gctcgtgagc atttcaaaaa gcatggggct gaacactact gggaccttgc gctgagtgaa 2100
tctgtgttag agtccactga ttgagactac tgcaagccct tgcctctcct cccttgcctt 2160
tgtctcttca gtcctctcac ttattctatt tcccaacccc ctcccacttg tttgtgtgat 2220
ctcagaactg tgccaagcag acactgggac aaaggagaaa tatcttgctc ccctcctgag 2280
tcagcctggt gttgcccttt attcccctta tgtgcatatg attaaagagt tatttttaaa 2340
aaaa 2344

<210> 36
<211> 683
<212> PRT
<213> homo sapiens

<220>
<221> misc_feature
<223> PRPF3 gene product

<400> 36

<210>  37

<211>  2280

<212>  DNA

<213>  homo sapiens

<220>

<221>  misc_feature

<223>  TRPC1 gene sequence made up of non-contigous segments of gene map
        locus 3q22-q24

<400>  37
atgatggcgg ccctgtaccc gagcacggac ctctcgggcg cctcctcctc ctccctgcct      60

tcctctccat cctcttcctc gccgaacgag gtgatggcgc tgaaggatgt gcgggaggtg     120

aaggaggaga atacgctgaa tgagaagctt ttcttgctgg cgtgcgacaa gggtgactat     180

tatatggtta aaaagatttt ggaggaaaac agttcaggtg acttgaacat aaattgcgta     240

gatgtgcttg ggagaaatgc tgttaccata actattgaaa acgaaaactt ggatatactg     300

cagcttcttt tggactacgg ttgtcagaaa ctaatggaac gaattcagaa tcctgagtat     360

tcaacaacta tggatgttgc acctgtcatt ttagctgctc atcgtaacaa ctatgaaatt     420

cttacaatgc tcttaaaaca ggatgtatct ctacccaagc cccatgcagt tggctgtgaa     480

tgcacattgt gttctgcaaa aaacaaaaag gatagcctcc ggcattccag gtttcgtctt     540

gatatatatc gatgtttggc cagtccagct ctaataatgt taacagagga ggatccaatt     600

ctgagagcat ttgaacttag tgctgattta aaagaactaa gtcttgtgga ggtggaattc     660

aggaatgatt atgaggaact agcccggcaa tgtaaaatgt ttgctaagga tttacttgca     720

caagcccgga attctcgtga attggaagtt attctaaacc atacgtctag tgacgagcct     780

cttgacaaac ggggattatt agaagaaaga atgaatttaa gtcgtctaaa acttgctatc 840
aaatataacc agaaagagtt tgtctcccag tctaactgcc agcagttcct gaacactgtt 900
tggtttggac agatgtcrgg ttaccgacgc aagcccacct gtaagaagat aatgactgtt 960
ttgacagtag gcatcttttg gccagttttg tcactttgtt atttgatagc tcccaaatct 1020
cagtttggca gaatcattca cacacctttt atgaaattta tcattcatgg agcatcatat 1080
ttcacatttc tgctgttgct taatctatac tctcttgtct acaatgagga taagaaaaac 1140
acaatggggc cagcccttga aagaatagac tatcttctta ttctgtggat tattgggatg 1200
atttggtcag acattaaaag actctggtat gaagggttgg aagacttttt agaagaatct 1260
cgtaatcaac tcagttttgt catgaattct ctttatttgg caacctttgc cctcaaagtg 1320
gttgctcaca acaagtttca tgattttgct gatcggaagg attgggatgc attccatcct 1380
acactggtgg cagaagggct ttttgcattt gcaaatgttc taagttatct tcgtctcttt 1440
tttatgtata caaccagctc tatcttgggt ccattacaga tttcaatggg acagatgtta 1500
caagattttg gaaaatttct tgggatgttt cttcttgttt tgttttcttt cacaattgga 1560
ctgacacaac tgtatgataa aggatatact tcaaaggagc agaaggactg tgtaggcatc 1620
ttctgtgaac agcaaagcaa tgataccttc cattcgttca ttggcacctg ctttgctttg 1680
ttctggtata ttttctcctt agcgcatgtg gcaatctttg tcacaagatt tagctatgga 1740
gaagaactgc agtcctttgt gggagctgtc attgttggta catacaatgt cgtggttgtg 1800
attgtgctta ccaaactgct ggtggcaatg cttcataaaa gctttcagtt gatagcaaat 1860
catgaagaca aagaatggaa gtttgctcga gcaaaattat ggcttagcta ctttgatgac 1920
aaatgtacgt tacctccacc tttcaacatc attccctcac caaagactat ctgctatatg 1980
attagtagcc tcagtaagtg gatttgctct catacatcaa aaggcaaggt caaacggcaa 2040
aacagtttaa aggaatggag aaatttgaaa cagaagagag atgaaaacta tcaaaaagtg 2100
atgtgctgcc tagtgcatcg ttacttgact tccatgagac agaagatgca aagtacagat 2160
caggcaactg tggaaaatct aaacgaactg cgccaagatc tgtcaaaatt ccgaaatgaa 2220
ataaggattt tacttggctt tcggacttct aaatatgcta tgttttatcc aagaaattaa 2280

<210> 38
<211> 4085
<212> DNA
<213> homo sapiens

<220>
<221> misc_feature
<223> TRPC1 mRNA

<400> 38
ccgggcctcg agccgaggca gcagtgggaa cgactcatcc tttttccagc cctggggcgt 60
ggctggggtc ggggtcgggg tcggggccgg tgggggcccc gcccccgtct cctggcctgc 120
ccccttcatg ggccgcgatg atggcggccc tgtacccgag cacggacctc tcgggcgcct 180
cctcctcctc cctgccttcc tctccatcct cttcctcgcc gaacgaggtg atggcgctga 240
aggatgtgcg ggaggtgaag gaggagaata cgctgaatga gaagcttttc ttgctggcgt 300
gcgacaaggg tgactattat atggttaaaa agattttgga ggaaaacagt tcaggtgact 360
tgaacataaa ttgcgtagat gtgcttggga gaaatgctgt taccataact attgaaaacg 420
aaaacttgga tatactgcag cttcttttgg actacggttg tcagaaacta atggaacgaa 480
ttcagaatcc tgagtattca acaactatgg atgttgcacc tgtcatttta gctgctcatc 540
gtaacaacta tgaaattctt acaatgctct taaaacagga tgtatctcta cccaagcccc 600
atgcagttgg ctgtgaatgc acattgtgtt ctgcaaaaaa caaaaaggat agcctccggc 660
attccaggtt tcgtcttgat atatatcgat gtttggccag tccagctcta ataatgttaa 720
cagaggagga tccaattctg agagcatttg aacttagtgc tgatttaaaa gaactaagtc 780
ttgtggaggt ggaattcagg aatgattatg aggaactagc ccggcaatgt aaaatgtttg 840
ctaaggattt acttgcacaa gcccggaatt ctcgtgaatt ggaagttatt ctaaaccata 900
cgtctagtga cgagcctctt gacaaacggg gattattaga agaaagaatg aatttaagtc 960
gtctaaaact tgctatcaaa tataaccaga aagagtttgt ctcccagtct aactgccagc 1020
agttcctgaa cactgtttgg tttggacaga tgtcrggtta ccgacgcaag cccacctgta 1080
agaagataat gactgttttg acagtaggca tcttttggcc agttttgtca ctttgttatt 1140
tgatagctcc caaatctcag tttggcagaa tcattcacac accttttatg aaatttatca 1200
ttcatggagc atcatatttc acatttctgc tgttgcttaa tctatactct cttgtctaca 1260
atgaggataa gaaaaacaca atggggccag cccttgaaag aatagactat cttcttattc 1320
tgtggattat tgggatgatt tggtcagaca ttaaaagact ctggtatgaa gggttggaag 1380
actttttaga agaatctcgt aatcaactca gttttgtcat gaattctctt tatttggcaa 1440
cctttgccct caaagtggtt gctcacaaca agtttcatga ttttgctgat cggaaggatt 1500
gggatgcatt ccatcctaca ctggtggcag aagggctttt tgcatttgca aatgttctaa 1560
gttatcttcg tctctttttt atgtatacaa ccagctctat cttgggtcca ttacagattt 1620
caatgggaca gatgttacaa gattttggaa aatttcttgg gatgtttctt cttgttttgt 1680
tttctttcac aattggactg acacaactgt atgataaagg atatacttca aaggagcaga 1740
aggactgtgt aggcatcttc tgtgaacagc aaagcaatga taccttccat tcgttcattg 1800
gcacctgctt tgctttgttc tggtatattt tctccttagc gcatgtggca atctttgtca 1860
caagatttag ctatggagaa gaactgcagt cctttgtggg agctgtcatt gttggtacat 1920
acaatgtcgt ggttgtgatt gtgcttacca aactgctggt ggcaatgctt cataaaagct 1980

ttcagttgat agcaaatcat gaagacaaag aatggaagtt tgctcgagca aaattatggc 2040
ttagctactt tgatgacaaa tgtacgttac ctccaccttt caacatcatt ccctcaccaa 2100
agactatctg ctatatgatt agtagcctca gtaagtggat ttgctctcat acatcaaaag 2160
gcaaggtcaa acggcaaaac agtttaaagg aatggagaaa tttgaaacag aagagagatg 2220
aaaactatca aaaagtgatg tgctgcctag tgcatcgtta cttgacttcc atgagacaga 2280
agatgcaaag tacagatcag gcaactgtgg aaaatctaaa cgaactgcgc caagatctgt 2340
caaaattccg aaatgaaata agggatttac ttggctttcg gacttctaaa tatgctatgt 2400
tttatccaag aaattaacca ttttctaaat catggagcga ataattttca ataacagatc 2460
caaaagacta tattgcataa cttgcaatga aattaatgag atatatattg aaataaagaa 2520
ttatgtaaaa gccattcttt aaaatattta tagcataaat atatgttatg taaagtgtgt 2580
atatagaatt agttttttaa accttctgtt agtggctttt tgcagaagca aaacagatta 2640
agtagataga ttttgttagc atgctgcttg gttttcttac ttagtgcttt aaaatgtttt 2700
tttttatgtt taagaggggc agttataaat ggacacattg cccagaatgt tttgtaaaat 2760
gaagaccagc aaatgtaggc tgatctcctt cacaggatac acttgaaata tagaagttat 2820
gttttaaata tctctgtttt aggagttcac atatagttca gcatttattg tttaggagta 2880
taattttatt ttatctaaaa taatagtcta tttttctttt tgtattttgt tataatctta 2940
agcaacaaag aaaaaaccct aatatttgaa tctatttatg tctttcaatt taaattcact 3000
tcagtttttg ttattgtaat atatttactt ttacatggtt ataatcactt tatattttta 3060
atgttttttt cacttaatat tttatatata catttccatg tattgatgta gttagtccac 3120
atttaaattt ttatagaatt atatagtttt tgaaaaatac agtcagtaga tgttttattt 3180
tttagctatt cagttatgtt tataagtttg catagctact tctcgacatt tggtttgttt 3240
taattttttt gtatcataat agtcctattt ttttttcaag ttggagtgaa tgtttttagt 3300
tttaagatag ataggagaca cttttttatc acatgtagtc acaacctgtt ttgtttttgt 3360
aaaacatagg aagtctcttt aatgcaatga tttgttttat atttggacta aggttcttga 3420
gcttatctcc caaggtactt tccataattt aacacagctt ctataaaagt gacttcatgc 3480
ttacttgtgg atcattcttg ctgcttaaga tgaaaagcat tggtttttta aaattagaga 3540
ataaaatatg tatttaaatt tttggtgtgt tcacataaag ggatgtagct aaaatgtttt 3600
cataggctat tatatattct cgcagcattt ccagttaaga ggatattagg tatataattc 3660
tcttcttaac cgaatgtcag atggtcttac gccacagggt gcaggtaacc cttggtctgt 3720
aagcaccacc gatccaggga tcattgtcta aataggttac tattgtttgt ttcatcttgc 3780
ttttgcattt ttatttttta atttccaaat tttaagtgtt ccctctttgg ggcaaattct 3840
tataaaaatg tttattgtaa agttatatat tttgtctacg atgggattat gcacttccca 3900
attgggattt tacatctgga tttttagtca ttctaaaaaa cacctaatta ttaaaacatt 3960
tatagagtgc ctactgtatg catgagttga gttgcttctg aggtacattt tgaatgacag 4020

catattgtaa gaaaaaaaaa ggtgaataaa atttgacatt agattataaa aaaaaaaagg 4080
aattc 4085

<210> 39
<211> 759
<212> PRT
<213> homo sapiens

<220>
<221> misc_feature
<223> TRPC1 gene product

<400> 39
Met Met Ala Ala Leu Tyr Pro Ser Thr Asp Leu Ser Gly Ala Ser Ser
1               5                   10                  15

Ser Ser Leu Pro Ser Ser Pro Ser Ser Ser Ser Pro Asn Glu Val Met
            20                  25                  30

Ala Leu Lys Asp Val Arg Glu Val Lys Glu Glu Asn Thr Leu Asn Glu
        35                  40                  45

Lys Leu Phe Leu Leu Ala Cys Asp Lys Gly Asp Tyr Tyr Met Val Lys
    50                  55                  60

Lys Ile Leu Glu Glu Asn Ser Ser Gly Asp Leu Asn Ile Asn Cys Val
65                  70                  75                  80

Asp Val Leu Gly Arg Asn Ala Val Thr Ile Thr Ile Glu Asn Glu Asn
                85                  90                  95

Leu Asp Ile Leu Gln Leu Leu Leu Asp Tyr Gly Cys Gln Lys Leu Met
            100                 105                 110

Glu Arg Ile Gln Asn Pro Glu Tyr Ser Thr Thr Met Asp Val Ala Pro
        115                 120                 125

Val Ile Leu Ala Ala His Arg Asn Asn Tyr Glu Ile Leu Thr Met Leu
    130                 135                 140

Leu Lys Gln Asp Val Ser Leu Pro Lys Pro His Ala Val Gly Cys Glu
145                 150                 155                 160

Cys Thr Leu Cys Ser Ala Lys Asn Lys Lys Asp Ser Leu Arg His Ser
                165                 170                 175

Arg Phe Arg Leu Asp Ile Tyr Arg Cys Leu Ala Ser Pro Ala Leu Ile
            180                 185                 190

Met Leu Thr Glu Glu Asp Pro Ile Leu Arg Ala Phe Glu Leu Ser Ala
        195                 200                 205

Asp Leu Lys Glu Leu Ser Leu Val Glu Val Glu Phe Arg Asn Asp Tyr
    210                 215                 220

Glu Glu Leu Ala Arg Gln Cys Lys Met Phe Ala Lys Asp Leu Leu Ala
225                 230                 235                 240

Gln Ala Arg Asn Ser Arg Glu Leu Glu Val Ile Leu Asn His Thr Ser
                245                 250                 255

Ser Asp Glu Pro Leu Asp Lys Arg Gly Leu Leu Glu Glu Arg Met Asn
            260                 265                 270

Leu Ser Arg Leu Lys Leu Ala Ile Lys Tyr Asn Gln Lys Glu Phe Val
        275                 280                 285

Ser Gln Ser Asn Cys Gln Gln Phe Leu Asn Thr Val Trp Phe Gly Gln
    290                 295                 300

Met Ser Gly Tyr Arg Arg Lys Pro Thr Cys Lys Lys Ile Met Thr Val
305                 310                 315                 320

Leu Thr Val Gly Ile Phe Trp Pro Val Leu Ser Leu Cys Tyr Leu Ile
                325                 330                 335

Ala Pro Lys Ser Gln Phe Gly Arg Ile Ile His Thr Pro Phe Met Lys
            340                 345                 350

Phe Ile Ile His Gly Ala Ser Tyr Phe Thr Phe Leu Leu Leu Leu Asn
        355                 360                 365

Leu Tyr Ser Leu Val Tyr Asn Glu Asp Lys Lys Asn Thr Met Gly Pro
    370                 375                 380

Ala Leu Glu Arg Ile Asp Tyr Leu Leu Ile Leu Trp Ile Ile Gly Met
385                 390                 395                 400

Ile Trp Ser Asp Ile Lys Arg Leu Trp Tyr Glu Gly Leu Glu Asp Phe
                405                 410                 415

Leu Glu Glu Ser Arg Asn Gln Leu Ser Phe Val Met Asn Ser Leu Tyr
            420                 425                 430

Leu Ala Thr Phe Ala Leu Lys Val Val Ala His Asn Lys Phe His Asp
        435                 440                 445

Phe Ala Asp Arg Lys Asp Trp Asp Ala Phe His Pro Thr Leu Val Ala
    450                 455                 460

Glu Gly Leu Phe Ala Phe Ala Asn Val Leu Ser Tyr Leu Arg Leu Phe
465                 470                 475                 480

Phe Met Tyr Thr Thr Ser Ser Ile Leu Gly Pro Leu Gln Ile Ser Met
                485                 490                 495

Gly Gln Met Leu Gln Asp Phe Gly Lys Phe Leu Gly Met Phe Leu Leu
            500                 505                 510

Val Leu Phe Ser Phe Thr Ile Gly Leu Thr Gln Leu Tyr Asp Lys Gly
        515                 520                 525

Tyr Thr Ser Lys Glu Gln Lys Asp Cys Val Gly Ile Phe Cys Glu Gln
    530                 535                 540

Gln Ser Asn Asp Thr Phe His Ser Phe Ile Gly Thr Cys Phe Ala Leu
545                 550                 555                 560

Phe Trp Tyr Ile Phe Ser Leu Ala His Val Ala Ile Phe Val Thr Arg
                565                 570                 575

Phe Ser Tyr Gly Glu Glu Leu Gln Ser Phe Val Gly Ala Val Ile Val
            580                 585                 590

Gly Thr Tyr Asn Val Val Val Val Ile Val Leu Thr Lys Leu Leu Val
        595                 600                 605

Ala Met Leu His Lys Ser Phe Gln Leu Ile Ala Asn His Glu Asp Lys
    610                 615                 620

Glu Trp Lys Phe Ala Arg Ala Lys Leu Trp Leu Ser Tyr Phe Asp Asp
625                 630                 635                 640

Lys Cys Thr Leu Pro Pro Pro Phe Asn Ile Ile Pro Ser Pro Lys Thr
                645                 650                 655

Ile Cys Tyr Met Ile Ser Ser Leu Ser Lys Trp Ile Cys Ser His Thr
            660                 665                 670

Ser Lys Gly Lys Val Lys Arg Gln Asn Ser Leu Lys Glu Trp Arg Asn
        675                 680                 685

Leu Lys Gln Lys Arg Asp Glu Asn Tyr Gln Lys Val Met Cys Cys Leu
    690                 695                 700

Val His Arg Tyr Leu Thr Ser Met Arg Gln Lys Met Gln Ser Thr Asp
705                 710                 715                 720

Gln Ala Thr Val Glu Asn Leu Asn Glu Leu Arg Gln Asp Leu Ser Lys
                725                 730                 735

Phe Arg Asn Glu Ile Arg Asp Leu Leu Gly Phe Arg Thr Ser Lys Tyr
            740                 745                 750

Ala Met Phe Tyr Pro Arg Asn
        755